(19)

Europäisches
Patentamt
European
Patent Office
Office européen
des brevets

(11) **EP 3 466 973 A1**

(12) **EUROPEAN PATENT APPLICATION**

(43) Date of publication:
**10.04.2019 Bulletin 2019/15**

(21) Application number: **18171273.8**

(22) Date of filing: **01.11.2013**

(51) Int Cl.:
*C07K 16/24* (2006.01)    *C07K 16/28* (2006.01)
*A61K 9/00* (2006.01)    *A61K 9/19* (2006.01)
*C07K 16/18* (2006.01)    *C07K 16/22* (2006.01)
*C07K 16/32* (2006.01)    *C07K 16/42* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.11.2012   US 201261721364 P
15.03.2013   US 201361794231 P**

(62) Document number(s) of the earlier application(s) in
accordance with Art. 76 EPC:
**13792165.6 / 2 914 626**

(27) Previously filed application:
**01.11.2013 PCT/US2013/068110**

(71) Applicant: **AbbVie Inc.
North Chicago, IL 60064 (US)**

(72) Inventors:
• **Siedler, Michael
69118 Heidelberg (DE)**
• **Kumar, Vineet
West Chester, Pennsylvania 19382 (US)**
• **Chari, Ravi
Worcester, MA 01606 (US)**
• **Patil, Vishwesh
Arlington, MA 02476 (US)**
• **Saluja, Sonal
Shrewsbury, MA 01545 (US)**

(74) Representative: **Cabinet Plasseraud
66, rue de la Chaussée d'Antin
75440 Paris Cedex 09 (FR)**

Remarks:
This application was filed on 08-05-2018 as a
divisional application to the application mentioned
under INID code 62.

(54) **STABLE DUAL VARIABLE DOMAIN IMMUNOGLOBULIN PROTEIN FORMULATIONS**

(57)    The disclosure provides stable aqueous formulations comprising an Aqueous Stable Dual Variable Domain Immunoglobulin (AS-DVD-Ig) protein. The disclosure also provides stable lyophilized formulations comprising a Lyophilized Stable Dual Variable Domain Immunoglobulin (LS-DVD-Ig) protein.

EP 3 466 973 A1

SEQUENCE LISTING

<110> ABBVIE INC., et al.

<120> STABLE DUAL VARIABLE DOMAIN IMMUNOGLOBULIN PROTEIN FORMULATIONS

<130> ABVI-005-EP-DIV1

<150> EP13792165.6
<151> 2013-11-01

<150> 61/794,231
<151> 2013-03-15

<150> 61/721,364
<151> 2012-11-01

<160> 75

<170> PatentIn version 3.5

<210> 1
<211> 16
<212> PRT
<213> Artificial Sequence

<220>
<223> Description of Artificial Sequence: Synthetic
      peptide

<400> 1
Ala Lys Thr Thr Pro Lys Leu Glu Glu Gly Glu Phe Ser Glu Ala Arg
1               5                   10                  15


<210> 2
<211> 17
<212> PRT
<213> Artificial Sequence

<220>
<223> Description of Artificial Sequence: Synthetic
      peptide

<400> 2
Ala Lys Thr Thr Pro Lys Leu Glu Glu Gly Glu Phe Ser Glu Ala Arg
1               5                   10                  15


Val



<210> 3
<211> 9
<212> PRT
<213> Artificial Sequence

<220>
<223> Description of Artificial Sequence: Synthetic
      peptide

<400> 3

```
Ala Lys Thr Thr Pro Lys Leu Gly Gly
1               5


<210> 4
<211> 10
<212> PRT
<213> Artificial Sequence

<220>
<223> Description of Artificial Sequence: Synthetic
      peptide

<400> 4
Ser Ala Lys Thr Thr Pro Lys Leu Gly Gly
1               5                   10


<210> 5
<211> 6
<212> PRT
<213> Artificial Sequence

<220>
<223> Description of Artificial Sequence: Synthetic
      peptide

<400> 5
Ser Ala Lys Thr Thr Pro
1               5


<210> 6
<211> 6
<212> PRT
<213> Artificial Sequence

<220>
<223> Description of Artificial Sequence: Synthetic
      peptide

<400> 6
Arg Ala Asp Ala Ala Pro
1               5


<210> 7
<211> 9
<212> PRT
<213> Artificial Sequence

<220>
<223> Description of Artificial Sequence: Synthetic
      peptide

<400> 7
Arg Ala Asp Ala Ala Pro Thr Val Ser
1               5


<210> 8
<211> 12
<212> PRT
```

<210> Artificial Sequence

<220>
<223> Description of Artificial Sequence: Synthetic
        peptide

<400> 8
Arg Ala Asp Ala Ala Ala Ala Gly Gly Pro Gly Ser
1               5               10


<210> 9
<211> 27
<212> PRT
<213> Artificial Sequence

<220>
<223> Description of Artificial Sequence: Synthetic
        peptide

<400> 9
Arg Ala Asp Ala Ala Ala Ala Gly Gly Gly Gly Ser Gly Gly Gly Gly
1               5               10              15


Ser Gly Gly Gly Gly Ser Gly Gly Gly Gly Ser
            20              25


<210> 10
<211> 18
<212> PRT
<213> Artificial Sequence

<220>
<223> Description of Artificial Sequence: Synthetic
        peptide

<400> 10
Ser Ala Lys Thr Thr Pro Lys Leu Glu Glu Gly Glu Phe Ser Glu Ala
1               5               10              15


Arg Val


<210> 11
<211> 5
<212> PRT
<213> Artificial Sequence

<220>
<223> Description of Artificial Sequence: Synthetic
        peptide

<400> 11
Ala Asp Ala Ala Pro
1               5


<210> 12
<211> 12

<212> PRT
<213> Artificial Sequence

<220>
<223> Description of Artificial Sequence: Synthetic
      peptide

<400> 12
Ala Asp Ala Ala Pro Thr Val Ser Ile Phe Pro Pro
1               5                   10


<210> 13
<211> 5
<212> PRT
<213> Artificial Sequence

<220>
<223> Description of Artificial Sequence: Synthetic
      peptide

<400> 13
Thr Val Ala Ala Pro
1               5


<210> 14
<211> 12
<212> PRT
<213> Artificial Sequence

<220>
<223> Description of Artificial Sequence: Synthetic
      peptide

<400> 14
Thr Val Ala Ala Pro Ser Val Phe Ile Phe Pro Pro
1               5                   10


<210> 15
<211> 6
<212> PRT
<213> Artificial Sequence

<220>
<223> Description of Artificial Sequence: Synthetic
      peptide

<400> 15
Gln Pro Lys Ala Ala Pro
1               5


<210> 16
<211> 13
<212> PRT
<213> Artificial Sequence

<220>
<223> Description of Artificial Sequence: Synthetic
      peptide

```
<400> 16
Gln Pro Lys Ala Ala Pro Ser Val Thr Leu Phe Pro Pro
1               5                   10


<210> 17
<211> 6
<212> PRT
<213> Artificial Sequence

<220>
<223> Description of Artificial Sequence: Synthetic
      peptide

<400> 17
Ala Lys Thr Thr Pro Pro
1               5


<210> 18
<211> 13
<212> PRT
<213> Artificial Sequence

<220>
<223> Description of Artificial Sequence: Synthetic
      peptide

<400> 18
Ala Lys Thr Thr Pro Pro Ser Val Thr Pro Leu Ala Pro
1               5                   10


<210> 19
<211> 6
<212> PRT
<213> Artificial Sequence

<220>
<223> Description of Artificial Sequence: Synthetic
      peptide

<400> 19
Ala Lys Thr Thr Ala Pro
1               5


<210> 20
<211> 13
<212> PRT
<213> Artificial Sequence

<220>
<223> Description of Artificial Sequence: Synthetic
      peptide

<400> 20
Ala Lys Thr Thr Ala Pro Ser Val Tyr Pro Leu Ala Pro
1               5                   10


<210> 21
<211> 6
```

148

```
<212> PRT
<213> Artificial Sequence

<220>
<223> Description of Artificial Sequence: Synthetic
      peptide

<400> 21
Ala Ser Thr Lys Gly Pro
1               5


<210> 22
<211> 13
<212> PRT
<213> Artificial Sequence

<220>
<223> Description of Artificial Sequence: Synthetic
      peptide

<400> 22
Ala Ser Thr Lys Gly Pro Ser Val Phe Pro Leu Ala Pro
1               5                   10


<210> 23
<211> 15
<212> PRT
<213> Artificial Sequence

<220>
<223> Description of Artificial Sequence: Synthetic
      peptide

<400> 23
Gly Gly Gly Gly Ser Gly Gly Gly Gly Ser Gly Gly Gly Gly Ser
1               5                   10                  15


<210> 24
<211> 15
<212> PRT
<213> Artificial Sequence

<220>
<223> Description of Artificial Sequence: Synthetic
      peptide

<400> 24
Gly Glu Asn Lys Val Glu Tyr Ala Pro Ala Leu Met Ala Leu Ser
1               5                   10                  15


<210> 25
<211> 15
<212> PRT
<213> Artificial Sequence

<220>
<223> Description of Artificial Sequence: Synthetic
      peptide
```

<400> 25
Gly Pro Ala Lys Glu Leu Thr Pro Leu Lys Glu Ala Lys Val Ser
1               5                   10                  15


<210> 26
<211> 15
<212> PRT
<213> Artificial Sequence

<220>
<223> Description of Artificial Sequence: Synthetic
      peptide

<400> 26
Gly His Glu Ala Ala Ala Val Met Gln Val Gln Tyr Pro Ala Ser
1               5                   10                  15


<210> 27
<211> 10
<212> PRT
<213> Artificial Sequence

<220>
<223> Description of Artificial Sequence: Synthetic
      peptide

<400> 27
Gly Gly Gly Gly Ser Gly Gly Gly Gly Ser
1               5                   10


<210> 28
<211> 334
<212> PRT
<213> Artificial Sequence

<220>
<223> Description of Artificial Sequence: Synthetic
      polypeptide

<400> 28
Asp Ile Gln Met Thr Gln Ser Pro Ser Ser Leu Ser Ala Ser Val Gly
1               5                   10                  15


Asp Arg Val Thr Ile Thr Cys Arg Ala Ser Glu Asp Ile Tyr Ser Asn
            20                  25                  30


Leu Ala Trp Tyr Gln Gln Lys Pro Gly Lys Ala Pro Lys Leu Leu Ile
            35                  40                  45


Tyr Asp Thr Asn Asn Leu Ala Asp Gly Val Pro Ser Arg Phe Ser Gly
        50                  55                  60


Ser Gly Ser Gly Thr Asp Phe Thr Leu Thr Ile Ser Ser Leu Gln Pro
65                  70                  75                  80


150

```
Glu Asp Phe Ala Thr Tyr Tyr Cys Gln Gln Tyr Asn Asn Tyr Pro Pro
                85              90                  95

Thr Phe Gly Gln Gly Thr Lys Leu Glu Ile Lys Arg Thr Val Ala Ala
            100             105                 110

Pro Ser Val Phe Ile Phe Pro Pro Asp Ile Gln Met Thr Gln Ser Pro
            115             120                 125

Ser Ser Leu Ser Ala Ser Val Gly Asp Arg Val Thr Ile Thr Cys Ser
    130             135                 140

Ala Ser Gln Asp Ile Ser Asn Tyr Leu Asn Trp Tyr Gln Gln Lys Pro
145                 150                 155                 160

Gly Lys Ala Pro Lys Val Leu Ile Tyr Phe Thr Ser Ser Leu His Ser
            165                 170                 175

Gly Val Pro Ser Arg Phe Ser Gly Ser Gly Ser Gly Thr Asp Phe Thr
            180                 185                 190

Leu Thr Ile Ser Ser Leu Gln Pro Glu Asp Phe Ala Thr Tyr Tyr Cys
        195             200             205

Gln Gln Tyr Ser Thr Val Pro Trp Thr Phe Gly Gln Gly Thr Lys Val
    210             215                 220

Glu Ile Lys Arg Thr Val Ala Ala Pro Ser Val Phe Ile Phe Pro Pro
225             230             235                 240

Ser Asp Glu Gln Leu Lys Ser Gly Thr Ala Ser Val Val Cys Leu Leu
            245             250                 255

Asn Asn Phe Tyr Pro Arg Glu Ala Lys Val Gln Trp Lys Val Asp Asn
            260             265                 270

Ala Leu Gln Ser Gly Asn Ser Gln Glu Ser Val Thr Glu Gln Asp Ser
            275             280             285

Lys Asp Ser Thr Tyr Ser Leu Ser Ser Thr Leu Thr Leu Ser Lys Ala
    290             295             300

Asp Tyr Glu Lys His Lys Val Tyr Ala Cys Glu Val Thr His Gln Gly
305             310             315                 320

Leu Ser Ser Pro Val Thr Lys Ser Phe Asn Arg Gly Glu Cys
            325             330
```

<210> 29
<211> 577
<212> PRT
<213> Artificial Sequence

<220>
<223> Description of Artificial Sequence: Synthetic
      polypeptide

<400> 29
```
Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Asn Phe
            20                  25                  30


Pro Met Ala Trp Val Arg Gln Ala Pro Gly Lys Gly Leu Glu Trp Val
        35                  40                  45


Ala Thr Ile Ser Ser Ser Asp Gly Thr Thr Tyr Tyr Arg Asp Ser Val
    50                  55                  60


Lys Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95


Ala Arg Gly Tyr Tyr Asn Ser Pro Phe Ala Tyr Trp Gly Gln Gly Thr
            100                 105                 110


Leu Val Thr Val Ser Ser Ala Ser Thr Lys Gly Pro Glu Val Gln Leu
            115                 120                 125


Val Glu Ser Gly Gly Gly Leu Val Gln Pro Gly Gly Ser Leu Arg Leu
    130                 135                 140


Ser Cys Ala Ala Ser Gly Tyr Thr Phe Thr Asn Tyr Gly Met Asn Trp
145                 150                 155                 160


Val Arg Gln Ala Pro Gly Lys Gly Leu Glu Trp Val Gly Trp Ile Asn
                165                 170                 175


Thr Tyr Thr Gly Glu Pro Thr Tyr Ala Ala Asp Phe Lys Arg Arg Phe
            180                 185                 190


Thr Phe Ser Leu Asp Thr Ser Lys Ser Thr Ala Tyr Leu Gln Met Asn
            195                 200                 205
```

```
Ser Leu Arg Ala Glu Asp Thr Ala Val Tyr Tyr Cys Ala Lys Tyr Pro
    210             215             220

His Tyr Tyr Gly Ser Ser His Trp Tyr Phe Asp Val Trp Gly Gln Gly
225             230             235                     240

Thr Leu Val Thr Val Ser Ser Ala Ser Thr Lys Gly Pro Ser Val Phe
            245             250                     255

Pro Leu Ala Pro Ser Ser Lys Ser Thr Ser Gly Gly Thr Ala Ala Leu
            260             265             270

Gly Cys Leu Val Lys Asp Tyr Phe Pro Glu Pro Val Thr Val Ser Trp
        275             280             285

Asn Ser Gly Ala Leu Thr Ser Gly Val His Thr Phe Pro Ala Val Leu
    290             295             300

Gln Ser Ser Gly Leu Tyr Ser Leu Ser Ser Val Val Thr Val Pro Ser
305             310             315             320

Ser Ser Leu Gly Thr Gln Thr Tyr Ile Cys Asn Val Asn His Lys Pro
            325             330             335

Ser Asn Thr Lys Val Asp Lys Lys Val Glu Pro Lys Ser Cys Asp Lys
        340             345             350

Thr His Thr Cys Pro Pro Cys Pro Ala Pro Glu Ala Ala Gly Gly Pro
        355             360             365

Ser Val Phe Leu Phe Pro Pro Lys Pro Lys Asp Thr Leu Met Ile Ser
    370             375             380

Arg Thr Pro Glu Val Thr Cys Val Val Val Asp Val Ser His Glu Asp
385             390             395             400

Pro Glu Val Lys Phe Asn Trp Tyr Val Asp Gly Val Glu Val His Asn
            405             410             415

Ala Lys Thr Lys Pro Arg Glu Glu Gln Tyr Asn Ser Thr Tyr Arg Val
        420             425             430

Val Ser Val Leu Thr Val Leu His Gln Asp Trp Leu Asn Gly Lys Glu
        435             440             445

Tyr Lys Cys Lys Val Ser Asn Lys Ala Leu Pro Ala Pro Ile Glu Lys
    450             455             460
```

```
Thr Ile Ser Lys Ala Lys Gly Gln Pro Arg Glu Pro Gln Val Tyr Thr
465             470             475             480

Leu Pro Pro Ser Arg Glu Glu Met Thr Lys Asn Gln Val Ser Leu Thr
                485             490             495

Cys Leu Val Lys Gly Phe Tyr Pro Ser Asp Ile Ala Val Glu Trp Glu
            500             505             510

Ser Asn Gly Gln Pro Glu Asn Asn Tyr Lys Thr Thr Pro Pro Val Leu
            515             520             525

Asp Ser Asp Gly Ser Phe Phe Leu Tyr Ser Lys Leu Thr Val Asp Lys
    530             535             540

Ser Arg Trp Gln Gln Gly Asn Val Phe Ser Cys Ser Val Met His Glu
545             550             555             560

Ala Leu His Asn His Tyr Thr Gln Lys Ser Leu Ser Leu Ser Pro Gly
            565             570             575

Lys
```

```
<210> 30
<211> 584
<212> PRT
<213> Artificial Sequence

<220>
<223> Description of Artificial Sequence: Synthetic
      polypeptide

<400> 30
Gln Val Gln Leu Gln Gln Pro Gly Ala Glu Leu Val Lys Pro Gly Ala
1               5               10              15

Ser Val Lys Met Ser Cys Lys Ala Ser Gly Tyr Thr Phe Thr Ser Tyr
            20              25              30

Asn Met His Trp Val Lys Gln Thr Pro Gly Arg Gly Leu Glu Trp Ile
        35              40              45

Gly Ala Ile Tyr Pro Gly Asn Gly Asp Thr Ser Tyr Asn Gln Lys Phe
    50              55              60

Lys Gly Lys Ala Thr Leu Thr Ala Asp Lys Ser Ser Ser Thr Ala Tyr
65              70              75              80
```

```
Met Gln Leu Ser Ser Leu Thr Ser Glu Asp Ser Ala Val Tyr Tyr Cys
             85                  90                  95

Ala Arg Ser Thr Tyr Tyr Gly Gly Asp Trp Tyr Phe Asn Val Trp Gly
             100                 105                 110

Ala Gly Thr Thr Val Thr Val Ser Ala Ala Ser Thr Lys Gly Pro Gln
             115                 120                 125

Val Gln Leu Gln Glu Ser Gly Pro Gly Leu Val Lys Pro Ser Glu Thr
    130                 135                 140

Leu Ser Leu Thr Cys Ala Val Ser Gly Gly Ser Ile Ser Gly Gly Tyr
145                 150                 155                 160

Gly Trp Gly Trp Ile Arg Gln Pro Pro Gly Lys Gly Leu Glu Trp Ile
             165                 170                 175

Gly Ser Phe Tyr Ser Ser Ser Gly Asn Thr Tyr Tyr Asn Pro Ser Leu
             180                 185                 190

Lys Ser Gln Val Thr Ile Ser Thr Asp Thr Ser Lys Asn Gln Phe Ser
             195                 200                 205

Leu Lys Leu Asn Ser Met Thr Ala Ala Asp Thr Ala Val Tyr Tyr Cys
    210                 215                 220

Val Arg Asp Arg Leu Phe Ser Val Val Gly Met Val Tyr Asn Asn Trp
225                 230                 235                 240

Phe Asp Val Trp Gly Pro Gly Val Leu Val Thr Val Ser Ser Ala Ser
             245                 250                 255

Thr Lys Gly Pro Ser Val Phe Pro Leu Ala Pro Ser Ser Lys Ser Thr
             260                 265                 270

Ser Gly Gly Thr Ala Ala Leu Gly Cys Leu Val Lys Asp Tyr Phe Pro
             275                 280                 285

Glu Pro Val Thr Val Ser Trp Asn Ser Gly Ala Leu Thr Ser Gly Val
    290                 295                 300

His Thr Phe Pro Ala Val Leu Gln Ser Ser Gly Leu Tyr Ser Leu Ser
305                 310                 315                 320

Ser Val Val Thr Val Pro Ser Ser Ser Leu Gly Thr Gln Thr Tyr Ile
             325                 330                 335
```

```
Cys Asn Val Asn His Lys Pro Ser Asn Thr Lys Val Asp Lys Lys Val
            340                 345             350

Glu Pro Lys Ser Cys Asp Lys Thr His Thr Cys Pro Pro Cys Pro Ala
            355                 360             365

Pro Glu Ala Ala Gly Gly Pro Ser Val Phe Leu Phe Pro Pro Lys Pro
    370                 375             380

Lys Asp Thr Leu Met Ile Ser Arg Thr Pro Glu Val Thr Cys Val Val
385                 390             395                 400

Val Asp Val Ser His Glu Asp Pro Glu Val Lys Phe Asn Trp Tyr Val
            405                 410             415

Asp Gly Val Glu Val His Asn Ala Lys Thr Lys Pro Arg Glu Glu Gln
            420                 425             430

Tyr Asn Ser Thr Tyr Arg Val Val Ser Val Leu Thr Val Leu His Gln
        435             440             445

Asp Trp Leu Asn Gly Lys Glu Tyr Lys Cys Lys Val Ser Asn Lys Ala
    450                 455             460

Leu Pro Ala Pro Ile Glu Lys Thr Ile Ser Lys Ala Lys Gly Gln Pro
465                 470             475                 480

Arg Glu Pro Gln Val Tyr Thr Leu Pro Pro Ser Arg Glu Glu Met Thr
                485             490                 495

Lys Asn Gln Val Ser Leu Thr Cys Leu Val Lys Gly Phe Tyr Pro Ser
            500             505             510

Asp Ile Ala Val Glu Trp Glu Ser Asn Gly Gln Pro Glu Asn Asn Tyr
        515             520                 525

Lys Thr Thr Pro Pro Val Leu Asp Ser Asp Gly Ser Phe Phe Leu Tyr
    530             535             540

Ser Lys Leu Thr Val Asp Lys Ser Arg Trp Gln Gln Gly Asn Val Phe
545                 550             555                 560

Ser Cys Ser Val Met His Glu Ala Leu His Asn His Tyr Thr Gln Lys
            565                 570             575

Ser Leu Ser Leu Ser Pro Gly Lys
            580
```

<210> 31
<211> 329
<212> PRT
<213> Artificial Sequence

<220>
<223> Description of Artificial Sequence: Synthetic
      polypeptide

<400> 31
Gln Ile Val Leu Ser Gln Ser Pro Ala Ile Leu Ser Pro Ser Pro Gly
1               5                   10                  15


Glu Lys Val Thr Met Thr Cys Arg Ala Ser Ser Ser Val Ser Tyr Ile
            20                  25                  30


His Trp Phe Gln Gln Lys Pro Gly Ser Ser Pro Lys Pro Trp Ile Tyr
        35                  40                  45


Ala Thr Ser Asn Leu Ala Ser Gly Val Pro Val Arg Phe Ser Gly Ser
    50                  55                  60


Gly Ser Gly Thr Ser Tyr Ser Leu Thr Ile Ser Arg Val Glu Ala Glu
65                  70                  75                  80


Asp Ala Ala Thr Tyr Tyr Cys Gln Gln Trp Thr Ser Asn Pro Pro Thr
                85                  90                  95


Phe Gly Gly Gly Thr Lys Leu Glu Ile Lys Arg Thr Val Ala Ala Pro
            100                 105                 110


Glu Ser Ala Leu Thr Gln Pro Pro Ser Val Ser Gly Ala Pro Gly Gln
        115                 120                 125


Lys Val Thr Ile Ser Cys Thr Gly Ser Thr Ser Asn Ile Gly Gly Tyr
    130                 135                 140


Asp Leu His Trp Tyr Gln Gln Leu Pro Gly Thr Ala Pro Lys Leu Leu
145                 150                 155                 160


Ile Tyr Asp Ile Asn Lys Arg Pro Ser Gly Ile Ser Asp Arg Phe Ser
                165                 170                 175


Gly Ser Lys Ser Gly Thr Ala Ala Ser Leu Ala Ile Thr Gly Leu Gln
            180                 185                 190


Thr Glu Asp Glu Ala Asp Tyr Tyr Cys Gln Ser Tyr Asp Ser Ser Leu
            195                 200                 205

```
Asn Ala Gln Val Phe Gly Gly Gly Thr Arg Leu Thr Val Leu Gly Thr
    210                 215             220

Val Ala Ala Pro Ser Val Phe Ile Phe Pro Pro Ser Asp Glu Gln Leu
225                 230             235                     240

Lys Ser Gly Thr Ala Ser Val Val Cys Leu Leu Asn Asn Phe Tyr Pro
                245             250                 255

Arg Glu Ala Lys Val Gln Trp Lys Val Asp Asn Ala Leu Gln Ser Gly
            260             265                 270

Asn Ser Gln Glu Ser Val Thr Glu Gln Asp Ser Lys Asp Ser Thr Tyr
        275             280                 285

Ser Leu Ser Ser Thr Leu Thr Leu Ser Lys Ala Asp Tyr Glu Lys His
    290             295                 300

Lys Val Tyr Ala Cys Glu Val Thr His Gln Gly Leu Ser Ser Pro Val
305                 310                 315                 320

Thr Lys Ser Phe Asn Arg Gly Glu Cys
                325
```

<210> 32
<211> 584
<212> PRT
<213> Artificial Sequence

<220>
<223> Description of Artificial Sequence: Synthetic
      polypeptide

<400> 32
```
Gln Val Gln Leu Gln Glu Ser Gly Pro Gly Leu Val Lys Pro Ser Glu
1                   5               10                  15

Thr Leu Ser Leu Thr Cys Ala Val Ser Gly Gly Ser Ile Ser Gly Gly
                20              25                  30

Tyr Gly Trp Gly Trp Ile Arg Gln Pro Pro Gly Lys Gly Leu Glu Trp
        35              40                  45

Ile Gly Ser Phe Tyr Ser Ser Ser Gly Asn Thr Tyr Tyr Asn Pro Ser
    50              55                  60

Leu Lys Ser Gln Val Thr Ile Ser Thr Asp Thr Ser Lys Asn Gln Phe
65                  70              75                  80
```

```
Ser Leu Lys Leu Asn Ser Met Thr Ala Ala Asp Thr Ala Val Tyr Tyr
                85                  90                  95

Cys Val Arg Asp Arg Leu Phe Ser Val Val Gly Met Val Tyr Asn Asn
                100             105                 110

Trp Phe Asp Val Trp Gly Pro Gly Val Leu Val Thr Val Ser Ser Ala
                115             120                 125

Ser Thr Lys Gly Pro Gln Val Gln Leu Gln Gln Pro Gly Ala Glu Leu
        130             135                 140

Val Lys Pro Gly Ala Ser Val Lys Met Ser Cys Lys Ala Ser Gly Tyr
145                 150                 155                 160

Thr Phe Thr Ser Tyr Asn Met His Trp Val Lys Gln Thr Pro Gly Arg
                165             170                 175

Gly Leu Glu Trp Ile Gly Ala Ile Tyr Pro Gly Asn Gly Asp Thr Ser
                180             185                 190

Tyr Asn Gln Lys Phe Lys Gly Lys Ala Thr Leu Thr Ala Asp Lys Ser
        195             200                 205

Ser Ser Thr Ala Tyr Met Gln Leu Ser Ser Leu Thr Ser Glu Asp Ser
        210             215                 220

Ala Val Tyr Tyr Cys Ala Arg Ser Thr Tyr Tyr Gly Gly Asp Trp Tyr
225                 230                 235                 240

Phe Asn Val Trp Gly Ala Gly Thr Thr Val Thr Val Ser Ala Ala Ser
                245             250                 255

Thr Lys Gly Pro Ser Val Phe Pro Leu Ala Pro Ser Ser Lys Ser Thr
                260             265                 270

Ser Gly Gly Thr Ala Ala Leu Gly Cys Leu Val Lys Asp Tyr Phe Pro
                275             280                 285

Glu Pro Val Thr Val Ser Trp Asn Ser Gly Ala Leu Thr Ser Gly Val
        290             295                 300

His Thr Phe Pro Ala Val Leu Gln Ser Ser Gly Leu Tyr Ser Leu Ser
305                 310                 315                 320

Ser Val Val Thr Val Pro Ser Ser Ser Leu Gly Thr Gln Thr Tyr Ile
                325             330                 335
```

Cys Asn Val Asn His Lys Pro Ser Asn Thr Lys Val Asp Lys Lys Val
                340                     345             350

Glu Pro Lys Ser Cys Asp Lys Thr His Thr Cys Pro Pro Cys Pro Ala
        355                 360             365

Pro Glu Ala Ala Gly Gly Pro Ser Val Phe Leu Phe Pro Pro Lys Pro
    370                 375             380

Lys Asp Thr Leu Met Ile Ser Arg Thr Pro Glu Val Thr Cys Val Val
385                 390             395                     400

Val Asp Val Ser His Glu Asp Pro Glu Val Lys Phe Asn Trp Tyr Val
                405             410                 415

Asp Gly Val Glu Val His Asn Ala Lys Thr Lys Pro Arg Glu Glu Gln
            420                 425             430

Tyr Asn Ser Thr Tyr Arg Val Val Ser Val Leu Thr Val Leu His Gln
        435                 440             445

Asp Trp Leu Asn Gly Lys Glu Tyr Lys Cys Lys Val Ser Asn Lys Ala
    450                 455             460

Leu Pro Ala Pro Ile Glu Lys Thr Ile Ser Lys Ala Lys Gly Gln Pro
465                 470             475                     480

Arg Glu Pro Gln Val Tyr Thr Leu Pro Pro Ser Arg Glu Glu Met Thr
            485             490                 495

Lys Asn Gln Val Ser Leu Thr Cys Leu Val Lys Gly Phe Tyr Pro Ser
        500             505             510

Asp Ile Ala Val Glu Trp Glu Ser Asn Gly Gln Pro Glu Asn Asn Tyr
        515             520             525

Lys Thr Thr Pro Pro Val Leu Asp Ser Asp Gly Ser Phe Phe Leu Tyr
    530             535             540

Ser Lys Leu Thr Val Asp Lys Ser Arg Trp Gln Gln Gly Asn Val Phe
545             550             555                     560

Ser Cys Ser Val Met His Glu Ala Leu His Asn His Tyr Thr Gln Lys
            565             570                 575

Ser Leu Ser Leu Ser Pro Gly Lys
            580

<210> 33
<211> 329
<212> PRT
<213> Artificial Sequence

<220>
<223> Description of Artificial Sequence: Synthetic
      polypeptide

<400> 33

```
Glu Ser Ala Leu Thr Gln Pro Pro Ser Val Ser Gly Ala Pro Gly Gln
1               5                   10                  15


Lys Val Thr Ile Ser Cys Thr Gly Ser Thr Ser Asn Ile Gly Gly Tyr
            20                  25                  30


Asp Leu His Trp Tyr Gln Gln Leu Pro Gly Thr Ala Pro Lys Leu Leu
        35                  40                  45


Ile Tyr Asp Ile Asn Lys Arg Pro Ser Gly Ile Ser Asp Arg Phe Ser
    50                  55                  60


Gly Ser Lys Ser Gly Thr Ala Ala Ser Leu Ala Ile Thr Gly Leu Gln
65                  70                  75                  80


Thr Glu Asp Glu Ala Asp Tyr Tyr Cys Gln Ser Tyr Asp Ser Ser Leu
                85                  90                  95


Asn Ala Gln Val Phe Gly Gly Gly Thr Arg Leu Thr Val Leu Gly Gln
                100                 105                 110


Pro Lys Ala Ala Pro Gln Ile Val Leu Ser Gln Ser Pro Ala Ile Leu
        115                 120                 125


Ser Pro Ser Pro Gly Glu Lys Val Thr Met Thr Cys Arg Ala Ser Ser
    130                 135                 140


Ser Val Ser Tyr Ile His Trp Phe Gln Gln Lys Pro Gly Ser Ser Pro
145                 150                 155                 160


Lys Pro Trp Ile Tyr Ala Thr Ser Asn Leu Ala Ser Gly Val Pro Val
                165                 170                 175


Arg Phe Ser Gly Ser Gly Ser Gly Thr Ser Tyr Ser Leu Thr Ile Ser
                180                 185                 190


Arg Val Glu Ala Glu Asp Ala Ala Thr Tyr Tyr Cys Gln Gln Trp Thr
                195                 200                 205
```

```
Ser Asn Pro Pro Thr Phe Gly Gly Gly Thr Lys Leu Glu Ile Lys Arg
    210             215             220

Gln Pro Lys Ala Ala Pro Ser Val Thr Leu Phe Pro Pro Ser Ser Glu
225             230             235             240

Glu Leu Gln Ala Asn Lys Ala Thr Leu Val Cys Leu Ile Ser Asp Phe
            245             250             255

Tyr Pro Gly Ala Val Thr Val Ala Trp Lys Ala Asp Ser Ser Pro Val
            260             265             270

Lys Ala Gly Val Glu Thr Thr Thr Pro Ser Lys Gln Ser Asn Asn Lys
            275             280             285

Tyr Ala Ala Ser Ser Tyr Leu Ser Leu Thr Pro Glu Gln Trp Lys Ser
    290             295             300

His Arg Ser Tyr Ser Cys Gln Val Thr His Glu Gly Ser Thr Val Glu
305             310             315             320

Lys Thr Val Ala Pro Thr Glu Cys Ser
                325
```

```
<210> 34
<211> 579
<212> PRT
<213> Artificial Sequence

<220>
<223> Description of Artificial Sequence: Synthetic
      polypeptide

<400> 34
```

```
Glu Val Gln Leu Val Glu Ser Gly Gly Gly Leu Val Gln Pro Gly Gly
1               5               10              15

Ser Leu Arg Leu Ser Cys Ala Ala Ser Gly Tyr Thr Phe Thr Asn Tyr
            20              25              30

Gly Met Asn Trp Val Arg Gln Ala Pro Gly Lys Gly Leu Glu Trp Val
        35              40              45

Gly Trp Ile Asn Thr Tyr Thr Gly Glu Pro Thr Tyr Ala Ala Asp Phe
    50              55              60

Lys Arg Arg Phe Thr Phe Ser Leu Asp Thr Ser Lys Ser Thr Ala Tyr
65              70              75              80
```

```
Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100             105                 110

Trp Gly Gln Gly Thr Leu Val Thr Val Ser Ser Ala Ser Thr Lys Gly
        115                 120                 125

Pro Glu Val Gln Leu Val Glu Ser Gly Gly Gly Leu Val Gln Pro Gly
    130                 135                 140

Gly Ser Leu Arg Leu Ser Cys Ala Ala Ser Gly Phe Asn Ile Lys Asp
145             150                 155                 160

Thr Tyr Ile His Trp Val Arg Gln Ala Pro Gly Lys Gly Leu Glu Trp
            165                 170                 175

Val Ala Arg Ile Tyr Pro Thr Asn Gly Tyr Thr Arg Tyr Ala Asp Ser
            180                 185                 190

Val Lys Gly Arg Phe Thr Ile Ser Ala Asp Thr Ser Lys Asn Thr Ala
            195                 200                 205

Tyr Leu Gln Met Asn Ser Leu Arg Ala Glu Asp Thr Ala Val Tyr Tyr
    210                 215                 220

Cys Ser Arg Trp Gly Gly Asp Gly Phe Tyr Ala Met Asp Tyr Trp Gly
225             230                 235                 240

Gln Gly Thr Leu Val Thr Val Ser Ser Ala Ser Thr Lys Gly Pro Ser
            245                 250                 255

Val Phe Pro Leu Ala Pro Ser Ser Lys Ser Thr Ser Gly Gly Thr Ala
            260                 265                 270

Ala Leu Gly Cys Leu Val Lys Asp Tyr Phe Pro Glu Pro Val Thr Val
        275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305             310                 315                 320

Pro Ser Ser Ser Leu Gly Thr Gln Thr Tyr Ile Cys Asn Val Asn His
            325                 330                 335
```

```
Lys Pro Ser Asn Thr Lys Val Asp Lys Lys Val Glu Pro Lys Ser Cys
        340                 345             350

Asp Lys Thr His Thr Cys Pro Pro Cys Pro Ala Pro Glu Ala Ala Gly
        355                 360             365

Gly Pro Ser Val Phe Leu Phe Pro Pro Lys Pro Lys Asp Thr Leu Met
        370             375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
                420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Ala Leu Pro Ala Pro Ile
        450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Glu Glu Met Thr Lys Asn Gln Val Ser
                485                 490                 495

Leu Thr Cys Leu Val Lys Gly Phe Tyr Pro Ser Asp Ile Ala Val Glu
                500                 505                 510

Trp Glu Ser Asn Gly Gln Pro Glu Asn Asn Tyr Lys Thr Thr Pro Pro
                515                 520                 525

Val Leu Asp Ser Asp Gly Ser Phe Phe Leu Tyr Ser Lys Leu Thr Val
        530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys
```

<210> 35
<211> 327
<212> PRT
<213> Artificial Sequence

<220>
<223> Description of Artificial Sequence: Synthetic
      polypeptide

<400> 35

```
Asp Ile Gln Met Thr Gln Ser Pro Ser Ser Leu Ser Ala Ser Val Gly
1               5                   10                  15


Asp Arg Val Thr Ile Thr Cys Ser Ala Ser Gln Asp Ile Ser Asn Tyr
            20                  25                  30


Leu Asn Trp Tyr Gln Gln Lys Pro Gly Lys Ala Pro Lys Val Leu Ile
        35                  40                  45


Tyr Phe Thr Ser Ser Leu His Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Tyr Ser Thr Val Pro Trp
                85                  90                  95


Thr Phe Gly Gln Gly Thr Lys Val Glu Ile Lys Arg Thr Val Ala Ala
            100                 105                 110


Pro Asp Ile Gln Met Thr Gln Ser Pro Ser Ser Leu Ser Ala Ser Val
        115                 120                 125


Gly Asp Arg Val Thr Ile Thr Cys Arg Ala Ser Gln Asp Val Asn Thr
    130                 135                 140


Ala Val Ala Trp Tyr Gln Gln Lys Pro Gly Lys Ala Pro Lys Leu Leu
145                 150                 155                 160


Ile Tyr Ser Ala Ser Phe Leu Tyr Ser Gly Val Pro Ser Arg Phe Ser
                165                 170                 175


Gly Ser Arg Ser Gly Thr Asp Phe Thr Leu Thr Ile Ser Ser Leu Gln
            180                 185                 190


Pro Glu Asp Phe Ala Thr Tyr Tyr Cys Gln Gln His Tyr Thr Thr Pro
        195                 200                 205
```

```
Pro Thr Phe Gly Gln Gly Thr Lys Val Glu Ile Lys Arg Thr Val Ala
    210             215             220

Ala Pro Ser Val Phe Ile Phe Pro Pro Ser Asp Glu Gln Leu Lys Ser
225             230             235                 240

Gly Thr Ala Ser Val Val Cys Leu Leu Asn Asn Phe Tyr Pro Arg Glu
                245             250                 255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
            275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315                 320

Ser Phe Asn Arg Gly Glu Cys
                325
```

```
<210> 36
<211> 579
<212> PRT
<213> Artificial Sequence

<220>
<223> Description of Artificial Sequence: Synthetic
      polypeptide

<400> 36
Glu Val Gln Leu Val Glu Ser Gly Gly Gly Leu Val Gln Pro Gly Gly
1               5               10                  15

Ser Leu Arg Leu Ser Cys Ala Ala Ser Gly Phe Asn Ile Lys Asp Thr
            20              25                  30

Tyr Ile His Trp Val Arg Gln Ala Pro Gly Lys Gly Leu Glu Trp Val
            35              40                  45

Ala Arg Ile Tyr Pro Thr Asn Gly Tyr Thr Arg Tyr Ala Asp Ser Val
    50              55              60

Lys Gly Arg Phe Thr Ile Ser Ala Asp Thr Ser Lys Asn Thr Ala Tyr
65              70              75                  80
```

```
Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100             105             110

Gly Thr Leu Val Thr Val Ser Ser Ala Ser Thr Lys Gly Pro Glu Val
            115             120             125

Gln Leu Val Glu Ser Gly Gly Gly Leu Val Gln Pro Gly Gly Ser Leu
        130             135             140

Arg Leu Ser Cys Ala Ala Ser Gly Tyr Thr Phe Thr Asn Tyr Gly Met
145             150             155             160

Asn Trp Val Arg Gln Ala Pro Gly Lys Gly Leu Glu Trp Val Gly Trp
                165             170             175

Ile Asn Thr Tyr Thr Gly Glu Pro Thr Tyr Ala Ala Asp Phe Lys Arg
            180             185             190

Arg Phe Thr Phe Ser Leu Asp Thr Ser Lys Ser Thr Ala Tyr Leu Gln
            195             200             205

Met Asn Ser Leu Arg Ala Glu Asp Thr Ala Val Tyr Tyr Cys Ala Lys
    210             215             220

Tyr Pro His Tyr Tyr Gly Ser Ser His Trp Tyr Phe Asp Val Trp Gly
225             230             235             240

Gln Gly Thr Leu Val Thr Val Ser Ser Ala Ser Thr Lys Gly Pro Ser
            245             250             255

Val Phe Pro Leu Ala Pro Ser Ser Lys Ser Thr Ser Gly Gly Thr Ala
            260             265             270

Ala Leu Gly Cys Leu Val Lys Asp Tyr Phe Pro Glu Pro Val Thr Val
            275             280             285

Ser Trp Asn Ser Gly Ala Leu Thr Ser Gly Val His Thr Phe Pro Ala
    290             295             300

Val Leu Gln Ser Ser Gly Leu Tyr Ser Leu Ser Ser Val Val Thr Val
305             310             315             320

Pro Ser Ser Ser Leu Gly Thr Gln Thr Tyr Ile Cys Asn Val Asn His
            325             330             335
```

167

```
Lys Pro Ser Asn Thr Lys Val Asp Lys Lys Val Glu Pro Lys Ser Cys
        340                 345                 350

Asp Lys Thr His Thr Cys Pro Pro Cys Pro Ala Pro Glu Ala Ala Gly
        355                 360                 365

Gly Pro Ser Val Phe Leu Phe Pro Pro Lys Pro Lys Asp Thr Leu Met
        370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
                420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Ala Leu Pro Ala Pro Ile
        450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Glu Glu Met Thr Lys Asn Gln Val Ser
                485                 490                 495

Leu Thr Cys Leu Val Lys Gly Phe Tyr Pro Ser Asp Ile Ala Val Glu
        500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
        530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys
```

<210> 37
<211> 327
<212> PRT
<213> Artificial Sequence

<220>
<223> Description of Artificial Sequence: Synthetic
      polypeptide

<400> 37
Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Asp Val Asn Thr Ala
            20                  25                  30


Val Ala Trp Tyr Gln Gln Lys Pro Gly Lys Ala Pro Lys Leu Leu Ile
            35                  40                  45


Tyr Ser Ala Ser Phe Leu Tyr Ser Gly Val Pro Ser Arg Phe Ser Gly
    50                  55                  60


Ser Arg Ser Gly Thr Asp Phe Thr Leu Thr Ile Ser Ser Leu Gln Pro
65                  70                  75                  80


Glu Asp Phe Ala Thr Tyr Tyr Cys Gln Gln His Tyr Thr Thr Pro Pro
                85                  90                  95


Thr Phe Gly Gln Gly Thr Lys Val Glu Ile Lys Arg Thr Val Ala Ala
            100                 105                 110


Pro Asp Ile Gln Met Thr Gln Ser Pro Ser Ser Leu Ser Ala Ser Val
            115                 120                 125


Gly Asp Arg Val Thr Ile Thr Cys Ser Ala Ser Gln Asp Ile Ser Asn
    130                 135                 140


Tyr Leu Asn Trp Tyr Gln Gln Lys Pro Gly Lys Ala Pro Lys Val Leu
145                 150                 155                 160


Ile Tyr Phe Thr Ser Ser Leu His Ser Gly Val Pro Ser Arg Phe Ser
                165                 170                 175


Gly Ser Gly Ser Gly Thr Asp Phe Thr Leu Thr Ile Ser Ser Leu Gln
            180                 185                 190


Pro Glu Asp Phe Ala Thr Tyr Tyr Cys Gln Gln Tyr Ser Thr Val Pro
            195                 200                 205

169

Trp Thr Phe Gly Gln Gly Thr Lys Val Glu Ile Lys Arg Thr Val Ala
210             215             220

Ala Pro Ser Val Phe Ile Phe Pro Pro Ser Asp Glu Gln Leu Lys Ser
225             230             235             240

Gly Thr Ala Ser Val Val Cys Leu Leu Asn Asn Phe Tyr Pro Arg Glu
                245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
            275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315             320

Ser Phe Asn Arg Gly Glu Cys
                325

<210> 38
<211> 579
<212> PRT
<213> Artificial Sequence

<220>
<223> Description of Artificial Sequence: Synthetic
      polypeptide

<400> 38
Glu Val Gln Leu Val Glu Ser Gly Gly Gly Leu Val Gln Pro Gly Arg
1               5               10              15

Ser Leu Arg Leu Ser Cys Ala Ala Ser Gly Phe Thr Phe Asp Asp Tyr
            20              25              30

Ala Met His Trp Val Arg Gln Ala Pro Gly Lys Gly Leu Glu Trp Val
            35              40              45

Ser Ala Ile Thr Trp Asn Ser Gly His Ile Asp Tyr Ala Asp Ser Val
    50              55              60

Glu Gly Arg Phe Thr Ile Ser Arg Asp Asn Ala Lys Asn Ser Leu Tyr
65              70              75              80

170

Leu Gln Met Asn Ser Leu Arg Ala Glu Asp Thr Ala Val Tyr Tyr Cys
                    85                  90                  95

Ala Lys Val Ser Tyr Leu Ser Thr Ala Ser Ser Leu Asp Tyr Trp Gly
                100                 105                 110

Gln Gly Thr Leu Val Thr Val Ser Ser Ala Ser Thr Lys Gly Pro Glu
                115                 120                 125

Val Gln Leu Leu Glu Ser Gly Gly Gly Leu Val Gln Pro Gly Gly Ser
        130                 135                 140

Leu Arg Leu Ser Cys Ala Ala Ser Gly Phe Thr Phe Ser Ser Tyr Ala
145                 150                 155                 160

Met Ser Trp Val Arg Gln Ala Pro Gly Lys Gly Leu Glu Trp Val Ser
                165                 170                 175

Gly Ile Thr Gly Ser Gly Gly Ser Thr Tyr Tyr Ala Asp Ser Val Lys
                180                 185                 190

Gly Arg Phe Thr Ile Ser Arg Asp Asn Ser Lys Asn Thr Leu Tyr Leu
                195                 200                 205

Gln Met Asn Ser Leu Arg Ala Glu Asp Thr Ala Val Tyr Tyr Cys Ala
        210                 215                 220

Lys Asp Pro Gly Thr Thr Val Ile Met Ser Trp Phe Asp Pro Trp Gly
225                 230                 235                 240

Gln Gly Thr Leu Val Thr Val Ser Ser Ala Ser Thr Lys Gly Pro Ser
                245                 250                 255

Val Phe Pro Leu Ala Pro Ser Ser Lys Ser Thr Ser Gly Gly Thr Ala
                260                 265                 270

Ala Leu Gly Cys Leu Val Lys Asp Tyr Phe Pro Glu Pro Val Thr Val
        275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
                325                 330                 335

Lys Pro Ser Asn Thr Lys Val Asp Lys Lys Val Glu Pro Lys Ser Cys
            340                     345                 350

Asp Lys Thr His Thr Cys Pro Pro Cys Pro Ala Pro Glu Ala Ala Gly
            355                     360                 365

Gly Pro Ser Val Phe Leu Phe Pro Pro Lys Pro Lys Asp Thr Leu Met
            370                     375                 380

Ile Ser Arg Thr Pro Glu Val Thr Cys Val Val Val Asp Val Ser His
385                     390                     395                 400

Glu Asp Pro Glu Val Lys Phe Asn Trp Tyr Val Asp Gly Val Glu Val
                405                     410                     415

His Asn Ala Lys Thr Lys Pro Arg Glu Glu Gln Tyr Asn Ser Thr Tyr
                420                     425                 430

Arg Val Val Ser Val Leu Thr Val Leu His Gln Asp Trp Leu Asn Gly
            435                     440                     445

Lys Glu Tyr Lys Cys Lys Val Ser Asn Lys Ala Leu Pro Ala Pro Ile
    450                     455                     460

Glu Lys Thr Ile Ser Lys Ala Lys Gly Gln Pro Arg Glu Pro Gln Val
465                     470                     475                 480

Tyr Thr Leu Pro Pro Ser Arg Glu Glu Met Thr Lys Asn Gln Val Ser
                485                     490                     495

Leu Thr Cys Leu Val Lys Gly Phe Tyr Pro Ser Asp Ile Ala Val Glu
            500                     505                     510

Trp Glu Ser Asn Gly Gln Pro Glu Asn Asn Tyr Lys Thr Thr Pro Pro
            515                     520                     525

Val Leu Asp Ser Asp Gly Ser Phe Phe Leu Tyr Ser Lys Leu Thr Val
    530                     535                     540

Asp Lys Ser Arg Trp Gln Gln Gly Asn Val Phe Ser Cys Ser Val Met
545                     550                     555                 560

His Glu Ala Leu His Asn His Tyr Thr Gln Lys Ser Leu Ser Leu Ser
                565                     570                     575

Pro Gly Lys

<210> 39
<211> 328
<212> PRT
<213> Artificial Sequence

<220>
<223> Description of Artificial Sequence: Synthetic
      polypeptide

<400> 39
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
        100                 105                 110

Pro Glu Ile Val Leu Thr Gln Ser Pro Gly Thr Leu Ser Leu Ser Pro
        115                 120                 125

Gly Glu Arg Ala Thr Leu Ser Cys Arg Ala Ser Gln Ser Val Arg Gly
    130                 135                 140

Arg Tyr Leu Ala Trp Tyr Gln Gln Lys Pro Gly Gln Ala Pro Arg Leu
145                 150                 155                 160

Leu Ile Tyr Gly Ala Ser Ser Arg Ala Thr Gly Ile Pro Asp Arg Phe
            165                 170                 175

Ser Gly Ser Gly Ser Gly Thr Asp Phe Thr Leu Thr Ile Ser Arg Leu
        180                 185                 190

Glu Pro Glu Asp Phe Ala Val Phe Tyr Cys Gln Gln Tyr Gly Ser Ser
        195                 200                 205

173

EP 3 466 973 A1

```
Pro Arg Thr Phe Gly Gln Gly Thr Lys Val Glu Ile Lys Arg Thr Val
    210             215                 220

Ala Ala Pro Ser Val Phe Ile Phe Pro Pro Ser Asp Glu Gln Leu Lys
225             230                 235                     240

Ser Gly Thr Ala Ser Val Val Cys Leu Leu Asn Asn Phe Tyr Pro Arg
                245                 250                 255

Glu Ala Lys Val Gln Trp Lys Val Asp Asn Ala Leu Gln Ser Gly Asn
            260                 265                 270

Ser Gln Glu Ser Val Thr Glu Gln Asp Ser Lys Asp Ser Thr Tyr Ser
        275                 280                 285

Leu Ser Ser Thr Leu Thr Leu Ser Lys Ala Asp Tyr Glu Lys His Lys
    290                 295                 300

Val Tyr Ala Cys Glu Val Thr His Gln Gly Leu Ser Ser Pro Val Thr
305             310                 315                     320

Lys Ser Phe Asn Arg Gly Glu Cys
                325
```

<210> 40
<211> 579
<212> PRT
<213> Artificial Sequence

<220>
<223> Description of Artificial Sequence: Synthetic
      polypeptide

<400> 40
```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Gly Ile Thr Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                      80
```

174

```
Leu Gln Met Asn Ser Leu Arg Ala Glu Asp Thr Ala Val Tyr Tyr Cys
            85                  90                  95

Ala Lys Asp Pro Gly Thr Thr Val Ile Met Ser Trp Phe Asp Pro Trp
            100                 105                 110

Gly Gln Gly Thr Leu Val Thr Val Ser Ser Ala Ser Thr Lys Gly Pro
            115                 120                 125

Glu Val Gln Leu Val Glu Ser Gly Gly Gly Leu Val Gln Pro Gly Arg
    130                 135                 140

Ser Leu Arg Leu Ser Cys Ala Ala Ser Gly Phe Thr Phe Asp Asp Tyr
145                 150                 155                 160

Ala Met His Trp Val Arg Gln Ala Pro Gly Lys Gly Leu Glu Trp Val
            165                 170                 175

Ser Ala Ile Thr Trp Asn Ser Gly His Ile Asp Tyr Ala Asp Ser Val
            180                 185                 190

Glu Gly Arg Phe Thr Ile Ser Arg Asp Asn Ala Lys Asn Ser Leu Tyr
            195                 200                 205

Leu Gln Met Asn Ser Leu Arg Ala Glu Asp Thr Ala Val Tyr Tyr Cys
    210                 215                 220

Ala Lys Val Ser Tyr Leu Ser Thr Ala Ser Ser Leu Asp Tyr Trp Gly
225                 230                 235                 240

Gln Gly Thr Leu Val Thr Val Ser Ser Ala Ser Thr Lys Gly Pro Ser
            245                 250                 255

Val Phe Pro Leu Ala Pro Ser Ser Lys Ser Thr Ser Gly Gly Thr Ala
            260                 265                 270

Ala Leu Gly Cys Leu Val Lys Asp Tyr Phe Pro Glu Pro Val Thr Val
            275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
            325                 330                 335
```

Lys Pro Ser Asn Thr Lys Val Asp Lys Lys Val Glu Pro Lys Ser Cys
        340                 345                 350

Asp Lys Thr His Thr Cys Pro Pro Cys Pro Ala Pro Glu Ala Ala Gly
        355                 360                 365

Gly Pro Ser Val Phe Leu Phe Pro Pro Lys Pro Lys Asp Thr Leu Met
        370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
        420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Ala Leu Pro Ala Pro Ile
        450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Glu Glu Met Thr Lys Asn Gln Val Ser
                485                 490                 495

Leu Thr Cys Leu Val Lys Gly Phe Tyr Pro Ser Asp Ile Ala Val Glu
        500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
        530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210> 41
<211> 328
<212> PRT
<213> Artificial Sequence

<220>
<223> Description of Artificial Sequence: Synthetic
      polypeptide

<400> 41

```
Glu Ile Val Leu Thr Gln Ser Pro Gly Thr Leu Ser Leu Ser Pro Gly
1               5                   10                  15


Glu Arg Ala Thr Leu Ser Cys Arg Ala Ser Gln Ser Val Arg Gly Arg
            20                  25                  30


Tyr Leu Ala Trp Tyr Gln Gln Lys Pro Gly Gln Ala Pro Arg Leu Leu
        35                  40                  45


Ile Tyr Gly Ala Ser Ser Arg Ala Thr Gly Ile Pro Asp Arg Phe Ser
    50                  55                  60


Gly Ser Gly Ser Gly Thr Asp Phe Thr Leu Thr Ile Ser Arg Leu Glu
65                  70                  75                  80


Pro Glu Asp Phe Ala Val Phe Tyr Cys Gln Gln Tyr Gly Ser Ser Pro
                85                  90                  95


Arg Thr Phe Gly Gln Gly Thr Lys Val Glu Ile Lys Arg Thr Val Ala
            100                 105                 110


Ala Pro Asp Ile Gln Met Thr Gln Ser Pro Ser Ser Leu Ser Ala Ser
        115                 120                 125


Val Gly Asp Arg Val Thr Ile Thr Cys Arg Ala Ser Gln Gly Ile Arg
    130                 135                 140


Asn Tyr Leu Ala Trp Tyr Gln Gln Lys Pro Gly Lys Ala Pro Lys Leu
145                 150                 155                 160


Leu Ile Tyr Ala Ala Ser Thr Leu Gln Ser Gly Val Pro Ser Arg Phe
                165                 170                 175


Ser Gly Ser Gly Ser Gly Thr Asp Phe Thr Leu Thr Ile Ser Ser Leu
            180                 185                 190


Gln Pro Glu Asp Val Ala Thr Tyr Tyr Cys Gln Arg Tyr Asn Arg Ala
        195                 200                 205
```

```
Pro Tyr Thr Phe Gly Gln Gly Thr Lys Val Glu Ile Lys Arg Thr Val
    210             215             220

Ala Ala Pro Ser Val Phe Ile Phe Pro Pro Ser Asp Glu Gln Leu Lys
225             230             235                     240

Ser Gly Thr Ala Ser Val Val Cys Leu Leu Asn Asn Phe Tyr Pro Arg
                245             250                 255

Glu Ala Lys Val Gln Trp Lys Val Asp Asn Ala Leu Gln Ser Gly Asn
            260             265                 270

Ser Gln Glu Ser Val Thr Glu Gln Asp Ser Lys Asp Ser Thr Tyr Ser
        275             280                 285

Leu Ser Ser Thr Leu Thr Leu Ser Lys Ala Asp Tyr Glu Lys His Lys
    290             295                 300

Val Tyr Ala Cys Glu Val Thr His Gln Gly Leu Ser Ser Pro Val Thr
305             310                 315                     320

Lys Ser Phe Asn Arg Gly Glu Cys
                325
```

<210> 42
<211> 577
<212> PRT
<213> Artificial Sequence

<220>
<223> Description of Artificial Sequence: Synthetic
      polypeptide

<400> 42

```
Glu Val Gln Leu Val Glu Ser Gly Gly Gly Leu Val Gln Pro Gly Arg
1               5               10                  15

Ser Leu Arg Leu Ser Cys Ala Ala Ser Gly Phe Thr Phe Asp Asp Tyr
            20              25                  30

Ala Met His Trp Val Arg Gln Ala Pro Gly Lys Gly Leu Glu Trp Val
        35              40                  45

Ser Ala Ile Thr Trp Asn Ser Gly His Ile Asp Tyr Ala Asp Ser Val
    50              55                  60

Glu Gly Arg Phe Thr Ile Ser Arg Asp Asn Ala Lys Asn Ser Leu Tyr
65              70              75                      80
```

Leu Gln Met Asn Ser Leu Arg Ala Glu Asp Thr Ala Val Tyr Tyr Cys
                    85                  90                  95

Ala Lys Val Ser Tyr Leu Ser Thr Ala Ser Ser Leu Asp Tyr Trp Gly
            100                 105                 110

Gln Gly Thr Leu Val Thr Val Ser Ser Ala Ser Thr Lys Gly Pro Glu
            115                 120                 125

Val Gln Leu Val Glu Ser Gly Gly Gly Leu Val Gln Pro Ala Asn Ser
    130                 135                 140

Leu Lys Leu Ser Cys Ala Ala Ser Gly Phe Thr Phe Ser Asp Tyr Ala
145                 150                 155                 160

Met Ala Trp Val Arg Gln Ser Pro Lys Lys Gly Leu Glu Trp Val Ala
            165                 170                 175

Thr Ile Ile Tyr Asp Gly Ser Ser Thr Tyr Tyr Arg Asp Ser Val Lys
            180                 185                 190

Gly Arg Phe Thr Ile Ser Arg Asp Asn Ala Lys Ser Thr Leu Tyr Leu
            195                 200                 205

Gln Met Asp Ser Leu Arg Ser Glu Asp Thr Ala Thr Tyr Tyr Cys Ala
    210                 215                 220

Thr Gly Leu Gly Ile Ala Thr Asp Tyr Phe Asp Tyr Trp Gly Gln Gly
225                 230                 235                 240

Val Leu Val Thr Val Ser Ser Ala Ser Thr Lys Gly Pro Ser Val Phe
            245                 250                 255

Pro Leu Ala Pro Ser Ser Lys Ser Thr Ser Gly Gly Thr Ala Ala Leu
            260                 265                 270

Gly Cys Leu Val Lys Asp Tyr Phe Pro Glu Pro Val Thr Val Ser Trp
            275                 280                 285

Asn Ser Gly Ala Leu Thr Ser Gly Val His Thr Phe Pro Ala Val Leu
    290                 295                 300

Gln Ser Ser Gly Leu Tyr Ser Leu Ser Ser Val Val Thr Val Pro Ser
305                 310                 315                 320

Ser Ser Leu Gly Thr Gln Thr Tyr Ile Cys Asn Val Asn His Lys Pro
            325                 330                 335

```
Ser Asn Thr Lys Val Asp Lys Lys Val Glu Pro Lys Ser Cys Asp Lys
            340             345         350

Thr His Thr Cys Pro Pro Cys Pro Ala Pro Glu Ala Ala Gly Gly Pro
            355             360         365

Ser Val Phe Leu Phe Pro Pro Lys Pro Lys Asp Thr Leu Met Ile Ser
            370             375         380

Arg Thr Pro Glu Val Thr Cys Val Val Val Asp Val Ser His Glu Asp
385                 390             395                 400

Pro Glu Val Lys Phe Asn Trp Tyr Val Asp Gly Val Glu Val His Asn
                405             410             415

Ala Lys Thr Lys Pro Arg Glu Glu Gln Tyr Asn Ser Thr Tyr Arg Val
            420             425             430

Val Ser Val Leu Thr Val Leu His Gln Asp Trp Leu Asn Gly Lys Glu
            435             440             445

Tyr Lys Cys Lys Val Ser Asn Lys Ala Leu Pro Ala Pro Ile Glu Lys
    450             455             460

Thr Ile Ser Lys Ala Lys Gly Gln Pro Arg Glu Pro Gln Val Tyr Thr
465             470             475                 480

Leu Pro Pro Ser Arg Glu Glu Met Thr Lys Asn Gln Val Ser Leu Thr
            485             490             495

Cys Leu Val Lys Gly Phe Tyr Pro Ser Asp Ile Ala Val Glu Trp Glu
            500             505             510

Ser Asn Gly Gln Pro Glu Asn Asn Tyr Lys Thr Thr Pro Pro Val Leu
            515             520             525

Asp Ser Asp Gly Ser Phe Phe Leu Tyr Ser Lys Leu Thr Val Asp Lys
    530             535             540

Ser Arg Trp Gln Gln Gly Asn Val Phe Ser Cys Ser Val Met His Glu
545             550             555                 560

Ala Leu His Asn His Tyr Thr Gln Lys Ser Leu Ser Leu Ser Pro Gly
            565             570             575

Lys
```

<210> 43
<211> 327
<212> PRT
<213> Artificial Sequence

<220>
<223> Description of Artificial Sequence: Synthetic
polypeptide

<400> 43
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
        100                 105                 110

Pro Asp Ile Arg Met Thr Gln Ser Pro Ala Ser Leu Ser Ala Ser Leu
        115                 120                 125

Gly Glu Thr Val Asn Ile Glu Cys Leu Ala Ser Glu Asp Ile Tyr Ser
    130                 135                 140

Asp Leu Ala Trp Tyr Gln Gln Lys Pro Gly Lys Ser Pro Gln Leu Leu
145                 150                 155                 160

Ile Tyr Asn Ala Asn Ser Leu Gln Asn Gly Val Pro Ser Arg Phe Ser
            165                 170                 175

Gly Ser Gly Ser Gly Thr Gln Tyr Ser Leu Lys Ile Asn Ser Leu Gln
        180                 185                 190

Ser Glu Asp Val Ala Thr Tyr Phe Cys Gln Gln Tyr Asn Asn Tyr Pro
        195                 200                 205

```
Pro Thr Phe Gly Gly Gly Thr Lys Leu Glu Leu Lys Arg Thr Val Ala
    210             215             220

Ala Pro Ser Val Phe Ile Phe Pro Pro Ser Asp Glu Gln Leu Lys Ser
225             230             235                 240

Gly Thr Ala Ser Val Val Cys Leu Leu Asn Asn Phe Tyr Pro Arg Glu
            245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
            275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315                 320

Ser Phe Asn Arg Gly Glu Cys
                325
```

<210> 44
<211> 577
<212> PRT
<213> Artificial Sequence

<220>
<223> Description of Artificial Sequence: Synthetic
      polypeptide

<400> 44
```
Glu Val Gln Leu Val Glu Ser Gly Gly Gly Leu Val Gln Pro Ala Asn
1               5               10              15

Ser Leu Lys Leu Ser Cys Ala Ala Ser Gly Phe Thr Phe Ser Asp Tyr
            20              25              30

Ala Met Ala Trp Val Arg Gln Ser Pro Lys Lys Gly Leu Glu Trp Val
        35              40              45

Ala Thr Ile Ile Tyr Asp Gly Ser Ser Thr Tyr Tyr Arg Asp Ser Val
    50              55              60

Lys Gly Arg Phe Thr Ile Ser Arg Asp Asn Ala Lys Ser Thr Leu Tyr
65              70              75              80
```

Leu Gln Met Asp Ser Leu Arg Ser Glu Asp Thr Ala Thr Tyr Tyr Cys
                    85                  90                  95

Ala Thr Gly Leu Gly Ile Ala Thr Asp Tyr Phe Asp Tyr Trp Gly Gln
            100                 105             110

Gly Val Leu Val Thr Val Ser Ser Ala Ser Thr Lys Gly Pro Glu Val
            115                 120             125

Gln Leu Val Glu Ser Gly Gly Gly Leu Val Gln Pro Gly Arg Ser Leu
    130                 135                 140

Arg Leu Ser Cys Ala Ala Ser Gly Phe Thr Phe Asp Asp Tyr Ala Met
145             150             155                 160

His Trp Val Arg Gln Ala Pro Gly Lys Gly Leu Glu Trp Val Ser Ala
            165                 170             175

Ile Thr Trp Asn Ser Gly His Ile Asp Tyr Ala Asp Ser Val Glu Gly
            180             185             190

Arg Phe Thr Ile Ser Arg Asp Asn Ala Lys Asn Ser Leu Tyr Leu Gln
        195             200             205

Met Asn Ser Leu Arg Ala Glu Asp Thr Ala Val Tyr Tyr Cys Ala Lys
    210             215             220

Val Ser Tyr Leu Ser Thr Ala Ser Ser Leu Asp Tyr Trp Gly Gln Gly
225             230             235             240

Thr Leu Val Thr Val Ser Ser Ala Ser Thr Lys Gly Pro Ser Val Phe
            245             250             255

Pro Leu Ala Pro Ser Ser Lys Ser Thr Ser Gly Gly Thr Ala Ala Leu
            260             265             270

Gly Cys Leu Val Lys Asp Tyr Phe Pro Glu Pro Val Thr Val Ser Trp
        275             280             285

Asn Ser Gly Ala Leu Thr Ser Gly Val His Thr Phe Pro Ala Val Leu
    290             295             300

Gln Ser Ser Gly Leu Tyr Ser Leu Ser Ser Val Val Thr Val Pro Ser
305             310             315             320

Ser Ser Leu Gly Thr Gln Thr Tyr Ile Cys Asn Val Asn His Lys Pro
            325             330             335

```
Ser Asn Thr Lys Val Asp Lys Lys Val Glu Pro Lys Ser Cys Asp Lys
            340             345         350

Thr His Thr Cys Pro Pro Cys Pro Ala Pro Glu Ala Ala Gly Gly Pro
            355             360         365

Ser Val Phe Leu Phe Pro Pro Lys Pro Lys Asp Thr Leu Met Ile Ser
            370             375         380

Arg Thr Pro Glu Val Thr Cys Val Val Val Asp Val Ser His Glu Asp
385                 390             395                 400

Pro Glu Val Lys Phe Asn Trp Tyr Val Asp Gly Val Glu Val His Asn
                405             410                 415

Ala Lys Thr Lys Pro Arg Glu Glu Gln Tyr Asn Ser Thr Tyr Arg Val
            420             425             430

Val Ser Val Leu Thr Val Leu His Gln Asp Trp Leu Asn Gly Lys Glu
            435             440             445

Tyr Lys Cys Lys Val Ser Asn Lys Ala Leu Pro Ala Pro Ile Glu Lys
    450             455             460

Thr Ile Ser Lys Ala Lys Gly Gln Pro Arg Glu Pro Gln Val Tyr Thr
465             470             475                 480

Leu Pro Pro Ser Arg Glu Glu Met Thr Lys Asn Gln Val Ser Leu Thr
                485             490             495

Cys Leu Val Lys Gly Phe Tyr Pro Ser Asp Ile Ala Val Glu Trp Glu
            500             505             510

Ser Asn Gly Gln Pro Glu Asn Asn Tyr Lys Thr Thr Pro Pro Val Leu
            515             520             525

Asp Ser Asp Gly Ser Phe Phe Leu Tyr Ser Lys Leu Thr Val Asp Lys
    530             535             540

Ser Arg Trp Gln Gln Gly Asn Val Phe Ser Cys Ser Val Met His Glu
545             550             555                 560

Ala Leu His Asn His Tyr Thr Gln Lys Ser Leu Ser Leu Ser Pro Gly
            565             570             575

Lys
```

<210> 45
<211> 327
<212> PRT
<213> Artificial Sequence

<220>
<223> Description of Artificial Sequence: Synthetic
      polypeptide

<400> 45
Asp Ile Arg Met Thr Gln Ser Pro Ala Ser Leu Ser Ala Ser Leu Gly
1               5                   10                  15


Glu Thr Val Asn Ile Glu Cys Leu Ala Ser Glu Asp Ile Tyr Ser Asp
                20                  25                  30


Leu Ala Trp Tyr Gln Gln Lys Pro Gly Lys Ser Pro Gln Leu Leu Ile
            35                  40                  45


Tyr Asn Ala Asn Ser Leu Gln Asn Gly Val Pro Ser Arg Phe Ser Gly
    50                  55                  60


Ser Gly Ser Gly Thr Gln Tyr Ser Leu Lys Ile Asn Ser Leu Gln Ser
65                  70                  75                  80


Glu Asp Val Ala Thr Tyr Phe Cys Gln Gln Tyr Asn Asn Tyr Pro Pro
                85                  90                  95


Thr Phe Gly Gly Gly Thr Lys Leu Glu Leu Lys Arg Thr Val Ala Ala
            100                 105                 110


Pro Asp Ile Gln Met Thr Gln Ser Pro Ser Ser Leu Ser Ala Ser Val
            115                 120                 125


Gly Asp Arg Val Thr Ile Thr Cys Arg Ala Ser Gln Gly Ile Arg Asn
    130                 135                 140


Tyr Leu Ala Trp Tyr Gln Gln Lys Pro Gly Lys Ala Pro Lys Leu Leu
145                 150                 155                 160


Ile Tyr Ala Ala Ser Thr Leu Gln Ser Gly Val Pro Ser Arg Phe Ser
                165                 170                 175


Gly Ser Gly Ser Gly Thr Asp Phe Thr Leu Thr Ile Ser Ser Leu Gln
            180                 185                 190


Pro Glu Asp Val Ala Thr Tyr Tyr Cys Gln Arg Tyr Asn Arg Ala Pro
            195                 200                 205

```
Tyr Thr Phe Gly Gln Gly Thr Lys Val Glu Ile Lys Arg Thr Val Ala
    210             215             220

Ala Pro Ser Val Phe Ile Phe Pro Pro Ser Asp Glu Gln Leu Lys Ser
225             230             235                     240

Gly Thr Ala Ser Val Val Cys Leu Leu Asn Asn Phe Tyr Pro Arg Glu
            245             250                     255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
            275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315                     320

Ser Phe Asn Arg Gly Glu Cys
                325
```

```
<210> 46
<211> 579
<212> PRT
<213> Artificial Sequence

<220>
<223> Description of Artificial Sequence: Synthetic
      polypeptide

<400> 46
Gln Val Gln Leu Gln Gln Pro Gly Ala Glu Leu Val Lys Pro Gly Ala
1               5               10                      15

Ser Val Lys Met Ser Cys Lys Ala Ser Gly Tyr Thr Phe Thr Ser Tyr
            20              25                      30

Asn Met His Trp Val Lys Gln Thr Pro Gly Arg Gly Leu Glu Trp Ile
            35              40                      45

Gly Ala Ile Tyr Pro Gly Asn Gly Asp Thr Ser Tyr Asn Gln Lys Phe
    50              55              60

Lys Gly Lys Ala Thr Leu Thr Ala Asp Lys Ser Ser Ser Thr Ala Tyr
65              70              75                      80
```

```
Met Gln Leu Ser Ser Leu Thr Ser Glu Asp Ser Ala Val Tyr Tyr Cys
             85                  90                  95

Ala Arg Ser Thr Tyr Tyr Gly Gly Asp Trp Tyr Phe Asn Val Trp Gly
            100                 105                110

Ala Gly Thr Thr Val Thr Val Ser Ala Ala Ser Thr Lys Gly Pro Glu
            115                 120                 125

Val Gln Leu Leu Glu Ser Gly Gly Gly Leu Val Gln Pro Gly Gly Ser
    130                 135                 140

Leu Arg Leu Ser Cys Ala Ala Ser Gly Phe Thr Phe Ser Ser Tyr Ala
145                 150                 155                 160

Met Ser Trp Val Arg Gln Ala Pro Gly Lys Gly Leu Glu Trp Val Ser
            165                 170                 175

Gly Ile Thr Gly Ser Gly Gly Ser Thr Tyr Tyr Ala Asp Ser Val Lys
            180                 185                 190

Gly Arg Phe Thr Ile Ser Arg Asp Asn Ser Lys Asn Thr Leu Tyr Leu
            195                 200                 205

Gln Met Asn Ser Leu Arg Ala Glu Asp Thr Ala Val Tyr Tyr Cys Ala
    210                 215                 220

Lys Asp Pro Gly Thr Thr Val Ile Met Ser Trp Phe Asp Pro Trp Gly
225                 230                 235                 240

Gln Gly Thr Leu Val Thr Val Ser Ser Ala Ser Thr Lys Gly Pro Ser
            245                 250                 255

Val Phe Pro Leu Ala Pro Ser Ser Lys Ser Thr Ser Gly Gly Thr Ala
            260                 265                 270

Ala Leu Gly Cys Leu Val Lys Asp Tyr Phe Pro Glu Pro Val Thr Val
            275                 280                 285

Ser Trp Asn Ser Gly Ala Leu Thr Ser Gly Val His Thr Phe Pro Ala
    290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
            325                 330                 335
```

187

```
Lys Pro Ser Asn Thr Lys Val Asp Lys Lys Val Glu Pro Lys Ser Cys
        340                 345                 350

Asp Lys Thr His Thr Cys Pro Pro Cys Pro Ala Pro Glu Ala Ala Gly
        355                 360                 365

Gly Pro Ser Val Phe Leu Phe Pro Pro Lys Pro Lys Asp Thr Leu Met
        370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
        420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Ala Leu Pro Ala Pro Ile
    450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Glu Glu Met Thr Lys Asn Gln Val Ser
                485                 490                 495

Leu Thr Cys Leu Val Lys Gly Phe Tyr Pro Ser Asp Ile Ala Val Glu
        500                 505                 510

Trp Glu Ser Asn Gly Gln Pro Glu Asn Asn Tyr Lys Thr Thr Pro Pro
        515                 520                 525

Val Leu Asp Ser Asp Gly Ser Phe Phe Leu Tyr Ser Lys Leu Thr Val
        530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys
```

<210> 47
<211> 327
<212> PRT
<213> Artificial Sequence

<220>
<223> Description of Artificial Sequence: Synthetic
      polypeptide

<400> 47
Gln Ile Val Leu Ser Gln Ser Pro Ala Ile Leu Ser Pro Ser Pro Gly
1               5                   10                  15


Glu Lys Val Thr Met Thr Cys Arg Ala Ser Ser Ser Val Ser Tyr Ile
            20                  25                  30


His Trp Phe Gln Gln Lys Pro Gly Ser Ser Pro Lys Pro Trp Ile Tyr
            35                  40                  45


Ala Thr Ser Asn Leu Ala Ser Gly Val Pro Val Arg Phe Ser Gly Ser
        50                  55                  60


Gly Ser Gly Thr Ser Tyr Ser Leu Thr Ile Ser Arg Val Glu Ala Glu
65                  70                  75                  80


Asp Ala Ala Thr Tyr Tyr Cys Gln Gln Trp Thr Ser Asn Pro Pro Thr
                85                  90                  95


Phe Gly Gly Gly Thr Lys Leu Glu Ile Lys Arg Thr Val Ala Ala Pro
            100                 105                 110


Glu Ile Val Leu Thr Gln Ser Pro Gly Thr Leu Ser Leu Ser Pro Gly
            115                 120                 125


Glu Arg Ala Thr Leu Ser Cys Arg Ala Ser Gln Ser Val Arg Gly Arg
        130                 135                 140


Tyr Leu Ala Trp Tyr Gln Gln Lys Pro Gly Gln Ala Pro Arg Leu Leu
145                 150                 155                 160


Ile Tyr Gly Ala Ser Ser Arg Ala Thr Gly Ile Pro Asp Arg Phe Ser
                165                 170                 175


Gly Ser Gly Ser Gly Thr Asp Phe Thr Leu Thr Ile Ser Arg Leu Glu
                180                 185                 190


Pro Glu Asp Phe Ala Val Phe Tyr Cys Gln Gln Tyr Gly Ser Ser Pro
            195                 200                 205

```
Arg Thr Phe Gly Gln Gly Thr Lys Val Glu Ile Lys Arg Thr Val Ala
    210             215             220

Ala Pro Ser Val Phe Ile Phe Pro Pro Ser Asp Glu Gln Leu Lys Ser
225             230             235                 240

Gly Thr Ala Ser Val Val Cys Leu Leu Asn Asn Phe Tyr Pro Arg Glu
                245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
            275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315                 320

Ser Phe Asn Arg Gly Glu Cys
                325
```

```
<210> 48
<211> 579
<212> PRT
<213> Artificial Sequence

<220>
<223> Description of Artificial Sequence: Synthetic
      polypeptide

<400> 48
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Ser Tyr
            20              25              30

Ala Met Ser Trp Val Arg Gln Ala Pro Gly Lys Gly Leu Glu Trp Val
            35              40              45

Ser Gly Ile Thr Gly Ser Gly Gly Ser Thr Tyr Tyr Ala Asp Ser Val
    50              55              60

Lys Gly Arg Phe Thr Ile Ser Arg Asp Asn Ser Lys Asn Thr Leu Tyr
65              70              75              80
```

```
Leu Gln Met Asn Ser Leu Arg Ala Glu Asp Thr Ala Val Tyr Tyr Cys
                85                  90                  95

Ala Lys Asp Pro Gly Thr Thr Val Ile Met Ser Trp Phe Asp Pro Trp
                100                 105                 110

Gly Gln Gly Thr Leu Val Thr Val Ser Ser Ala Ser Thr Lys Gly Pro
                115                 120                 125

Gln Val Gln Leu Gln Gln Pro Gly Ala Glu Leu Val Lys Pro Gly Ala
        130                 135                 140

Ser Val Lys Met Ser Cys Lys Ala Ser Gly Tyr Thr Phe Thr Ser Tyr
145                 150                 155                 160

Asn Met His Trp Val Lys Gln Thr Pro Gly Arg Gly Leu Glu Trp Ile
                165                 170                 175

Gly Ala Ile Tyr Pro Gly Asn Gly Asp Thr Ser Tyr Asn Gln Lys Phe
                180                 185                 190

Lys Gly Lys Ala Thr Leu Thr Ala Asp Lys Ser Ser Ser Thr Ala Tyr
        195                 200                 205

Met Gln Leu Ser Ser Leu Thr Ser Glu Asp Ser Ala Val Tyr Tyr Cys
        210                 215                 220

Ala Arg Ser Thr Tyr Tyr Gly Gly Asp Trp Tyr Phe Asn Val Trp Gly
225                 230                 235                 240

Ala Gly Thr Thr Val Thr Val Ser Ala Ala Ser Thr Lys Gly Pro Ser
                245                 250                 255

Val Phe Pro Leu Ala Pro Ser Ser Lys Ser Thr Ser Gly Gly Thr Ala
                260                 265                 270

Ala Leu Gly Cys Leu Val Lys Asp Tyr Phe Pro Glu Pro Val Thr Val
        275                 280                 285

Ser Trp Asn Ser Gly Ala Leu Thr Ser Gly Val His Thr Phe Pro Ala
        290                 295                 300

Val Leu Gln Ser Ser Gly Leu Tyr Ser Leu Ser Ser Val Val Thr Val
305                 310                 315                 320

Pro Ser Ser Ser Leu Gly Thr Gln Thr Tyr Ile Cys Asn Val Asn His
                325                 330                 335
```

Lys Pro Ser Asn Thr Lys Val Asp Lys Lys Val Glu Pro Lys Ser Cys
        340                 345                 350

Asp Lys Thr His Thr Cys Pro Pro Cys Pro Ala Pro Glu Ala Ala Gly
        355                 360                 365

Gly Pro Ser Val Phe Leu Phe Pro Pro Lys Pro Lys Asp Thr Leu Met
        370                 375                 380

Ile Ser Arg Thr Pro Glu Val Thr Cys Val Val Val Asp Val Ser His
385                 390                 395                 400

Glu Asp Pro Glu Val Lys Phe Asn Trp Tyr Val Asp Gly Val Glu Val
                405                 410                 415

His Asn Ala Lys Thr Lys Pro Arg Glu Glu Gln Tyr Asn Ser Thr Tyr
                420                 425                 430

Arg Val Val Ser Val Leu Thr Val Leu His Gln Asp Trp Leu Asn Gly
        435                 440                 445

Lys Glu Tyr Lys Cys Lys Val Ser Asn Lys Ala Leu Pro Ala Pro Ile
        450                 455                 460

Glu Lys Thr Ile Ser Lys Ala Lys Gly Gln Pro Arg Glu Pro Gln Val
465                 470                 475                 480

Tyr Thr Leu Pro Pro Ser Arg Glu Glu Met Thr Lys Asn Gln Val Ser
                485                 490                 495

Leu Thr Cys Leu Val Lys Gly Phe Tyr Pro Ser Asp Ile Ala Val Glu
                500                 505                 510

Trp Glu Ser Asn Gly Gln Pro Glu Asn Asn Tyr Lys Thr Thr Pro Pro
                515                 520                 525

Val Leu Asp Ser Asp Gly Ser Phe Phe Leu Tyr Ser Lys Leu Thr Val
        530                 535                 540

Asp Lys Ser Arg Trp Gln Gln Gly Asn Val Phe Ser Cys Ser Val Met
545                 550                 555                 560

His Glu Ala Leu His Asn His Tyr Thr Gln Lys Ser Leu Ser Leu Ser
                565                 570                 575

Pro Gly Lys

<210> 49
<211> 327
<212> PRT
<213> Artificial Sequence

<220>
<223> Description of Artificial Sequence: Synthetic
     polypeptide

<400> 49

```
Glu Ile Val Leu Thr Gln Ser Pro Gly Thr Leu Ser Leu Ser Pro Gly
1               5                   10                  15


Glu Arg Ala Thr Leu Ser Cys Arg Ala Ser Gln Ser Val Arg Gly Arg
            20                  25                  30


Tyr Leu Ala Trp Tyr Gln Gln Lys Pro Gly Gln Ala Pro Arg Leu Leu
        35                  40                  45


Ile Tyr Gly Ala Ser Ser Arg Ala Thr Gly Ile Pro Asp Arg Phe Ser
    50                  55                  60


Gly Ser Gly Ser Gly Thr Asp Phe Thr Leu Thr Ile Ser Arg Leu Glu
65                  70                  75                  80


Pro Glu Asp Phe Ala Val Phe Tyr Cys Gln Gln Tyr Gly Ser Ser Pro
                85                  90                  95


Arg Thr Phe Gly Gln Gly Thr Lys Val Glu Ile Lys Arg Thr Val Ala
            100                 105                 110


Ala Pro Gln Ile Val Leu Ser Gln Ser Pro Ala Ile Leu Ser Pro Ser
        115                 120                 125


Pro Gly Glu Lys Val Thr Met Thr Cys Arg Ala Ser Ser Ser Val Ser
    130                 135                 140


Tyr Ile His Trp Phe Gln Gln Lys Pro Gly Ser Ser Pro Lys Pro Trp
145                 150                 155                 160


Ile Tyr Ala Thr Ser Asn Leu Ala Ser Gly Val Pro Val Arg Phe Ser
    165                 170                 175


Gly Ser Gly Ser Gly Thr Ser Tyr Ser Leu Thr Ile Ser Arg Val Glu
        180                 185                 190


Ala Glu Asp Ala Ala Thr Tyr Tyr Cys Gln Gln Trp Thr Ser Asn Pro
        195                 200                 205
```

```
Pro Thr Phe Gly Gly Gly Thr Lys Leu Glu Ile Lys Arg Thr Val Ala
    210             215             220

Ala Pro Ser Val Phe Ile Phe Pro Pro Ser Asp Glu Gln Leu Lys Ser
225             230             235                 240

Gly Thr Ala Ser Val Val Cys Leu Leu Asn Asn Phe Tyr Pro Arg Glu
                245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
            275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315                 320

Ser Phe Asn Arg Gly Glu Cys
            325
```

```
<210> 50
<211> 240
<212> PRT
<213> Artificial Sequence

<220>
<223> Description of Artificial Sequence: Synthetic
      polypeptide

<400> 50
Glu Val Gln Leu Val Glu Ser Gly Gly Gly Leu Val Gln Pro Gly Gly
1               5               10              15

Ser Leu Arg Leu Ser Cys Ala Ala Ser Gly Phe Thr Phe Thr Asp Asn
            20              25              30

Trp Ile Ser Trp Val Arg Gln Ala Pro Gly Lys Gly Leu Glu Trp Val
            35              40              45

Gly Tyr Ile Ser Pro Asn Ser Gly Phe Thr Tyr Tyr Ala Asp Ser Val
    50              55              60

Lys Gly Arg Phe Thr Ile Ser Ala Asp Thr Ser Lys Asn Thr Ala Tyr
65              70              75              80
```

```
Leu Gln Met Asn Ser Leu Arg Ala Glu Asp Thr Ala Val Tyr Tyr Cys
                85                  90                  95

Ala Arg Asp Asn Phe Gly Gly Tyr Phe Asp Tyr Trp Gly Gln Gly Thr
            100                 105                 110

Leu Val Thr Val Ser Ser Ala Ser Thr Lys Gly Pro Gln Val Gln Leu
        115                 120                 125

Gln Gln Ser Gly Ala Glu Leu Val Lys Pro Gly Ala Ser Val Lys Ile
    130                 135                 140

Ser Cys Lys Ala Ser Gly Tyr Thr Phe Thr Asp Tyr Tyr Ile Asn Trp
145                 150                 155                 160

Val Lys Leu Ala Pro Gly Gln Gly Leu Glu Trp Ile Gly Trp Ile Tyr
            165                 170                 175

Pro Gly Ser Gly Asn Thr Lys Tyr Asn Glu Lys Phe Lys Gly Lys Ala
            180                 185                 190

Thr Leu Thr Ile Asp Thr Ser Ser Ser Thr Ala Tyr Met Gln Leu Ser
        195                 200                 205

Ser Leu Thr Ser Glu Asp Thr Ala Val Tyr Phe Cys Val Arg Asp Ser
    210                 215                 220

Pro Phe Phe Asp Tyr Trp Gly Gln Gly Thr Leu Leu Thr Val Ser Ser
225                 230                 235                 240
```

```
<210> 51
<211> 227
<212> PRT
<213> Artificial Sequence

<220>
<223> Description of Artificial Sequence: Synthetic
      polypeptide

<400> 51
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
```

```
                    50                    55                        60


        Ser Gly Ser Gly Thr Asp Phe Thr Leu Thr Ile Ser Ser Leu Gln Pro
        65                  70                  75                      80


        Glu Asp Phe Ala Thr Thr Tyr Tyr Cys Gln Gln Ser Tyr Thr Gly Thr
                        85                  90                  95


        Val Thr Phe Gly Gln Gly Thr Lys Val Glu Ile Lys Arg Thr Val Ala
                    100                 105                 110


        Ala Pro Asp Ile Val Leu Thr Gln Ser Pro Asp Ser Leu Ala Val Ser
                115                 120                 125


        Leu Gly Glu Arg Val Thr Met Asn Cys Lys Ser Ser Gln Ser Leu Leu
                130                 135                 140


        Asn Ser Gly Met Arg Lys Ser Phe Leu Ala Trp Tyr Gln Gln Lys Pro
        145                 150                 155                     160


        Gly Gln Ser Pro Lys Leu Leu Ile Tyr Trp Ala Ser Thr Arg Glu Ser
                    165                 170                 175


        Gly Val Pro Asp Arg Phe Thr Gly Ser Gly Ser Gly Thr Asp Phe Thr
                    180                 185                 190


        Leu Thr Ile Ser Ser Val Gln Ala Glu Asp Val Ala Val Tyr Tyr Cys
                195                 200                 205


        Lys Gln Ser Tyr His Leu Phe Thr Phe Gly Ser Gly Thr Lys Leu Glu
                210                 215                 220


        Ile Lys Arg
        225


        <210> 52
        <211> 240
        <212> PRT
        <213> Artificial Sequence

        <220>
        <223> Description of Artificial Sequence: Synthetic
              polypeptide

        <400> 52
        Gln Val Gln Leu Gln Gln Ser Gly Ala Glu Leu Val Lys Pro Gly Ala
        1               5                   10                  15


        Ser Val Lys Ile Ser Cys Lys Ala Ser Gly Tyr Thr Phe Thr Asp Tyr
                    20                  25                  30
```

```
Tyr Ile Asn Trp Val Lys Leu Ala Pro Gly Gln Gly Leu Glu Trp Ile
        35              40                  45

Gly Trp Ile Tyr Pro Gly Ser Gly Asn Thr Lys Tyr Asn Glu Lys Phe
        50              55                  60

Lys Gly Lys Ala Thr Leu Thr Ile Asp Thr Ser Ser Ser Thr Ala Tyr
65              70              75                  80

Met Gln Leu Ser Ser Leu Thr Ser Glu Asp Thr Ala Val Tyr Phe Cys
                85              90                  95

Val Arg Asp Ser Pro Phe Phe Asp Tyr Trp Gly Gln Gly Thr Leu Leu
            100             105             110

Thr Val Ser Ser Ala Ser Thr Lys Gly Pro Glu Val Gln Leu Val Glu
        115             120             125

Ser Gly Gly Gly Leu Val Gln Pro Gly Gly Ser Leu Arg Leu Ser Cys
    130             135             140

Ala Ala Ser Gly Phe Thr Phe Thr Asp Asn Trp Ile Ser Trp Val Arg
145             150             155             160

Gln Ala Pro Gly Lys Gly Leu Glu Trp Val Gly Tyr Ile Ser Pro Asn
            165             170             175

Ser Gly Phe Thr Tyr Tyr Ala Asp Ser Val Lys Gly Arg Phe Thr Ile
        180             185             190

Ser Ala Asp Thr Ser Lys Asn Thr Ala Tyr Leu Gln Met Asn Ser Leu
        195             200             205

Arg Ala Glu Asp Thr Ala Val Tyr Tyr Cys Ala Arg Asp Asn Phe Gly
    210             215             220

Gly Tyr Phe Asp Tyr Trp Gly Gln Gly Thr Leu Val Thr Val Ser Ser
225             230             235             240
```

<210> 53
<211> 227
<212> PRT
<213> Artificial Sequence

<220>
<223> Description of Artificial Sequence: Synthetic
      polypeptide

197

<400> 53

Asp Ile Val Leu Thr Gln Ser Pro Asp Ser Leu Ala Val Ser Leu Gly
1               5                   10                  15

Glu Arg Val Thr Met Asn Cys Lys Ser Ser Gln Ser Leu Leu Asn Ser
            20                  25                  30

Gly Met Arg Lys Ser Phe Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Val Ala Val Tyr Tyr Cys Lys Gln
                85                  90                  95

Ser Tyr His Leu Phe Thr Phe Gly Ser Gly Thr Lys Leu Glu Ile Lys
            100                 105                 110

Arg Thr Val Ala Ala Pro Asp Ile Gln Met Thr Gln Ser Pro Ser Ser
        115                 120                 125

Leu Ser Ala Ser Val Gly Asp Arg Val Thr Ile Thr Cys Arg Ala Ser
    130                 135                 140

Gln Asp Val Ser Thr Ala Val Ala Trp Tyr Gln Gln Lys Pro Gly Lys
145                 150                 155                 160

Ala Pro Lys Leu Leu Ile Tyr Ser Ala Ser Phe Leu Tyr Ser Gly Val
            165                 170                 175

Pro Ser Arg Phe Ser Gly Ser Gly Ser Gly Thr Asp Phe Thr Leu Thr
        180                 185                 190

Ile Ser Ser Leu Gln Pro Glu Asp Phe Ala Thr Thr Tyr Tyr Cys Gln
    195                 200                 205

Gln Ser Tyr Thr Gly Thr Val Thr Phe Gly Gln Gly Thr Lys Val Glu
    210                 215                 220

Ile Lys Arg
225

<210> 54
<211> 580

```
<212> PRT
<213> Artificial Sequence

<220>
<223> Description of Artificial Sequence: Synthetic
      polypeptide

<400> 54
Glu Val Gln Leu Val Glu Ser Gly Gly Gly Leu Val Gln Pro Gly Arg
1               5                   10                  15


Ser Leu Arg Leu Ser Cys Ala Ala Ser Gly Phe Thr Phe Asp Asp Tyr
            20                  25                  30


Ala Met His Trp Val Arg Gln Ala Pro Gly Lys Gly Leu Glu Trp Val
            35                  40                  45


Ser Ala Ile Thr Trp Asn Ser Gly His Ile Asp Tyr Ala Asp Ser Val
        50                  55                  60


Glu Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95


Ala Lys Val Ser Tyr Leu Ser Thr Ala Ser Ser Leu Asp Tyr Trp Gly
            100                 105                 110


Gln Gly Thr Leu Val Thr Val Ser Ser Ala Ser Thr Lys Gly Pro Glu
            115                 120                 125


Val Gln Leu Gln Gln Ser Gly Pro Glu Leu Met Lys Pro Gly Ala Ser
    130                 135                 140


Val Lys Met Ser Cys Lys Ala Ser Gly Tyr Thr Phe Thr Asp Tyr Asn
145                 150                 155                 160


Met His Trp Met Lys Gln Asn Gln Gly Lys Ser Leu Glu Trp Ile Gly
                165                 170                 175


Glu Ile Asn Pro Asn Ser Gly Gly Ser Gly Tyr Asn Gln Lys Phe Lys
            180                 185                 190


Gly Lys Ala Thr Leu Thr Val Asp Lys Ser Ser Ser Thr Ala Tyr Met
            195                 200                 205


Glu Leu Arg Ser Leu Thr Ser Glu Asp Ser Ala Val Tyr Tyr Cys Ala
            210                 215                 220
```

199

```
Arg Leu Gly Tyr Tyr Gly Asn Tyr Glu Asp Trp Tyr Phe Asp Val Trp
225                 230             235             240

Gly Ala Gly Thr Thr Val Thr Val Ser Ser Ala Ser Thr Lys Gly Pro
            245             250             255

Ser Val Phe Pro Leu Ala Pro Ser Ser Lys Ser Thr Ser Gly Gly Thr
        260             265             270

Ala Ala Leu Gly Cys Leu Val Lys Asp Tyr Phe Pro Glu Pro Val Thr
        275             280             285

Val Ser Trp Asn Ser Gly Ala Leu Thr Ser Gly Val His Thr Phe Pro
    290             295             300

Ala Val Leu Gln Ser Ser Gly Leu Tyr Ser Leu Ser Ser Val Val Thr
305             310             315             320

Val Pro Ser Ser Ser Leu Gly Thr Gln Thr Tyr Ile Cys Asn Val Asn
            325             330             335

His Lys Pro Ser Asn Thr Lys Val Asp Lys Lys Val Glu Pro Lys Ser
            340             345             350

Cys Asp Lys Thr His Thr Cys Pro Pro Cys Pro Ala Pro Glu Ala Ala
        355             360             365

Gly Gly Pro Ser Val Phe Leu Phe Pro Pro Lys Pro Lys Asp Thr Leu
    370             375             380

Met Ile Ser Arg Thr Pro Glu Val Thr Cys Val Val Val Asp Val Ser
385             390             395             400

His Glu Asp Pro Glu Val Lys Phe Asn Trp Tyr Val Asp Gly Val Glu
            405             410             415

Val His Asn Ala Lys Thr Lys Pro Arg Glu Glu Gln Tyr Asn Ser Thr
        420             425             430

Tyr Arg Val Val Ser Val Leu Thr Val Leu His Gln Asp Trp Leu Asn
        435             440             445

Gly Lys Glu Tyr Lys Cys Lys Val Ser Asn Lys Ala Leu Pro Ala Pro
    450             455             460

Ile Glu Lys Thr Ile Ser Lys Ala Lys Gly Gln Pro Arg Glu Pro Gln
465             470             475             480
```

200

```
Val Tyr Thr Leu Pro Pro Ser Arg Glu Glu Met Thr Lys Asn Gln Val
                485             490             495

Ser Leu Thr Cys Leu Val Lys Gly Phe Tyr Pro Ser Asp Ile Ala Val
                500             505             510

Glu Trp Glu Ser Asn Gly Gln Pro Glu Asn Asn Tyr Lys Thr Thr Pro
        515             520             525

Pro Val Leu Asp Ser Asp Gly Ser Phe Phe Leu Tyr Ser Lys Leu Thr
    530             535             540

Val Asp Lys Ser Arg Trp Gln Gln Gly Asn Val Phe Ser Cys Ser Val
545             550             555             560

Met His Glu Ala Leu His Asn His Tyr Thr Gln Lys Ser Leu Ser Leu
                565             570             575

Ser Pro Gly Lys
                580
```

```
<210> 55
<211> 327
<212> PRT
<213> Artificial Sequence

<220>
<223> Description of Artificial Sequence: Synthetic
      polypeptide

<400> 55
Asp Ile Gln Met Thr Gln Ser Pro Ser Ser Leu Ser Ala Ser Val Gly
1               5               10              15

Asp Arg Val Thr Ile Thr Cys Arg Ala Ser Gln Gly Ile Arg Asn Tyr
                20              25              30

Leu Ala Trp Tyr Gln Gln Lys Pro Gly Lys Ala Pro Lys Leu Leu Ile
            35              40              45

Tyr Ala Ala Ser Thr Leu Gln Ser Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Val Ala Thr Tyr Tyr Cys Gln Arg Tyr Asn Arg Ala Pro Tyr
                85              90              95
```

```
Thr Phe Gly Gln Gly Thr Lys Val Glu Ile Lys Arg Thr Val Ala Ala
        100             105             110

Pro Asp Leu Gln Met Thr Gln Thr Thr Ser Ser Leu Ser Ala Ser Leu
        115             120             125

Gly Asp Arg Val Thr Ile Ser Cys Arg Ala Ser Gln Asp Ile Ser Asn
        130             135             140

Tyr Leu Asn Trp Tyr Gln Gln Lys Pro Asp Gly Thr Val Lys Leu Leu
145             150             155             160

Ile Phe Tyr Thr Ser Thr Leu Gln Ser Gly Val Pro Ser Arg Phe Ser
                165             170             175

Gly Ser Gly Ser Gly Thr Asn Tyr Ser Leu Thr Ile Thr Asn Leu Glu
            180             185             190

Gln Asp Asp Ala Ala Thr Tyr Phe Cys Gln Gln Gly Asp Thr Leu Pro
        195             200             205

Tyr Thr Phe Gly Gly Gly Thr Lys Leu Glu Ile Lys Arg Thr Val Ala
    210             215             220

Ala Pro Ser Val Phe Ile Phe Pro Pro Ser Asp Glu Gln Leu Lys Ser
225             230             235             240

Gly Thr Ala Ser Val Val Cys Leu Leu Asn Asn Phe Tyr Pro Arg Glu
                245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
        275             280             285

Ser Ser Thr Leu Thr Leu Ser Lys Ala Asp Tyr Glu Lys His Lys Val
290             295             300

Tyr Ala Cys Glu Val Thr His Gln Gly Leu Ser Ser Pro Val Thr Lys
305             310             315             320

Ser Phe Asn Arg Gly Glu Cys
            325
```

<210> 56
<211> 580

<212> PRT
<213> Artificial Sequence

<220>
<223> Description of Artificial Sequence: Synthetic
      polypeptide

<400> 56
Glu Val Gln Leu Gln Gln Ser Gly Pro Glu Leu Met Lys Pro Gly Ala
1               5                   10                  15

Ser Val Lys Met Ser Cys Lys Ala Ser Gly Tyr Thr Phe Thr Asp Tyr
            20                  25                  30

Asn Met His Trp Met Lys Gln Asn Gln Gly Lys Ser Leu Glu Trp Ile
        35                  40                  45

Gly Glu Ile Asn Pro Asn Ser Gly Gly Ser Gly Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Ala Arg Leu Gly Tyr Tyr Gly Asn Tyr Glu Asp Trp Tyr Phe Asp Val
            100                 105                 110

Trp Gly Ala Gly Thr Thr Val Thr Val Ser Ser Ala Ser Thr Lys Gly
        115                 120                 125

Pro Glu Val Gln Leu Val Glu Ser Gly Gly Gly Leu Val Gln Pro Gly
    130                 135                 140

Arg Ser Leu Arg Leu Ser Cys Ala Ala Ser Gly Phe Thr Phe Asp Asp
145                 150                 155                 160

Tyr Ala Met His Trp Val Arg Gln Ala Pro Gly Lys Gly Leu Glu Trp
                165                 170                 175

Val Ser Ala Ile Thr Trp Asn Ser Gly His Ile Asp Tyr Ala Asp Ser
            180                 185                 190

Val Glu Gly Arg Phe Thr Ile Ser Arg Asp Asn Ala Lys Asn Ser Leu
        195                 200                 205

Tyr Leu Gln Met Asn Ser Leu Arg Ala Glu Asp Thr Ala Val Tyr Tyr
    210                 215                 220

```
Cys Ala Lys Val Ser Tyr Leu Ser Thr Ala Ser Ser Leu Asp Tyr Trp
225             230             235             240

Gly Gln Gly Thr Leu Val Thr Val Ser Ser Ala Ser Thr Lys Gly Pro
                245             250             255

Ser Val Phe Pro Leu Ala Pro Ser Ser Lys Ser Thr Ser Gly Gly Thr
            260             265             270

Ala Ala Leu Gly Cys Leu Val Lys Asp Tyr Phe Pro Glu Pro Val Thr
        275             280             285

Val Ser Trp Asn Ser Gly Ala Leu Thr Ser Gly Val His Thr Phe Pro
    290             295             300

Ala Val Leu Gln Ser Ser Gly Leu Tyr Ser Leu Ser Ser Val Val Thr
305             310             315             320

Val Pro Ser Ser Ser Leu Gly Thr Gln Thr Tyr Ile Cys Asn Val Asn
            325             330             335

His Lys Pro Ser Asn Thr Lys Val Asp Lys Lys Val Glu Pro Lys Ser
            340             345             350

Cys Asp Lys Thr His Thr Cys Pro Pro Cys Pro Ala Pro Glu Ala Ala
        355             360             365

Gly Gly Pro Ser Val Phe Leu Phe Pro Pro Lys Pro Lys Asp Thr Leu
    370             375             380

Met Ile Ser Arg Thr Pro Glu Val Thr Cys Val Val Val Asp Val Ser
385             390             395             400

His Glu Asp Pro Glu Val Lys Phe Asn Trp Tyr Val Asp Gly Val Glu
            405             410             415

Val His Asn Ala Lys Thr Lys Pro Arg Glu Glu Gln Tyr Asn Ser Thr
        420             425             430

Tyr Arg Val Val Ser Val Leu Thr Val Leu His Gln Asp Trp Leu Asn
    435             440             445

Gly Lys Glu Tyr Lys Cys Lys Val Ser Asn Lys Ala Leu Pro Ala Pro
    450             455             460

Ile Glu Lys Thr Ile Ser Lys Ala Lys Gly Gln Pro Arg Glu Pro Gln
465             470             475             480
```

```
        Val Tyr Thr Leu Pro Pro Ser Arg Glu Glu Met Thr Lys Asn Gln Val
                    485             490             495

        Ser Leu Thr Cys Leu Val Lys Gly Phe Tyr Pro Ser Asp Ile Ala Val
                    500             505             510

        Glu Trp Glu Ser Asn Gly Gln Pro Glu Asn Asn Tyr Lys Thr Thr Pro
                    515             520             525

        Pro Val Leu Asp Ser Asp Gly Ser Phe Phe Leu Tyr Ser Lys Leu Thr
            530             535             540

        Val Asp Lys Ser Arg Trp Gln Gln Gly Asn Val Phe Ser Cys Ser Val
        545             550             555             560

        Met His Glu Ala Leu His Asn His Tyr Thr Gln Lys Ser Leu Ser Leu
                    565             570             575

        Ser Pro Gly Lys
                    580


<210> 57
<211> 327
<212> PRT
<213> Artificial Sequence

<220>
<223> Description of Artificial Sequence: Synthetic
      polypeptide

<400> 57
Asp Leu Gln Met Thr Gln Thr Thr Ser Ser Leu Ser Ala Ser Leu Gly
1               5               10              15

Asp Arg Val Thr Ile Ser Cys Arg Ala Ser Gln Asp Ile Ser Asn Tyr
            20              25              30

Leu Asn Trp Tyr Gln Gln Lys Pro Asp Gly Thr Val Lys Leu Leu Ile
            35              40              45

Phe Tyr Thr Ser Thr Leu Gln Ser Gly Val Pro Ser Arg Phe Ser Gly
        50              55              60

Ser Gly Ser Gly Thr Asn Tyr Ser Leu Thr Ile Thr Asn Leu Glu Gln
65              70              75              80

Asp Asp Ala Ala Thr Tyr Phe Cys Gln Gln Gly Asp Thr Leu Pro Tyr
                85              90              95
```

```
Thr Phe Gly Gly Gly Thr Lys Leu Glu Ile Lys Arg Thr Val Ala Ala
            100                 105                 110

Pro Asp Ile Gln Met Thr Gln Ser Pro Ser Ser Leu Ser Ala Ser Val
            115                 120                 125

Gly Asp Arg Val Thr Ile Thr Cys Arg Ala Ser Gln Gly Ile Arg Asn
            130                 135                 140

Tyr Leu Ala Trp Tyr Gln Gln Lys Pro Gly Lys Ala Pro Lys Leu Leu
145                 150                 155                 160

Ile Tyr Ala Ala Ser Thr Leu Gln Ser Gly Val Pro Ser Arg Phe Ser
                165                 170                 175

Gly Ser Gly Ser Gly Thr Asp Phe Thr Leu Thr Ile Ser Ser Leu Gln
            180                 185                 190

Pro Glu Asp Val Ala Thr Tyr Tyr Cys Gln Arg Tyr Asn Arg Ala Pro
            195                 200                 205

Tyr Thr Phe Gly Gln Gly Thr Lys Val Glu Ile Lys Arg Thr Val Ala
    210                 215                 220

Ala Pro Ser Val Phe Ile Phe Pro Pro Ser Asp Glu Gln Leu Lys Ser
225                 230                 235                 240

Gly Thr Ala Ser Val Val Cys Leu Leu Asn Asn Phe Tyr Pro Arg Glu
                245                 250                 255

Ala Lys Val Gln Trp Lys Val Asp Asn Ala Leu Gln Ser Gly Asn Ser
            260                 265                 270

Gln Glu Ser Val Thr Glu Gln Asp Ser Lys Asp Ser Thr Tyr Ser Leu
            275                 280                 285

Ser Ser Thr Leu Thr Leu Ser Lys Ala Asp Tyr Glu Lys His Lys Val
290                 295                 300

Tyr Ala Cys Glu Val Thr His Gln Gly Leu Ser Ser Pro Val Thr Lys
305                 310                 315                 320

Ser Phe Asn Arg Gly Glu Cys
                325
```

```
<210> 58
<211> 577
```

```
<212> PRT
<213> Artificial Sequence

<220>
<223> Description of Artificial Sequence: Synthetic
      polypeptide

<400> 58
Gln Val Gln Leu Gln Gln Ser Gly Ala Glu Leu Met Lys Pro Gly Ala
1               5                   10                  15


Ser Val Lys Leu Ser Cys Lys Ala Thr Gly Tyr Thr Phe Thr Gly Ser
            20                  25                  30


Trp Ile Glu Trp Ile Lys Gln Arg Pro Gly His Gly Leu Glu Trp Ile
            35                  40                  45


Gly Gln Ile Leu Pro Gly Ser Gly Ser Ala Tyr Tyr Asn Glu Lys Phe
        50                  55                  60


Lys Gly Lys Ala Thr Phe Thr Ala Asp Thr Ser Ser Lys Thr Val Tyr
65                  70                  75                  80


Ile Gln Leu Ile Ser Leu Thr Thr Glu Asp Ser Ala Ile Tyr Tyr Cys
                85                  90                  95


Ala Arg Glu Asp Asn Tyr Gly Ser Ser Ser Leu Ala Tyr Trp Gly Gln
            100                 105                 110


Gly Thr Leu Leu Thr Val Ser Ala Ala Ser Thr Lys Gly Pro Glu Val
            115                 120                 125


Gln Leu Val Glu Ser Gly Gly Gly Leu Val Gln Pro Gly Gly Ser Leu
        130                 135                 140


Arg Leu Ser Cys Ala Val Ser Gly Tyr Ser Ile Thr Ser Gly Tyr Ser
145                 150                 155                 160


Trp Asn Trp Ile Arg Gln Ala Pro Gly Lys Gly Leu Glu Trp Val Ala
                165                 170                 175


Ser Ile Thr Tyr Asp Gly Ser Thr Asn Tyr Asn Pro Ser Val Lys Gly
            180                 185                 190


Arg Ile Thr Ile Ser Arg Asp Asp Ser Lys Asn Thr Phe Tyr Leu Gln
            195                 200                 205


Met Asn Ser Leu Arg Ala Glu Asp Thr Ala Val Tyr Tyr Cys Ala Arg
        210                 215                 220
```

```
Gly Ser His Tyr Phe Gly His Trp His Phe Ala Val Trp Gly Gln Gly
225             230             235             240

Thr Leu Val Thr Val Ser Ser Ala Ser Thr Lys Gly Pro Ser Val Phe
                245             250             255

Pro Leu Ala Pro Ser Ser Lys Ser Thr Ser Gly Gly Thr Ala Ala Leu
            260             265             270

Gly Cys Leu Val Lys Asp Tyr Phe Pro Glu Pro Val Thr Val Ser Trp
        275             280             285

Asn Ser Gly Ala Leu Thr Ser Gly Val His Thr Phe Pro Ala Val Leu
    290             295             300

Gln Ser Ser Gly Leu Tyr Ser Leu Ser Ser Val Val Thr Val Pro Ser
305             310             315             320

Ser Ser Leu Gly Thr Gln Thr Tyr Ile Cys Asn Val Asn His Lys Pro
            325             330             335

Ser Asn Thr Lys Val Asp Lys Lys Val Glu Pro Lys Ser Cys Asp Lys
        340             345             350

Thr His Thr Cys Pro Pro Cys Pro Ala Pro Glu Ala Ala Gly Gly Pro
    355             360             365

Ser Val Phe Leu Phe Pro Pro Lys Pro Lys Asp Thr Leu Met Ile Ser
    370             375             380

Arg Thr Pro Glu Val Thr Cys Val Val Val Asp Val Ser His Glu Asp
385             390             395             400

Pro Glu Val Lys Phe Asn Trp Tyr Val Asp Gly Val Glu Val His Asn
            405             410             415

Ala Lys Thr Lys Pro Arg Glu Glu Gln Tyr Asn Ser Thr Tyr Arg Val
        420             425             430

Val Ser Val Leu Thr Val Leu His Gln Asp Trp Leu Asn Gly Lys Glu
    435             440             445

Tyr Lys Cys Lys Val Ser Asn Lys Ala Leu Pro Ala Pro Ile Glu Lys
    450             455             460

Thr Ile Ser Lys Ala Lys Gly Gln Pro Arg Glu Pro Gln Val Tyr Thr
465             470             475             480
```

```
Leu Pro Pro Ser Arg Glu Glu Met Thr Lys Asn Gln Val Ser Leu Thr
                485             490                 495

Cys Leu Val Lys Gly Phe Tyr Pro Ser Asp Ile Ala Val Glu Trp Glu
            500             505             510

Ser Asn Gly Gln Pro Glu Asn Asn Tyr Lys Thr Thr Pro Pro Val Leu
            515             520             525

Asp Ser Asp Gly Ser Phe Phe Leu Tyr Ser Lys Leu Thr Val Asp Lys
        530             535             540

Ser Arg Trp Gln Gln Gly Asn Val Phe Ser Cys Ser Val Met His Glu
545             550             555                     560

Ala Leu His Asn His Tyr Thr Gln Lys Ser Leu Ser Leu Ser Pro Gly
                565                 570                 575

Lys
```

```
<210> 59
<211> 331
<212> PRT
<213> Artificial Sequence

<220>
<223> Description of Artificial Sequence: Synthetic
      polypeptide

<400> 59
Asp Ile Leu Leu Thr Gln Ser Pro Ala Ile Leu Ser Val Ser Pro Gly
1               5               10              15

Glu Arg Val Ser Phe Ser Cys Arg Ala Ser Gln Ser Ile Gly Thr Asn
            20              25              30

Ile His Trp Tyr Gln Gln Arg Thr Asn Gly Ser Pro Arg Leu Leu Ile
            35              40              45

Lys Tyr Ala Ser Glu Ser Ile Ser Gly Ile Pro Ser Arg Phe Ser Gly
        50              55              60

Gly Gly Ser Gly Thr Asp Phe Thr Leu Ser Ile Asn Ser Val Glu Ser
65              70              75              80

Glu Asp Ile Ala Asp Tyr Tyr Cys Gln Gln Ser Asn Asn Trp Pro Leu
            85              90              95
```

```
Thr Phe Gly Ala Gly Thr Lys Leu Glu Leu Lys Arg Thr Val Ala Ala
        100             105             110

Pro Asp Ile Gln Leu Thr Gln Ser Pro Ser Ser Leu Ser Ala Ser Val
        115             120             125

Gly Asp Arg Val Thr Ile Thr Cys Arg Ala Ser Gln Ser Val Asp Tyr
        130             135             140

Asp Gly Asp Ser Tyr Met Asn Trp Tyr Gln Gln Lys Pro Gly Lys Ala
145             150             155             160

Pro Lys Leu Leu Ile Tyr Ala Ala Ser Tyr Leu Glu Ser Gly Val Pro
                165             170             175

Ser Arg Phe Ser Gly Ser Gly Ser Gly Thr Asp Phe Thr Leu Thr Ile
        180             185             190

Ser Ser Leu Gln Pro Glu Asp Phe Ala Thr Tyr Tyr Cys Gln Gln Ser
        195             200             205

His Glu Asp Pro Tyr Thr Phe Gly Gln Gly Thr Lys Val Glu Ile Lys
        210             215             220

Arg Thr Val Ala Ala Pro Ser Val Phe Ile Phe Pro Pro Ser Asp Glu
225             230             235             240

Gln Leu Lys Ser Gly Thr Ala Ser Val Val Cys Leu Leu Asn Asn Phe
                245             250             255

Tyr Pro Arg Glu Ala Lys Val Gln Trp Lys Val Asp Asn Ala Leu Gln
                260             265             270

Ser Gly Asn Ser Gln Glu Ser Val Thr Glu Gln Asp Ser Lys Asp Ser
                275             280             285

Thr Tyr Ser Leu Ser Ser Thr Leu Thr Leu Ser Lys Ala Asp Tyr Glu
        290             295             300

Lys His Lys Val Tyr Ala Cys Glu Val Thr His Gln Gly Leu Ser Ser
305             310             315             320

Pro Val Thr Lys Ser Phe Asn Arg Gly Glu Cys
                325             330
```

<210> 60
<211> 577

<212> PRT
<213> Artificial Sequence

<220>
<223> Description of Artificial Sequence: Synthetic
     polypeptide

<400> 60
Glu Val Gln Leu Val Glu Ser Gly Gly Gly Leu Val Gln Pro Gly Gly
1               5                   10                  15

Ser Leu Arg Leu Ser Cys Ala Val Ser Gly Tyr Ser Ile Thr Ser Gly
            20                  25                  30

Tyr Ser Trp Asn Trp Ile Arg Gln Ala Pro Gly Lys Gly Leu Glu Trp
        35                  40                  45

Val Ala Ser Ile Thr Tyr Asp Gly Ser Thr Asn Tyr Asn Pro Ser Val
    50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
            100                 105                 110

Gln Gly Thr Leu Val Thr Val Ser Ser Ala Ser Thr Lys Gly Pro Gln
            115                 120                 125

Val Gln Leu Gln Gln Ser Gly Ala Glu Leu Met Lys Pro Gly Ala Ser
    130                 135                 140

Val Lys Leu Ser Cys Lys Ala Thr Gly Tyr Thr Phe Thr Gly Ser Trp
145                 150                 155                 160

Ile Glu Trp Ile Lys Gln Arg Pro Gly His Gly Leu Glu Trp Ile Gly
                165                 170                 175

Gln Ile Leu Pro Gly Ser Gly Ser Ala Tyr Tyr Asn Glu Lys Phe Lys
            180                 185                 190

Gly Lys Ala Thr Phe Thr Ala Asp Thr Ser Ser Lys Thr Val Tyr Ile
        195                 200                 205

Gln Leu Ile Ser Leu Thr Thr Glu Asp Ser Ala Ile Tyr Tyr Cys Ala
    210                 215                 220

```
Arg Glu Asp Asn Tyr Gly Ser Ser Ser Leu Ala Tyr Trp Gly Gln Gly
225             230             235             240

Thr Leu Leu Thr Val Ser Ala Ala Ser Thr Lys Gly Pro Ser Val Phe
                245             250             255

Pro Leu Ala Pro Ser Ser Lys Ser Thr Ser Gly Gly Thr Ala Ala Leu
                260             265             270

Gly Cys Leu Val Lys Asp Tyr Phe Pro Glu Pro Val Thr Val Ser Trp
                275             280             285

Asn Ser Gly Ala Leu Thr Ser Gly Val His Thr Phe Pro Ala Val Leu
                290             295             300

Gln Ser Ser Gly Leu Tyr Ser Leu Ser Ser Val Val Thr Val Pro Ser
305             310             315             320

Ser Ser Leu Gly Thr Gln Thr Tyr Ile Cys Asn Val Asn His Lys Pro
                325             330             335

Ser Asn Thr Lys Val Asp Lys Lys Val Glu Pro Lys Ser Cys Asp Lys
                340             345             350

Thr His Thr Cys Pro Pro Cys Pro Ala Pro Glu Ala Ala Gly Gly Pro
                355             360             365

Ser Val Phe Leu Phe Pro Pro Lys Pro Lys Asp Thr Leu Met Ile Ser
        370             375             380

Arg Thr Pro Glu Val Thr Cys Val Val Val Asp Val Ser His Glu Asp
385             390             395             400

Pro Glu Val Lys Phe Asn Trp Tyr Val Asp Gly Val Glu Val His Asn
                405             410             415

Ala Lys Thr Lys Pro Arg Glu Glu Gln Tyr Asn Ser Thr Tyr Arg Val
                420             425             430

Val Ser Val Leu Thr Val Leu His Gln Asp Trp Leu Asn Gly Lys Glu
                435             440             445

Tyr Lys Cys Lys Val Ser Asn Lys Ala Leu Pro Ala Pro Ile Glu Lys
        450             455             460

Thr Ile Ser Lys Ala Lys Gly Gln Pro Arg Glu Pro Gln Val Tyr Thr
465             470             475             480
```

```
Leu Pro Pro Ser Arg Glu Glu Met Thr Lys Asn Gln Val Ser Leu Thr
            485                 490                 495

Cys Leu Val Lys Gly Phe Tyr Pro Ser Asp Ile Ala Val Glu Trp Glu
            500                 505                 510

Ser Asn Gly Gln Pro Glu Asn Asn Tyr Lys Thr Thr Pro Pro Val Leu
            515                 520                 525

Asp Ser Asp Gly Ser Phe Phe Leu Tyr Ser Lys Leu Thr Val Asp Lys
        530                 535                 540

Ser Arg Trp Gln Gln Gly Asn Val Phe Ser Cys Ser Val Met His Glu
545                 550                 555                 560

Ala Leu His Asn His Tyr Thr Gln Lys Ser Leu Ser Leu Ser Pro Gly
                565                 570                 575

Lys
```

```
<210> 61
<211> 331
<212> PRT
<213> Artificial Sequence

<220>
<223> Description of Artificial Sequence: Synthetic
      polypeptide

<400> 61
Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Val Asp Tyr Asp
            20                  25                  30

Gly Asp Ser Tyr Met Asn Trp Tyr Gln Gln Lys Pro Gly Lys Ala Pro
            35                  40                  45

Lys Leu Leu Ile Tyr Ala Ala Ser Tyr Leu Glu Ser Gly Val Pro Ser
        50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser His
                85                  90                  95
```

Glu Asp Pro Tyr Thr Phe Gly Gln Gly Thr Lys Val Glu Ile Lys Arg
100 105 110

Thr Val Ala Ala Pro Asp Ile Leu Leu Thr Gln Ser Pro Ala Ile Leu
115 120 125

Ser Val Ser Pro Gly Glu Arg Val Ser Phe Ser Cys Arg Ala Ser Gln
130 135 140

Ser Ile Gly Thr Asn Ile His Trp Tyr Gln Gln Arg Thr Asn Gly Ser
145 150 155 160

Pro Arg Leu Leu Ile Lys Tyr Ala Ser Glu Ser Ile Ser Gly Ile Pro
165 170 175

Ser Arg Phe Ser Gly Gly Gly Ser Gly Thr Asp Phe Thr Leu Ser Ile
180 185 190

Asn Ser Val Glu Ser Glu Asp Ile Ala Asp Tyr Tyr Cys Gln Gln Ser
195 200 205

Asn Asn Trp Pro Leu Thr Phe Gly Ala Gly Thr Lys Leu Glu Leu Lys
210 215 220

Arg Thr Val Ala Ala Pro Ser Val Phe Ile Phe Pro Pro Ser Asp Glu
225 230 235 240

Gln Leu Lys Ser Gly Thr Ala Ser Val Val Cys Leu Leu Asn Asn Phe
245 250 255

Tyr Pro Arg Glu Ala Lys Val Gln Trp Lys Val Asp Asn Ala Leu Gln
260 265 270

Ser Gly Asn Ser Gln Glu Ser Val Thr Glu Gln Asp Ser Lys Asp Ser
275 280 285

Thr Tyr Ser Leu Ser Ser Thr Leu Thr Leu Ser Lys Ala Asp Tyr Glu
290 295 300

Lys His Lys Val Tyr Ala Cys Glu Val Thr His Gln Gly Leu Ser Ser
305 310 315 320

Pro Val Thr Lys Ser Phe Asn Arg Gly Glu Cys
325 330

<210> 62
<211> 587

EP 3 466 973 A1

<212> PRT
<213> Artificial Sequence

<220>
<223> Description of Artificial Sequence: Synthetic
polypeptide

<400> 62
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
            35                  40                  45

Ser Ala Ile Thr Trp Asn Ser Gly His Ile Asp Tyr Ala Asp Ser Val
            50                  55                  60

Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Lys Val Ser Tyr Leu Ser Thr Ala Ser Ser Leu Asp Tyr Trp Gly
            100                 105                 110

Gln Gly Thr Leu Val Thr Val Ser Ser Gly Gly Gly Gly Ser Gly Gly
            115                 120                 125

Gly Gly Ser Glu Val Gln Leu Val Gln Ser Gly Ala Glu Val Lys Lys
            130                 135                 140

Pro Gly Ser Ser Val Lys Val Ser Cys Lys Ala Ser Gly Gly Ser Phe
145                 150                 155                 160

Gly Gly Tyr Gly Ile Gly Trp Val Arg Gln Ala Pro Gly Gln Gly Leu
                165                 170                 175

Glu Trp Met Gly Gly Ile Thr Pro Phe Phe Gly Phe Ala Asp Tyr Ala
                180                 185                 190

Gln Lys Phe Gln Gly Arg Val Thr Ile Thr Ala Asp Glu Ser Thr Thr
            195                 200                 205

Thr Ala Tyr Met Glu Leu Ser Gly Leu Thr Ser Asp Asp Thr Ala Val
            210                 215                 220

215

```
Tyr Tyr Cys Ala Arg Asp Pro Asn Glu Phe Trp Asn Gly Tyr Tyr Ser
225                 230             235                 240

Thr His Asp Phe Asp Ser Trp Gly Gln Gly Thr Thr Val Thr Val Ser
                245             250             255

Ser Ala Ser Thr Lys Gly Pro Ser Val Phe Pro Leu Ala Pro Ser Ser
            260             265             270

Lys Ser Thr Ser Gly Gly Thr Ala Ala Leu Gly Cys Leu Val Lys Asp
            275             280             285

Tyr Phe Pro Glu Pro Val Thr Val Ser Trp Asn Ser Gly Ala Leu Thr
    290             295             300

Ser Gly Val His Thr Phe Pro Ala Val Leu Gln Ser Ser Gly Leu Tyr
305             310             315             320

Ser Leu Ser Ser Val Val Thr Val Pro Ser Ser Ser Leu Gly Thr Gln
            325             330             335

Thr Tyr Ile Cys Asn Val Asn His Lys Pro Ser Asn Thr Lys Val Asp
            340             345             350

Lys Lys Val Glu Pro Lys Ser Cys Asp Lys Thr His Thr Cys Pro Pro
            355             360             365

Cys Pro Ala Pro Glu Leu Leu Gly Gly Pro Ser Val Phe Leu Phe Pro
    370             375             380

Pro Lys Pro Lys Asp Thr Leu Met Ile Ser Arg Thr Pro Glu Val Thr
385             390             395             400

Cys Val Val Val Asp Val Ser His Glu Asp Pro Glu Val Lys Phe Asn
            405             410             415

Trp Tyr Val Asp Gly Val Glu Val His Asn Ala Lys Thr Lys Pro Arg
            420             425             430

Glu Glu Gln Tyr Asn Ser Thr Tyr Arg Val Val Ser Val Leu Thr Val
            435             440             445

Leu His Gln Asp Trp Leu Asn Gly Lys Glu Tyr Lys Cys Lys Val Ser
    450             455             460

Asn Lys Ala Leu Pro Ala Pro Ile Glu Lys Thr Ile Ser Lys Ala Lys
465             470             475             480
```

216

```
Gly Gln Pro Arg Glu Pro Gln Val Tyr Thr Leu Pro Pro Ser Arg Asp
            485             490                 495

Glu Leu Thr Lys Asn Gln Val Ser Leu Thr Cys Leu Val Lys Gly Phe
            500             505                 510

Tyr Pro Ser Asp Ile Ala Val Glu Trp Glu Ser Asn Gly Gln Pro Glu
            515             520             525

Asn Asn Tyr Lys Thr Thr Pro Pro Val Leu Asp Ser Asp Gly Ser Phe
        530             535             540

Phe Leu Tyr Ser Lys Leu Thr Val Asp Lys Ser Arg Trp Gln Gln Gly
545             550             555             560

Asn Val Phe Ser Cys Ser Val Met His Glu Ala Leu His Asn His Tyr
            565             570                 575

Thr Gln Lys Ser Leu Ser Leu Ser Pro Gly Lys
            580             585
```

<210> 63
<211> 332
<212> PRT
<213> Artificial Sequence

<220>
<223> Description of Artificial Sequence: Synthetic
      polypeptide

<400> 63
```
Asp Ile Gln Met Thr Gln Ser Pro Ser Ser Leu Ser Ala Ser Val Gly
1               5               10              15

Asp Arg Val Thr Ile Thr Cys Arg Ala Ser Gln Gly Ile Arg Asn Tyr
            20              25              30

Leu Ala Trp Tyr Gln Gln Lys Pro Gly Lys Ala Pro Lys Leu Leu Ile
            35              40              45

Tyr Ala Ala Ser Thr Leu Gln Ser Gly Val Pro Ser Arg Phe Ser Gly
        50              55              60

Ser Gly Ser Gly Thr Asp Phe Thr Leu Thr Ile Ser Ser Leu Gln Pro
65              70              75              80

Glu Asp Val Ala Thr Tyr Tyr Cys Gln Arg Tyr Asn Arg Ala Pro Tyr
            85              90              95
```

217

```
Thr Phe Gly Gln Gly Thr Lys Val Glu Ile Lys Arg Gly Gly Ser Gly
            100                 105                 110

Gly Gly Gly Ser Gly Ser Glu Ile Val Leu Thr Gln Ser Pro Asp Phe
            115                 120                 125

Gln Ser Val Thr Pro Lys Glu Lys Val Thr Ile Thr Cys Arg Ala Ser
        130                 135                 140

Gln Asp Ile Gly Ser Glu Leu His Trp Tyr Gln Gln Lys Pro Asp Gln
145                 150                 155                 160

Pro Pro Lys Leu Leu Ile Lys Tyr Ala Ser His Ser Thr Ser Gly Val
            165                 170                 175

Pro Ser Arg Phe Ser Gly Ser Gly Ser Gly Thr Asp Phe Thr Leu Thr
            180                 185                 190

Ile Asn Gly Leu Glu Ala Glu Asp Ala Gly Thr Tyr Tyr Cys His Gln
            195                 200                 205

Thr Asp Ser Leu Pro Tyr Thr Phe Gly Pro Gly Thr Lys Val Asp Ile
        210                 215                 220

Lys Arg Thr Val Ala Ala Pro Ser Val Phe Ile Phe Pro Pro Ser Asp
225                 230                 235                 240

Glu Gln Leu Lys Ser Gly Thr Ala Ser Val Val Cys Leu Leu Asn Asn
            245                 250                 255

Phe Tyr Pro Arg Glu Ala Lys Val Gln Trp Lys Val Asp Asn Ala Leu
            260                 265                 270

Gln Ser Gly Asn Ser Gln Glu Ser Val Thr Glu Gln Asp Ser Lys Asp
            275                 280                 285

Ser Thr Tyr Ser Leu Ser Ser Thr Leu Thr Leu Ser Lys Ala Asp Tyr
        290                 295                 300

Glu Lys His Lys Val Tyr Ala Cys Glu Val Thr His Gln Gly Leu Ser
305                 310                 315                 320

Ser Pro Val Thr Lys Ser Phe Asn Arg Gly Glu Cys
            325                 330
```

<210> 64
<211> 573

<212> PRT
<213> Artificial Sequence

<220>
<223> Description of Artificial Sequence: Synthetic
      polypeptide

<400> 64

```
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Ala Ile Thr Trp Asn Ser Gly His Ile Asp Tyr Ala Asp Ser Val
        50                  55                  60

Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Lys Val Ser Tyr Leu Ser Thr Ala Ser Ser Leu Asp Tyr Trp Gly
            100                 105                 110

Gln Gly Thr Leu Val Thr Val Ser Ser Ala Ser Thr Lys Gly Pro Glu
            115                 120                 125

Val Gln Leu Val Gln Ser Gly Ala Glu Val Lys Lys Pro Gly Ala Ser
    130                 135                 140

Val Lys Val Ser Cys Lys Ala Ser Gly Tyr Thr Phe Thr Lys Tyr Trp
145                 150                 155                 160

Leu Gly Trp Val Arg Gln Ala Pro Gly Gln Gly Leu Glu Trp Met Gly
                165                 170                 175

Asp Ile Tyr Pro Gly Tyr Asp Tyr Thr His Tyr Asn Glu Lys Phe Lys
            180                 185                 190

Asp Arg Val Thr Leu Thr Thr Asp Thr Ser Thr Ser Thr Ala Tyr Met
            195                 200                 205

Glu Leu Arg Ser Leu Arg Ser Asp Asp Thr Ala Val Tyr Tyr Cys Ala
    210                 215                 220
```

```
Arg Ser Asp Gly Ser Ser Thr Tyr Trp Gly Gln Gly Thr Leu Val Thr
225             230             235             240

Val Ser Ser Ala Ser Thr Lys Gly Pro Ser Val Phe Pro Leu Ala Pro
                245             250             255

Ser Ser Lys Ser Thr Ser Gly Gly Thr Ala Ala Leu Gly Cys Leu Val
            260             265             270

Lys Asp Tyr Phe Pro Glu Pro Val Thr Val Ser Trp Asn Ser Gly Ala
            275             280             285

Leu Thr Ser Gly Val His Thr Phe Pro Ala Val Leu Gln Ser Ser Gly
    290             295             300

Leu Tyr Ser Leu Ser Ser Val Val Thr Val Pro Ser Ser Ser Leu Gly
305             310             315             320

Thr Gln Thr Tyr Ile Cys Asn Val Asn His Lys Pro Ser Asn Thr Lys
            325             330             335

Val Asp Lys Lys Val Glu Pro Lys Ser Cys Asp Lys Thr His Thr Cys
            340             345             350

Pro Pro Cys Pro Ala Pro Glu Leu Leu Gly Gly Pro Ser Val Phe Leu
        355             360             365

Phe Pro Pro Lys Pro Lys Asp Thr Leu Met Ile Ser Arg Thr Pro Glu
    370             375             380

Val Thr Cys Val Val Val Asp Val Ser His Glu Asp Pro Glu Val Lys
385             390             395             400

Phe Asn Trp Tyr Val Asp Gly Val Glu Val His Asn Ala Lys Thr Lys
                405             410             415

Pro Arg Glu Glu Gln Tyr Asn Ser Thr Tyr Arg Val Val Ser Val Leu
            420             425             430

Thr Val Leu His Gln Asp Trp Leu Asn Gly Lys Glu Tyr Lys Cys Lys
        435             440             445

Val Ser Asn Lys Ala Leu Pro Ala Pro Ile Glu Lys Thr Ile Ser Lys
    450             455             460

Ala Lys Gly Gln Pro Arg Glu Pro Gln Val Tyr Thr Leu Pro Pro Ser
465             470             475             480
```

```
Arg Asp Glu Leu Thr Lys Asn Gln Val Ser Leu Thr Cys Leu Val Lys
                485                 490                 495

Gly Phe Tyr Pro Ser Asp Ile Ala Val Glu Trp Glu Ser Asn Gly Gln
            500                 505                 510

Pro Glu Asn Asn Tyr Lys Thr Thr Pro Pro Val Leu Asp Ser Asp Gly
        515                 520                 525

Ser Phe Phe Leu Tyr Ser Lys Leu Thr Val Asp Lys Ser Arg Trp Gln
    530                 535                 540

Gln Gly Asn Val Phe Ser Cys Ser Val Met His Glu Ala Leu His Asn
545                 550                 555                 560

His Tyr Thr Gln Lys Ser Leu Ser Leu Ser Pro Gly Lys
                565                 570
```

<210> 65
<211> 332
<212> PRT
<213> Artificial Sequence

<220>
<223> Description of Artificial Sequence: Synthetic
      polypeptide

<400> 65
```
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Ala Trp Tyr Gln Gln Lys Pro Gly Lys Ala Pro Lys Leu Leu Ile
            35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
            100                 105                 110
```

221

```
Pro Asp Val Leu Met Thr Gln Thr Pro Leu Ser Leu Pro Val Thr Pro
        115                 120             125

Gly Glu Pro Ala Ser Ile Ser Cys Thr Ser Ser Gln Asn Ile Val His
        130                 135             140

Ser Asn Gly Asn Thr Tyr Leu Glu Trp Tyr Leu Gln Lys Pro Gly Gln
145                 150             155                 160

Ser Pro Gln Leu Leu Ile Tyr Lys Val Ser Asn Arg Phe Ser Gly Val
                165             170             175

Pro Asp Arg Phe Ser Gly Ser Gly Ser Gly Thr Asp Phe Thr Leu Lys
            180             185             190

Ile Ser Arg Val Glu Ala Glu Asp Val Gly Val Tyr Tyr Cys Phe Gln
        195                 200             205

Val Ser His Val Pro Tyr Thr Phe Gly Gly Gly Thr Lys Val Glu Ile
    210             215             220

Lys Arg Thr Val Ala Ala Pro Ser Val Phe Ile Phe Pro Pro Ser Asp
225             230             235                 240

Glu Gln Leu Lys Ser Gly Thr Ala Ser Val Val Cys Leu Leu Asn Asn
                245             250             255

Phe Tyr Pro Arg Glu Ala Lys Val Gln Trp Lys Val Asp Asn Ala Leu
            260             265             270

Gln Ser Gly Asn Ser Gln Glu Ser Val Thr Glu Gln Asp Ser Lys Asp
            275             280             285

Ser Thr Tyr Ser Leu Ser Ser Thr Leu Thr Leu Ser Lys Ala Asp Tyr
    290             295             300

Glu Lys His Lys Val Tyr Ala Cys Glu Val Thr His Gln Gly Leu Ser
305             310             315                 320

Ser Pro Val Thr Lys Ser Phe Asn Arg Gly Glu Cys
                325             330
```

<210> 66
<211> 577
<212> PRT
<213> Artificial Sequence

<220>

<223> Description of Artificial Sequence: Synthetic
      polypeptide

<400> 66

Glu Val Gln Leu Val Glu Ser Gly Gly Gly Val Val Gln Pro Gly Arg
1               5                   10                  15


Ser Leu Arg Leu Ser Cys Ser Ala Ser Gly Phe Ile Phe Ser Arg Tyr
            20                  25                  30


Asp Met Ser Trp Val Arg Gln Ala Pro Gly Lys Gly Leu Glu Trp Val
            35                  40                  45


Ala Tyr Ile Ser His Gly Gly Ala Gly Thr Tyr Tyr Pro Asp Ser Val
        50                  55                  60


Lys Gly Arg Phe Thr Ile Ser Arg Asp Asn Ser Lys Asn Thr Leu Phe
65                  70                  75                  80


Leu Gln Met Asp Ser Leu Arg Pro Glu Asp Thr Gly Val Tyr Phe Cys
                85                  90                  95


Ala Arg Gly Gly Val Thr Lys Gly Tyr Phe Asp Val Trp Gly Gln Gly
            100                 105                 110


Thr Pro Val Thr Val Ser Ser Ala Ser Thr Lys Gly Pro Gln Val Gln
            115                 120                 125


Leu Val Glu Ser Gly Gly Gly Val Val Gln Pro Gly Arg Ser Leu Arg
        130                 135                 140


Leu Ser Cys Thr Ala Ser Gly Phe Thr Phe Ser Met Phe Gly Val His
145                 150                 155                 160


Trp Val Arg Gln Ala Pro Gly Lys Gly Leu Glu Trp Val Ala Ala Val
                165                 170                 175


Ser Tyr Asp Gly Ser Asn Lys Tyr Tyr Ala Glu Ser Val Lys Gly Arg
            180                 185                 190


Phe Thr Ile Ser Arg Asp Asn Ser Lys Asn Ile Leu Phe Leu Gln Met
            195                 200                 205


Asp Ser Leu Arg Leu Glu Asp Thr Ala Val Tyr Tyr Cys Ala Arg Gly
        210                 215                 220


Arg Pro Lys Val Val Ile Pro Ala Pro Leu Ala His Trp Gly Gln Gly
225                 230                 235                 240


223

```
Thr Leu Val Thr Phe Ser Ser Ala Ser Thr Lys Gly Pro Ser Val Phe
            245             250             255

Pro Leu Ala Pro Ser Ser Lys Ser Thr Ser Gly Gly Thr Ala Ala Leu
            260             265             270

Gly Cys Leu Val Lys Asp Tyr Phe Pro Glu Pro Val Thr Val Ser Trp
            275             280             285

Asn Ser Gly Ala Leu Thr Ser Gly Val His Thr Phe Pro Ala Val Leu
            290             295             300

Gln Ser Ser Gly Leu Tyr Ser Leu Ser Ser Val Val Thr Val Pro Ser
305             310             315             320

Ser Ser Leu Gly Thr Gln Thr Tyr Ile Cys Asn Val Asn His Lys Pro
            325             330             335

Ser Asn Thr Lys Val Asp Lys Lys Val Glu Pro Lys Ser Cys Asp Lys
            340             345             350

Thr His Thr Cys Pro Pro Cys Pro Ala Pro Glu Ala Ala Gly Gly Pro
            355             360             365

Ser Val Phe Leu Phe Pro Pro Lys Pro Lys Asp Thr Leu Met Ile Ser
370             375             380

Arg Thr Pro Glu Val Thr Cys Val Val Val Asp Val Ser His Glu Asp
385             390             395             400

Pro Glu Val Lys Phe Asn Trp Tyr Val Asp Gly Val Glu Val His Asn
            405             410             415

Ala Lys Thr Lys Pro Arg Glu Glu Gln Tyr Asn Ser Thr Tyr Arg Val
            420             425             430

Val Ser Val Leu Thr Val Leu His Gln Asp Trp Leu Asn Gly Lys Glu
            435             440             445

Tyr Lys Cys Lys Val Ser Asn Lys Ala Leu Pro Ala Pro Ile Glu Lys
    450             455             460

Thr Ile Ser Lys Ala Lys Gly Gln Pro Arg Glu Pro Gln Val Tyr Thr
465             470             475             480

Leu Pro Pro Ser Arg Glu Glu Met Thr Lys Asn Gln Val Ser Leu Thr
            485             490             495
```

```
        Cys Leu Val Lys Gly Phe Tyr Pro Ser Asp Ile Ala Val Glu Trp Glu
                    500             505             510

        Ser Asn Gly Gln Pro Glu Asn Asn Tyr Lys Thr Thr Pro Pro Val Leu
                    515             520             525

        Asp Ser Asp Gly Ser Phe Phe Leu Tyr Ser Lys Leu Thr Val Asp Lys
                    530             535             540

        Ser Arg Trp Gln Gln Gly Asn Val Phe Ser Cys Ser Val Met His Glu
        545             550             555             560

        Ala Leu His Asn His Tyr Thr Gln Lys Ser Leu Ser Leu Ser Pro Gly
                    565             570             575

        Lys


        <210> 67
        <211> 327
        <212> PRT
        <213> Artificial Sequence

        <220>
        <223> Description of Artificial Sequence: Synthetic
              polypeptide

        <400> 67
        Asp Ile Gln Met Thr Gln Ser Pro Ser Ser Leu Ser Ala Ser Val Gly
        1               5               10              15

        Asp Arg Val Thr Ile Thr Cys Arg Ala Ser Gly Asn Ile His Asn Tyr
                    20              25              30

        Leu Thr Trp Tyr Gln Gln Thr Pro Gly Lys Ala Pro Lys Leu Leu Ile
                    35              40              45

        Tyr Asn Ala Lys Thr Leu Ala Asp Gly Val Pro Ser Arg Phe Ser Gly
            50              55              60

        Ser Gly Ser Gly Thr Asp Tyr Thr Phe Thr Ile Ser Ser Leu Gln Pro
        65              70              75              80

        Glu Asp Ile Ala Thr Tyr Tyr Cys Gln His Phe Trp Ser Ile Pro Tyr
                    85              90              95

        Thr Phe Gly Gln Gly Thr Lys Leu Gln Ile Thr Arg Thr Val Ala Ala
                    100             105             110
```

```
Pro Asp Ile Gln Met Thr Gln Ser Pro Ser Ser Val Ser Ala Ser Val
        115             120             125

Gly Asp Arg Val Thr Ile Thr Cys Arg Ala Ser Gln Gly Ile Ser Ser
        130             135             140

Trp Leu Ala Trp Tyr Gln Gln Lys Pro Gly Lys Ala Pro Lys Leu Leu
145             150             155             160

Ile Tyr Glu Ala Ser Asn Leu Glu Thr Gly Val Pro Ser Arg Phe Ser
                165             170             175

Gly Ser Gly Ser Gly Ser Asp Phe Thr Leu Thr Ile Ser Ser Leu Gln
            180             185             190

Pro Glu Asp Phe Ala Thr Tyr Tyr Cys Gln Gln Thr Ser Ser Phe Leu
        195             200             205

Leu Ser Phe Gly Gly Gly Thr Lys Val Glu His Lys Arg Thr Val Ala
    210             215             220

Ala Pro Ser Val Phe Ile Phe Pro Pro Ser Asp Glu Gln Leu Lys Ser
225             230             235             240

Gly Thr Ala Ser Val Val Cys Leu Leu Asn Asn Phe Tyr Pro Arg Glu
            245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
        275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315             320

Ser Phe Asn Arg Gly Glu Cys
                325
```

<210> 68
<211> 584
<212> PRT
<213> Artificial Sequence

<220>

<223> Description of Artificial Sequence: Synthetic polypeptide

<400> 68

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Arg His Phe
            20                  25                  30

Pro Met Ala Trp Val Arg Gln Ala Pro Gly Lys Gly Leu Glu Trp Val
        35                  40                  45

Ala Thr Ile Ser Ser Ser Asp Ala Trp Pro Ser Tyr Arg Asp Ser Val
        50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ser Arg Gly Tyr Tyr Asn Ser Pro Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Glu Val Gln Leu Val Glu Ser Gly Gly Gly Leu Val Gln
        130                 135                 140

Pro Gly Gly Ser Leu Arg Leu Ser Cys Ala Ala Ser Gly Tyr Thr Phe
145                 150                 155                 160

Thr Asn Tyr Gly Met Asn Trp Val Arg Gln Ala Pro Gly Lys Gly Leu
                165                 170                 175

Glu Trp Val Gly Trp Ile Asn Thr Tyr Thr Gly Glu Pro Thr Tyr Ala
            180                 185                 190

Ala Asp Phe Lys Arg Arg Phe Thr Phe Ser Leu Asp Thr Ser Lys Ser
        195                 200                 205

Thr Ala Tyr Leu Gln Met Asn Ser Leu Arg Ala Glu Asp Thr Ala Val
        210                 215                 220

Tyr Tyr Cys Ala Lys Tyr Pro His Tyr Tyr Gly Ser Ser His Trp Tyr
225                 230                 235                 240
```

```
Phe Asp Val Trp Gly Gln Gly Thr Leu Val Thr Val Ser Ser Ala Ser
              245              250              255

Thr Lys Gly Pro Ser Val Phe Pro Leu Ala Pro Ser Ser Lys Ser Thr
              260              265              270

Ser Gly Gly Thr Ala Ala Leu Gly Cys Leu Val Lys Asp Tyr Phe Pro
              275              280              285

Glu Pro Val Thr Val Ser Trp Asn Ser Gly Ala Leu Thr Ser Gly Val
          290              295              300

His Thr Phe Pro Ala Val Leu Gln Ser Ser Gly Leu Tyr Ser Leu Ser
305              310              315              320

Ser Val Val Thr Val Pro Ser Ser Ser Leu Gly Thr Gln Thr Tyr Ile
              325              330              335

Cys Asn Val Asn His Lys Pro Ser Asn Thr Lys Val Asp Lys Lys Val
              340              345              350

Glu Pro Lys Ser Cys Asp Lys Thr His Thr Cys Pro Pro Cys Pro Ala
          355              360              365

Pro Glu Ala Ala Gly Gly Pro Ser Val Phe Leu Phe Pro Pro Lys Pro
          370              375              380

Lys Asp Thr Leu Met Ile Ser Arg Thr Pro Glu Val Thr Cys Val Val
385              390              395              400

Val Asp Val Ser His Glu Asp Pro Glu Val Lys Phe Asn Trp Tyr Val
              405              410              415

Asp Gly Val Glu Val His Asn Ala Lys Thr Lys Pro Arg Glu Glu Gln
          420              425              430

Tyr Asn Ser Thr Tyr Arg Val Val Ser Val Leu Thr Val Leu His Gln
          435              440              445

Asp Trp Leu Asn Gly Lys Glu Tyr Lys Cys Lys Val Ser Asn Lys Ala
          450              455              460

Leu Pro Ala Pro Ile Glu Lys Thr Ile Ser Lys Ala Lys Gly Gln Pro
465              470              475              480

Arg Glu Pro Gln Val Tyr Thr Leu Pro Pro Ser Arg Glu Glu Met Thr
              485              490              495
```

```
Lys Asn Gln Val Ser Leu Thr Cys Leu Val Lys Gly Phe Tyr Pro Ser
            500                 505                 510

Asp Ile Ala Val Glu Trp Glu Ser Asn Gly Gln Pro Glu Asn Asn Tyr
            515                 520                 525

Lys Thr Thr Pro Pro Val Leu Asp Ser Asp Gly Ser Phe Phe Leu Tyr
            530                 535                 540

Ser Lys Leu Thr Val Asp Lys Ser Arg Trp Gln Gln Gly Asn Val Phe
545                 550                 555                 560

Ser Cys Ser Val Met His Glu Ala Leu His Asn His Tyr Thr Gln Lys
                565                 570                 575

Ser Leu Ser Leu Ser Pro Gly Lys
                580
```

<210> 69
<211> 327
<212> PRT
<213> Artificial Sequence

<220>
<223> Description of Artificial Sequence: Synthetic
      polypeptide

<400> 69
```
Asp Ile Gln Met Thr Gln Ser Pro Ser Ser Leu Ser Ala Ser Val Gly
1               5                   10                  15

Asp Arg Val Thr Ile Thr Cys Arg Ala Ser Glu Asp Ile Tyr Ser Asn
            20                  25                  30

Leu Ala Trp Tyr Gln Gln Lys Pro Gly Lys Ala Pro Lys Leu Leu Ile
            35                  40                  45

Tyr Asp Thr Asn Asn Leu Ala Asp Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Asn Tyr Pro Pro
                85                  90                  95

Thr Phe Gly Gln Gly Thr Lys Leu Glu Ile Lys Arg Thr Val Ala Ala
            100                 105                 110
```

Pro Asp Ile Gln Met Thr Gln Ser Pro Ser Ser Leu Ser Ala Ser Val
        115             120             125

Gly Asp Arg Val Thr Ile Thr Cys Ser Ala Ser Gln Asp Ile Ser Asn
        130             135             140

Tyr Leu Asn Trp Tyr Gln Gln Lys Pro Gly Lys Ala Pro Lys Val Leu
145             150             155             160

Ile Tyr Phe Thr Ser Ser Leu His Ser Gly Val Pro Ser Arg Phe Ser
                165             170             175

Gly Ser Gly Ser Gly Thr Asp Phe Thr Leu Thr Ile Ser Ser Leu Gln
                180             185             190

Pro Glu Asp Phe Ala Thr Tyr Tyr Cys Gln Gln Tyr Ser Thr Val Pro
        195             200             205

Trp Thr Phe Gly Gln Gly Thr Lys Val Glu Ile Lys Arg Thr Val Ala
        210             215             220

Ala Pro Ser Val Phe Ile Phe Pro Pro Ser Asp Glu Gln Leu Lys Ser
225             230             235             240

Gly Thr Ala Ser Val Val Cys Leu Leu Asn Asn Phe Tyr Pro Arg Glu
                245             250             255

Ala Lys Val Gln Trp Lys Val Asp Asn Ala Leu Gln Ser Gly Asn Ser
                260             265             270

Gln Glu Ser Val Thr Glu Gln Asp Ser Lys Asp Ser Thr Tyr Ser Leu
        275             280             285

Ser Ser Thr Leu Thr Leu Ser Lys Ala Asp Tyr Glu Lys His Lys Val
290             295             300

Tyr Ala Cys Glu Val Thr His Gln Gly Leu Ser Ser Pro Val Thr Lys
305             310             315             320

Ser Phe Asn Arg Gly Glu Cys
                325

<210> 70
<211> 576
<212> PRT
<213> Artificial Sequence

<220>

<223> Description of Artificial Sequence: Synthetic
      polypeptide

<400> 70
Glu Val Thr Leu Arg Glu Ser Gly Pro Ala Leu Val Lys Pro Thr Gln
1               5                   10                  15


Thr Leu Thr Leu Thr Cys Thr Phe Ser Gly Phe Ser Leu Ser Lys Ser
            20                  25                  30


Val Met Gly Val Ser Trp Ile Arg Gln Pro Pro Gly Lys Ala Leu Glu
        35                  40                  45


Trp Leu Ala His Ile Tyr Trp Asp Asp Asp Lys Tyr Tyr Asn Pro Ser
    50                  55                  60


Leu Lys Ser Arg Leu Thr Ile Ser Lys Asp Thr Ser Lys Asn Gln Val
65                  70                  75                  80


Val Leu Thr Met Thr Asn Met Asp Pro Val Asp Thr Ala Thr Tyr Tyr
                85                  90                  95


Cys Ala Arg Arg Gly Ile Arg Ser Ala Met Asp Tyr Trp Gly Gln Gly
            100                 105                 110


Thr Thr Val Thr Val Ser Ser Ala Ser Thr Lys Gly Pro Glu Val Gln
            115                 120                 125


Leu Val Gln Ser Gly Thr Glu Val Lys Lys Pro Gly Glu Ser Leu Lys
    130                 135                 140


Ile Ser Cys Lys Gly Ser Gly Tyr Thr Val Thr Ser Tyr Trp Ile Gly
145                 150                 155                 160


Trp Val Arg Gln Met Pro Gly Lys Gly Leu Glu Trp Met Gly Phe Ile
                165                 170                 175


Tyr Pro Gly Asp Ser Glu Thr Arg Tyr Ser Pro Thr Phe Gln Gly Gln
                180                 185                 190


Val Thr Ile Ser Ala Asp Lys Ser Phe Asn Thr Ala Phe Leu Gln Trp
        195                 200                 205


Ser Ser Leu Lys Ala Ser Asp Thr Ala Met Tyr Tyr Cys Ala Arg Val
    210                 215                 220


Gly Ser Gly Trp Tyr Pro Tyr Thr Phe Asp Ile Trp Gly Gln Gly Thr
225                 230                 235                 240

```
Met Val Thr Val Ser Ser Ala Ser Thr Lys Gly Pro Ser Val Phe Pro
            245                 250                 255

Leu Ala Pro Ser Ser Lys Ser Thr Ser Gly Gly Thr Ala Ala Leu Gly
            260                 265                 270

Cys Leu Val Lys Asp Tyr Phe Pro Glu Pro Val Thr Val Ser Trp Asn
            275                 280                 285

Ser Gly Ala Leu Thr Ser Gly Val His Thr Phe Pro Ala Val Leu Gln
        290                 295                 300

Ser Ser Gly Leu Tyr Ser Leu Ser Ser Val Val Thr Val Pro Ser Ser
305                 310                 315                 320

Ser Leu Gly Thr Gln Thr Tyr Ile Cys Asn Val Asn His Lys Pro Ser
            325                 330                 335

Asn Thr Lys Val Asp Lys Lys Val Glu Pro Lys Ser Cys Asp Lys Thr
            340                 345                 350

His Thr Cys Pro Pro Cys Pro Ala Pro Glu Ala Ala Gly Gly Pro Ser
        355                 360                 365

Val Phe Leu Phe Pro Pro Lys Pro Lys Asp Thr Leu Met Ile Ser Arg
        370                 375                 380

Thr Pro Glu Val Thr Cys Val Val Val Asp Val Ser His Glu Asp Pro
385                 390                 395                 400

Glu Val Lys Phe Asn Trp Tyr Val Asp Gly Val Glu Val His Asn Ala
            405                 410                 415

Lys Thr Lys Pro Arg Glu Glu Gln Tyr Asn Ser Thr Tyr Arg Val Val
            420                 425                 430

Ser Val Leu Thr Val Leu His Gln Asp Trp Leu Asn Gly Lys Glu Tyr
        435                 440                 445

Lys Cys Lys Val Ser Asn Lys Ala Leu Pro Ala Pro Ile Glu Lys Thr
        450                 455                 460

Ile Ser Lys Ala Lys Gly Gln Pro Arg Glu Pro Gln Val Tyr Thr Leu
465                 470                 475                 480

Pro Pro Ser Arg Glu Glu Met Thr Lys Asn Gln Val Ser Leu Thr Cys
            485                 490                 495
```

```
Leu Val Lys Gly Phe Tyr Pro Ser Asp Ile Ala Val Glu Trp Glu Ser
            500             505             510

Asn Gly Gln Pro Glu Asn Asn Tyr Lys Thr Thr Pro Pro Val Leu Asp
            515             520             525

Ser Asp Gly Ser Phe Phe Leu Tyr Ser Lys Leu Thr Val Asp Lys Ser
            530             535             540

Arg Trp Gln Gln Gly Asn Val Phe Ser Cys Ser Val Met His Glu Ala
545             550             555             560

Leu His Asn His Tyr Thr Gln Lys Ser Leu Ser Leu Ser Pro Gly Lys
                565             570             575
```

<210> 71
<211> 328
<212> PRT
<213> Artificial Sequence

<220>
<223> Description of Artificial Sequence: Synthetic
      polypeptide

<400> 71
```
Asp Ile Val Met Thr Gln Ser Pro Asp Ser Leu Ala Val Ser Leu Gly
1               5               10              15

Glu Arg Ala Thr Ile Asn Cys Lys Ala Ser Gln Ser Val Ser Asn Asp
            20              25              30

Val Ala Trp Tyr Gln Gln Lys Pro Gly Gln Pro Pro Lys Leu Leu Ile
            35              40              45

Tyr Tyr Ala Ser Asn Arg Tyr Thr Gly Val Pro Asp Arg Phe Ser Gly
    50              55              60

Ser Gly Ser Gly Thr Asp Phe Thr Leu Thr Ile Ser Ser Leu Gln Ala
65              70              75              80

Glu Asp Val Ala Val Tyr Tyr Cys Gln Gln Asp Tyr Asn Ser Pro Trp
                85              90              95

Thr Phe Gly Gly Gly Thr Lys Val Glu Ile Lys Arg Thr Val Ala Ala
            100             105             110

Pro Glu Ile Val Met Thr Gln Ser Pro Ala Thr Leu Ser Val Ser Pro
            115             120             125
```

Gly Glu Arg Ala Thr Leu Ser Cys Arg Ala Ser Glu Ser Ile Ser Ser
        130                 135                 140

Asn Leu Ala Trp Tyr Gln Gln Lys Pro Gly Gln Ala Pro Arg Leu Phe
        145                 150                 155                 160

Ile Tyr Thr Ala Ser Thr Arg Ala Thr Asp Ile Pro Ala Arg Phe Ser
                165                 170                 175

Gly Ser Gly Ser Gly Thr Glu Phe Thr Leu Thr Ile Ser Ser Leu Gln
                180                 185                 190

Ser Glu Asp Phe Ala Val Tyr Tyr Cys Gln Gln Tyr Asn Asn Trp Pro
                195                 200                 205

Ser Ile Thr Phe Gly Gln Gly Thr Arg Leu Glu Ile Lys Arg Thr Val
        210                 215                 220

Ala Ala Pro Ser Val Phe Ile Phe Pro Pro Ser Asp Glu Gln Leu Lys
225                 230                 235                 240

Ser Gly Thr Ala Ser Val Val Cys Leu Leu Asn Asn Phe Tyr Pro Arg
                245                 250                 255

Glu Ala Lys Val Gln Trp Lys Val Asp Asn Ala Leu Gln Ser Gly Asn
        260                 265                 270

Ser Gln Glu Ser Val Thr Glu Gln Asp Ser Lys Asp Ser Thr Tyr Ser
        275                 280                 285

Leu Ser Ser Thr Leu Thr Leu Ser Lys Ala Asp Tyr Glu Lys His Lys
        290                 295                 300

Val Tyr Ala Cys Glu Val Thr His Gln Gly Leu Ser Ser Pro Val Thr
305                 310                 315                 320

Lys Ser Phe Asn Arg Gly Glu Cys
                325

<210> 72
<211> 584
<212> PRT
<213> Artificial Sequence

<220>
<223> Description of Artificial Sequence: Synthetic
        polypeptide

<400> 72

Glu Val Gln Leu Val Glu Ser Gly Gly Gly Leu Val Gln Pro Gly Gly
1               5               10                  15

Ser Leu Arg Leu Ser Cys Ala Ala Ser Gly Phe Thr Phe Ser Asn Phe
            20                  25                  30

Pro Met Ala Trp Val Arg Gln Ala Pro Gly Lys Gly Leu Glu Trp Val
            35                  40                  45

Ala Thr Ile Ser Ser Ser Asp Gly Thr Thr Tyr Tyr Arg Asp Ser Val
            50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Gly Tyr Tyr Asn Ser Pro Phe Ala Tyr Trp Gly Gln Gly Thr
            100                 105                 110

Leu Val Thr Val Ser Ser Ala Ser Thr Lys Gly Pro Ser Val Phe Pro
            115                 120                 125

Leu Ala Pro Glu Val Gln Leu Val Glu Ser Gly Gly Gly Leu Val Gln
            130                 135                 140

Pro Gly Gly Ser Leu Arg Leu Ser Cys Ala Ala Ser Gly Tyr Thr Phe
145                 150                 155                 160

Thr Asn Tyr Gly Met Asn Trp Val Arg Gln Ala Pro Gly Lys Gly Leu
                165                 170                 175

Glu Trp Val Gly Trp Ile Asn Thr Tyr Thr Gly Glu Pro Thr Tyr Ala
            180                 185                 190

Ala Asp Phe Lys Arg Arg Phe Thr Phe Ser Leu Asp Thr Ser Lys Ser
            195                 200                 205

Thr Ala Tyr Leu Gln Met Asn Ser Leu Arg Ala Glu Asp Thr Ala Val
            210                 215                 220

Tyr Tyr Cys Ala Lys Tyr Pro His Tyr Tyr Gly Ser Ser His Trp Tyr
225                 230                 235                 240

Phe Asp Val Trp Gly Gln Gly Thr Leu Val Thr Val Ser Ser Ala Ser
                245                 250                 255

Thr Lys Gly Pro Ser Val Phe Pro Leu Ala Pro Ser Ser Lys Ser Thr
        260             265             270

Ser Gly Gly Thr Ala Ala Leu Gly Cys Leu Val Lys Asp Tyr Phe Pro
        275             280             285

Glu Pro Val Thr Val Ser Trp Asn Ser Gly Ala Leu Thr Ser Gly Val
    290             295             300

His Thr Phe Pro Ala Val Leu Gln Ser Ser Gly Leu Tyr Ser Leu Ser
305             310             315             320

Ser Val Val Thr Val Pro Ser Ser Ser Leu Gly Thr Gln Thr Tyr Ile
            325             330             335

Cys Asn Val Asn His Lys Pro Ser Asn Thr Lys Val Asp Lys Lys Val
        340             345             350

Glu Pro Lys Ser Cys Asp Lys Thr His Thr Cys Pro Pro Cys Pro Ala
        355             360             365

Pro Glu Ala Ala Gly Gly Pro Ser Val Phe Leu Phe Pro Pro Lys Pro
    370             375             380

Lys Asp Thr Leu Met Ile Ser Arg Thr Pro Glu Val Thr Cys Val Val
385             390             395             400

Val Asp Val Ser His Glu Asp Pro Glu Val Lys Phe Asn Trp Tyr Val
            405             410             415

Asp Gly Val Glu Val His Asn Ala Lys Thr Lys Pro Arg Glu Glu Gln
        420             425             430

Tyr Asn Ser Thr Tyr Arg Val Val Ser Val Leu Thr Val Leu His Gln
        435             440             445

Asp Trp Leu Asn Gly Lys Glu Tyr Lys Cys Lys Val Ser Asn Lys Ala
    450             455             460

Leu Pro Ala Pro Ile Glu Lys Thr Ile Ser Lys Ala Lys Gly Gln Pro
465             470             475             480

Arg Glu Pro Gln Val Tyr Thr Leu Pro Pro Ser Arg Glu Glu Met Thr
            485             490             495

Lys Asn Gln Val Ser Leu Thr Cys Leu Val Lys Gly Phe Tyr Pro Ser

236

```
                    500                    505                    510


        Asp Ile Ala Val Glu Trp Glu Ser Asn Gly Gln Pro Glu Asn Asn Tyr
                515                    520                    525


        Lys Thr Thr Pro Pro Val Leu Asp Ser Asp Gly Ser Phe Phe Leu Tyr
                530                    535                    540


        Ser Lys Leu Thr Val Asp Lys Ser Arg Trp Gln Gln Gly Asn Val Phe
        545                    550                    555                    560


        Ser Cys Ser Val Met His Glu Ala Leu His Asn His Tyr Thr Gln Lys
                565                    570                    575


        Ser Leu Ser Leu Ser Pro Gly Lys
                580


        <210> 73
        <211> 327
        <212> PRT
        <213> Artificial Sequence

        <220>
        <223> Description of Artificial Sequence: Synthetic
              polypeptide

        <400> 73
        Asp Ile Gln Met Thr Gln Ser Pro Ser Ser Leu Ser Ala Ser Val Gly
        1                    5                    10                    15


        Asp Arg Val Thr Ile Thr Cys Arg Ala Ser Glu Asp Ile Tyr Ser Asn
                20                    25                    30


        Leu Ala Trp Tyr Gln Gln Lys Pro Gly Lys Ala Pro Lys Leu Leu Ile
                35                    40                    45


        Tyr Asp Thr Asn Asn Leu Ala Asp Gly Val Pro Ser Arg Phe Ser Gly
                50                    55                    60


        Ser Gly Ser Gly Thr Asp Phe Thr Leu Thr Ile Ser Ser Leu Gln Pro
        65                    70                    75                    80


        Glu Asp Phe Ala Thr Tyr Tyr Cys Gln Gln Tyr Asn Asn Tyr Pro Pro
                85                    90                    95


        Thr Phe Gly Gln Gly Thr Lys Leu Glu Ile Lys Arg Thr Val Ala Ala
                100                    105                    110


        Pro Asp Ile Gln Met Thr Gln Ser Pro Ser Ser Leu Ser Ala Ser Val
                115                    120                    125
```

```
Gly Asp Arg Val Thr Ile Thr Cys Ser Ala Ser Gln Asp Ile Ser Asn
    130                 135             140

Tyr Leu Asn Trp Tyr Gln Gln Lys Pro Gly Lys Ala Pro Lys Val Leu
    145             150             155                 160

Ile Tyr Phe Thr Ser Ser Leu His Ser Gly Val Pro Ser Arg Phe Ser
            165             170                 175

Gly Ser Gly Ser Gly Thr Asp Phe Thr Leu Thr Ile Ser Ser Leu Gln
            180             185                 190

Pro Glu Asp Phe Ala Thr Tyr Tyr Cys Gln Gln Tyr Ser Thr Val Pro
        195             200             205

Trp Thr Phe Gly Gln Gly Thr Lys Val Glu Ile Lys Arg Thr Val Ala
    210             215             220

Ala Pro Ser Val Phe Ile Phe Pro Pro Ser Asp Glu Gln Leu Lys Ser
225             230             235                 240

Gly Thr Ala Ser Val Val Cys Leu Leu Asn Asn Phe Tyr Pro Arg Glu
            245             250             255

Ala Lys Val Gln Trp Lys Val Asp Asn Ala Leu Gln Ser Gly Asn Ser
            260             265             270

Gln Glu Ser Val Thr Glu Gln Asp Ser Lys Asp Ser Thr Tyr Ser Leu
        275             280             285

Ser Ser Thr Leu Thr Leu Ser Lys Ala Asp Tyr Glu Lys His Lys Val
    290             295             300

Tyr Ala Cys Glu Val Thr His Gln Gly Leu Ser Ser Pro Val Thr Lys
305             310             315                 320

Ser Phe Asn Arg Gly Glu Cys
                325
```

```
<210> 74
<211> 577
<212> PRT
<213> Artificial Sequence

<220>
<223> Description of Artificial Sequence: Synthetic
      polypeptide
```

<400> 74

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Arg His Phe
            20                  25                  30

Pro Met Ala Trp Val Arg Gln Ala Pro Gly Lys Gly Leu Glu Trp Val
        35                  40                  45

Ala Thr Ile Ser Ser Ser Asp Ala Trp Pro Ser Tyr Arg Asp Ser Val
        50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ser Arg Gly Tyr Tyr Asn Ser Pro Phe Ala Tyr Trp Gly Gln Gly Thr
            100                 105                 110

Leu Val Thr Val Ser Ser Ala Ser Thr Lys Gly Pro Glu Val Gln Leu
        115                 120                 125

Val Glu Ser Gly Gly Gly Leu Val Gln Pro Gly Gly Ser Leu Arg Leu
        130                 135                 140

Ser Cys Ala Ala Ser Gly Tyr Thr Phe Thr Asn Tyr Gly Met Asn Trp
145                 150                 155                 160

Val Arg Gln Ala Pro Gly Lys Gly Leu Glu Trp Val Gly Trp Ile Asn
                165                 170                 175

Thr Tyr Thr Gly Glu Pro Thr Tyr Ala Ala Asp Phe Lys Arg Arg Phe
            180                 185                 190

Thr Phe Ser Leu Asp Thr Ser Lys Ser Thr Ala Tyr Leu Gln Met Asn
        195                 200                 205

Ser Leu Arg Ala Glu Asp Thr Ala Val Tyr Tyr Cys Ala Lys Tyr Pro
        210                 215                 220

His Tyr Tyr Gly Ser Ser His Trp Tyr Phe Asp Val Trp Gly Gln Gly
225                 230                 235                 240

Thr Leu Val Thr Val Ser Ser Ala Ser Thr Lys Gly Pro Ser Val Phe

239

                    245                          250                          255

Pro Leu Ala Pro Ser Ser Lys Ser Thr Ser Gly Gly Thr Ala Ala Leu
            260                  265              270

Gly Cys Leu Val Lys Asp Tyr Phe Pro Glu Pro Val Thr Val Ser Trp
            275              280              285

Asn Ser Gly Ala Leu Thr Ser Gly Val His Thr Phe Pro Ala Val Leu
            290              295              300

Gln Ser Ser Gly Leu Tyr Ser Leu Ser Ser Val Val Thr Val Pro Ser
305              310              315              320

Ser Ser Leu Gly Thr Gln Thr Tyr Ile Cys Asn Val Asn His Lys Pro
            325              330              335

Ser Asn Thr Lys Val Asp Lys Lys Val Glu Pro Lys Ser Cys Asp Lys
            340              345              350

Thr His Thr Cys Pro Pro Cys Pro Ala Pro Glu Ala Ala Gly Gly Pro
            355              360              365

Ser Val Phe Leu Phe Pro Pro Lys Pro Lys Asp Thr Leu Met Ile Ser
370              375              380

Arg Thr Pro Glu Val Thr Cys Val Val Val Asp Val Ser His Glu Asp
385              390              395              400

Pro Glu Val Lys Phe Asn Trp Tyr Val Asp Gly Val Glu Val His Asn
            405              410              415

Ala Lys Thr Lys Pro Arg Glu Glu Gln Tyr Asn Ser Thr Tyr Arg Val
            420              425              430

Val Ser Val Leu Thr Val Leu His Gln Asp Trp Leu Asn Gly Lys Glu
            435              440              445

Tyr Lys Cys Lys Val Ser Asn Lys Ala Leu Pro Ala Pro Ile Glu Lys
            450              455              460

Thr Ile Ser Lys Ala Lys Gly Gln Pro Arg Glu Pro Gln Val Tyr Thr
465              470              475              480

Leu Pro Pro Ser Arg Glu Glu Met Thr Lys Asn Gln Val Ser Leu Thr
            485              490              495

Cys Leu Val Lys Gly Phe Tyr Pro Ser Asp Ile Ala Val Glu Trp Glu
                500                 505             510

Ser Asn Gly Gln Pro Glu Asn Asn Tyr Lys Thr Thr Pro Pro Val Leu
            515                 520             525

Asp Ser Asp Gly Ser Phe Phe Leu Tyr Ser Lys Leu Thr Val Asp Lys
            530                 535             540

Ser Arg Trp Gln Gln Gly Asn Val Phe Ser Cys Ser Val Met His Glu
545                 550                 555                 560

Ala Leu His Asn His Tyr Thr Gln Lys Ser Leu Ser Leu Ser Pro Gly
                565                 570                 575

Lys


<210> 75
<211> 334
<212> PRT
<213> Artificial Sequence

<220>
<223> Description of Artificial Sequence: Synthetic
      polypeptide

<400> 75
Asp Ile Gln Met Thr Gln Ser Pro Ser Ser Leu Ser Ala Ser Val Gly
1                   5                   10                  15

Asp Arg Val Thr Ile Thr Cys Arg Ala Ser Glu Asp Ile Tyr Ser Asn
                20                  25                  30

Leu Ala Trp Tyr Gln Gln Lys Pro Gly Lys Ala Pro Lys Leu Leu Ile
            35                  40                  45

Tyr Asp Thr Asn Asn Leu Ala Asp Gly Val Pro Ser Arg Phe Ser Gly
        50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Asn Asn Tyr Pro Pro
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Asp Ile Gln Met Thr Gln Ser Pro

```
              115                    120                    125

        Ser Ser Leu Ser Ala Ser Val Gly Asp Arg Val Thr Ile Thr Cys Ser
            130                    135                    140

        Ala Ser Gln Asp Ile Ser Asn Tyr Leu Asn Trp Tyr Gln Gln Lys Pro
        145                150                155                    160

        Gly Lys Ala Pro Lys Val Leu Ile Tyr Phe Thr Ser Ser Leu His Ser
                        165                170                    175

        Gly Val Pro Ser Arg Phe Ser Gly Ser Gly Ser Gly Thr Asp Phe Thr
                    180                    185                190

        Leu Thr Ile Ser Ser Leu Gln Pro Glu Asp Phe Ala Thr Tyr Tyr Cys
                    195                    200                    205

        Gln Gln Tyr Ser Thr Val Pro Trp Thr Phe Gly Gln Gly Thr Lys Val
            210                    215                    220

        Glu Ile Lys Arg Thr Val Ala Ala Pro Ser Val Phe Ile Phe Pro Pro
        225                    230                    235                240

        Ser Asp Glu Gln Leu Lys Ser Gly Thr Ala Ser Val Val Cys Leu Leu
                    245                    250                    255

        Asn Asn Phe Tyr Pro Arg Glu Ala Lys Val Gln Trp Lys Val Asp Asn
                    260                    265                    270

        Ala Leu Gln Ser Gly Asn Ser Gln Glu Ser Val Thr Glu Gln Asp Ser
                    275                    280                    285

        Lys Asp Ser Thr Tyr Ser Leu Ser Ser Thr Leu Thr Leu Ser Lys Ala
            290                    295                    300

        Asp Tyr Glu Lys His Lys Val Tyr Ala Cys Glu Val Thr His Gln Gly
        305                    310                    315                320

        Leu Ser Ser Pro Val Thr Lys Ser Phe Asn Arg Gly Glu Cys
                        325                    330
```

**Description**

**RELATED APPLICATIONS**

This application claims the benefit of priority to US Provisional Appln. No. 61/721364, filed on November 1, 2012. This application also claims the benefit of priority to US Provisional Appln. No. 61/794231, filed on March 15, 2013. The contents of both the priority applications are hereby incorporated by reference.

**BACKGROUND OF THE INVENTION**

A basic principle of pharmaceutical protein formulations is that certain instabilities, *e.g.*, chemical instability and physical instability, must be overcome. Chemical instabilities often lead to the modification of the protein through bond formation or cleavage. Examples of problems associated with chemical instability include deamidation, racemization, hydrolysis, oxidation, beta elimination and disulfide exchange. While physical instabilities do not lead to covalent changes in proteins, they are just as problematic and difficult to overcome. Physical instabilities involve changes in the higher order structure (secondary and above) of proteins, which can result in denaturation, adsorption to surfaces, aggregation, and/or precipitation (Manning et al. (1989) Pharm. Res. 6:903). For therapeutic proteins, chemical and physical instabilities can create significant challenges in formulating the protein for delivery to a patient. Aggregation is often considered the most common type of physical instability. For example, exposure to hydrophobic interfaces fosters physical instability by alignment of protein molecules at the interface, unfolding the protein and maximizing exposure of hydrophobic residues to air, and initiating aggregation.

Highly concentrated protein formulations, especially those in liquid form, are often desirable for therapeutic purposes since they allow for dosages with smaller volumes, and provide for the possibility of subcutaneous delivery. The development of high protein concentration formulations, however, presents many challenges. For example, a high protein concentration often results in increased protein aggregation, insolubility and degradation (for review, see Shire et al. (2004) J. Pharm. Sci. 93:1390).

To date, the majority of approved therapeutic proteins are antibodies. The development of commercially viable antibody pharmaceutical formulations has not, however, been straightforward despite the fact that antibodies generally have the same structure (see Wang et al. (2007) J. Pharm. Sci. 96:1). Concentration dependent aggregation is considered one of the greatest challenges in formulating antibodies (see Shire et al. (2004) J. Pharm. Sci. 93: 1390).

Dual Variable Domain Immunoglobulin (DVD-Ig™) proteins are multivalent binding proteins that are engineered to combine the function and specificity of two monoclonal antibodies into one molecular entity (See Wu et al., US Patent No. 7,612,181). Given the multivalent nature of DVD-Ig proteins, these molecules hold tremendous promise as therapeutics. However, DVD-Ig proteins present a new formulation challenge given their much larger size (approximately 200 kDa) and complexity, compared to antibodies.

**SUMMARY OF THE INVENTION**

While the majority of Dual Variable Domain Immunoglobulin (DVD-Ig™) proteins are prone to destabilization (*e.g.*, aggregation) in aqueous formulations, a subset of DVD-Ig proteins can be stably formulated. Some DVD-Ig proteins in the aqueous state suffer from certain problems, such as aggregation and/or fragmentation of the DVD-Ig protein monomer. Unpredictably, a subset of DVD-Ig proteins can be stably formulated in the aqueous state even at high concentrations. Such stable DVD-Ig proteins are referred to herein as Aqueous Stable Dual Variable Domain Immunoglobulin proteins or "AS-DVD-Ig" proteins.

In certain embodiments, the disclosure provides stable aqueous formulations comprising AS-DVD-Ig proteins, including high concentration AS-DVD-Ig formulations. AS-DVD-Ig proteins are a subpopulation of DVD-Ig proteins characterized by their ability to remain stable, *e.g.*, in concentrations 50 mg/ml or greater, during storage (*e.g.*, exhibit an aggregation increase of less than 3 % (determined by size exclusion chromatography (SEC)) following accelerated storage (40°C) in an aqueous formulation at a concentration of at least 50 mg/ml). In certain embodiments, the AS-DVD-Ig protein is characterized as a DVD-Ig protein having less than about 15% loss in relative percentage monomers as determined by size exclusion chromatography (SEC) when formulated in a histidine or citrate phosphate buffer at a concentration of at least about 50 mg/ml, following 14 days storage at about 40 °C. In certain embodiments, the AS-DVD-Ig protein is characterized as a DVD-Ig protein having less than about 10% loss in relative percentage monomers as determined by SEC when formulated in a histidine or citrate phosphate buffer at a concentration of at least about 50 mg/ml, following 14 days storage at about 40 °C. In certain embodiments, the AS-DVD-Ig protein is characterized as a DVD-Ig protein having less than about 5% loss in relative percentage monomers as determined by SEC when formulated in a histidine or citrate phosphate buffer at a concentration of at least about 50 mg/ml, following 14 days storage at about 40 °C. In certain embodiments, an AS-DVD-Ig protein is characterized as having less than 10% aggregation as determined by SEC when formulated in a citrate phosphate buffer at a concentration of at least 50 mg/ml following 14 days of storage at 40°C. In certain embodiments, the AS-DVD-Ig protein is characterized as having 6% or less aggregation as determined by SEC following 14 days of storage at 40°C, wherein the AS-DVD-Ig protein at a concentration of at least 50 mg/ml is stored in a citrate phosphate buffer or a histidine buffer. In certain embodiments, the AS-DVD-Ig protein is characterized as having 5% or less aggregation as determined by SEC following 14 days of storage at 40°C, wherein the AS-DVD-Ig protein at a concentration of at least 50 mg/ml is stored in a citrate phosphate buffer or a histidine buffer. In certain embodiments, the AS-DVD-Ig protein is characterized as having 4% or less aggregation as determined by SEC following 14 days of storage at 40°C, wherein the AS-DVD-Ig protein at a concentration of at least 50 mg/ml is stored in a citrate phosphate buffer or a histidine buffer. In certain embodiments, the AS-DVD-Ig protein is characterized as having 3% or less aggregation as determined by SEC following 14 days of storage at 40°C, wherein the AS-DVD-Ig protein at a concentration of at least 50 mg/ml is stored in a citrate phosphate buffer or a histidine buffer. In certain embodiments, the AS-DVD-Ig protein is characterized as having 2% or less aggregation as determined by SEC following 14 days of storage at 40°C, wherein the AS-DVD-Ig protein at a concentration of at least 50 mg/ml is stored in a citrate phosphate buffer or a histidine buffer. In certain embodiments, the AS-DVD-Ig protein is characterized as having 1 % or less aggregation as determined by SEC following 14 days of storage at 40°C, wherein the AS-DVD-Ig protein at a concentration of at least 50 mg/ml is stored in a citrate phosphate buffer or a histidine buffer. In certain embodiments, the AS-DVD-Ig protein is characterized as a DVD-Ig protein having about a 10% relative (rel.) peak area or less change in monomers at about 40 °C after 21 days of storage at a concentration of about 100 mg/ml in an aqueous formulation at a pH between about 5.5 to about 6.5. In other embodiments, the AS-DVD-Ig protein is characterized as a DVD-Ig protein having about a 1% rel. peak area or less change in monomers at about 5 °C after 21 days of storage at a concentration of about 100 mg/ml at a pH between about 5.5 to about 6.5 in an aqueous formulation. Examples of AS-DVD-Ig proteins that may be included in the formulations of the disclosure include, but are not limited to, an AS-DVD-Ig protein having binding specificity for IL4 and IL13; IL1$\alpha$ and IE1$\beta$; TNF$\alpha$ and IL17; and DLL4 and VEGF.

In certain embodiments, the formulation comprises less than about 10% aggregate AS-DVD-Ig protein. In certain embodiments, the formulation comprises less than about 9% aggregate AS-DVD-Ig protein. In certain embodiments, the formulation comprises less than about 8% aggregate AS-DVD-Ig protein. In certain embodiments, the formulation comprises less than about 7% aggregate AS-DVD-Ig protein. In certain embodiments, the formulation comprises less than about 6% aggregate AS-DVD-Ig protein. In certain embodiments, the formulation comprises less than about 5% aggregate AS-DVD-Ig protein. In certain embodiments, the formulation comprises less than about 4% aggregate AS-DVD-Ig protein. In a further embodiment, the formulation comprises less than about 3% aggregate AS-DVD-Ig protein. Aggregation may be determined by SEC analysis.

In certain embodiments, the AS-DVD-Ig protein is characterized as a DVD-Ig protein having a about 10% relative (rel.) peak area or less change in monomers at about 40 °C after 21 days of storage at a concentration of about 100 mg/ml in an aqueous formulation at a pH between about 5.0 to about 6.5, *e.g.*, about 5.5 to about 6.0. In certain embodiments, the AS-DVD-Ig protein is characterized as a DVD-Ig protein having a about 1% rel. peak area or less change in monomers at about 5 °C after 21 days of storage at a concentration of about 100 mg/ml at a pH between about 5.0 to about 6.5, *e.g.*, about 5.5 to about 6.0, in an aqueous formulation.

In certain embodiments, the disclosure provides an aqueous formulation comprising an AS-DVD-Ig protein and a buffer. For example, the disclosure provides an aqueous formulation comprising an AS-DVD-Ig protein and a buffer having a molarity of about 5 to about 50 mM, wherein the formulation has a pH of about 4.5 to about 7.5, *e.g.*, a pH of about 5 to about 6.5. In certain embodiments, the AS-DVD-Ig protein is characterized as a DVD-Ig protein having less than about 15% loss in relative percentage monomers as determined by size exclusion chromatography (SEC) when formulated in a histidine or citrate phosphate buffer at a concentration of at least about 50 mg/ml, following 14 days storage at about 40 degrees C. In certain embodiments, the AS-DVD-Ig protein is characterized as a DVD-Ig protein having less than about 10% loss in relative percentage monomers as determined by SEC when formulated in a histidine or citrate phosphate buffer at a concentration of at least about 50 mg/ml, following 14 days storage at about 40 degrees C. In certain embodiments, the AS-DVD-Ig protein is characterized as a DVD-Ig protein having less than about 5% loss in relative percentage monomers as determined by SEC when formulated in a histidine or citrate phosphate buffer at a concentration of at least about 50 mg/ml, following 14 days storage at about 40 degrees C. In certain embodiments, the formulation comprises about 6% or less aggregation as determined by SEC analysis. In other embodiment, the formulation comprises about 5% or less aggregation as determined by SEC analysis. In certain embodiments, the AS-DVD-Ig protein is characterized as a DVD-Ig protein having about a 10% relative (rel.) peak area or less change in monomers at about 40 °C after 21 days of storage at a concentration of about 100 mg/ml in an aqueous formulation at a pH between about 5.5 to about 6.5. In other embodiments, the AS-DVD-Ig protein is characterized as a DVD-Ig protein having about a 1% rel. peak area or less change in monomers at about 5 °C after 21 days of storage at a concentration of about 100 mg/ml at a pH between about 5.5 to about 6.5 in an aqueous formulation. In certain embodiments, the formulation comprises about 1 to about 250 mg/ml, about 1 to 200 mg/ml, about 10 to about 230 mg/ml, about 20 to about 210 mg/ml, about 30 to about 190 mg/ml, about 40 to about 170 mg/ml, about 50 to about 150 mg/ml, about 60 to about 130 mg/ml, about 70 to about 110 mg/ml, or about 80 to about 105 mg/ml of the AS-DVD-Ig protein. In certain embodiments, the buffer used in the formulation is acetate, histidine, glycine, arginine, phosphate, citrate, or citrate / phosphate buffer. In certain embodiments, the molarity of the buffer ranges from 5 to 50 mM, *e.g.*, 10 to 20 mM.

In certain embodiments, the disclosure provides an aqueous formulation comprising an AS-DVD-Ig protein, a buffer and a surfactant. For example, the disclosure provides an aqueous formulation comprising an AS-DVD-Ig protein, a buffer having a molarity of about 5 to about 50 mM, and a surfactant, wherein the formulation has a pH of about 4.5 to about 7.5. In certain embodiments, the formulation comprises about 1 to about 250 mg/ml, about 10 to about 230 mg/ml, about 20 to about 210 mg/ml, about 30 to about 190 mg/ml, about 40 to about 170 mg/ml, about 50 to about 150 mg/ml, about 60 to about 130 mg/ml, about 70 to about 110 mg/ml, or about 80 to about 105 mg/ml of the AS-DVD-Ig protein. In certain embodiments, the surfactant is a polysorbate, *e.g.*, polysorbate 80 or polysorbate 20. In certain embodiments, the concentration of polysorbate in the formulation is about 0.001% to about 1%, about 0.005% to about 0.05%, about 0.01% to about 0.05%, or about 0.1%. In certain embodiments, the concentration of polysorbate 80 or polysorbate 20 in the formulation is about 0.005% to about 0.02%. In certain embodiments, the buffer used in the formulation is acetate, histidine, glycine, arginine, phosphate, citrate, or citrate / phosphate buffer. In certain embodiments, the molarity of the buffer ranges from 5 to 50 mM, *e.g.*, 10 to 20 mM.

In certain embodiments, the disclosure includes an aqueous formulation comprising an AS-DVD-Ig protein, a buffer, and a polyol. For example, the disclosure provides an aqueous formulation comprising an AS-DVD-Ig protein, a buffer having a molarity of about 5 to about 50 mM, and a polyol, wherein the formulation has a pH of about 4.5 to about 7.5. In certain embodiments, the formulation comprises about 1 to about 250 mg/ml, about 10 to about 230 mg/ml, about 20 to about 210 mg/ml, about 30 to about 190 mg/ml, about 40 to about 170 mg/ml, about 50 to about 150 mg/ml, about 60 to about 130 mg/ml, about 70 to about 110 mg/ml, or about 80 to about 105 mg/ml of the AS-DVD-Ig protein. In certain embodiments, the polyol is sorbitol. In certain embodiments, the concentration of sorbitol in the formulation is about 20 to about 60 mg/ml sorbitol, about 25 to about 55 mg/ml, about 30 to about 50 mg/ml, or about 35 to about 45 mg/ml. In certain embodiments, the polyol is sucrose. In certain embodiments, the concentration of sucrose in the formulation is about 60 to about 100 mg/ml, about 65 to about 95 mg/ml, about 70 to about 90 mg/ml, or about 75 to about 85 mg/ml. In certain embodiments, the polyol is mannitol. In certain embodiments, the concentration of mannitol in the formulation is about 10 to about 100 mg/ml, or about 20 to about 80, about 20 to about 70, about 30 to about 60, or about 30 to about 50 mg/ml. In certain embodiments, the buffer used in the formulation is acetate, histidine, glycine, arginine, phosphate, citrate, or citrate / phosphate buffer. In certain embodiments, the molarity of the buffer ranges from 5 to 50 mM, *e.g.*, 10 to 20 mM.

In certain embodiments, the disclosure provides an aqueous formulation comprising an AS-DVD-Ig protein, a buffer, a polyol, and a surfactant. For example, the disclosure provides an aqueous formulation comprising an AS-DVD-Ig protein, a buffer having a molarity of about 5 to about 50 mM, a surfactant, and a polyol, wherein the formulation has a pH of about 4.5 to about 7.5. In certain embodiments, the formulation comprises about 1 to about 250 mg/ml, about 10 to about 230 mg/ml, about 20 to about 210 mg/ml, about 30 to about 190 mg/ml, about 40 to about 170 mg/ml, about 50 to about 150 mg/ml, about 60 to about 130 mg/ml, about 70 to about 110 mg/ml, or about 80 to about 105 mg/ml of the AS-DVD-Ig. In certain embodiments, the polyol is sorbitol. In certain embodiment, the sorbitol concentration in the formulation is about 20 to about 60 mg/ml, about 25 to about 55 mg/ml, about 30 to about 50 mg/ml, or about 35 to about 45 mg/ml. In certain embodiments, the polyol is sucrose. In certain embodiments, the concentration of sucrose in the formulation is about 60 to about 100 mg/ml, about 65 to about 95 mg/ml, about 70 to about 90 mg/ml, or about 75 to about 85 mg/ml. In certain embodiments, the polyol is mannitol. In certain embodiments, the concentration of mannitol in the formulation is about 10 to about 100 mg/ml, or about 20 to about 80, about 20 to about 70, about 30 to about 60, or about 30 to about 50 mg/ml. In certain embodiments, the surfactant is a polysorbate, *e.g.*, polysorbate 80 or polysorbate 20. In certain embodiments, the concentration of polysorbate in the formulation is about 0.001% to about 1%, about 0.005% to about 0.05%, about 0.01% to about 0.05%, or about 0.1%. In certain embodiments, the concentration of polysorbate 80 or polysorbate 20 in the formulation is about 0.005% to about 0.02%. In certain embodiments, the buffer used in the formulation is acetate, histidine, glycine, arginine, phosphate, citrate, or citrate / phosphate buffer. In certain embodiments, the molarity of the buffer ranges from 5 to 50 mM, *e.g.*, 10 to 20 mM.

In certain embodiment, the disclosure provides a formulation comprising an AS-DVD-Ig protein, a polyol (*e.g.*, sorbitol, mannitol, or sucrose), a buffer (*e.g.*, acetate, histidine, glycine, arginine, phosphate, citrate, or citrate / phosphate), and a surfactant (*e.g.*, a polysorbate), wherein said formulation has a pH of about 5 to about 7, and wherein the AS-DVD-Ig protein is characterized as a DVD-Ig protein having less than about 15% loss in relative percentage monomers as determined by SEC when formulated in a histidine or citrate phosphate buffer at a concentration of about 60 mg/ml following 14 days storage at about 40 degrees C. In certain embodiments, the polysorbate is polysorbate 80 or polysorbate 20. In certain embodiments, the concentration of polysorbate 80 or polysorbate 20 is about 0.005% to about 0.02%.

In certain embodiments, the disclosure provides a formulation comprising an AS-DVD-Ig protein, a polyol, histidine buffer, and a polysorbate, wherein said formulation has a pH of about 5 to about 7, and wherein the AS-DVD-Ig protein is characterized as having 6% aggregation or less as determined by SEC, where the AS-DVD-Ig protein is formulated in a citrate phosphate buffer or histidine buffer at a concentration of at least 60 mg/ml, following 14 days storage at 40° C.

In certain embodiments, the disclosure provides a formulation comprising an AS-DVD-Ig protein and a buffer having a molarity of about 5 to about 50 mM, wherein the AS-DVD-Ig protein is characterized as a DVD-Ig protein having less than about 10% loss in relative percentage monomers as determined by SEC when formulated in a histidine or citrate phosphate buffer at a concentration of about 60 mg/ml, following 14 days storage at about 40 degrees C, and the formulation has a pH of 4.5 to 7.5. In certain embodiments, the formulation further comprises a surfactant, a polyol, or combinations thereof.

The disclosure is also based, in part, on the surprising discovery that while the majority of DVD-Ig™ proteins are prone to destabilization in a lyophilized state, a subset of DVD-Ig proteins are able to be stably formulated in a lyophilized form. Such stable DVD-Ig proteins are referred to herein as Lyophilized Stable Dual Variable Domain Immunoglobulin proteins or "LS-DVD-Ig" proteins. Formulations wherein the DVD-Ig protein was in the lyophilized state suffered from certain problems such as aggregation and/or fragmentation of the DVD-Ig protein monomer. Unpredictably, a subset of DVD-Ig proteins can be stably formulated in the lyophilized state even at high concentrations.

In certain embodiments, the disclosure is a lyophilized formulation comprising a Lyophilized-Stable DVD Immunoglobulin (LS-DVD-Ig) protein, wherein when said formulation is reconstituted said formulation comprises about 1 to about 100 mg/ml of the LS-DVD-Ig protein, about 10 to about 50 mM of a buffer, a polyol, about 0.01 to about 0.2 mg/ml of a polysorbate, and has a pH of about 5 to about 7, *e.g.*, about 5.5 to about 6.5. In certain embodiments, the LS-DVD-Ig protein has more than 10% rel. peak area change in monomers observed, following accelerated storage at a pH between about 5.5 and about 6.5 in an aqueous formulation for 21 days at about 40 °C, when formulated at a concentration over about 100 mg/ml.

In certain embodiments, the LS-DVD-Ig protein has more than about 10% rel. peak area change in monomers observed, following accelerated storage at a pH between about 5.0 to about 6.5, *e.g.*, about 5.5 to about 6.0, in an aqueous formulation for 21 days at about 40 °C, when formulated at a concentration of about 100 mg/ml or more.

In certain embodiments, the disclosure provides a lyophilized formulation prepared by lyophilizing an aqueous formulation comprising a buffer have a molarity (*e.g.*, histidine, succinate or citrate and/or phosphate) of about 5 to about 50 mM, a surfactant, and a polyol (*e.g.*, mannitol, sorbitol, sucrose, or trehalose), wherein the formulation has a pH of about 4.5 to about 7.5, *e.g.*, about 5.5 to about 6.5.

In certain embodiments, the disclosure provides LS-DVD-Ig proteins or AS-DVD-Ig proteins that are stable in formulations having a pH of about 4.5 to about 7.5. In certain embodiments, the formulation has a pH of 5 to 6.5. In certain embodiments, the formulation has a pH of about 5.7 to about 6.3. In certain embodiments, the formulation the formulation of the disclosure has a pH of about 5.5 to about 6.5. In certain embodiments, the formulation of the disclosure has a pH of 5.8 to about 6.2, or a pH of 6.

In certain embodiments, the disclosure provides a formulation comprising an AS-DVD-Ig protein or an LS-DVD-Ig protein, a polyol, histidine buffer, and a polysorbate, wherein said formulation has a pH of about 5 to about 7, and wherein the AS-DVD-Ig protein or an LS-DVD-Ig protein is characterized as having 15% aggregation or less as determined by SEC, where the AS-DVD-Ig protein or an LS-DVD-Ig protein is formulated in a citrate phosphate buffer or histidine buffer at a concentration of at least 60 mg/ml, following 14 days storage at 40°C. Such formulations may be either in a lyophilized or an aqueous state, as AS-DVD-Ig protein or an LS-DVD-Ig protein identified as having 15% aggregation or less as determined by SEC, where the AS-DVD-Ig protein or an LS-DVD-Ig protein is formulated in a citrate phosphate buffer or a histidine buffer at a concentration of at least 60 mg/ml, following 14 days storage at 40°C.

One advantage of the compositions of the disclosure is that the AS-DVD-Ig protein or LS-DVD-Ig protein can be stably formulated in liquid form at a high concentration. In certain embodiments, the AS-DVD-Ig protein or LS-DVD-Ig protein has a concentration of about 1 to about 200 mg/ml. In certain embodiments, the AS-DVD-Ig protein or LS-DVD-Ig protein has a concentration of about 20 to about 100 mg/ml. In certain embodiments, the formulation comprises about 1 to about 250 mg/ml, about 10 to about 230 mg/ml, about 20 to about 210 mg/ml, about 30 to about 190 mg/ml, about 40 to about 170 mg/ml, about 50 to about 150 mg/ml, about 60 to about 130 mg/ml, about 70 to about 110 mg/ml, or about 80 to about 105 mg/ml of the AS-DVD-Ig protein or LS-DVD-Ig.

Examples of buffers that may be used in the formulations of the disclosure include, but are not limited to, acetate, histidine, glycine, arginine, phosphate, and citrate. In certain embodiments, the molarity of the buffer in the formulation is about 5 to about 50 mM. In certain embodiments, the buffer molarity is about 10 mM to about 20 mM.

Examples of polyols that may be used in the formulations of the disclosure include, but are not limited to, sorbitol, mannitol, and sucrose. In certain embodiments, the polyol is sorbitol. In certain embodiments, about 30 to about 50 mg/ml of sorbitol is used in the formulation. In certain embodiments, the polyol is sucrose. In certain embodiments, about 70 to about 90 mg/ml of sucrose is used in the formulation. In a further embodiment, the polyol is mannitol. In certain embodiments, about 30 to about 50 mg/ml of mannitol is used in the formulation.

Examples of surfactants that may be used in the formulations of the disclosure include, but are not limited to, polysorbates and poloxamers. In certain embodiments, the surfactant is a polysorbate, examples of which are polysorbate 80 and polysorbate 20. Other examples include poloxamer Pluronic F-68, albumin, lecithin, and cyclodextrins. In certain embodiments, the polysorbate has a concentration of about 0.05 mg/ml to about 2mg/ml. In a further embodiment, the polysorbate has a concentration of about 0.01 to about 0.2 mg/ml.

In certain embodiments, the AS-DVD-Ig protein or LS-DVD-Ig protein comprises first and second polypeptide chains, each independently comprising VD1-(X1)n-VD2-C-(X2)n, wherein VD1 is a first variable domain; VD2 is a second variable domain; C is a constant domain; X1 is a linker with the proviso that it is not CH1; X2 is an Fc region; n is 0 or 1, wherein the VD1 domains on the first and second polypeptide chains form a first functional target binding site and the VD2 domains on the first and second polypeptide chains form a second functional target binding site. In a further embodiment, the first polypeptide chain comprises a first VD1-(X1)n-VD2-C-(X2)n, wherein VD1 is a first heavy chain variable domain; VD2 is a second heavy chain variable domain; C is a heavy chain constant domain; X1 is a linker with the proviso that it is not CH1; X2 is an Fc region; n is 0 or 1, and wherein the second polypeptide chain comprises a second VD1-(X1)n-VD2-C-(X2)n, wherein VD1 is a first light chain variable domain; VD2 is a second light chain variable domain; C is a light chain constant domain; X1 is a linker with the proviso that it is not CH1; X2 does not comprise an Fc region; n is 0 or 1, wherein the VD1 domains on the first and second polypeptide chains form a first functional target binding site and the VD2 domains on the first and second polypeptide chains form a second functional target binding site. In certain embodiments, the two first polypeptide chains and two second polypeptide chains, wherein the binding protein comprises four functional target binding sites. In certain embodiments, X1 is not CL.

In certain embodiments, the AS-DVD-Ig protein or LS-DVD-Ig protein comprises a polypeptide chain comprising VD1-(X1)n-VD2-C-(X2)n, wherein VD1 is a first variable domain, VD2 is a second variable domain, C is a constant domain, X1 represents an amino acid or polypeptide, X2 represents an Fc region and n is 0 or 1.

In certain embodiments, the AS-DVD-Ig protein or LS-DVD-Ig protein used in the compositions and methods of the disclosure comprises four polypeptide chains, wherein two polypeptide chains comprise VD1-(X1)n-VD2-C-(X2)n, wherein VD1 is a first heavy chain variable domain, VD2 is a second heavy chain variable domain, C is a heavy chain constant domain, X1 is a linker with the proviso that it is not CH1, and X2 is an Fc region; and two polypeptide chains comprise VD1-(X1)n-VD2-C-(X2)n, wherein VD1 is a first light chain variable domain, VD2 is a second light chain variable domain, C is a light chain constant domain, X1 is a linker with the proviso that it is not CH1, and X2 does not comprise an Fc region; and n is 0 or 1; and wherein said four polypeptide chains of said binding protein form four functional antigen binding sites.

In certain embodiments, the AS-DVD-Ig protein or LS-DVD-Ig protein comprises a polypeptide chain wherein the polypeptide chain comprises VD1-(X1)n-VD2-C-(X2)n, wherein VD1 is a first heavy chain variable domain; VD2 is a second heavy chain variable domain; C is a heavy chain constant domain; X1 is a linker with the proviso that it is not CH1; X2 is an Fc region; and n is 0 or 1. In certain embodiments, the AS-DVD-Ig protein or LS-DVD-Ig protein comprises a polypeptide chain, wherein the polypeptide chain comprises VD1-(X1)n-VD2-C-(X2)n, wherein VD1 is a first light chain variable domain; VD2 is a second light chain variable domain; C is a light chain constant domain; X1 is a linker with the proviso that it is not a CH1 or CL; X2 does not comprise an Fc region; and n is 0 or 1. In a further embodiment, (X1)n on the heavy and/or light chain is (X1)0 and/or (X2)n on the heavy and/or light chain is (X2)0.

In certain embodiments, the AS-DVD-Ig protein or LS-DVD-Ig protein comprises first and second polypeptide chains, wherein the first polypeptide chain comprises a first VD1-(X1)n-VD2-C-(X2)n, wherein VD1 is a first heavy chain variable domain; VD2 is a second heavy chain variable domain; C is a heavy chain constant domain; X1 is a first linker with the proviso that it is not CH2; X2 is an Fc region; n is 0 or 1; and wherein the second polypeptide chain comprises a second VD1-(X1)n-VD2-C-(X2)n, wherein VD1 is a first light chain variable domain; VD2 is a second light chain variable domain; C is a light chain constant domain; X1 is a second linker with the proviso that it is not CH1 or CL; X2 does not comprise an Fc region; and n is 0 or 1.

In certain embodiments, the VD1 of the first polypeptide chain and the VD1 of the second polypeptide chain are from different first and second parent antibodies, respectively, or binding portions thereof. In certain embodiments, the VD2 of the first polypeptide chain and the VD2 of the second polypeptide chain are from different first and second parent antibodies, respectively, or binding portions thereof. In certain embodiments, the first and the second parent antibodies bind different epitopes on the same target or different targets. In certain embodiments, the first parent antibody or binding portion thereof binds the first target with a potency that is different from the potency with which the second parent antibody or binding portion thereof binds the second target. In certain embodiments, the first parent antibody or binding portion thereof binds the first target with an affinity different from the affinity with which the second parent antibody or binding portion thereof binds the second target.

In certain embodiments, the AS-DVD-Ig protein or LS-DVD-Ig protein comprise, two first polypeptide chains and two second polypeptide chains.

In certain embodiments, the AS-DVD-Ig protein or LS-DVD-Ig protein comprises first and second polypeptide chains, each independently comprising VD1-(X1)n-VD2-C-(X2)n, wherein VD1 is a first variable domain; VD2 is a second variable domain; C is a constant domain; X1 is a linker with the proviso that it is not CH1; X2 is an Fc region; n is 0 or 1, and wherein the VD1 domains on the first and second polypeptide chains form a first functional target binding site and the VD2 domains on the first and second polypeptide chains form a second functional target binding site. IN a further embodiment, the first polypeptide chain comprises a first VD1-(X1)n-VD2-C-(X2)n, wherein VD1 is a first heavy chain variable domain; VD2 is a second heavy chain variable domain; C is a heavy chain constant domain; X1 is a linker with the proviso that it is not CH1; X2 is an Fc region; n is 0 or 1, and wherein the second polypeptide chain comprises a second VD1-(X1)n-VD2-C-(X2)n, wherein VD1 is a first light chain variable domain; VD2 is a second light chain variable domain; C is a light chain constant domain; X1 is a linker with the proviso that it is not CH1; X2 does not comprise an Fc region; n is 0 or 1, wherein the VD1 domains on the first and second polypeptide chains form a first functional target binding site and the VD2 domains on the first and second polypeptide chains form a second functional target binding site.

In certain embodiments, the formulation of the disclosure comprises a DVD-Ig protein comprising a heavy or light chain amino acid sequence as set forth in Table 58 or 65.

In certain embodiments, the formulation of the disclosure comprises a DVD-Ig protein comprising a heavy or light chain variable region amino acid sequence as set forth in Table 58 or 65 (SEQ ID NOs: 28-75). Alternatively, the formulation of the disclosure comprises a DVD-Ig protein comprising CDRs as set forth in the heavy or light chain variable region amino acid sequences as set forth in Table 58 or 65 (SEQ ID NOs: 28-75).

In certain embodiments, the formulation of the disclosure comprises an anti-TNF/IL-17 DVD-Ig protein. In certain embodiments, the anti-TNF/IL-17 DVD-Ig protein comprises a heavy and light chain sequences having an amino acid sequence as set forth in SEQ ID NOs: 62 and 63, respectively.

In certain embodiments, the formulation of the disclosure comprises an anti-IL1α/IL1β DVD-Ig. In certain embodiments, the anti-IL1α/IL1β DVD-Ig protein comprises a heavy and light chain sequences having an amino acid sequence as set forth in SEQ ID NOs: 66 and 67, respectively.

In certain embodiments, the DVD-Ig protein used in the formulation of the disclosure binds one of the following target combinations (in either target order): CD20/CD80, VEGF/Her2, TNF/RANKL, TNF/DKK, CD20/RANKL, DLL4/PLGF, TNF/SOST (S2), IL-9(S2)/IgE, IL-12/IL-18, TNF/IL-17, TNF/PGE2, IL1α/IL1β, or DLL4/VEGF.

In certain embodiments, the formulation of the disclosure is a pharmaceutical formulation, including a pharmaceutical aqueous formulation or a pharmaceutical lyophilized formulation.

Also included in the disclosure are methods of making and using AS-DVD-Ig protein or LS-DVD-Ig protein formulations.

In certain embodiments, the formulations of the disclosure are used for treating a disorder in a subject.

A further embodiment of the disclosure is a method of identifying either an AS-DVD-Ig protein or an LS-DVD-Ig protein. Such methods include aggregation testing (*e.g.*, by SEC analysis) following accelerated storage (*e.g.*, 14 days at about 40 degrees C) of a liquid formulation comprising the DVD-Ig protein, a citrate/phosphate buffer, and a high concentration of DVD-Ig protein (*e.g.*, about 50 mg/ml or greater).

## BRIEF DESCRIPTION OF DRAWINGS

**Figure 1** shows a graphic description of a comparison of DSC profiles of an IgG1 antibody (Briakinumab) to that of a DVD-Ig protein (TNF/PGE2; DVD-B) showing the difference in three vs. four domain unfolding, respectively. The sample composition of the DVD-Ig solution used was 1 mg/ml DVD-Ig protein, 1 mM ionic strength Histidine pH 6, 1 °C/minute scan rate.

**Figure 2A** shows a graphic description of serum stability of various DVD-Ig proteins.

**Figure 2B** shows the domain orientation concept for different variable domain combinations.

**Figure 3** shows a graphic description of the correlation between pharmacokinetic parameters of different DVD-Ig proteins and high molecular weight (HMW) aggregate formation.

**Figure 4** provides a graphic description of a thermodynamic comparison of 14 monoclonal antibodies and 16 DVD-Ig proteins showing the melting temperatures of the molecules relative to the number of molecules (y axis) for each temperature. The mean onset melting temperature for the antibodies was 59.2 °C $\pm$ 3.4 °C. The mean onset melting temperature for the DVD-Ig proteins was 53.6°C $\pm$ 3.6°C.

**Figure 5** graphically depicts the molar ellipticity in the DVD-Ig proteins as measured using near UV-CD scans between 250-320 nm wavelengths.

## DETAILED DESCRIPTION

### I. Definitions

The term "multivalent binding protein" is used to denote a binding protein comprising two or more target binding sites. The multivalent binding protein may be engineered to have the three or more antigen binding sites, and is generally not a naturally occurring antibody.

The term "multispecific binding protein" refers to a binding protein capable of binding two or more related or unrelated targets. An example of a multivalent binding protein is a Dual Variable Domain (DVD) binding protein, such as a DVD-Ig™. In certain embodiments, DVD binding proteins comprise two or more antigen binding sites and are tetravalent or multivalent binding proteins. DVDs may be monospecific, *i.e.*, capable of binding one target, or multispecific, *i.e.*, capable of binding two or more targets.

The term "Dual Variable Domain Immunoglobulin" or "DVD-Ig™" or "DVD-Ig protein" refers to a DVD binding protein comprising two heavy chain DVD polypeptides and two light chain DVD polypeptides. Each half of a DVD-Ig comprises a heavy chain DVD polypeptide and a light chain DVD polypeptide, and two target binding sites. Each binding site comprises a heavy chain variable domain and a light chain variable domain with a total of 6 CDRs involved in target binding. Each variable domain (VD) in a DVD-Ig protein may be obtained from one or more "parent" monoclonal antibodies (mAbs) that bind one or more desired antigens or epitopes. In certain embodiments, the resulting DVD-Ig molecule retains activities of both parental mAbs. The term "DVD-Ig protein" is inclusive of the terms AS-DVD-Ig protein and LS-DVD-Ig protein described below.

The term "Aqueous Stable Dual Variable Domain Immunoglobulin" or "AS-DVD-Ig" or "AS-DVD-Ig protein" refers to a subset of DVD-Ig proteins that have low aggregation or a low change in monomer content due to physical degradation following stability tests at a given temperature and time period, *e.g.*, 5 °C or 40 °C for 14 to 21 days, at a concentration ranging from about 1 to about 100 mg/ml (*e.g.*, about 1-10 mg/ml or about 50-100 mg/ml) and at a pH between about 5.0 to about 6.5, *e.g,* about 5.5 to about 6.0. Different stability tests may be used to define an AS-DVD-Ig. In certain embodiments, an AS-DVD-Ig protein is defined as a DVD-Ig protein that has about 1% relative peak area or less change in monomers as determined by SEC analysis at about 5 °C after 21 days of storage at a concentration of about 50 mg/ml to about 100 mg/ml and at a pH between about 5.0 to about 6.5, *e.g,* about 5.5 to about 6.0, in a buffered aqueous formulation. In certain embodiments, an AS-DVD-Ig protein has 10% relative (rel.) peak area or less change in monomers as determined by SEC analysis following accelerated storage for 21 days at about 40 °C at a concentration of about 50 mg/ml to about 100 mg/ml and at a pH between about 5.0 to about 6.5, *e.g,* about 5.5 to about 6.0, in a buffered aqueous formulation. In certain embodiments, stability testing for AS-DVD-Ig proteins may be performed by testing the stability, *e.g.*, loss of monomers, of a solution having a DVD-Ig protein concentration of 50 to 100 mg/ml in a citrate phosphate buffer or histidine buffer at a pH between 5.0 - 6.5, *e.g,* about 5.5 to about 6.0.

The term "aqueous formulation" refers to a liquid solution in which the solvent is water. In certain embodiments, the term "aqueous formulation" refers to a liquid formulation in which the solvent is water wherein the formulation was not previously lyophilized (*i.e.*, does not result from reconstitution of a lyophilized formulation).

The term "Lyophilized Stable Dual Variable Domain Immunoglobulin" or "LS-DVD-Ig" or "LS-DVD-Ig protein" refers to a subset of DVD-Ig proteins that have low aggregation or elevated levels of change in monomers (*i.e.*, loss of monomers) in the liquid state. Different stability tests may be used to define an LS-DVD-Ig. In certain embodiments, an LS-DVD-Ig protein has more than 10% rel. peak area change in monomers observed, following accelerated storage, *e.g.*, 21 days at about 40 °C, when formulated at a concentration of about 50 to 100 mg/ml at a pH between about 5.0 and about 6.5, *e.g,* about 5.5 to about 6.0, in an aqueous formulation. DVD-Ig proteins may be tested in aqueous formulations containing citrate and phosphate buffer, or histidine buffer. Change in monomers can be determined according to methods known in the art, including, but not limited to, SEC. In certain embodiments, the accelerated storage conditions include storing the DVD-Ig protein in the absence of light at about 40 °C. In certain embodiments, an LS-DVD-Ig protein has 50% rel. peak area or less change in monomers as determined by SEC analysis following accelerated storage for 14 days at about 40 °C, where the LS-DVD-Ig protein is formulated at a concentration of at least 50 mg/ml in a citrate phosphate buffer in an aqueous formulation. In certain embodiments, stability testing for LS-DVD-Ig proteins may be performed by testing the stability, *e.g.*, loss of monomers, of a solution having a DVD-Ig protein concentration of about 50 mg/ml to 100 mg/ml) in a citrate phosphate buffer or histidine buffer at a pH between about 5.0 - about 6.5, *e.g*, about 5.5 to about 6.0.

The term "pharmaceutical formulation" refers to preparations that are in such a form as to permit the biological activity of the active ingredients to be effective and, therefore, may be administered to a subject for therapeutic use. In certain embodiments, the disclosure provides an aqueous pharmaceutical formulation comprising an AS-DVD-Ig. In other embodiments, the disclosure provides a lyophilized pharmaceutical formulation comprising an LS-DVD-Ig.

A "stable" formulation is one in which the DVD-Ig protein therein essentially retains its physical stability and/or chemical stability and/or biological activity upon storage. Various analytical techniques for measuring protein stability are available in the art and are reviewed in, *e.g.*, Peptide and Protein Drug Delivery, pp. 247-301, Vincent Lee Ed., Marcel Dekker, Inc., New York, N.Y., Pubs. (1991) and Jones (1993) Adv. Drug Delivery Rev. 10: 29-90. In certain embodiments, the stability of the DVD-Ig protein is determined according to the percentage of monomer protein in the solution, with a low percentage of degraded (*e.g.*, fragmented) and/or aggregated protein. For example, an aqueous formulation comprising a stable DVD-Ig protein may include at least 95% monomer DVD-Ig protein, *e.g.*, AS-DVD-Ig protein. Alternatively, an aqueous formulation of the disclosure may include no more than 5% aggregate and/or degraded DVD-Ig protein, *e.g.*, AS-DVD-Ig protein.

A DVD-Ig protein "retains its physical stability" in a pharmaceutical formulation if it shows substantially no signs of aggregation, precipitation and/or denaturation upon visual examination of color and/or clarity, or as measured by UV light scattering or by size exclusion chromatography. In certain embodiments of the disclosure, a stable aqueous formulation is a formulation having less than about 10% aggregation, and less than about 5% AS-DVD-Ig protein aggregation in the formulation.

A DVD-Ig protein "retains its chemical stability" in a pharmaceutical formulation if the chemical stability at a given time is such that the DVD-Ig protein is considered to still retain its biological activity as defined below. Chemical stability can be assessed by detecting and quantifying chemically altered forms of the DVD-Ig. Chemical alteration may involve size modifications (*e.g.*, clipping) which can be evaluated using size exclusion chromatography, SDS-PAGE and/or matrix-assisted laser desorption ionization / time of flight mass spectrometry (MALDI/TOF MS), for example. Other types of chemical alternation include charge alteration (*e.g.*, occurring as a result of deamidation), which can be evaluated by, *e.g.*, ion-exchange chromatography.

A DVD-Ig protein "retains its biological activity" in a pharmaceutical formulation, if the protein in a pharmaceutical formulation is biologically active for its intended purpose. For example, biological activity of a DVD-Ig protein is retained if the biological activity of the DVD-Ig protein in the pharmaceutical formulation is within about 30%, about 20%, or about 10% (within the errors of the assay) of the biological activity exhibited at the time the pharmaceutical formulation was prepared (*e.g.*, as determined in an antigen binding assay).

The term "surfactant", as used herein, refers to organic substances having amphipathic structures; namely, they are composed of groups of opposing solubility tendencies, typically an oil-soluble hydrocarbon chain and a water-soluble ionic group. Surfactants can be classified, depending on the charge of the surface-active moiety, into anionic, cationic, and nonionic surfactants. Surfactants are often used as wetting, emulsifying, solubilizing, and dispersing agents for various pharmaceutical compositions and preparations of biological materials. Examples of suitable surfactants include, but are not limited to, sodium lauryl sulfate, polysorbates such as polyoxyethylene sorbitan monooleate, monolaurate, monopalmitate, monostearate or another ester of polyoxyethylene sorbitan (*e.g.*, the commercially available Tweens™, such as, Tween™ 20 and Tween™ 80 (ICI Speciality Chemicals)), sodium dioctylsulfosuccinate (DOSS), lecithin, stearylic alcohol, cetostearylic alcohol, cholesterol, polyoxyethylene ricin oil, polyoxyethylene fatty acid glycerides, poloxamers (*e.g.*, Pluronics F68™ and F108™, which are block copolymers of ethylene oxide and propylene oxide); polyoxyethylene castor oil derivatives or mixtures thereof. In certain embodiments, a formulation of the disclosure comprises Polysorbate 20, Polysorbate 40, Polysorbate 60, or Polysorbate 80.

The term "tonicity modifier" or "tonicity agent" refers to a compound that can be used to adjust the tonicity of a liquid formulation. Examples of tonicity modifiers include glycerin, lactose, mannitol, dextrose, sodium chloride, magnesium sulfate, magnesium chloride, sodium sulfate, sorbitol, trehalose, sucrose, raffinose, maltose and others known to those or ordinary skill in the art.

The term "polyol" refers to a substance with multiple hydroxyl groups, and includes sugars (reducing and nonreducing sugars), sugar alcohols and sugar acids. In certain embodiments, polyols have a molecular weight that is less than about 600 kD (*e.g.*, in the range from about 120 to about 400 kD). A "reducing sugar" is one that contains a free aldehyde or ketone group and can reduce metal ions or react covalently with lysine and other amino groups in proteins. A "nonreducing sugar" is one that lacks a free aldehyde or ketonic group and is not oxidised by mild oxidising agents such as Fehling's or Benedict's solutions.. Examples of reducing sugars are fructose, mannose, maltose, lactose, arabinose, xylose, ribose, rhamnose, galactose and glucose. Nonreducing sugars include sucrose, trehalose, sorbose, melezitose and raffinose. Mannitol, xylitol, erythritol, threitol, sorbitol and glycerol are examples of sugar alcohols. As to sugar acids, these include L-gluconate and metallic salts thereof. The polyol may also act as a tonicity agent. In certain embodiments of the disclosure, one ingredient of the formulation is sorbitol in a concentration of about 10 to about 70 mg/ml. In a particular embodiment of the disclosure, the concentration of sorbitol is about 30 to about 50 mg/ml. In certain embodiments, the concentration of sucrose is about 60 to about 100 mg/ml. In a particular embodiment of the disclosure, the concentration of sucrose is about 70 to about 90 mg/ml.

The term "buffer" refers to a buffered solution that resists changes in pH by the action of its acid-base conjugate components. A buffer used in this disclosure has a pH in the range from about 4.5 to about 7.5. Examples of buffers that will control the pH in this range include acetate (*e.g.*, sodium acetate), succinate (such as sodium succinate), gluconate, methionine, imidazole, histidine, glycine, arginine, citrate, phosphate, citrate and phosphate, Tris, and other organic acid buffers. In certain embodiments, the buffer used in the formulation of the disclosure is histidine, glycine, arginine, acetate, citrate, and/or phosphate buffered saline (PBS).

A "reconstituted" formulation is one which has been prepared by dissolving a lyophilized protein formulation in a diluent such that the protein is dispersed in the reconstituted formulation. The reconstituted formulation is suitable for administration (*e.g.* parenteral administration) to a patient to be treated with the protein of interest (*e.g.*, LS-DVD-Ig).

A "diluent" of interest herein is one which is pharmaceutically acceptable (safe and non-toxic for administration to a human) and is useful for the preparation of a liquid formulation, such as a formulation reconstituted after lyophilization.

Exemplary diluents include sterile water, bacteriostatic water for injection (BWFI), a pH buffered solution (*e.g.* phosphate-buffered saline), sterile saline solution, Ringer's solution or dextrose solution. In an alternative embodiment, diluents can include aqueous solutions of salts and/or buffers.

A "therapeutically effective amount" or "effective amount" of a binding protein refers to an amount effective in the prevention or treatment of a disorder for the treatment of which the antibody is effective.

The term "disorder" refers to any condition that would benefit from treatment with the formulations of the disclosure. This includes chronic and acute disorders or diseases including those pathological conditions that predispose the subject to the disorder in question.

The term "treatment" refers to both therapeutic treatment and prophylactic or preventative measures. Those patients in need of treatment include those already with the disorder as well as those in which the disorder is to be prevented.

The terms "parenteral administration" and "administered parenterally" means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion. The phrases "systemic administration," "administered systemically," "peripheral administration" and "administered peripherally" mean the administration of a compound, drug or other material other than directly into the central nervous system, such that it enters the patient's system and is subject to metabolism and other like processes, for example, subcutaneous administration.

The term "antibody" broadly refers to an immunoglobulin (Ig) molecule, generally comprised of four polypeptide chains, two heavy (H) chains and two light (L) chains, or any functional fragment, mutant, variant, or derivative thereof, that retains the essential target binding features of an Ig molecule.

In a full-length antibody, each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. Immunoglobulin molecules can be of any type (*e.g.*, IgG, IgE, IgM, IgD, IgA and IgY) and class (*e.g.*, IgG1, IgG2, IgG 3, IgG4, IgA1 and IgA2) or subclass.

The term "Fc region" means the C-terminal region of an immunoglobulin heavy chain, which may be generated by papain digestion of an intact antibody. The Fc region may be a native sequence Fc region or a variant sequence Fc region. The Fc region of an immunoglobulin generally comprises two constant domains, a CH2 domain and a CH3 domain, and optionally comprises a CH4 domain.

The term "antigen-binding portion" refers to one or more fragments of a binding protein that specifically binds to a target or an antigen. Such embodiments may be monospecific, or may be bispecific, dual specific, or multi-specific (may specifically bind two or more different antigens).

A "functional antigen binding site" of a binding protein is one that is capable of binding a target antigen. The antigen binding affinity of the functional antigen binding site is not necessarily as strong as the parent antibody from which the antigen binding site is derived, but the ability to bind antigen must be measurable using a known method for evaluating antibody binding to an antigen. Moreover, the antigen binding affinity of each of the functional antigen binding sites of a multivalent binding protein need not be quantitatively the same.

The term "linker" denotes polypeptides comprising two or more amino acid residues joined by peptide bonds that are used to link one or more antigen binding portions. Such linker polypeptides are well known in the art (see, *e.g.,* Holliger et al. (1993) Proc. Natl. Acad. Sci. USA 90:6444-6448; Poljak et al. (1994) Structure 2:1121-1123).

An "immunoglobulin constant domain" refers to a heavy or light chain constant domain. Human heavy chain and light chain (*e.g.*, IgG) constant domain amino acid sequences are known in the art.

The term "monoclonal antibody" refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different epitopes, each monoclonal antibody is directed against a single epitope on the antigen. The modifier "monoclonal" is not to be construed as requiring production of the antibody by any particular method.

The term "human antibody" includes antibodies that have variable and constant regions derived from human germline immunoglobulin sequences. The human antibodies may include amino acid residues not encoded by human germline immunoglobulin sequences (*e.g.*, mutations introduced by random or site-specific mutagenesis in vitro or by somatic mutation in vivo). However, the term "human antibody" is not intended to include antibodies in which CDR sequences derived from the germline of another mammalian species, such as a mouse, have been grafted onto human framework sequences.

The term "chimeric antibody" means antibodies that comprise heavy and light chain variable region sequences from one species and constant region sequences from another species, such as antibodies having murine heavy and light chain variable regions linked to human constant regions.

The term "CDR-grafted antibody" means antibodies that comprise heavy and light chain variable region sequences from one species but in which the sequences of one or more of the CDR regions of their VH and/or VL are replaced with the CDR sequences of another species, such as antibodies having human heavy and light chain variable regions in which one or more of the murine CDRs (*e.g.*, CDR3) has been replaced with murine CDR sequences.

The term "humanized antibody" means an antibody that comprises heavy and light chain variable region sequences from a non-human species (*e.g.*, a mouse) but in which at least a portion of the VH and/or VL sequence has been altered to be more "human-like", *i.e.*, more similar to human germline variable sequences. One type of humanized antibody comprises non-human CDR sequences and human framework sequences.

The term "CDR" means the complementarity determining region within antibody variable sequences. There are three CDRs in each of the variable regions of the heavy chain and the light chain, which are designated CDR1, CDR2 and CDR3, for each of the variable regions. The term "CDR set" as used herein refers to a group of three CDRs that occur in a single variable region capable of binding the target. The exact boundaries of these CDRs have been defined differently according to different systems.

The terms "Kabat numbering", "Kabat definitions and "Kabat labeling" are used interchangeably herein. These terms refer to a system of numbering amino acid residues that are more variable (*i.e.*, hypervariable) than other amino acid residues in the heavy and light chain variable regions of an antibody, or an antigen binding portion thereof (Kabat et al. (1971) Ann. NY Acad. Sci. 190:382-391 and Kabat et al. (1991) Sequences of Proteins of Immunological Interest, Fifth Edition, U.S. Department of Health and Human Services, NIH Publication No. 91-3242). For the heavy chain variable region, the hypervariable region generally ranges from amino acid positions 31 to 35 for CDR1, amino acid positions 50 to 65 for CDR2, and amino acid positions 95 to 102 for CDR3. For the light chain variable region, the hypervariable region generally ranges from amino acid positions 24 to 34 for CDR1, amino acid positions 50 to 56 for CDR2, and amino acid positions 89 to 97 for CDR3.

Chothia and coworkers (Chothia and Lesk (1987) J. Mol. Biol. 196:901-917 and Chothia et al. (1989) Nature 342:877-883) found that certain sub-portions within Kabat CDRs adopt nearly identical peptide backbone conformations, despite having great diversity at the level of amino acid sequence. These sub-portions were designated as L1, L2 and L3 or HI, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (1995) FASEB J. 9:133-139 and MacCallum (1996) J. Mol. Biol. 262(5):732-45. Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although embodiments use Kabat or Chothia defined CDRs.

The term "framework" or "framework sequence" refers to the remaining sequences of a variable region minus the CDRs. Because the exact definition of a CDR sequence can be determined by different systems, the meaning of a framework sequence is subject to correspondingly different interpretations. The six CDRs (CDR-H1, -H2, and -H3 of the heavy chain and CDR-L1, -L2, and -L3 of the light chain) also divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. The term "activity" includes activities such as the binding specificity and binding affinity of a DVD-Ig protein for two or more antigens.

The term "epitope" includes any polypeptide determinant capable of specific binding to an immunoglobulin or T-cell receptor. In certain embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. In certain embodiments, an epitope is a region of an antigen that is bound by an antibody or multispecific binding protein.

The term "surface plasmon resonance" or "SPR", refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, N.J.). For further descriptions, see Jonsson et al. (1993) Ann. Biol. Clin. 51:19-26; Jonsson et al. (1991) Biotechniques 11:620-627; Jonsson et al. (1995) J. Mol. Recognit. 8:125-131; and Johnsson et al. (1991) Anal. Biochem. 198:268-277.

The term "$K_{on}$" refers to the on rate constant for association of a binding protein to the antigen to form the binding protein /antigen complex as is known in the art.

The term "$K_{off}$" refers to the off rate constant for dissociation of a binding protein from the binding protein/antigen complex as is known in the art.

The term "$K_d$" refers to the dissociation constant of a particular antibody-antigen interaction as is known in the art.

The terms "specific binding", "specifically binding" or "specifically binds", as used herein, in reference to the interaction of a binding protein with a second chemical species, mean that the interaction is dependent upon the presence of a particular structure (e.g., an antigenic determinant or epitope) on the chemical species; for example, a DVD-Ig protein recognizes and binds to a specific protein structure rather than to proteins generally. If a DVD-Ig protein is specific for epitope "A", the presence of a molecule containing epitope A (or free, unlabeled A), in a reaction containing labeled "A" and the DVD-Ig protein, will reduce the amount of labeled A bound to the DVD-Ig protein.

**II. Dual Variable Domain Immunoglobulin (DVD-Ig) Proteins for Use in Formulations of the Disclosure**

The disclosure pertains to formulations, and uses thereof, of DVD-Ig proteins, particularly those identified as AS-DVD-Ig protein or LS-DVD-Ig protein (described in more detail below).

A. General DVD-Ig Protein Structure

In certain embodiments, the DVD-Ig protein used in the formulations and methods of the disclosure comprises a polypeptide chain, wherein said polypeptide chain comprises VD1-(X1)n-VD2-C-(X2)n, wherein VD1 is a first variable domain, VD2 is a second variable domain, C is a constant domain, X1 represents an amino acid or polypeptide, X2 represents an Fc region and n is 0 or 1.

In certain embodiments, a DVD-Ig protein contains two polypeptide chains, wherein a first polypeptide chain comprises VD1-(X1)n-VD2-C-(X2)n, wherein VD1 is a first heavy chain variable domain, VD2 is a second heavy chain variable domain, C is a heavy chain constant domain, X1 is a linker with the proviso that it is not CH1, and X2 is an Fc region; and a second polypeptide chain comprises VD1-(X1)n-VD2-C-(X2)n, wherein VD1 is a first light chain variable domain, VD2 is a second light chain variable domain, C is a light chain constant domain, X1 is a linker with the proviso that it is not CH1, and X2 does not comprise an Fc region; and n is 0 or 1; and wherein said two polypeptide chains of said binding protein form two functional antigen binding sites.

In certain embodiments, a DVD-Ig protein contains four polypeptide chains, wherein two polypeptide chains comprise VD1-(X1)n-VD2-C-(X2)n, wherein VD1 is a first heavy chain variable domain, VD2 is a second heavy chain variable domain, C is a heavy chain constant domain, X1 is a linker with the proviso that it is not CH1, and X2 is an Fc region; and two polypeptide chains comprise VD1-(X1)n-VD2-C-(X2)n, wherein VD1 is a first light chain variable domain, VD2 is a second light chain variable domain, C is a light chain constant domain, X1 is a linker with the proviso that it is not CH1, and X2 does not comprise an Fc region; and n is 0 or 1; and wherein said four polypeptide chains of said binding protein form four functional antigen binding sites.

In certain embodiments, the AS-DVD-Ig protein or LS-DVD-Ig protein comprises a polypeptide chain wherein the polypeptide chain comprises VD1-(X1)n-VD2-C-(X2)n, wherein VD1 is a first heavy chain variable domain; VD2 is a second heavy chain variable domain; C is a heavy chain constant domain; X1 is a linker with the proviso that it is not CH1; X2 is an Fc region; and n is 0 or 1. In certain embodiments, the AS-DVD-Ig protein or LS-DVD-Ig protein comprises a polypeptide chain, wherein the polypeptide chain comprises VD1-(X1)n-VD2-C-(X2)n, wherein VD1 is a first light chain variable domain; VD2 is a second light chain variable domain; C is a light chain constant domain; X1 is a linker with the proviso that it is not a CH1 or CL; X2 does not comprise an Fc region; and n is 0 or 1. In a further embodiment, (X1)n on the heavy and/or light chain is (X1)0 and/or (X2)n on the heavy and/or light chain is (X2)0.

In certain embodiments, the AS-DVD-Ig protein or LS-DVD-Ig protein comprises first and second polypeptide chains, wherein the first polypeptide chain comprises a first VD1-(X1)n-VD2-C-(X2)n, wherein VD1 is a first heavy chain variable domain; VD2 is a second heavy chain variable domain; C is a heavy chain constant domain; X1 is a first linker with the proviso that it is not CH2; X2 is an Fc region; n is 0 or 1; and wherein the second polypeptide chain comprises a second VD1-(X1)n-VD2-C-(X2)n, wherein VD1 is a first light chain variable domain; VD2 is a second light chain variable domain; C is a light chain constant domain; X1 is a second linker with the proviso that it is not CH1 or CL; X2 does not comprise an Fc region; and n is 0 or 1.

In certain embodiments, the VD1 of the first polypeptide chain and the VD1 of the second polypeptide chain are from different first and second parent antibodies, respectively, or binding portions thereof. In certain embodiments, the VD2 of the first polypeptide chain and the VD2 of the second polypeptide chain are from different first and second parent antibodies, respectively, or binding portions thereof. In certain embodiments, the first and the second parent antibodies bind different epitopes on the same target or different targets. In certain embodiments, the first parent antibody or binding portion thereof binds the first target with a potency that is different from the potency with which the second parent antibody or binding portion thereof binds the second target. In certain embodiments, the first parent antibody or binding portion thereof binds the first target with an affinity different from the affinity with which the second parent antibody or binding portion thereof binds the second target.

In certain embodiments, the AS-DVD-Ig protein or LS-DVD-Ig protein comprises, two first polypeptide chains and two second polypeptide chains.

In certain embodiments, the AS-DVD-Ig protein or LS-DVD-Ig protein comprises first and second polypeptide chains, each independently comprising VD1-(X1)n-VD2-C-(X2)n, wherein VD1 is a first variable domain; VD2 is a second variable domain; C is a constant domain; X1 is a linker with the proviso that it is not CH1; X2 is an Fc region; n is 0 or 1, wherein the VD1 domains on the first and second polypeptide chains form a first functional target binding site and the VD2 domains on the first and second polypeptide chains form a second functional target binding site. In a further embodiment, the first polypeptide chain comprises a first VD1-(X1)n-VD2-C-(X2)n, wherein VD1 is a first heavy chain variable domain; VD2 is a second heavy chain variable domain; C is a heavy chain constant domain; X1 is a linker with the proviso that it is not CH1; X2 is an Fc region; n is 0 or 1, and wherein the second polypeptide chain comprises a second VD1-(X1)n-VD2-C-(X2)n, wherein VD1 is a first light chain variable domain; VD2 is a second light chain variable domain; C is a light chain constant domain; X1 is a linker with the proviso that it is not CH1; X2 does not comprise an Fc region; n is 0 or 1, wherein the VD1 domains on the first and second polypeptide chains form a first functional target binding site and the VD2 domains on the first and second polypeptide chains form a second functional target binding site.

In certain embodiments, the AS-DVD-Ig protein or LS-DVD-Ig protein comprises first and second polypeptide chains, each independently comprising VD1-(X1)n-VD2-C-(X2)n, wherein VD1 is a first variable domain; VD2 is a second variable domain; C is a constant domain; X1 is a linker with the proviso that it is not CH1; X2 is an Fc region; n is 0 or 1, and wherein the VD1 domains on the first and second polypeptide chains form a first functional target binding site and the VD2 domains on the first and second polypeptide chains form a second functional target binding site. In a further embodiment, the first polypeptide chain comprises a first VD1-(X1)n-VD2-C-(X2)n, wherein VD1 is a first heavy chain variable domain; VD2 is a second heavy chain variable domain; C is a heavy chain constant domain; X1 is a linker with the proviso that it is not CH1; X2 is an Fc region; n is 0 or 1, and wherein the second polypeptide chain comprises a second VD1-(X1)n-VD2-C-(X2)n, wherein VD1 is a first light chain variable domain; VD2 is a second light chain variable domain; C is a light chain constant domain; X1 is a linker with the proviso that it is not CH1; X2 does not comprise an Fc region; n is 0 or 1, wherein the VD1 domains on the first and second polypeptide chains form a first functional target binding site and the VD2 domains on the first and second polypeptide chains form a second functional target binding site.

Examples of DVD-Ig proteins are described in US Patent No. 7,612,181, which is incorporated by reference herein.

Examples of DVD-Ig proteins that may be used in the methods and compositions of the disclosure are described below, including Tables 58 and 65. Further examples of DVD-Ig proteins that may be used in the methods and compositions of the disclosure are described in SEQ ID NOs: 28 to 75.

B. Generation of DVD-Ig Proteins

The variable domains of a DVD-Ig protein can be obtained from parent antibodies, including polyclonal and monoclonal antibodies capable of binding targets of interest. These antibodies may be naturally occurring or may be generated by recombinant technology. Examples of antibodies that may be used in making DVD-Ig proteins include chimeric antibodies, human antibodies, and humanized antibodies. Monoclonal antibodies can be prepared using a wide variety of techniques known in the art including, for example, the use of hybridoma, recombinant, and phage display technologies, or any combination thereof. Monoclonal antibodies may also be produced by immunizing a non-human animal comprising some, or all, of the human immunoglobulin locus with an antigen of interest, such as, for example, XENOMOUSE™ transgenic mouse, an engineered mouse strain that comprises large fragments of the human immunoglobulin loci and is deficient in mouse antibody production. Methods of generating DVD-Ig proteins are described in US Patent No. 7,612,181, the teachings of which are incorporated by reference herein. DVD-Ig proteins used in the compositions and methods of the disclosure may be made from antibodies capable of binding specific targets and well known in the art. These include, but are not limited to an anti-TNF antibody (U.S. Patent No. 6,258,562), anti-IL-12 and or anti-IL-12p40 antibody (U.S. Patent No. 6,914,128); anti-IL-18 antibody (US Patent Publication No. 20050147610), as well as anti-C5, anti-CBL, anti-CD147, anti-gp120, anti-VLA4, anti-CD 11a, anti-CD 18, anti-VEGF, anti-CD40L, anti-Id, anti-ICAM-1, anti-CXCL13, anti-CD2, anti-EGFR, anti-TGF-beta 2, anti-E-selectin, anti-Fact VII, anti-Her2/neu, anti-F gp, anti-CD11/18, anti-CD14, anti-ICAM-3, anti-CD80, anti-CD4, anti-CD3, anti-CD23, anti-beta2-integrin, anti-alpha4beta7, anti-CD52, anti-HLA DR, anti-CD22, anti-CD20, anti-MIF, anti-CD64 (FcR), anti-TCR alpha beta, anti-CD2, anti-Hep B, anti-CA 125, anti-EpCAM, anti-gp120, anti-CMV, anti-gpIIbIIIa, anti-IgE, anti-CD25, anti-CD33, anti-HLA, anti-VNRintegrin, anti-IL-1alpha, anti-IL-1beta, anti-IL-1 receptor, anti-IL-2 receptor, anti-IL-4, anti-IL4 receptor, anti-IL5, anti-IL-5 receptor, anti-IL-6, anti-IL-8, anti-IL-9, anti-IL-13, anti-IL-13 receptor, anti-IL-17, and anti-IL-23 antibodies (see Presta (2005) J. Allergy Clin. Immunol. 116:731-6 and Clark "Antibodies for Therapeutic Applications," Department of Pathology, Cambridge University, UK (2000), published online at M. Clark's home page at the website for the Department of Pathology, Cambridge University.

Parent monoclonal antibodies may also be selected from various therapeutic antibodies approved for use, in clinical trials, or in development for clinical use. Such therapeutic antibodies include, but are not limited to: rituzimab (RITUXAN™ Biogen Idec, Genentech/Roche) (see for example U.S. Patent No. 5,736,137) a chimeric anti-CD20 antibody approved to treat non-Hodgkin's lymphoma; ofatumumab (HUMAX-CD20™ Genmab, GlaxoSmithKlein) (described in U.S. Patent 5,500,362) an anti-CD20 antibody approved to treat chronic lymphocytic leukemia that is refractory to fludarabine and alemtuzumab; AME-133v (Mentrik Biotech) an anti-CD20 antibody; veltuzumab (hA20) (Immunomedics) an anti-CD20 antibody; HumaLYM (Intracel); PRO70769 (Genentech/Roche) (PCT/US2003/040426) an anti-CD20 antibody; trastuzumab (HERCEPTIN™ Genentech/Roche) (described in U.S. Patent No. 5,677,171) a humanized anti-Her2/neu antibody approved to treat breast cancer; pertuzumab (rhuMab-2C4, OMNITARG™ Genentech/Roche) (described in U.S. Patent No. 4,753,894) ; cetuximab (ERBITUX™ Imclone) (described in U.S. Patent No. 4,943,533; PCT WO 96/40210) a chimeric anti-EGFR antibody approved to treat colorectal and head and neck cancer; panitumumab (ABX-EGF VECTEBIX® Amgen) (described in U.S. Patent No. 6,235,883) an anti-EGFR antibody approved to treat colorectal cancer; zalutumumab (HUMAX-EGFR™ Genmab) (described in U.S. Patent Application Serial No. 10/172,317) an anti-EGFR antibody ; EMD55900 (Mab 425 Merck) an anti-EGFR antibody; EMD62000 and EMD72000 (Mab 425 Merck) anti-EGFR antibodies (described in U.S. Patent No. 5,558,864; Murthy et al. (1987) Arch. Biochem. Biophys. 252(2):549-60; Rodeck et al. (1987) J. Cell. Biochem. 35(4):315-20; Kettleborough et al. (1991) Protein Eng. 4(7):773-83; ICR62 (Institute of Cancer Research) an anti-EGFR antibody (described in PCT Publication No. WO 95/20045; Modjtahedi et al. (1993) J. Cell. Biophys. 22(1-3):129-46; Modjtahedi et al. (1993) Br. J. Cancer 67(2):247-53; Modjtahedi et al. (1996) Br. J. Cancer 73(2):228-35; Modjtahedi et al. (2003) Int. J. Cancer 105(2):273-80); nimotuzumab (TheraCIM hR3, THERALOC® YM Biosciences, Oncoscience AG) (described in U.S. Patent No. 5,891,996; U.S. Patent No. 6,506,883; Mateo et al. (1997) Immunotechnol. 3(1):71-81) an anti-EGFR antibody; ABT-806 (Ludwig Institute for Cancer Research, Memorial Sloan-Kettering) (Jungbluth et al. (2003) Proc. Natl. Acad. Sci. USA 100(2):639-44) an anti-EGFR antibody; KSB-102 (KS Biomedix); MR1-1 (IVAX, National Cancer Institute) (PCT Publication No. WO 0162931A2) an anti-EGFRvIII antibody; SC100 (Scancell) (PCT Publication No. WO 01/88138) an anti-EGFR antibody; alemtuzumab (CAMPATH™ Genzyme/Sanofi) an anti-CD52 antibody approved to treat B-cell chronic lymphocytic leukemia; muromonab-CD3 (Orthoclone OKT3™ Johnson and Johnson) an anti-CD3 antibody approved to treat organ transplant rejection; ibritumomab tiuxetan (ZEVALIN™ Spectrum Pharmaceuticals) an anti-CD20 antibody approved to treat non-Hogkins Lymphoma; gemtuzumab ozogamicin (hP67.6 MYLOTARG™ Pfizer) an anti-CD33 antibody conjugated to calicheamicin; alefacept (AMEVIVE™ Astellas Pharma) an anti-CD2 LFA-3 Fc fusion; abciximab (REOPRO™ Centocor Ortho Biotech Products, Lilly) a chimeric human-mouse anti-glycoprotein IIb/IIIa receptor and anti-vitronectic $\alpha_v\beta_3$ receptor antibody approved as an adjunct to percutaneous coronary intervention to prevent cardiac ishemia; basiliximab (SIMULECT™ Novartis) an anti-CF25 antibody approved to treat organ transplant rejection; palivizumab (SYNAGIS™ Medimmune) an antibody to the A antigenic site of F protein of RSV approved to treat RSV infection; infliximab (REMICADE™ Janssen Biotech) an anti-TNFalpha$\alpha$ antibody approved to treat Crohn's disease, ulcerative colitis, arthritis, ankylosing spondylitis, psoriatic arthritis, and plaque psoriasis; adalimumab (HUMIRA™ AbbVie) an anti-TNFa antibody approved to treat rheumatoid arthritis, juvenile idiopathic arthritis, psoriatic arthritis, ankylosing spondylitis, Crohn's disease, ulcerative colitis, plaque psoriasis; CDP571 (HUMICADE™ Celltech, Biogen IDEC) an anti-TNFa antibody ; etanercept (ENBREL™ Amgen, Pfizer) an anti-TNFa Fc fusion antibody approved to treat rheumatoid arthritis, juvenile idiopathic arthritis, psoriatic arthritis, ankylosing spondylitis, plaque psoriasis; certolizumab pegol (CIMZIA)UCB Pharma) an anti-TNFa antibody approved to treat rheumatoid arthritis and Crohn's disease; ustekinumab (STELARA Janssen Biotech) a human anti-p40 subunit of IL-12 and IL-23 antibody approved to treat plaque psoriasis; galilimomab (ABX-CBL Abgenix) a mouse anti-CD 147 antibody; ABX-IL8 (Abgenix) an anti-IL8 antibody ; ABX-MA1 (Abgenix) an anti-MUC18 antibody ; pemtumomab (Theragyn, R1549, 90Y-muHMFG1Antisoma) a mouse anti-MUCI-Yttrium 90 antibody conjugate; Therex (R1550 Antisoma) an anti-MUC1 antibody; AngioMab (muBC-1, AS1405 Antisoma); HuBC-1 (Antisoma); Thioplatin (AS1407 Antisoma); natalizumab (TYSABRI® Biogen Idec, Elan) an anti-$\alpha$4 integrin antibody approved to treat multiple sclerosis and Crohn's disease; VLA-1 (Santarus) a humanized anti-VLA-1 antibody ; LTBR mAb (Biogen Idec) an anti-lymphotoxin $\beta$ receptor antibody; lerdelimumab (CAT-152 Cambridge Antibody Technology/Abbott) an anti-TGF-$\beta$2 antibody ; briakinumab (AbbVie) an anti-IL-12 and 23 antibody ; metelimumab (CAT-192 Cambridge Antibody Technology, Genzyme) an anti-TGF$\beta$1 antibody ; bertilimumab (CAT-213, iCO-008 Cambridge Antibody Technology, iCo Therapeutics, Immune Pharmaceuticals) an anti-eotaxinl antibody ; belimumab (BENLYSTA® GlaxoSmithKline) an anti-B lymphocyte stimulator protein antibody approved to treat systemic lupus erythematosus; maputumumab (HGS-ETR1 Cambridge Antibody Technology, Human Genome Sciences) an anti-TRAIL-RI antibody; bevacizumab (AVASTIN™ Genentech/Roche) an anti-VEGF antibody approved to treat metastatic colorectal cancer, non-squamous non-small cell lung cancer, glioblastoma, metastatic renal cell cancer; anti-HER3/EGFR antibody (Genentech/Roche); an Anti-Tissue Factor antibody (Genentech/Roche); omalizumab (XOLAIR™ Genentech/Roche, Novartis) an anti-IgE antibody approved to treat severe allergic asthma; efalizumab (RAPTIVA™ Genentech/Roche, Merck Serono) an anti-CD11 a antibody; MLN-02 (Millenium, Genentech/Roche) an anti-$\alpha$4$\beta$7 integrin antibody; zanolimumab (HUMAX CD4™ Emergent BioSolutions) an anti-CD4 antibody ; HUMAX-IL15™ (AMG-714 Genmab, Amgen) an anti-IL15 antibody ; HuMax-IL8 (HUMAX -Inflam™, MDX-018 Genmab, Cormorant Pharmaceuticals) an anti-IL8 antibody; HUMAX™-Cancer, (Genmab, Medarex, Oxford GlycoSciences) an anti-Heparanase I antibody; HUMAX™-Lymphoma (Genmab) an anti-IL8 antibody; HUMAX™-TAC (Genmab) an anti-IL-2Rα, CD25 antibody ; daratumumab (HuMax®-CD38, Genmab, Janssen Biotech) an anti-CD38 antibody; toralizumab (IDEC-131 Biogen Idec) an anti-CD40L antibody ; clenolimimab (IDEC-151 Biogen Idec) an anti-CD4 antibody ; glaiximab (IDEC-114 Biogen Idec) an anti-CD80 antibody ; lumilixmab (IDEC-152 Biogen Idec) an anti-CD23 ; anti-macrophage migration factor (MIF) antibodies (Biogen Idec, Taisho Pharmaceutical); mitumomab (BEC2 Imclone) a mouse anti-idiotypic antibody ; IMC-1C11 (Imclone) a chimeric anti-VEGFR2 antibody; DC101 (Imclone) murine anti-VEGFR2 antibody;; anti-VE cadherin antibody (Imclone); labetuzumab (CEA-CIDE™ Immunomedics) an anti-carcinoembryonic antigen antibody ; epratuzumab (LYMPHOCIDE ™ Immunomedics) an anti-CD22 antibody ; yttrium ($^{90}$Y) tacatuzumab tetraxetan (AFP-Cide® Immunomedics) an anti-afetoprotein antibody; milatuzumab (MyelomaCide® Immunomedics) an anti-CF74 antibody; LeukoCide® (Immunomedics); ProstaCide® (Immunomedics); ipilimumab (Yervoy™, MDX-010 Bristol-Myers Squibb) an anti-CTLA4 antibody approved to treat melanoma; iratumumab (MDX-060 Medarex) an anti-CD30 antibody ; MDX-070 (Medarex) an anti-prostate specific membrane antigen; OSIDEM™ (IDM-1 Medarex, Immuno-Designed Molecules) an anti-Her2 antibody; HUMAX™ -CD4, an anti-CD4 antibody being developed by Medarex and Genmab; HuMax-IL15, an anti-IL15 antibody being developed by Medarex and Genmab; golimumab (SIMPONI™ Janssen Biotech) an anti-TNFa antibody approved to treat rheumatoid arthritis, psoriatic arthritis, ankylosing spondylitis; ustekinumab (STELARA®, CNTO 1275 Janssen Biotech) an anti-IL-12 antibody approved to treat plaque psoriasis; MOR101 and MOR102 (MorphoSys) anti-intercellular adhesion molecule-1 (ICAM-1) (CD54) antibodies; MOR201 (MorphoSys) an anti-fibroblast growth factor receptor 3 antibody ; visilizumab (NUVION™ PDL BioPharma) an anti-CD3 antibody ; fontolizumab (HUZAF™ PDL BioPharma) an anti-INFγ antibody ; volociximab (M200 PDL BioPharma, Biogen Idec) an anti-α5β1 integrin antibody ; SMART® IL-12 (PDL BioPharma) an anti-IL-12 ; ING-1 (Xoma) an anti-Ep-CAM antibody; omalizumab (XOLAIR™ Genentech/Roche, Novartis) an anti-IgE antibody approved to treat allergic asthma; MLN01 (Xoma) an anti-β integrin antibody; and tocilizumab (ACTEMRA™ Genentech/Roche) an anti-IL6 antibody approved to treat rhemuatoid arthritis and systemic juvenile idiopathic arthritis.

## C. Construction of DVD-Ig Proteins

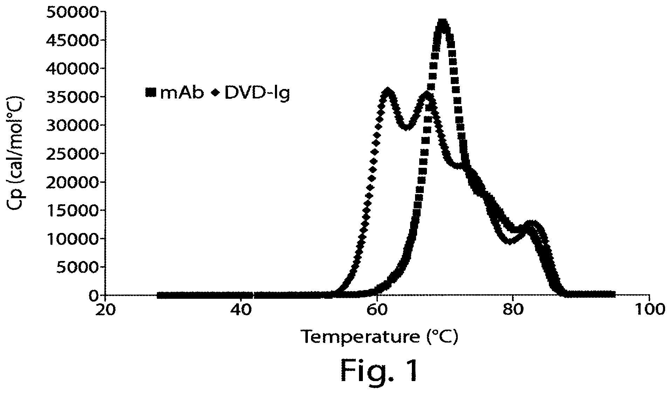

A DVD-Ig protein is formed by combining two heavy chain DVD polypeptides and two light chain DVD polypeptides. The dual variable domain immunoglobulin (DVD-Ig) heavy chain comprises two heavy chain variable domains (VH) linked in tandem, directly or by a linker, followed by the constant domain CH1 and Fc region. The dual variable domain immunoglobulin (DVD-Ig) light chain is designed such that two light chain variable domains (VL) from the two parent mAbs are linked in tandem, directly or via a linker, followed by the light chain constant domain (CL) (see Figure 1A of U.S. Patent No. 7,612,181, incorporated by reference herein). Methods of making DVD-Ig proteins are also described in U.S. Patent No. 7,612,181, incorporated by reference herein.

The variable domains of the DVD-Ig protein can be obtained using recombinant DNA techniques from a parent antibody generated by any one of the methods described above. In certain embodiments, the variable domain is a CDR grafted or a humanized variable heavy or light chain domain. In certain embodiments, the variable domain is a human heavy or light chain variable domain.

The linker sequence may be a single amino acid or a polypeptide sequence. Examples of linker sequences that may be used to link variable domains include, but are not limited to, AKTTPKLEEGEFSEAR (SEQ ID NO:1); AKTTPKLEEGEFSEARV (SEQ ID NO:2); AKTTPKLGG (SEQ ID NO:3); SAKTTPKLGG (SEQ ID NO:4); SAKTTP (SEQ ID NO:5); RADAAP (SEQ ID NO:6); RADAAPTVS (SEQ ID NO:7); RADAAAAGGPGS (SEQ ID NO:8); RADAAAA(G$_{4S}$)$_{.4}$ (SEQ ID NO:9), SAKTTPKLEEGEFSEARV (SEQ ID NO:10); ADAAP (SEQ ID NO:11); ADAAPTVSIFPP (SEQ ID NO:12); TVAAP (SEQ ID NO:13); TVAAPSVFIFPP (SEQ ID NO:14); QPKAAP (SEQ ID NO:15); QPKAAPSVTLFPP (SEQ ID NO:16); AKTTPP (SEQ ID NO:17); AKTTPPSVTPLAP (SEQ ID NO:18); AKTTAP (SEQ ID NO:19); AKTTAPSVYPLAP (SEQ ID NO:20); ASTKGP (SEQ ID NO:21); ASTKGPSVFPLAP (SEQ ID NO:22); GGGGSGGGGSGGGGS (SEQ ID NO:23); GENKVEYAPALMALS (SEQ ID NO:24); GPAKELTPLKEAKVS (SEQ ID NO:25); GHEAAAVMQVQYPAS (SEQ ID NO:26); and GGGGSGGGGS (SEQ ID NO: 27). Other examples of linkers are described in U.S. Patent Publication No. 20100226923, incorporated by reference herein. The choice of linker sequences may be determined based on crystal structure analysis of several antibody Fab molecules. There is a natural flexible linkage between the variable domain and the CH1/CL constant domain in Fab or antibody molecular structure. This natural linkage comprises approximately 10-12 amino acid residues, contributed by 4-6 residues from C-terminus of V domain and 4-6 residues from the N-terminus of the CL or CH1 domain. DVD-Ig proteins of the disclosure were generated using N-terminal 5-6 amino acid residues, or 11-12 amino acid residues, of CL or CH1 as the linker in the light chain and the heavy chain of the DVD-Ig protein, respectively. The N-terminal residues of the CL or the CH1 domains, particularly the first 5-6 amino acid residues, adopt a loop conformation without strong secondary structure, and therefore can act as flexible linkers between the two variable domains. The N-terminal residues of the CL or CH1 domains are natural extensions of the variable domains, as they are part of the Ig sequences, and therefore immunogenicity potentially arising from the linkers or junctions is minimized. Further examples of linkers are described in Table 65 (see underlined amino acids).

Other linker sequences may include a sequence of any length of the CL or CH1 domain but not all residues of a CL/CH1 domain; for example the first 5-12 amino acid residues of the CL or CH1 domain; the light chain linkers can be from Cκ or Cλ; and the heavy chain linkers can be derived from CH1 of any isotype, including Cγ1, Cγ2, Cγ3, Cγ4, Cα1, Cα2, Cδ, Cε, and Cμ. Linker sequences may also be derived from other proteins such as Ig-like proteins, (*e.g.*, TCR, FcR, KIR); G/S based sequences (*e.g.*, G4S repeats); hinge region-derived sequences; and other natural sequences from proteins.

In certain embodiments, a constant domain is linked to the two linked variable domains using recombinant DNA techniques. For example, a sequence comprising linked heavy chain variable domains is linked to a heavy chain constant domain and sequence comprising linked light chain variable domains is linked to a light chain constant domain. In certain embodiments, the constant domains are a human heavy chain constant domain and a human light chain constant domain, respectively. In certain embodiments, the DVD-Ig heavy chain is further linked to an Fc region. The Fc region may comprise a native Fc region sequence, or a variant Fc region sequence. In certain embodiments, the Fc region is a human Fc region. For example, the Fc region comprises an Fc region from an IgG1, IgG2, IgG3, IgG4, IgA, IgM, IgE, or IgD.

In certain embodiments, the DVD-Ig protein used in the methods and compositions of the disclosure is a dual-specific tetravalent binding protein. In certain embodiments, the DVD-Ig protein used in the methods and compositions of the disclosure binds CD20 and CD80 In certain embodiments, the DVD-Ig protein used in the methods and compositions of the disclosure binds VEGF and HER2. In certain embodiments, the DVD-Ig protein used in the methods and compositions of the disclosure binds TNF and RANKL. In certain embodiments, the DVD-Ig protein used in the methods and compositions of the disclosure binds TNF and DKK. In certain embodiments, the DVD-Ig protein used in the methods and compositions of the disclosure binds CD20 and RANKL. In certain embodiments, the DVD-Ig protein used in the methods and compositions of the disclosure binds DLL4 and PLGF. In certain embodiments, the DVD-Ig protein used in the methods and compositions of the disclosure binds DLL4 and VEGF. In certain embodiments, the DVD-Ig protein used in the methods and compositions of the disclosure binds TNF and SOST. In certain embodiments, the DVD-Ig protein used in the methods and compositions of the disclosure binds IL-9 and IgE. In certain embodiments, the DVD-Ig protein used in the methods and compositions of the disclosure binds IL-12 and IL-18. An example of an IL-12 and IL-18 DVD-Ig protein is described in U.S. Patent No.7,612,181. In certain embodiments, the DVD-Ig protein used in the methods and compositions of the disclosure binds TNF and IL-17. In certain embodiments, the DVD-Ig protein used in the methods and compositions of the disclosure binds TNF and PGE2. Examples of PGE2 DVD-Ig proteins are provided in U.S. Patent Publication No. 20100074900. In certain embodiments, the DVD-Ig protein used in the methods and compositions of the disclosure binds IL-1α and IL-1β. An example of an IL-1α and IL-1β DVD-Ig protein is described in U.S. Patent No. 7,612,181. In certain embodiments, the DVD-Ig protein used in the methods and compositions of the disclosure binds IL-4 and IL-1. An example of an IL-4 and IL-13 DVD-Ig protein is described in U.S. Publication No. 20100226923. The amino acid and nucleic acid sequences of the DVD-Ig proteins described in the aforementioned patents and patent applications are incorporated by reference herein. Amino acid sequences of DVD-Ig proteins that may be used in the methods and compositions of the disclosure are described in SEQ ID NOs: 28-75.

In certain embodiments, the DVD-Ig protein used in the methods and compositions of the disclosure specifically binds CD20/CD80, and comprises amino acid sequences corresponding to the heavy and light chain CDRs set forth in SEQ ID NOs: 30 and 31. In certain embodiments, the anti-CD20/CD80 DVD-Ig protein comprises amino acid sequences corresponding to the heavy and light chain variable regions set forth in SEQ ID NOs: 30 and 31. In certain embodiments, the anti-CD20/CD80 DVD-Ig protein comprises the amino acid sequences corresponding to the heavy and light chains set forth in SEQ ID NOs: 30 and 31.

In certain embodiments, the DVD-Ig protein used in the methods and compositions of the disclosure specifically binds CD80/CD20, and comprises amino acid sequences corresponding to the heavy and light chain CDRs set forth in SEQ ID NOs: 32 and 33. In certain embodiments, the anti-CD80/CD20 DVD-Ig protein comprises amino acid sequences corresponding to the heavy and light chain variable regions set forth in SEQ ID NOs: 32 and 33. In certain embodiments, the anti-CD80/CD20 DVD-Ig protein comprises the amino acid sequences corresponding to the heavy and light chains set forth in SEQ ID NOs: 32 and 33.

In certain embodiments, the DVD-Ig protein used in the methods and compositions of the disclosure specifically binds VEGF/HER2, and comprises amino acid sequences corresponding to the heavy and light chain CDRs set forth in SEQ ID NOs: 34 and 35. In certain embodiments, the anti-VEGF/HER2-Ig protein comprises amino acid sequences corresponding to the heavy and light chain variable regions set forth in SEQ ID NOs: 34 and 35. In certain embodiments, the anti-VEGF/HER2 DVD-Ig protein comprises the amino acid sequences corresponding to the heavy and light chains set forth in SEQ ID NOs: 34 and 35.

In certain embodiments, the DVD-Ig protein used in the methods and compositions of the disclosure specifically binds HER2/VEGF, and comprises amino acid sequences corresponding to the heavy and light chain CDRs set forth in SEQ ID NOs: 36 and 37. In certain embodiments, the anti-HER2/VEGF-Ig protein comprises amino acid sequences corresponding to the heavy and light chain variable regions set forth in SEQ ID NOs: 36 and 37. In certain embodiments, the anti-HER2/VEGF DVD-Ig protein comprises the amino acid sequences corresponding to the heavy and light chains set forth in SEQ ID NOs: 36 and 37.

In certain embodiments, the DVD-Ig protein used in the methods and compositions of the disclosure specifically binds TNF/RANKL, and comprises amino acid sequences corresponding to the heavy and light chain CDRs set forth in SEQ ID NOs: 38 and 39. In certain embodiments, the anti-TNF/RANKL-Ig protein comprises amino acid sequences corresponding to the heavy and light chain variable regions set forth in SEQ ID NOs: 38 and 39. In certain embodiments, the anti-TNF/RANKL DVD-Ig protein comprises the amino acid sequences corresponding to the heavy and light chains set forth in SEQ ID NOs: 38 and 39.

In certain embodiments, the DVD-Ig protein used in the methods and compositions of the disclosure specifically binds RANKL/TNF, and comprises amino acid sequences corresponding to the heavy and light chain CDRs set forth in SEQ ID NOs: 40 and 41. In certain embodiments, the anti-RANKL/TNF-Ig protein comprises amino acid sequences corresponding to the heavy and light chain variable regions set forth in SEQ ID NOs: 40 and 41. In certain embodiments, the anti-RANKL/TNF DVD-Ig protein comprises the amino acid sequences corresponding to the heavy and light chains set forth in SEQ ID NOs:40 and 41.

In certain embodiments, the DVD-Ig protein used in the methods and compositions of the disclosure specifically binds TNF/DKK, and comprises amino acid sequences corresponding to the heavy and light chain CDRs set forth in SEQ ID NOs: 42 and 43. In certain embodiments, the anti-TNF/DKK-Ig protein comprises amino acid sequences corresponding to the heavy and light chain variable regions set forth in SEQ ID NOs: 42 and 43. In certain embodiments, the anti-TNF/DKK-Ig protein comprises the amino acid sequences corresponding to the heavy and light chains set forth in SEQ ID NOs: 42 and 43.

In certain embodiments, the DVD-Ig protein used in the methods and compositions of the disclosure specifically binds DKK/TNF, and comprises amino acid sequences corresponding to the heavy and light chain CDRs set forth in SEQ ID NOs: 44 and 45. In certain embodiments, the anti-DKK/TNF-Ig protein comprises amino acid sequences corresponding to the heavy and light chain variable regions set forth in SEQ ID NOs: 44 and 45. In certain embodiments, the anti-DKK/TNF-Ig protein comprises the amino acid sequences corresponding to the heavy and light chains set forth in SEQ ID NOs: 44 and 45.

In certain embodiments, the DVD-Ig protein used in the methods and compositions of the disclosure specifically binds CD20/RANKL, and comprises amino acid sequences corresponding to the heavy and light chain CDRs set forth in SEQ ID NOs: 46 and 47. In certain embodiments, the anti-CD20/RANKL-Ig protein comprises amino acid sequences corresponding to the heavy and light chain variable regions set forth in SEQ ID NOs: 46 and 47. In certain embodiments, the anti-CD20/RANKL-Ig protein comprises the amino acid sequences corresponding to the heavy and light chains set forth in SEQ ID NOs: 46 and 47.

In certain embodiments, the DVD-Ig protein used in the methods and compositions of the disclosure specifically binds RANKL/CD20, and comprises amino acid sequences corresponding to the heavy and light chain CDRs set forth in SEQ ID NOs: 48 and 49. In certain embodiments, the anti-RANKL/CD20-Ig protein comprises amino acid sequences corresponding to the heavy and light chain variable regions set forth in SEQ ID NOs: 48 and 49. In certain embodiments, the anti-RANKL/CD20-Ig protein comprises the amino acid sequences corresponding to the heavy and light chains set forth in SEQ ID NOs: 48 and 49.

In certain embodiments, the DVD-Ig protein used in the methods and compositions of the disclosure specifically binds DLL4/PLGF, and comprises amino acid sequences corresponding to the heavy and light chain CDRs set forth in SEQ ID NOs: 50 and 51. In certain embodiments, the anti-DLL4/PLGF-Ig protein comprises amino acid sequences corresponding to the heavy and light chain variable regions set forth in SEQ ID NOs: 50 and 51. In certain embodiments, the anti-DLL4/PLGF-Ig protein comprises the amino acid sequences corresponding to the heavy and light chains set forth in SEQ ID NOs: 50 and 51.

In certain embodiments, the DVD-Ig protein used in the methods and compositions of the disclosure specifically binds PLGF/DLL4, and comprises amino acid sequences corresponding to the heavy and light chain CDRs set forth in SEQ ID NOs: 52 and 53. In certain embodiments, the anti-PLGF/DLL4-Ig protein comprises amino acid sequences corresponding to the heavy and light chain variable regions set forth in SEQ ID NOs: 52 and 53. In certain embodiments, the anti-PLGF/DLL4-Ig protein comprises the amino acid sequences corresponding to the heavy and light chains set forth in SEQ ID NOs: 52 and 53.

In certain embodiments, the DVD-Ig protein used in the methods and compositions of the disclosure specifically binds TNF/SOST (S2), and comprises amino acid sequences corresponding to the heavy and light chain CDRs set forth in SEQ ID NOs: 54 and 55. In certain embodiments, the anti-TNF/SOST (S2)-Ig protein comprises amino acid sequences corresponding to the heavy and light chain variable regions set forth in SEQ ID NOs: 54 and 55. In certain embodiments, the anti-TNF/SOST (S2)-Ig protein comprises the amino acid sequences corresponding to the heavy and light chains set forth in SEQ ID NOs: 54 and 55.

In certain embodiments, the DVD-Ig protein used in the methods and compositions of the disclosure specifically binds SOST (S2)/TNF, and comprises amino acid sequences corresponding to the heavy and light chain CDRs set forth in SEQ ID NOs: 56 and 57. In certain embodiments, the anti-SOST (S2)/TNF-Ig protein comprises amino acid sequences corresponding to the heavy and light chain variable regions set forth in SEQ ID NOs: 56 and 57. In certain embodiments, the anti-SOST (S2)/TNF-Ig protein comprises the amino acid sequences corresponding to the heavy and light chains set forth in SEQ ID NOs: 56 and 57.

In certain embodiments, the DVD-Ig protein used in the methods and compositions of the disclosure specifically binds IL-9 (S2)/IgE, and comprises amino acid sequences corresponding to the heavy and light chain CDRs set forth in SEQ ID NOs: 58 and 59. In certain embodiments, the anti-IL-9 (S2)/IgE-Ig protein comprises amino acid sequences corresponding to the heavy and light chain variable regions set forth in SEQ ID NOs: 58 and 59. In certain embodiments, the anti-IL-9 (S2)/IgE-Ig protein comprises the amino acid sequences corresponding to the heavy and light chains set forth in SEQ ID NOs: 58 and 59.

In certain embodiments, the DVD-Ig protein used in the methods and compositions of the disclosure specifically binds IgE/IL-9 (S2), and comprises amino acid sequences corresponding to the heavy and light chain CDRs set forth in SEQ ID NOs: 60 and 61. In certain embodiments, the anti-IgE/IL-9 (S2)-Ig protein comprises amino acid sequences corresponding to the heavy and light chain variable regions set forth in SEQ ID NOs: 60 and 61. In certain embodiments, the anti-IgE/IL-9 (S2)-Ig protein comprises the amino acid sequences corresponding to the heavy and light chains set forth in SEQ ID NOs: 60 and 61.

In certain embodiments, the DVD-Ig protein used in the methods and compositions of the disclosure specifically binds TNF/IL-17, and comprises amino acid sequences corresponding to the heavy and light chain CDRs set forth in SEQ ID NOs: 62 and 63. In certain embodiments, the anti-TNF/IL-17-Ig protein comprises amino acid sequences corresponding to the heavy and light chain variable regions set forth in SEQ ID NOs: 62 and 63. In certain embodiments, the anti-TNF/IL-17-Ig protein comprises the amino acid sequences corresponding to the heavy and light chains set forth in SEQ ID NOs: 62 and 63.

In certain embodiments, the DVD-Ig protein used in the methods and compositions of the disclosure specifically binds TNF/PGE2, and comprises amino acid sequences corresponding to the heavy and light chain CDRs set forth in SEQ ID NOs: 64 and 65. In certain embodiments, the anti-TNF/PGE2-Ig protein comprises amino acid sequences corresponding to the heavy and light chain variable regions set forth in SEQ ID NOs: 64 and 65. In certain embodiments, the anti-TNF/PGE2-Ig protein comprises the amino acid sequences corresponding to the heavy and light chains set forth in SEQ ID NOs: 64 and 65. In certain embodiments, the DVD-Ig protein used in the methods and compositions of the disclosure specifically binds IL-1α/IL-1β, and comprises amino acid sequences corresponding to the heavy and light chain CDRs set forth in SEQ ID NOs: 66 and 67. In certain embodiments, the anti-IL-1α/IL-1β-Ig protein comprises amino acid sequences corresponding to the heavy and light chain variable regions set forth in SEQ ID NOs: 66 and 67. In certain embodiments, the anti-IL-1α/IL-1β-Ig protein comprises the amino acid sequences corresponding to the heavy and light chains set forth in SEQ ID NOs: 66 and 67.

In certain embodiments, the DVD-Ig protein used in the methods and compositions of the disclosure specifically binds DLL4/VEGF, and comprises amino acid sequences corresponding to the heavy and light chain CDRs set forth in SEQ ID NOs: 28 and 29. In certain embodiments, the anti-DLL4/VEGF-Ig protein comprises amino acid sequences corresponding to the heavy and light chain variable regions set forth in SEQ ID NOs: 28 and 29. In certain embodiments, the anti-DLL4/VEGF-Ig protein comprises the amino acid sequences corresponding to the heavy and light chains set forth in SEQ ID NOs: 28 and 29.

In certain embodiments, the DVD-Ig protein used in the methods and compositions of the disclosure specifically binds DLL4/VEGF, and comprises amino acid sequences corresponding to the heavy and light chain CDRs set forth in SEQ ID NOs: 72 and 73. In certain embodiments, the anti-DLL4/VEGF-Ig protein comprises amino acid sequences corresponding to the heavy and light chain variable regions set forth in SEQ ID NOs: 72 and 73. In certain embodiments, the anti-DLL4/VEGF-Ig protein comprises the amino acid sequences corresponding to the heavy and light chains set forth in SEQ ID NOs: 72 and 73.

In certain embodiments, the DVD-Ig protein used in the methods and compositions of the disclosure specifically binds IL12/IL18, and comprises amino acid sequences corresponding to the heavy and light chain CDRs set forth in SEQ ID NOs: 70 and 71. In certain embodiments, the anti-IL12/IL18-Ig protein comprises amino acid sequences corresponding to the heavy and light chain variable regions set forth in SEQ ID NOs: 70 and 71. In certain embodiments, the anti-IL12/IL18-Ig protein comprises the amino acid sequences corresponding to the heavy and light chains set forth in SEQ ID NOs: 70 and 71.

D. Expression of DVD-Ig Proteins

DVD-Ig proteins of the present disclosure may be produced by any of a number of techniques known in the art. For example, expression from host cells, wherein expression vector(s) encoding the DVD heavy and DVD light chains is (are) transfected into a host cell by standard techniques. The various forms of the term "transfection" are intended to encompass a wide variety of techniques commonly used for the introduction of exogenous DNA into a prokaryotic or eukaryotic host cell, *e.g.*, electroporation, calcium-phosphate precipitation, DEAE-dextran transfection and the like.

Mammalian host cells for expressing the recombinant antibodies of the disclosure include Chinese Hamster Ovary (CHO cells) (including dhfr-CHO cells, described in Urlaub and Chasin (1980) Proc. Natl. Acad. Sci. USA 77:4216-4220, used with a DHFR selectable marker, *e.g.*, as described in Kaufman and Sharp (1982) J. Mol. Biol. 159:601-621) and DG44 or DUXB11 cells (Urlaub et al. (1986) Som. Cell Molec. Genet. 12:555; Haynes et al. (1983) Nuc. Acid. Res. 11:687-706; Lau et al. (1984) Mol. Cell. Biol. 4:1469-1475), NS0 myeloma cells, monkey kidney line (*e.g.*, CVI and COS, such as a COS 7 cell), SP2 cells, human embryonic kidney (HEK) cells, such as a HEK-293 cell, Chinese hamster fibroblast (*e.g.*, R1610), human cervical carcinoma (*e.g.*, HELA), murine fibroblast (*e.g.*, BALBc/3T3), murine myeloma (P3x63-Ag3.653; NS0; SP2/O), hamster kidney line (*e.g.*, HAK), murine L cell (*e.g.*, L-929), human lymphocyte (*e.g.*, RAJI), human kidney (*e.g.*, 293 and 293T). Host cell lines are typically commercially available (*e.g.*, from BD Biosciences, Lexington, Ky.; Promega, Madison, Wis.; Life Technologies, Gaithersburg, Md.) or from the American Type Culture Collection (ATCC, Manassas, Va.).

When recombinant expression vectors encoding DVD-Ig proteins are introduced into mammalian host cells, the DVD-Ig proteins are produced by culturing the host cells for a period of time sufficient to allow for expression of the DVD-Ig proteins in the host cells or secretion of the DVD-Ig proteins into the culture medium in which the host cells are grown. DVD-Ig proteins can be recovered from the culture medium using standard protein purification methods.

In an exemplary system for recombinant expression of DVD-Ig proteins, a recombinant expression vector encoding both the DVD-Ig heavy chain and the DVD-Ig light chain is introduced into dhfr-CHO cells by calcium phosphate-mediated transfection. Within the recombinant expression vector, the DVD-Ig heavy and light chain cDNAs are each operatively linked to CMV enhancer/AdMLP promoter regulatory elements to drive high levels of transcription of the cDNAs. The recombinant expression vector also carries cDNA encoding DHFR, which allows for selection of CHO cells that have been transfected with the vector using methotrexate selection/amplification. The selected transformant host cells are cultured to allow for expression of the DVD-Ig heavy and light chains and intact DVD-Ig protein is recovered from the culture medium. Standard molecular biology techniques are used to prepare the recombinant expression vector, transfect the host cells, select for transformants, culture the host cells and recover the DVD-Ig protein from the culture medium. Still further, the disclosure provides a method of synthesizing a DVD-Ig protein of the disclosure by culturing a host cell of the disclosure in a suitable culture medium until a DVD-Ig protein of the disclosure is synthesized. The method can further comprise isolating the DVD-Ig protein from the culture medium. An important feature of DVD-Ig protein is that it can be produced and purified in a similar way as a conventional antibody.

E. Methods for Identifying Aqueous Stable DVD-Ig (AS-DVD-Ig) Proteins and Lyophilized Stable DVD-Ig (LS-DVD-Ig) Proteins

An unexpected and surprising finding is that a certain subset of DVD-Ig proteins (referred to as AS-DVD-Ig protein and LS-DVD-Ig proteins) are stable - even at high concentrations - in aqueous formulations, while a large number of DVD-Ig proteins are unstable and prone to aggregation. In addition, while the majority of DVD-Ig proteins have been found not to be stable in a lyophilized state, a certain subset of DVD-Ig proteins (referred to as LS-DVD-Ig proteins) are stable and can be successfully lyophilized using the formulations of the disclosure. Notably, DVD-Ig proteins identified as AS-DVD-Ig proteins are also LS-DVD-Ig proteins given that AS-DVD-Ig proteins are generally more stable than LS-DVD-Ig proteins. In addition, a non-AS-DVD Ig protein can be an LS DVD-Ig protein. The distinction between the two subpopulations may be based on the level of aggregation, freeze/thaw characteristics, or monomer content following storage, as described in the below assays. Generally, an increase in aggregation indicates a decrease in monomer content.

Thus, in certain embodiments, the disclosure comprises a method for distinguishing between AS-DVD-Ig proteins and non-AS-DVD-Ig proteins. The disclosure also comprises a method for distinguishing between LS-DVD-Ig proteins and non-LS-DVD-Ig proteins. In another alternative, the disclosure provides methods for distinguishing AS-DVD-Ig proteins from LS-DVD-Ig proteins. Following identification, AS-DVD-Ig protein and LS-DVD-Ig proteins may be successfully formulated in the compositions of the disclosure, while non-AS-DVD-Ig protein and non-LS-DVD-Ig proteins fail to remain stable in such formulations and are prone to aggregation and/or loss of monomer content. Generally, an increase in aggregation indicates a decrease in monomer content.

In certain embodiments, a freeze/thaw (F/T) (*e.g.*, - 80 ° C / 30° C) test may be used to identify DVD-Ig proteins that are AS-DVD-Ig proteins or LS-DVD-Ig proteins. Such a method relies upon determining the percentage of high molecular weight (HMW) species in a solution having a high concentration of DVD-Ig protein (*e.g.*, 100 mg/ml). In one embodiment, an AS-DVD-Ig protein or an LS-DVD-Ig protein is defined as a DVD-Ig protein that shows 1% or less increase in high molecular weight (HMW) species (aggregates) (*e.g.*, 0.5% or less aggregate) or less than a 1 % increase (*e.g.*, 0.5%) in change in relative % monomer as determined by SEC following a F/T cycle. A F/T cycle includes freezing the DVD-Ig protein solution, thawing the solution, optionally repeating, and testing the thawed solution for aggregate levels using, *e.g.*, SEC analysis. Freeze/thaw testing may be performed on a solution comprising a DVD-Ig protein at a concentration of about 1 mg/ml to about 10 mg/ml. In certain embodiments, the F/T testing includes measuring aggregation or alternatively change in monomer content by SEC after 4 freeze/thaw cycles from about 30 °C to - 80 °C. If testing results in more than about a 1 % increase in relative percent aggregates or change in relative % monomer after 4 cycles, then the DVD-Ig protein would be considered a non- LS DVD Ig.

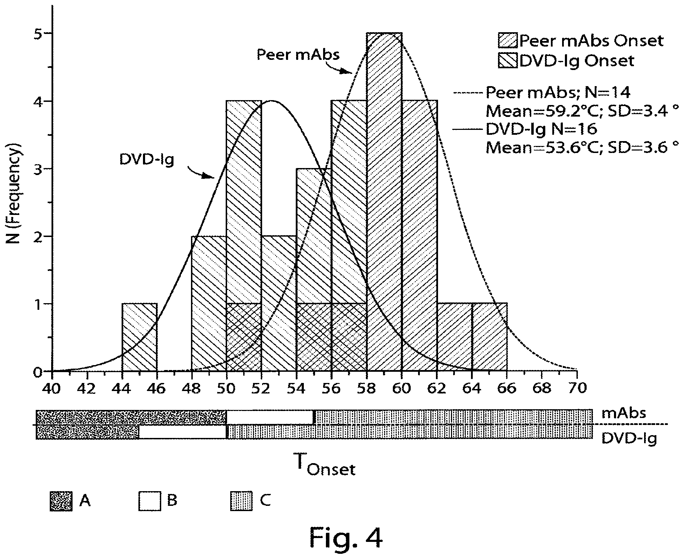

In certain embodiments, the unfolding temperature of a DVD-Ig protein may be used to determine whether the DVD-Ig protein is an AS-DVD-Ig protein or an LS-DVD-Ig protein. As described in Figure 4, a DVD-Ig protein that shows a temperature of unfolding of less than about 45 °C is generally not characterized as an AS-DVD-Ig protein or an LS-DVD-Ig protein ("A" as described in Figure 4). In certain other embodiments, a DVD-Ig protein that shows a temperature of unfolding of about 45-50 °C is unlikely to be an AS-DVD-Ig protein but may be an LS-DVD-Ig protein ("B" as described in Figure 4). In certain other embodiments, a DVD-Ig protein that shows a temperature of unfolding that is higher than 50 °C is generally considered to be an AS-DVD-Ig protein and an LS-DVD-Ig protein ("C" as described in Figure 4). Tests to determine DVD-Ig protein unfolding temperatures are known in the art (see also Example 1 describing thermodynamic testing) and are generally performed at a pH of between about 5 and about 7, *e.g.*, about 5.5 to about 6.5.

In order to determine whether a DVD-Ig protein is an AS-DVD-Ig protein or an LS-DVD-Ig, storage testing of the solution can be performed. For example, storage testing may be performed at 5 °C or 40 °C for 14 to 21 days at a DVD-Ig protein in solution at a concentration ranging from 1 to 100 mg/ml, *e.g.*, about 50-100 mg/ml.

Testing at temperatures greater than ambient temperatures, *e.g.,* 40 °C, are often referred to as accelerated conditions. In certain embodiments, the accelerated storage conditions include storing the DVD-Ig protein in the absence of light at 40 °C. In certain embodiments, testing is based on a solution's DVD-Ig protein aggregation levels at a high temperature (*e.g.*, 40 °C) and a high concentration (*e.g.*, 50 mg/ml) as determined by SEC. For example, the DVD-Ig protein may be formulated at a concentration of at least about 50 mg/ml, *e.g.*, 50 to 100 mg/ml, in an aqueous formulation using a citrate phosphate buffer or a histidine buffer, and stored under accelerated conditions. Accelerated conditions may include temperatures higher than room temperature, *e.g.*, storage temperatures of about 35 to about 45 ° Celsius (C), for extended periods of time, *e.g.*, about 10 to about 21 days. In certain embodiments, the accelerated storage conditions used to screen for an AS-DVD-Ig protein or LS-DVD-Ig protein are 14 days of storage at a temperature of 40 ° C at a DVD-Ig protein concentration of 50 mg/ml or greater, *e.g.,* about 60 mg/ml or 50-100 mg/ml. Following accelerated storage testing at a concentration of 50 mg/ml or greater, *e.g.* 50-100 mg/ml, the solution may be tested for signs of DVD-Ig protein aggregation or change in monomer content.

In certain embodiments, DVD-Ig proteins may be tested to determine whether they are an AS-DVD-Ig protein or an LS-DVD-Ig protein in buffered solutions (*e.g.*, histidine or citrate / phosphate buffer) having a pH of 5.0 to 6.5, *e.g.,* a pH of about 6, and a concentration of about 50 mg/ml to about 100 mg/ml.

Notably, lower levels of DVD-Ig protein concentration (*e.g.*, 1 mg/ml) may also be used to test the protein, wherein lower levels of aggregate would be expected for an AS-DVD-Ig protein or an LS-DVD-Ig. For example, an AS-DVD-Ig protein is a DVD-Ig that has 3% or less aggregation when stored at about 40 °C after 21 days at a concentration of 1 mg/ml in an aqueous formulation.

Protein aggregation may be determined according to methods known in the art, including, but not limited to, Size Exclusion Chromatography (SEC). Generally, an increase in aggregation indicates a decrease in monomer content, which can also be determined using SEC analysis.

In certain embodiments, the DVD-Ig protein is considered an AS-DVD-Ig protein if the solution has 10% or less aggregation of the DVD-Ig protein as determined by Size Exclusion Chromatography (SEC) analysis following accelerated storage at a concentration of 1-100 mg/ml, preferably about 50 mg/ml to about 100 mg/ml. In certain embodiments, the DVD-Ig protein is considered an AS-DVD-Ig protein if the solution has 6% or less aggregation of the DVD-Ig protein as determined by SEC analysis following accelerated storage at a concentration of about 50 mg/ml to about 100 mg/ml. In certain embodiments, the DVD-Ig protein is considered an AS-DVD-Ig protein if the DVD-Ig protein has less than 10%, alternatively less than 9%, less than 8 %, less than 7%, less than 6%, less than 5%, less than 4%, less than 3%, less than 2%, or less than 1% aggregation as determined by SEC analysis following accelerated storage at a concentration of about 50 mg/ml to about 100 mg/ml. In certain embodiments, an AS-DVD-Ig protein is defined as a DVD-Ig that has less than 8% aggregation following 14 days of accelerated storage (at, for example, about 40 °C). In certain embodiments, an AS-DVD-Ig protein is defined as a DVD-Ig that has 6% or less aggregation following 14 days of accelerated storage (at, for example, about 40 °C). In a further embodiment, an AS-DVD-Ig protein is defined as a DVD-Ig that has less than 5% aggregation following 14 days of accelerated storage (at, for example, about 40 °C). In certain embodiments, an AS-DVD-Ig protein is defined as a DVD-Ig that has 10% or less aggregation at about 40 °C after 21 days of storage at a concentration of 100 mg/ml in an aqueous formulation or has 10% or less aggregate following accelerated storage after 14 days at about 40 °C, when formulated at a concentration of 50 mg/ml or more in an aqueous formulation.

In certain embodiments, an AS-DVD-Ig protein is defined as a DVD-Ig protein that has 10% or less aggregation at about 40 °C after 21 days of storage at a concentration of 100 mg/ml in an aqueous formulation or, alternatively, a DVD-Ig protein that has 1% or less aggregation at about 5 °C after 21 days of storage at a concentration of 100 mg/ml in an aqueous formulation. Alternatively, an AS-DVD-Ig protein is a DVD-Ig protein that has 1.5% or less aggregation at about 5 °C after 21 days of storage at a concentration of 1 mg/ml in an aqueous formulation or 3% or less aggregation at about 40 °C after 21 days of storage at a concentration of 1 mg/ml in an aqueous formulation. In certain embodiments, an AS-DVD-Ig protein is defined as a DVD-Ig protein that has less than 10% aggregate formed following accelerated storage after 14 days at about 40 °C, when formulated at a concentration over 50 mg/ml in an aqueous formulation. In certain embodiments, an AS-DVD-Ig protein is defined as a DVD-Ig protein that has less than 10% aggregation following 14 days of accelerated storage (at, for example, about 40 °C). In certain embodiments, an AS-DVD-Ig protein is defined as a DVD-Ig protein that has less than 8% aggregation following 14 days of accelerated storage (at, for example, about 40 °C). In certain embodiments, an AS-DVD-Ig protein is defined as a DVD-Ig protein that has 6% or less aggregation following 14 days of accelerated storage (at, for example, about 40 °C). In a further embodiment, an AS-DVD-Ig protein is defined as a DVD-Ig protein that has less than 5% aggregation following 14 days of accelerated storage (at, for example, about 40 °C). Aggregation can be determined according to methods known in the art, including, but not limited to, size exclusion chromatography (SEC). In certain embodiments, the accelerated storage conditions include storing the DVD-Ig protein in the absence of light at 40 °C. DVD-Ig proteins may be tested in aqueous formulations containing citrate and phosphate buffer, or histidine buffer. In certain embodiments, an AS-DVD-Ig protein has 10% or less aggregation as determined by SEC analysis following accelerated storage for 21 days at about 40 °C, where the AS-DVD-Ig protein is formulated at a concentration of 50 to 100 mg/ml in a citrate phosphate buffer or histidine buffer in an aqueous formulation. In certain embodiments, an AS-DVD-Ig protein has less than 6% aggregation as determined by SEC analysis following accelerated storage for 14 days at about 40 °C, where the AS-DVD-Ig protein is formulated at a concentration of at least 50 mg/ml in a citrate phosphate buffer or histidine buffer in an aqueous formulation.

While percent aggregation may be used to determine whether aggregation is present following accelerated storage, monomer content of the DVD-Ig protein solution may also be used. Generally, an increase in percent aggregation indicates a decrease in monomer content. Alternatively, a DVD-Ig protein may be considered an AS-DVD-Ig protein if the protein has 6% or less monomer loss (determined by SEC) after 14 days at 40 °C or 3% or less monomer loss (determined by SEC) after 7 days at 40 °C in a solution having a concentration of 50 mg/ml or more DVD-Ig protein at pH about 5.0 to about 6.5, *e.g.*, about 5.5 to about 6.0 in 15 mM histidine. Monomer content may be used under any testing conditions, including, but not limited to, storage at 40 °C and/or at a pH of about 5.0 to about 6.5, *e.g.*, about 5.5 to about 6.0.

In certain embodiments, an AS-DVD-Ig protein is a DVD-Ig protein that has about 10% relative (rel.) peak area or less change in monomers at about 40 °C after 21 days of storage at a concentration of about 100 mg/ml in an aqueous formulation or, alternatively, a DVD-Ig protein that has about 1% rel. peak area or less change in monomers at about 5 °C after 21 days of storage at a concentration of about 100 mg/ml and at a pH between about 5.5 to about 6.5 in an aqueous formulation. Alternatively, an AS-DVD-Ig protein is a DVD-Ig protein that has about 1.5% rel. peak area or less change in monomers at about 5 °C after 21 days of storage at a concentration of about 1 mg/ml in an aqueous formulation or about 3% rel. peak area or less change in monomers at about 40 °C after 21 days of storage at a concentration of 1 mg/ml and at a pH between about 5.5 to about 6.5 in an aqueous formulation. In certain embodiments, an AS-DVD-Ig protein is defined as a DVD-Ig protein that has a change in monomers less than about 10% rel. peak area following accelerated storage after 14 days at about 40 °C, when formulated at a concentration over about 50 mg/ml and at a pH between about 5.5 and about 6.5 in an aqueous formulation. In certain embodiments, an AS-DVD-Ig protein is defined as a DVD-Ig protein that has less than 8% rel. peak area change in monomers following 14 days of accelerated storage (at, for example, about 40 °C) when formulated at a concentration over 60 mg/ml and at a pH between 5.5 - 6.5 in an aqueous formulation. In certain embodiments, an AS-DVD-Ig protein is defined as a DVD-Ig protein that has 6% rel. peak area or less change in monomers following 14 days of accelerated storage (at, for example, about 40 °C). In a further embodiment, an AS-DVD-Ig protein is defined as a DVD-Ig protein that has less than 5% rel. peak area change in monomers following 14 days of accelerated storage (at, for example, about 40 °C).

In certain embodiments, an AS-DVD-Ig protein has 10% rel. peak area or less change in monomers as determined by SEC analysis following accelerated storage for 21 days at about 40 °C, where the AS-DVD-Ig protein is formulated at a concentration of 50 to 100 mg/ml in a citrate phosphate buffer or histidine buffer at a pH between about 5.0 to about 6.5, *e.g.*, about 5.5 to about 6.0, in an aqueous formulation. In certain embodiments, an AS-DVD-Ig protein has less than 6% rel. peak area change in monomers as determined by SEC analysis following accelerated storage for 14 days at about 40 °C, where the AS-DVD-Ig protein is formulated at a concentration of at least 50 mg/ml in a citrate phosphate buffer or histidine buffer in an aqueous formulation.

In another alternative, AS-DVD-Ig proteins are identified based on a solution's stability aggregation and/or monomer content at a low temperature (*e.g.*, 5 °C) and a high concentration (*e.g.*, 50 mg/ml) of DVD-Ig as determined by SEC. For example, a solution containing 50 mg/ml of an AS-DVD-Ig protein at a pH of about 5.0 to about 6.5, *e.g.*, about 5.5 to about 6.0 in 15 mM histidine may have 1% or less monomer (determined by SEC) loss after 7 days at 5° C (determined by SEC). In another example, a solution containing 50 mg/ml of an AS-DVD-Ig protein at a pH of about 5.0 to about 6.5, *e.g.*, about 5.5 to about 6.0 in 15 mM histidine may have 2% or less monomer loss after 14 days at 5 ° C. Alternatively, an AS-DVD-Ig has 1% or less aggregation at about 5 °C after 21 days of storage at a concentration of 100 mg/ml in an aqueous formulation, or 1.5% or less aggregation at about 5°C after 21 days of storage at a concentration of 1 mg/ml in an aqueous formulation. In certain embodiments, monomer loss is determined at a pH of about 5.0 to about 6.5, *e.g.*, about 5.5 to about 6.0.

In certain embodiments, the test solution conditions described herein also contain 0.02% (w/v) sodium azide as a bacteriostatic.

In certain embodiments, the DVD-Ig protein is considered an LS-DVD-Ig protein if the solution has 15% or less aggregation of the DVD-Ig protein as determined by Size Exclusion Chromatography (SEC) analysis. In certain embodiments, the LS-DVD-Ig protein is considered an LS-DVD-Ig protein if the DVD-Ig protein has 15% or less, alternatively less than 14%, less than 13%, less than 12%, less than 11%, less than aggregation as determined by SEC analysis.

In certain embodiments, an LS-DVD-Ig protein is defined as having less than 15% aggregate formed, following accelerated storage, *e.g.*, 14 days of accelerated storage at about 40 °C, when formulated at a concentration over 50 mg/ml in an aqueous formulation. In certain embodiments, an LS-DVD-Ig protein has 15% or less aggregation following 14 days of accelerated storage at, for example, 40 °C. In certain embodiments, an LS-DVD-Ig protein has less than 14% aggregation following 14 days of accelerated storage, at, for example, about 40 °C. In a further embodiment, an LS-DVD-Ig protein has less than 13% aggregation following 14 days of accelerated storage, at, for example, about 40 °C. Alternatively, an LS-DVD-Ig protein is defined as a DVD-Ig protein that has 1% or less aggregation following 4 freeze thaw cycles. DVD-Ig proteins may be tested in aqueous formulations containing citrate and phosphate buffer, or histidine buffer. In certain embodiments, an LS-DVD-Ig protein has 15% or less aggregation as determined by SEC analysis following accelerated storage for 14 days at about 40 °C, where the LS-DVD-Ig protein is formulated at a concentration of at least 50 mg/ml in a citrate phosphate buffer in an aqueous formulation. As described above, aggregation can be determined according to methods known in the art, including, but not limited to, SEC.

In certain embodiments, an LS-DVD-Ig protein has more than 10% rel. peak area change in monomers observed, following accelerated storage, *e.g.*, 21 days at about 40 °C, when formulated at a concentration of about 50 to 100 mg/ml at a pH between about 5.0 and about 6.5, *e,g,* about 5.5 to about 6.0, in an aqueous formulation. In certain embodiments, an LS-DVD-Ig protein has 50% rel. peak area or less change in monomers as determined by SEC analysis following accelerated storage for 14 days at about 40 °C, where the LS-DVD-Ig protein is formulated at a concentration of at least 50 mg/ml in a citrate phosphate buffer in an aqueous formulation. In certain embodiments, an LS-DVD-Ig protein has 20 % rel. peak area or less change in monomers following 21 days of accelerated storage at about 40 °C, when formulated at a concentration over 100 mg/ml at a pH between about 5.0 to about 6.5, *e.g.*, about 5.5 to about 6.0 in an aqueous formulation. In certain embodiments, an LS-DVD-Ig protein has less than 18% rel. peak area change in monomers following 14 days of accelerated storage, at, for example, about 40 °C. In a further embodiment, an LS-DVD-Ig protein has less than 13% rel. peak area change in monomers following 14 days of accelerated storage, at, for example, about 40 °C. Alternatively, an LS-DVD-Ig protein is defined as a DVD-Ig protein that has 1% rel. peak area or less change in monomers following 4 freeze thaw cycles. Alternatively, an LS-DVD-Ig protein is defined as a DVD-Ig protein that has 4% rel. peak area or less change in monomers following 7 days at about 25 °C at a concentration between 1-100 mg/mL, *e.g.*, about 1 to 10 mg/ml or about 50 to 100 mg/ml, in aqueous solution at the most stable pH. Alternatively, an LS-DVD-Ig protein is defined as a DVD-Ig protein that has 1 % rel. peak area or less change in monomers following 7 days at about 5 °C in aqueous solution at the most stable

Notably, the assays described herein used to determine whether a DVD-Ig protein is an AS-DVD-Ig protein or an LS-DVD-Ig protein are not exclusive, meaning that, for example, an AS-DVD-Ig may be characterized as having an unfolding temperature of at least 50 °C and also have about a 1% relative peak area or less change in monomers at about 5 °C after 21 days of storage at a concentration of about 50 to about 100 mg/ml and at a pH between about 5.5 to about 6.5 in an aqueous, buffered formulation. In certain embodiments, an AS-DVD-Ig protein has 10% rel. peak area or less change in monomers as determined by SEC analysis following accelerated storage for 21 days at about 40 °C and has an unfolding temperature of at least 50 °C.

Once the DVD-Ig protein is identified as being an AS-DVD-Ig protein or LS-DVD-Ig protein according to the aforementioned tests, the AS-DVD-Ig protein or LS-DVD-Ig protein can be stably formulated. Further identification of AS-DVD-Ig protein and LS-DVD-Ig proteins is described below in Example 4.

## III. Aqueous Stable Dual Variable Domain Immunoglobulin (AS-DVD-Ig) Formulations of the Disclosure

The disclosure provides stable aqueous formulations comprising AS-DVD-Ig proteins. The present disclosure features aqueous formulations having improved properties as compared to art-recognized formulations, in that AS-DVD-Ig proteins can be stably formulated, even at high concentrations.

Thus, the disclosure is based, at least in part, on the discovery that a subpopulation of DVD-Ig proteins can be stably formulated in an aqueous formulation having a pH of about 4.5 to about 7.5, and containing a buffer, a surfactant, and/or a polyol. These "Aqueous Stable DVD-Ig proteins" are referred to as AS-DVD-Ig proteins and can be identified using an accelerated storage assay where the DVD-Ig protein is formulated in a liquid form at a concentration greater than 50 mg/ml, *e.g.*, 50 mg/ml to 100 mg/ml (see also Section II.E. above).

In certain embodiments, the aqueous formulation of the disclosure has a pH of about 4.5 to about 7.5. As described in the working examples, pH was found to have an impact on the stability of the AS-DVD-Ig protein in a buffered formulation. In certain embodiments, the pH of the formulation containing the AS-DVD-Ig protein ranges from about 4.5 to about 7.5; alternatively, the pH of the AS-DVD-Ig protein formulation ranges from about 5.0 to about 7.0; alternatively the pH may range from about 5 to about 6.5; alternatively the pH of the formulation may range from about 5.5 to about 6.5. In a further embodiment, the pH ranges from about 5.8 to about 6.2. The ranges intermediate to the aforementioned pH values, *e.g.*, about 5.6 to about 6.4, are also intended to be part of the disclosure. Ranges of values using a combination of any of the aforementioned values as upper / lower limits are also intended to be included, *e.g.*, a pH range of about 5.5 to about 6.2. In certain embodiments, the pH of the formulation of the disclosure is about 6.0.

In certain embodiments, the aqueous formulation of the disclosure includes an AS-DVD-Ig protein and a buffer. Examples of buffers that may be used in the formulation of the disclosure include, but are not limited to, acetate, histidine, glycine, arginine, phosphate, Tris, and citrate. The molarity of the buffer used in the formulation of the disclosure may range from about 1 to about 50 mM. In certain embodiments, the aqueous formulation of the disclosure has a buffer with a molarity of about 5 to about 50 mM. Alternatively, the molarity of the buffer is about 10 to about 20 mM.

In certain embodiments of the disclosure, the buffer system comprises about 1 to about 200 mM histidine (*e.g.*, about 2 to about 100 mM; about 5 to about 70 mM; about 5 to about 60 mM; about 5 to about 50 mM; about 10 to about 40 mM, about 10 to about 30 mM, or about 10 to about 20 mM) with a pH of about 4.5 to about 7.5, *e.g.*, a pH of about 5 to about 7, or a pH of about 5.5 to about 6.5. In certain embodiments, the buffer system of the disclosure comprises about 15 mM histidine with a pH of about 4.5 to about 7.5, *e.g.*, a pH of about 5 to about 7, or a pH of about 5.5 to about 6.5.

In certain embodiments, the buffer system comprises about 1 to about 50 mM (*e.g.*, about 5 to about 40 mM) glycine with a pH of about 4.5 to about 7.5. In a particular embodiment, the buffer system comprises glycine at a concentration of about 20 mM. In a more particular embodiment, the buffer system comprises glycine at a concentration of about 20 mM, and glycerol at a concentration of about 20 to about 30 mg/ml, *e.g.*, about 26 mg/ml, with a pH of about 4.5 to about 7.5, *e.g.*, a pH of about 5 to about 7, or a pH of about 5.5 to about 6.5.

In certain embodiments, the buffer system comprises about 1 to about 50 mM acetate (*e.g.*, about 5 to about 50 mM, about 2 to about 40 mM; about 5 to about 30 mM; or about 2 to about 15 mM) with a pH of about 4.5 to about 7.5, *e.g.*, a pH of about 5 to about 7, or a pH of about 5.5 to about 6.5. In a particular embodiment, the buffer system comprises acetate at a concentration of about 2 to about 15 mM.

In certain embodiments of the disclosure, the buffer system comprises about 1 to about 50 mM (*e.g.*, about 5 to about 50 mM, about 2 to about 40 mM; about 5 to about 30 mM; or about 2 to about 15 mM) arginine with a pH of about 4.5 to about 7.5, *e.g.*, a pH of about 5 to about 7, or a pH of about 5.5 to about 6.5. In a particular embodiment, the buffer system comprises arginine at a concentration of about 15 mM.

In still another embodiment of the disclosure, the buffer system comprises about 1 to about 50 mM (*e.g.*, about 5 to about 50 mM) citrate with a pH of about 4.5 to about 7.5, *e.g.*, a pH of about 5 to about 7, or a pH of about 5.5 to about 6.5. In a particular embodiment, the buffer system comprises citrate at a concentration of about 15 mM.

In still another embodiment of the disclosure, the buffer system comprises about 1 to about 50 mM (*e.g.*, about 5 to about 50 mM) phosphate with a pH of about 4.5 to about 7.5, *e.g.*, a pH of about 5 to about 7, or a pH of about 5.5 to about 6.5. In a particular embodiment, the buffer system comprises phosphate at a concentration of about 10 mM. In a one embodiment, the buffer system comprises phosphate at a concentration of about 10 mM, and sodium chloride at a concentration of about 125 mM.

In certain embodiments, the buffer system comprises about 1 to about 50 mM (*e.g.*, about 5 to about 50 mM) Tris with a pH of about 4.5 to about 7.5, *e.g.*, a pH of about 5 to about 7, or a pH of about 5.5 to about 6.5. In a particular embodiment, the buffer system comprises Tris at a concentration of about 2 to about 10 mM.

In yet another embodiment, the buffer system comprises phosphate and citrate, *e.g.*, phosphate (*e.g.*, sodium hydrogen phosphate) at a concentration of about 1 to about 50 mM (*e.g.*, about 5 to about 50 mM, about 5 to about 10 mM), and citrate (citric acid) at a concentration of about 1 to about 50 mM (*e.g.*, about 5 to about 10 mM). In a particular embodiment, the buffer system comprises phosphate at a concentration of about 5 mM and citrate (citric acid) at a concentration of about 5mM. In certain embodiments, the buffer system comprises phosphate at a concentration of about 10 mM and citrate (citric acid) at a concentration of about 10 mM.

In addition to the buffer, a polyol may be added to the aqueous formulation, *e.g.*, for added stability. The polyol may be added to the formulation in an amount that may vary with respect to the desired isotonicity of the formulation. In certain embodiments, the aqueous formulation is isotonic.

Examples of polyols that may be used in the aqueous formulations of the disclosure include, but are not limited to, sorbitol, mannitol, and sucrose fructose, mannose, maltose, lactose, arabinose, xylose, ribose, rhamnose, galactose and glucose. Nonreducing sugars include sucrose, trehalose, sorbose, melezitose, raffinose, mannitol, xylitol, erythritol, threitol, sorbitol and glycerol. The amount of polyol added may also vary with respect to the molecular weight of the polyol. For example, a lower amount of a monosaccharide (*e.g.*, mannitol) may be added, compared to a disaccharide (*e.g.*, trehalose).

In certain embodiments, the concentration of a polyol such as sorbitol is about 30 to about 50 mg/ml. In a one embodiment, the composition comprises about 20 to about 60 mg/ml sorbitol, about 25 to about 55 mg/ml, about 30 to about 50 mg/ml, about 35 to about 45 mg/ml, and ranges in between, *e.g.*, about 33 to about 48 mg/ml of sorbitol.

In certain embodiments, sucrose has a concentration of about 70 to about 90 mg/ml. In certain embodiments, the composition comprises about 60 to about 100 mg/ml sucrose, about 65 to about 95 mg/ml, about 70 to about 90 mg/ml, about 75 to about 85 mg/ml, and ranges in between, *e.g.*, about 72 to about 84 mg/ml of sucrose.

In certain embodiments, the polyol is mannitol. In certain embodiments, the composition comprises about 10 to about 100 mg/ml, or about 20 to about 80, about 20 to about 70, about 30 to about 60, about 30 to about 50 mg/ml of mannitol, for example, about 10, about 20, about 30, about 40, about 50, about 60, about 70, about 80, about 90, or about 100 mg/ml.

In certain embodiments, the aqueous formulation of the disclosure includes an AS-DVD-Ig, a buffer having a molarity of about 5 to about 50 mM, and a polyol, wherein the formulation has a pH of about 4.5 to about 7.5.

In addition to the buffer, a surfactant may be added to the aqueous formulations, *e.g.*, for added stability. Exemplary surfactants include nonionic detergents such as polysorbates (*e.g.*, polysorbates 20, 80) or poloxamers (*e.g.*, poloxamer 188). In certain embodiments, the amount of surfactant added is such that it reduces aggregation of the formulated AS-DVD-Ig protein and/or minimizes the formation of particulates in the formulation and/or reduces adsorption.

In certain embodiments, the aqueous formulation contains the detergent polysorbate 80 or Tween 80. Tween 80 is a term used to describe polyoxyethylene (20) sorbitan monooleate. In certain embodiments, the formulation contains about 0.001 to about 1% polysorbate 80, or about 0.005 and about 0.05% polysorbate 80, for example, about 0.001%, about 0.005, about 0.01%, about 0.05%, or about 0.1% polysorbate 80. In certain embodiments, about 0.01% polysorbate 80 is found in the formulation of the disclosure.

In certain embodiments, the aqueous formulation of the disclosure includes an AS-DVD-Ig, a buffer having a molarity of about 5 to about 50 mM, and a surfactant, wherein the formulation has a pH of about 4.5 to about 7.5. In certain embodiments, the surfactant is a polysorbate, *e.g.*, polysorbate 80 or polysorbate 20. In certain embodiments, the polysorbate has a concentration of about 0.005% to about 0.02%.

In certain embodiments, the aqueous formulation of the disclosure includes an AS-DVD-Ig, a buffer having a molarity of about 5 to about 50 mM, a surfactant, and a polyol, wherein the formulation has a pH of about 4.5 to about 7.5. In certain embodiments, the formulation includes an AS-DVD-Ig, a buffer (*e.g.,* histidine), a polysorbate, *e.g.,* polysorbate 80, and a sugar alcohol, *e.g.,* mannitol or sorbitol. In certain embodiments, the formulation includes an AS-DVD-Ig, a buffer (*e.g.,* histidine), a polysorbate, *e.g.,* polysorbate 80, and a non-reducing sugar, *e.g.*, sucrose.

One advantage of the aqueous formulation of the disclosure is that high concentrations of AS-DVD-Ig proteins may be stably maintained in an aqueous solution. Thus, in certain embodiments, the formulations of the disclosure comprise a high protein concentration, including, for example, a protein concentration greater than about 10 mg/ml, greater than about 20 mg/ml, greater than about 30 mg/ml, greater than about 40 mg/ml, greater than about 50 mg/ml, greater than about 100 mg/ml, greater than about 110 mg/ml, greater than about 120 mg/ml, greater than about 130 mg/ml, greater than about 140 mg/ml, greater than about 150 mg/ml, greater than about 160 mg/ml, greater than about 170 mg/ml, greater than about 180 mg/ml, greater than about 190 mg/ml, or greater than about 200 mg/ml.

In various embodiments of the disclosure, the concentration of the AS-DVD-Ig protein in the aqueous formulation is about 0.1-250 mg/ml, about 0.5-220 mg/ml, about 1-210 mg/ml, about 5-200 mg/ml, about 10-195 mg/ml, about 15-190 mg/ml, about 20-185 mg/ml, about 25-180 mg/ml, about 30-175 mg/ml, about 35-170 mg/ml, about 40-165 mg/ml, about 45-160 mg/ml, about 50-155 mg/ml, about 55-150 mg/ml, about 60-145 mg/ml, about 65-140 mg/ml, about 70-135 mg/ml, about 75-130 mg/ml, about 80-125 mg/ml, about 85-120 mg/ml, about 90-H5 mg/ml, about 95-110 mg/ml, about 95-105 mg/ml, or about 100 mg/ml. Ranges intermediate to the above recited concentrations, *e.g.,* about 31-174 mg/ml, are also intended to be part of this disclosure. For example, ranges of values using a combination of any of the above recited values as upper and/or lower limits are intended to be included.

The present disclosure features aqueous formulations having improved properties as compared to art-recognized formulations. For example, the formulations of the disclosure have an AS-DVD-Ig protein aggregation level of less than 7% aggregate, less than 6% aggregate, or less than 5% aggregate.

In certain embodiments, the AS-DVD-Ig protein used in the aqueous formulation of the disclosure is an anti-TNF/IL-17 DVD-Ig protein having heavy and light chain sequences having an amino acid sequence as set forth in SEQ ID NOs: 62 and 63, respectively.

In certain embodiments, the aqueous formulation of the disclosure comprises an anti-TNF/IL-17 DVD-Ig protein (*e.g.*, an anti-TNF/IL-17 DVD-Ig protein comprising a heavy and a light chain comprising amino acid sequences corresponding to the heavy and light chain CDRs set forth in SEQ ID NOs: 62 and 63; or an anti-TNF/IL-17 DVD-Ig protein comprising a heavy and a light chain comprising amino acid sequences corresponding to the heavy and light chain variable regions set forth in SEQ ID NOs: 62 and 63; or DVD-A), histidine or acetate buffer, and sucrose or sorbitol, wherein the formulation has a pH of about 5 to about 6 and wherein the protein concentration is about 50 mg/ml or more. In further embodiments, the aqueous formulation has a pH ranging from about 5 to about 5.5, *e.g.,* about 5.2. In a further embodiment, the aqueous formulation comprises a surfactant, *e.g.,* a polysorbate. Ranges for the recited buffers, excipients, and pH are described above.

In certain embodiments, the AS-DVD-Ig protein is an anti-IL1α/IL-1β DVD-Ig comprising an anti-1L1α/IL1β DVD-Ig protein having a heavy and light chain sequences having an amino acid sequence as set forth in SEQ ID NOs: 66 and 67, respectively.

In certain embodiments, the aqueous formulation of the disclosure comprises an anti-IL1α/IL1β DVD-Ig protein (*e.g.*, an anti-IL1α/IL1β DVD-Ig protein comprising a heavy and a light chain comprising amino acid sequences corresponding to the heavy and light chain CDRs set forth in SEQ ID NOs: 66 and 67; or an anti-TNF/IL-17 DVD-Ig protein comprising a heavy and a light chain comprising amino acid sequences corresponding to the heavy and light chain variable regions set forth in SEQ ID NOs: 66 and 67; or DVD-C), histidine buffer, and sucrose or sorbitol, wherein the formulation has a pH of about 5 to about 6 and wherein the protein concentration is about 50 mg/ml or more. In further embodiments, the aqueous formulation has a pH ranging from about 5 to about 5.5, *e.g.,* about 5.4. In a further embodiment, the aqueous formulation comprises a surfactant, *e.g.,* a polysorbate. Ranges for the recited buffers, excipients, and pH are described above.

In certain embodiments, the aqueous formulation of the disclosure comprises an anti-DLL4/VEGF DVD-Ig protein (*e.g.*, an anti-DLL4/VEGF DVD-Ig protein comprising a heavy and a light chain comprising amino acid sequences corresponding to the heavy and light chain CDRs set forth in SEQ ID NOs: 28 and 29 or SEQ ID NOs: 72 and 73; or an anti-DLL4/VEGF DVD-Ig protein comprising a heavy and a light chain comprising amino acid sequences corresponding to the heavy and light chain variable regions set forth in SEQ ID NOs: 28 and 29 or SEQ ID NOs: 72 and 73), histidine buffer, and sucrose or arginine, wherein the formulation has a pH of about 5 to about 6 and wherein the protein concentration is about 20 mg/mL to about 50 mg/ml. In further embodiments, the aqueous formulation has a pH ranging from about 5.5 to about 6 In a further embodiment, the aqueous formulation comprises a surfactant, *e.g.,* a polysorbate. Ranges for the recited buffers, excipients, and pH are described above.

**IV. Lyophilized Stable Dual Variable Domain Immunoglobulin (LS-DVD-Ig) Protein Formulations of the Disclosure**

The disclosure further provides stable lyophilized formulations comprising LS-DVD-Ig proteins. Thus, the disclosure is based, at least in part, on the discovery that a subpopulation of DVD-Ig proteins can be stably formulated in a lyophilized formulation having a pH of about 4.5 to about 7.5, and containing a buffer, a surfactant, and/or a polyol. These "Lyophilized Stable DVD-Ig proteins" or "LS-DVD-Ig proteins" can be identified using an accelerated storage assay (described above) where the DVD-Ig protein is formulated in a liquid form at a concentration greater than 50 mg/ml.

The below-mentioned features of the lyophilized formulations of the disclosure may refer to the solution prior to lyophilization or, alternatively, the reconstituted formulation, for example, where liquid concentrations (mg/ml) are referenced.

In certain embodiments, the lyophilized formulation of the disclosure has a pH of about 4.5 to about 7.5. In certain embodiments, the pH of the formulation containing the LS-DVD-Ig protein ranges from about 4.5 to about 7.5; alternatively, the pH of the LS-DVD-Ig protein formulation ranges from about 5.0 to about 7.0; alternatively the pH may range from about 5 to about 6.5; alternatively the pH of the formulation may range from about 5.5 to about 6.5. In a further embodiment, the pH ranges from about 5.8 to about 6.2. The ranges intermediate to the aforementioned pH values, *e.g.,* about 5.6 to about 6.4, are also intended to be part of the disclosure. Ranges of values using a combination of any of the aforementioned values as upper / lower limits are also intended to be included, *e.g.,* a pH range of about 5.5 to about 6.2. In certain embodiments, the pH of the formulation of the disclosure is about 6.0.

In certain embodiments, the lyophilized formulation of the disclosure includes an LS-DVD-Ig protein and a buffer. Examples of buffers that may be used in the formulation of the disclosure include, but are not limited to, acetate, histidine, glycine, arginine, phosphate, Tris, and citrate. The molarity of the buffer used in the formulation of the disclosure may range from about 1 to about 50 mM. In certain embodiments, the aqueous formulation of the disclosure has a buffer with a molarity of about 5 to about 50 mM. Alternatively, the molarity of the buffer is about 10 to about 20 mM.

In certain embodiments of the disclosure, the buffer system comprises about 1 to about 200 mM histidine (*e.g.*, about 2 to about 100 mM; about 5 to about 70 mM; about 5 to about 60 mM; about 5 to about 50 mM; about 10 to about 40 mM, about 10 to about 30 mM, or about 10 to about 20 mM) with a pH of about 4.5 to about 7.5, *e.g.,* a pH of about 5 to about 7, or a pH of about 5.5 to about 6.5. In certain embodiments, the buffer system of the disclosure comprises about 15 mM histidine with a pH of about 4.5 to about 7.5, *e.g.,* a pH of about 5 to about 7, or a pH of about 5.5 to about 6.5.

In certain embodiments, the buffer system comprises about 1 to about 50 mM (*e.g.*, about 5 to about 40 mM) glycine with a pH of about 4.5 to about 7.5. In a particular embodiment, the buffer system comprises glycine at a concentration of about 20 mM. In a more particular embodiment, the buffer system comprises glycine at a concentration of about 20 mM, and glycerol at a concentration of about 20 to about 30 mg/ml, *e.g.,* about 26 mg/ml, with a pH of about 4.5 to about 7.5, *e.g.,* a pH of about 5 to about 7, or a pH of about 5.5 to about 6.5.

In certain embodiments, the buffer system comprises about 1 to about 50 mM acetate (*e.g.*, about 5 to about 50 mM, about 2 to about 40 mM; about 5 to about 30 mM; or about 2 to about 15 mM) with a pH of about 4.5 to about 7.5, *e.g.,* a pH of about 5 to about 7, or a pH of about 5.5 to about 6.5. In a particular embodiment, the buffer system comprises acetate at a concentration of about 2 to about 15 mM.

In certain embodiments of the disclosure, the buffer system comprises about 1 to about 50 mM (*e.g.,* about 5 to about 50 mM, about 2 to about 40 mM; about 5 to about 30 mM; or about 2 to about 15 mM) arginine with a pH of about 4.5 to about 7.5, *e.g.,* a pH of about 5 to about 7, or a pH of about 5.5 to about 6.5. In a particular embodiment, the buffer system comprises arginine at a concentration of about 15 mM.

In still another embodiment of the disclosure, the buffer system comprises about 1 to about 50 mM (*e.g.*, about 5 to about 50 mM) citrate with a pH of about 4.5 to about 7.5, *e.g.*, a pH of about 5 to about 7, or a pH of about 5.5 to about 6.5. In a particular embodiment, the buffer system comprises citrate at a concentration of about 15 mM.

In still another embodiment of the disclosure, the buffer system comprises about 1 to about 50 mM (*e.g.,* about 5 to about 50 mM) phosphate with a pH of about 4.5 to about 7.5, *e.g.,* a pH of about 5 to about 7, or a pH of about 5.5 to about 6.5. In a particular embodiment, the buffer system comprises phosphate at a concentration of about 10 mM. In a one embodiment, the buffer system comprises phosphate at a concentration of about 10 mM, and sodium chloride at a concentration of about 125 mM.

In certain embodiments, the buffer system comprises about 1 to about 50 mM (*e.g.*, about 5 to about 50 mM) Tris with a pH of about 4.5 to about 7.5, *e.g.,* a pH of about 5 to about 7, or a pH of about 5.5 to about 6.5. In a particular embodiment, the buffer system comprises Tris at a concentration of about 2 to about 10 mM.

In yet another embodiment, the buffer system comprises phosphate and citrate, *e.g.*, phosphate (*e.g.,* sodium hydrogen phosphate) at a concentration of about 1 to about 50 mM (*e.g.*, about 5 to about 50 mM, about 5 to about 10 mM), and citrate (citric acid) at a concentration of about 1 to about 50 mM (*e.g.*, about 5 to about 10 mM). In a particular embodiment, the buffer system comprises phosphate at a concentration of about 5 mM and citrate (citric acid) at a concentration of about 5mM. In certain embodiments, the buffer system comprises phosphate at a concentration of about 10 mM and citrate (citric acid) at a concentration of about 10 mM.

In addition to the buffer, a polyol may be added to the formulation, *e.g.,* for added stability. The polyol may be added to the formulation in an amount that may vary with respect to the desired isotonicity of the formulation. In certain embodiments, the lyophilized formulation is isotonic upon reconstitution.

Examples of polyols that may be used in the lyophilized formulations of the disclosure include, but are not limited to, mannitol, sucrose, trehalose and raffinose. The amount of polyol added may also vary with respect to the molecular weight of the polyol. For example, a lower amount of a monosaccharide (*e.g.*, mannitol) may be added, compared to a disaccharide (*e.g.*, trehalose).

In certain embodiments, the concentration of a polyol such as sorbitol is about 30 to about 50 mg/ml. In a one embodiment, the composition comprises about 20 to about 60 mg/ml sorbitol, about 25 to about 55 mg/ml, about 30 to about 50 mg/ml, about 35 to about 45 mg/ml, and ranges in between, *e.g.,* about 33 to about 48 mg/ml of sorbitol.

In certain embodiments, sucrose has a concentration of about 70 to about 90 mg/ml. In certain embodiments, the composition comprises about 60 to about 100 mg/ml sucrose, about 65 to about 95 mg/ml, about 70 to about 90 mg/ml, about 75 to about 85 mg/ml, and ranges in between, *e.g.,* about 72 to about 84 mg/ml of sucrose.

In certain embodiments, the polyol is mannitol. In certain embodiments, the composition comprises about 10 to about 100 mg/ml, or about 20 to about 80, about 20 to about 70, about 30 to about 60, about 30 to about 50 mg/ml of mannitol, for example, about 10, about 20, about 30, about 40, about 50, about 60, about 70, about 80, about 90, or about 100 mg/ml.

In certain embodiments, the lyophilized formulation of the disclosure includes an AS-DVD-Ig, a buffer having a molarity of about 5 to about 50 mM, and a polyol, wherein the formulation has a pH of about 4.5 to about 7.5.

In addition to the buffer, a surfactant may be added to the lyophilized formulations, *e.g.,* for added stability. Exemplary surfactants include nonionic detergents such as polysorbates (*e.g.*, polysorbates 20, 60, 80,) or poloxamers (*e.g.*, poloxamer 188). In certain embodiments, the amount of surfactant added is such that it reduces aggregation of the formulated LS-DVD-Ig protein and/or minimizes the formation of particulates in the formulation and/or reduces adsorption.

In certain embodiments, the lyophilized formulation contains the detergent polysorbate 80 or Tween 80. Tween 80 is a term used to describe polyoxyethylene (20) sorbitan monooleate. In certain embodiments, the formulation contains about 0.001 to about 0.1% polysorbate 80, or about 0.005 and about 0.05%, 20 polysorbate 80, for example, about 0.001, about 0.005, about 0.01, about 0.05, or about 0.1% polysorbate 80. In certain embodiments, about 0.01% polysorbate 80 is found in the formulation of the disclosure.

In certain embodiments, the lyophilized formulation of the disclosure includes an LS-DVD-Ig, a buffer having a molarity of about 5 to about 50 mM, and a surfactant, wherein the formulation has a pH of about 4.5 to about 7.5. In certain embodiments, the surfactant is a polysorbate, *e.g.*, polysorbate 80 or polysorbate 20. In certain embodiments, the polysorbate has a concentration of about 0.005% to about 0.02%.

In certain embodiments, the lyophilized formulation of the disclosure includes an LS-DVD-Ig, a buffer having a molarity of about 5 to about 50 mM, a surfactant, and a polyol, wherein the formulation has a pH of about 4.5 to about 7.5. In certain embodiments, the formulation includes an LS-DVD-Ig, a buffer (*e.g.,* histidine), a polysorbate (*e.g.,* polysorbate 80), and a sugar alcohol (*e.g.,* mannitol or sorbitol). In certain embodiments, the formulation includes an LS-DVD-Ig, a buffer (*e.g.,* histidine), a polysorbate, *e.g.,* polysorbate 80, and a non-reducing sugar, *e.g.,* sucrose.

The lyophilized formulation described herein is initially made as a "pre-lyophilized formulation," which is the formulation prior to the lyophilization process. The amount of protein present in the pre-lyophilized formulation is determined taking into account the desired dose volumes, mode(s) of administration etc. For example, the starting concentration of an LS-DVD-Ig protein can be from about 2 mg/ml to about 50 mg/ml.

In certain embodiments, the DVD-Ig protein used in the lyophilized formulations of the disclosure specifically binds TNF/IL-17, and comprises amino acid sequences corresponding to the heavy and light chain CDRs set forth in SEQ ID NOs: 62 and 63. In certain embodiments, the anti-TNF/IL-17-Ig protein comprises amino acid sequences corresponding to the heavy and light chain variable regions set forth in SEQ ID NOs: 62 and 63. In certain embodiments, the anti-TNF/IL-17-Ig protein comprises the amino acid sequences corresponding to the heavy and light chains set forth in SEQ ID NOs: 62 and 63.

In certain embodiments, the lyophilized formulation of the disclosure comprises an anti-TNF/IL-17 DVD-Ig protein ((*e.g.,* an anti-TNF/IL-17 DVD-Ig protein comprising a heavy and a light chain comprising amino acid sequences corresponding to the heavy and light chain CDRs set forth in SEQ ID NOs: 62 and 63; or an anti-TNF/IL-17 DVD-Ig protein comprising a heavy and a light chain comprising amino acid sequences corresponding to the heavy and light chain variable regions set forth in SEQ ID NOs: 62 and 63; or DVD-A), histidine or acetate buffer, and sucrose or sorbitol, where the formulation has a pH of about 5 to about 6 and wherein the protein concentration is greater than about 50 mg/ml upon reconstitution..

In certain embodiments, the DVD-Ig protein used in the lyophilized formulations of the disclosure specifically binds IL1α/IL-1β and comprises a heavy and a light chain sequence comprising an amino acid sequence as set forth in SEQ ID NOs: 66 and 67, respectively. In certain embodiments, the DVD-Ig protein used in the lyophilized formulations specifically binds IL-1α/IL-1β, and comprises amino acid sequences corresponding to the heavy and light chain CDRs set forth in SEQ ID NOs: 66 and 67. In certain embodiments, the anti-IL-1α/IL-1β-Ig protein comprises amino acid sequences corresponding to the heavy and light chain variable regions set forth in SEQ ID NOs: 66 and 67. In certain embodiments, the anti-IL-1α/IL-1β-Ig protein comprises the amino acid sequences corresponding to the heavy and light chains set forth in SEQ ID NOs: 66 and 67.

In certain embodiments, the lyophilized formulation of the disclosure comprises an anti-anti-IL1α/IL1β DVD-Ig protein (*e.g.,* an anti-IL1α/IL1β DVD-Ig protein comprising a heavy and a light chain comprising amino acid sequences corresponding to the heavy and light chain CDRs set forth in SEQ ID NOs: 66 and 67; or an anti-TNF/IL-17 DVD-Ig protein comprising a heavy and a light chain comprising amino acid sequences corresponding to the heavy and light chain variable regions set forth in SEQ ID NOs: 66 and 67; or DVD-C), histidine buffer, and sucrose or sorbitol, where the formulation has a pH of about 5 to about 6 and wherein the protein concentration is greater than about 50 mg/ml upon reconstitution. In certain embodiments, the aqueous formulation has a pH ranging from about 5 to about 5.5, *e.g.,* about 5.4.

In certain embodiments, the DVD-Ig protein used in the lyophilized formulations of the disclosure specifically binds TNF/PGE2, and comprises amino acid sequences corresponding to the heavy and light chain CDRs set forth in SEQ ID NOs: 64 and 65. In certain embodiments, the anti-TNF/PGE2-Ig protein comprises amino acid sequences corresponding to the heavy and light chain variable regions set forth in SEQ ID NOs: 64 and 65. In certain embodiments, the anti-TNF/PGE2-Ig protein comprises the amino acid sequences corresponding to the heavy and light chains set forth in SEQ ID NOs: 64 and 65.

In certain embodiments, the lyophilized formulation of the disclosure comprises a DVD-Ig protein that specifically binds DLL4/VEGF, and comprises amino acid sequences corresponding to the heavy and light chain CDRs set forth in SEQ ID NOs: 28 and 29 or SEQ ID NOs: 72 and 73. In certain embodiments, the anti-DLL4/VEGF-Ig protein comprises amino acid sequences corresponding to the heavy and light chain variable regions set forth in SEQ ID NOs: 28 and 29 or SEQ ID NOs: 72 and 73. In certain embodiments, the anti-DLL4/VEGF-Ig protein comprises the amino acid sequences corresponding to the heavy and light chains set forth in SEQ ID NOs: 28 and 29 or SEQ ID NOs: 72 and 73.

In certain embodiments, the lyophilized formulation of the disclosure comprises a DVD-Ig protein that specifically binds IL12/IL18, and comprises amino acid sequences corresponding to the heavy and light chain CDRs set forth in SEQ ID NOs: 70 and 71. In certain embodiments, the anti-IL12/IL18-Ig protein comprises amino acid sequences corresponding to the heavy and light chain variable regions set forth in SEQ ID NOs: 70 and 71. In certain embodiments, the anti-IL12/IL18-Ig protein comprises the amino acid sequences corresponding to the heavy and light chains set forth in SEQ ID NOs: 70 and 71.

Lyophilization may be performed according to methods known in the art. Many different freeze-dryers are available for this purpose such as Hull50™ (Hull, USA) or GT20.TM. (Leybold-Heraeus, Germany) freeze-dryers. Freeze-drying is accomplished by freezing the formulation and subsequently subliming ice from the frozen content at a temperature suitable for primary drying. Under this condition, the product temperature is below the eutectic point or the collapse temperature of the formulation. Typically, the shelf temperature for the primary drying will range from about -30 to 25°C. (provided the product remains frozen during primary drying) at a suitable pressure, ranging typically from about 50 to 250 mTorr. The formulation, size and type of the container holding the sample (*e.g.*, glass vial) and the volume of liquid will mainly dictate the time required for drying, which can range from a few hours to several days (*e.g.* 40-60 hours). Optionally, a secondary drying stage may also be performed depending upon the desired residual moisture level in the product. The temperature at which the secondary drying is carried out ranges from about 0-40°C, depending primarily on the type and size of container and the type of protein employed. For example, the shelf temperature throughout the entire water removal phase of lyophilization may be from about 15-30°C (*e.g.*, about 20°C). The time and pressure required for secondary drying will be that which produces a suitable lyophilized cake, dependent, *e.g.*, on the temperature and other parameters. The secondary drying time is dictated by the desired residual moisture level in the product and typically takes at least about 5 hours (*e.g.* 10-15 hours). The pressure may be the same as that employed during the primary drying step. Freeze-drying conditions can be varied depending on the formulation and vial size.

Prior to administration to the patient, the lyophilized formulation is reconstituted with a pharmaceutically acceptable diluent such that the protein concentration in the reconstituted formulation is at least about 2 mg/ml, for example from about 2 mg/ml to about 100 mg/ml, alternatively from about 10 mg/ml to about 90 mg/ml, alternatively from about 30 mg/ml to about 50 mg/ml. Such high protein concentrations in the reconstituted formulation are considered to be particularly useful where subcutaneous delivery of the reconstituted formulation is intended. However, for other routes of administration, such as intravenous administration, lower concentrations of the protein in the reconstituted formulation may be desired (for example from about 2-50 mg/ml, or from about 3-40 mg/ml protein in the reconstituted formulation). In certain embodiments, the protein concentration in the reconstituted formulation is significantly higher than that in the pre-lyophilized formulation. Reconstitution generally takes place at a temperature of about 25.degree C to ensure complete hydration, although other temperatures may be employed as desired. The time required for reconstitution will depend, *e.g.,* on the type of diluent, amount of excipient(s) and protein. Exemplary diluents include sterile water, bacteriostatic water for injection (BWFI), a pH buffered solution (*e.g.* phosphate-buffered saline), sterile saline solution, Ringer's solution or dextrose solution. The diluent optionally contains a preservative. Exemplary preservatives have been described above, with aromatic alcohols such as benzyl or phenol alcohol being the preferred preservatives. The amount of preservative employed is determined by assessing different preservative concentrations for compatibility with the protein and preservative efficacy testing. For example, if the preservative is an aromatic alcohol (such as benzyl alcohol), it can be present in an amount from about 0.1-2.0% and preferably from about 0.5-1.5%, but most preferably about 1.0-1.2%.

## V. Uses of the Disclosure

The formulations of the disclosure may be used both therapeutically, *i.e.*, in vivo, or as reagents for in vitro or in situ purposes. The methods of the disclosure may also be used to make a water-based formulation having characteristics that are advantageous for therapeutic use. The aqueous formulation may be used as a pharmaceutical formulation to treat a disorder in a subject.

The formulation of the disclosure may be used to treat any disorder for which the therapeutic protein is appropriate for treating. A "disorder" is any condition that would benefit from treatment with the protein. This includes chronic and acute disorders or diseases including those pathological conditions which predispose the mammal to the disorder in question. In the case of an anti-TNF DVD-Ig, a therapeutically effective amount of the DVD-Ig protein may be administered to treat an autoimmune disease, such as rheumatoid arthritis, an intestinal disorder, such as Crohn's disease, a spondyloarthropathy, such as ankylosing spondylitis, or a skin disorder, such as psoriasis. In the case of an anti-IL-12 DVD-Ig, a therapeutically effective amount of the DVD-Ig protein may be administered to treat a neurological disorder, such as multiple sclerosis, or a skin disorder, such as psoriasis. Other examples of disorders in which the formulation of the disclosure may be used to treat include cancer, including breast cancer, leukemia, lymphoma, and colon cancer.

The term "subject" is intended to include living organisms, *e.g.*, prokaryotes and eukaryotes. Examples of subjects include mammals, *e.g.*, humans, dogs, cows, horses, pigs, sheep, goats, cats, mice, rabbits, rats, and transgenic non-human animals. In specific embodiments of the disclosure, the subject is a human.

The term "treatment" refers to both therapeutic treatment and prophylactic or preventative measures. Those in need of treatment include those already with the disorder, as well as those in which the disorder is to be prevented.

The aqueous formulation may be administered to a mammal, including a human, in need of treatment in accordance with known methods of administration. Examples of methods of administration include parenteral, subcutaneous, intramuscular, intravenous, intraarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal.

The appropriate dosage ("therapeutically effective amount") of the protein will depend, for example, on the condition to be treated, the severity and course of the condition, whether the protein is administered for preventive or therapeutic purposes, previous therapy, the patient's clinical history and response to the protein, the type of protein used, and the discretion of the attending physician. The protein is administered to the patient at one time or over a series of treatments and may be administered to the patient at any time from diagnosis onwards. The protein may be administered as the sole treatment or in conjunction with other drugs or therapies useful in treating the condition in question.

Actual dosage levels of the AS-DVD-Ig protein or LS-DVD-Ig protein, the active ingredient, in the pharmaceutical formulation of this disclosure may be varied so as to obtain an amount of the active ingredient that is effective to achieve the desired therapeutic response for a particular patient, composition, and mode of administration, without being toxic to the patient.

The selected dosage level will depend upon a variety of factors including the activity of the AS-DVD-Ig protein or LS-DVD-Ig protein found in the formulation, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

In certain embodiments of the disclosure, the dosage of the AS-DVD-Ig protein in the formulation is about 1 to about 250 mg. In certain embodiments, the dosage of the AS-DVD-Ig protein in the formulation is about 30 to about 140 mg, about 40 to about 120 mg, about 50 to about 110 mg, about 60 to about 100 mg, or about 70 to about 90 mg. In a further embodiment, the composition includes an AS-DVD-Ig protein dosage of about 1, 10, 20, 30, 40, 50, 60, 70, 80, 90, 100, 110, 120, 130, 140, 150, 160, 170, 180, 190, 200, 210, 220, 230, 240 or 250 mg.

In certain embodiments of the disclosure, the dosage of the LS-DVD-Ig protein in the formulation (upon reconstitution) is about 1 to about 250 mg. In a further embodiment, the dosage of the LS-DVD-Ig in the formulation is about 30 to about 140 mg, about 40 to about 120 mg, about 50 to about 110 mg, about 60 to about 100 mg, or about 70 to about 90 mg. In a further embodiment, the composition includes an LS-DVD-Ig dosage of about 1, 10, 20, 30, 40, 50, 60, 70, 80, 90, 100, 110, 120, 130, 140, 150, 160, 170, 180, 190, 200, 210, 220, 230, 240 or 250 mg.

The formulations of the disclosure overcome common problems known in formulation development, including the problem of protein aggregation often associated with high concentrations of protein, particularly complex proteins such as DVD-Ig proteins. Thus, in certain embodiments, the formulations of the disclosure provide a new means by which high levels of this new therapeutic protein format may be administered to a patient.

## EXAMPLES

The Examples presented herein describe formulations containing dual variable domain immunoglobulin (DVD-Ig) proteins that have unexpected stability characteristics. The experiments were surprising in that certain DVD-Ig proteins, referred to as Aqueous Stable DVD-Ig (AS-DVD-Ig) proteins or Lyophilized Stable DVD-Ig (LS-DVD-Ig) proteins, were stable in aqueous or lyophilized formulations, respectively, whereas other DVD-Ig proteins showed aggregation and instability when similarly formulated. The experiments exemplify methods for identifying AS-DVD-Ig proteins and LS-DVD-Ig proteins, as well as stable formulations thereof.

### Materials and Methods

The methods described herein were used in experiments performed to assess and monitor the stability of DVD-Ig proteins in aqueous and lyophilized formulations.

### *General Methods*

DVD-Ig protein formulations were tested for general quality parameters (*e.g.,* pH), parameters of physical stability (*e.g.*, clarity, color, particle contamination and purity), and parameters of chemical stability (*e.g.,* deamidation, oxidation, and general chemical stability). Exemplary tests included tests for visible particulate contamination, for light obscuration particle count for subvisible particles, and for purity such as size exclusion high pressure liquid chromatography (also referred to herein as size exclusion chromatography (SEC)) and ion exchange chromatography (IEC).

Particulate contamination (*e.g.,* visible particles) was determined by visual inspection. Subvisible particles were monitored by light obscuration assays according to the United States Pharmacopeia (USP). The physicochemical stability of formulations was assessed by SEC, which allows for the detection of fragments and aggregates. To monitor chemical stability, SEC (for the detection of fragments and hydrolysis) and IEC were performed.

*DVD-Ig Proteins Tested*

The DVD-Ig proteins that were tested in the Examples provided herein are listed in Table 1. The sequences of the DVD-Ig proteins described in Table 1 are provided in Table 65.

Table 1: Dual Variable Domain Immunoglobulin (DVD-Ig) Proteins and Their Targets

| DVD-I Protein Name | Targets |
|---|---|
| DVD 5 | CD20/CD80 |
| DVD 6 | CD80/CD20 |
| DVD 37 | VEGF/HER2 |
| DVD 38 | HER2/VEGF |
| DVD 53 | TNF/RANKL |
| DVD 54 | RANKL/TNF |
| DVD 65 | TNF/DKK |
| DVD 66 | DKK/TNF |
| DVD 165 | CD20/RANKL |
| DVD 166 | RANKL/CD20 |
| DVD 257 | DLL4/PLGF |
| DVD 258 | PLGF/DLL4 |
| DVD 277 | TNF/SOST (S2) |
| DVD 278 | SOST(S2)/TNF |
| DVD 281 | IL-9(S2)/IgE |
| DVD 282 | IpE/IL-9(S2) |
| IL12IL18 | IL-12/IL-18 |
| DVD-A | TNF/IL-17 |
| DVD-B | TNF/PGE2 |
| DVD-C | IL-1$\alpha$/IL-1$\beta$ |

DVD-Ig protein as starting material was provided following purification and was > 95 % monomeric form.

*Cation Exchange HPLC Methods*

Cation exchange HPLC, a form of IEC, was used to determine the identity and purity of the DVD-Ig protein formulations. The assay was performed with the parameters detailed below.

A Dionex ProPac® WCX-10 analytical column (Dionex Corp., Sunnyvale, CA), combined with a Dionex WCX-10G guard column (Dionex Corp., Sunnyvale, CA), was run with upper column pressure being limited to 210 bar. The mobile phase A consisted of 10 mM $Na_2HPO_4$, pH 6.0. This buffer was created by dissolving 4.97 g anhydrous disodium hydrogen phosphate in approximately 3300 mL Milli-Q water, adjusting the pH to 7.0 using 1 M phosphoric acid, increasing the buffer volume to 3500 mL with Milli-Q water and filtering the solution through a membrane filter. The mobile phase B consisted of 10 mM $Na_2HPO_4$, 500 mM NaCl, pH 6.0. This buffer was created by dissolving 2.56 g anhydrous disodium hydrogen phosphate in approximately 1500 ml Milli-Q water, adjusting the pH to 6.0 using 1 M phosphoric acid, increasing the buffer volume to 1800 ml with Milli-Q water and filtering the solution through a membrane filter. A summary of the cation exchange HPLC methods is described in Table 2.

Table 2: Summary of Cation Exchange HPLC Methods

| Item | Description/Operating Conditions |
|---|---|
| Guard column | ProPac WCX-10G, 4.0 x 50 mm |

(continued)

| Item | Description/Operating Conditions | |
|---|---|---|
| Column | ProPac WCX-10, 4.0 x 250 mm | |
| Mobile phase A* | 10 mM disodium hydrogen phosphate, pH 6.0 | |
| Mobile phase B* | 10 mM disodium hydrogen phosphate/500 mM sodium chloride, pH 6.0 | |
| | **Binary Gradient** | |
| | **Time (minute)** | **Mobile Phase B%** |
| | 0.01 | 15.0 |
| | 30.00 | 30.0 |
| | 32.00 | 100.0 |
| | 37.00 | 100.0 |
| | 39.00 | 15.0 |
| | 44.00 | 15.0 |
| Gradient | 44.10 | Stop |
| Flow rate | 1.0 ml/minute | |
| Detector wavelength | 280 nm | |
| Autosampler temperature | Nominal 4°C | |
| Column oven temperature | 35°C | |
| Sample load | 100 μL/100 μg | |
| Run time | 44.0 minutes | |

For the IL12IL18 and DVD 66 DVD-Ig proteins, the mobile phases used were changed as follows:

Mobile phase A: 10 mM MES, pH 5.6; and
Mobile phase B: 10 mM MES + 500 mM NaCl, pH 5.6.

For IL1αILβ, the mobile phases used were changed as follows:

Mobile phase A: 20 mM Phosphate, pH 8.0; and
Mobile phase B: 20 mM Phosphate + 500 mM NaCl, pH 8.0.

Table 3. Gradient for IL1αILβ

| Time (minutes) | % B |
|---|---|
| 0 | 7 |
| 5 | 7 |
| 25 | 25 |
| 27 | 100 |
| 32 | 100 |
| 34 | 7 |
| 37 | 7 |

Similar versions of IEC were used for various other DVD-Ig proteins.

*Size Exclusion HPLC Methods*

Size exclusion HPLC was used to determine the purity of DVD-Ig protein solutions. The assay was performed as outlined below.

A TSK gel guard (VWR Scientific, Bridgeport, NJ; cat. no. 08543, 6.0 mm x 4.0 cm, 7 $\mu$m), was combined with a TSK gel G3000SW (VWR Scientific, Bridgeport, NJ; cat. no. 08541, 7.8 mm x 30 cm, 5 $\mu$m) and run with an upper column pressure limit of 70 bar. The mobile phase consisted of 100 mM $Na_2HPO_4$ / 200 mM $Na_2SO_4$, pH 7.0. This buffer was created by dissolving 49.68 g anhydrous disodium hydrogen phosphate and 99.44 g anhydrous sodium sulfate in approximately 3300 ml Milli-Q water, adjusting the pH to 7.0 using 1 M phosphoric acid, increasing the buffer volume to 3500 ml with Milli-Q water and filtering the solution through a membrane filter.

The experimental parameters were listed as follows:

Flow rate: 0.3 ml/minute;
Injection volume (equivalent to 20 $\mu$g sample): 20 $\mu$l;
Column temperature: room temperature;
Autosampler temperature: 2 to 8°C;
Run time: 50 minute;
Elution: isocratic gradient.

Detection was performed using a diode array detector using a 214 nm wavelength (> 0.1 min peak width and 8 nm band width) and a 360 nm reference wavelength (100 nm band width).

Test samples were injected in duplicate. Purity was determined by comparing the area of DVD-Ig protein peak to the total area of all 214 nm absorbing components in the sample, excluding buffer-related peaks. High molecular weight aggregates and antibody fragments were resolved from intact DVD-Ig protein using this method.

*Freeze/Thaw Assays*

The stability of DVD-Ig protein solutions was measured using repeated freeze/thaw assays. The DVD-Ig proteins were frozen at -80°C and then thawed at 30°C in a water bath and the resulting solutions were analyzed for aggregation by SEC and/or for subvisible particle formation by light obscuration assays.

*Accelerated and Real Time Storage Stability Studies*

The pH and storage temperature of formulations are two important factors influencing protein stability during long-term storage of protein liquid and lyophilisate formulations. To assess the impact of these factors, the protein formulations were exposed to short-term storage at elevated temperatures, *e.g.,* 40°C, (accelerated storage) in order to mimic long term storage and quickly gain insight in the formulation feasibility for long-term storage at lower temperatures *(e.g.,* 2-8°C). To assess the real time storage stability, the samples were also kept at 2-8°C.

*Light Obscuration Assays*

Light obscuration assays were performed to measure the insoluble particulate content of aggregating DVD-Ig protein solutions. Light obscuration measurement equipment (particle counter, model syringe, Klotz Bad Liebenzell, Germany, series S20037) was equipped with laminar air hood (Thermo Electron Corp., Asheville, NC; Model No. ULT2586-9-A40) to minimize foreign particle contamination during measurements. Light obscuration analysis was performed as follows. A 3.5 ml sample was placed in a 5 ml roundbottom tube under laminar air flow conditions. Measurements were performed according to manufacturer's specifications in n=3 mode (0.8 mL per single measurement), after an initial 0.8 ml rinse.

*Differential Scanning Calorimetry (DSC)*

Prior to DSC analysis, DVD-Ig proteins were dialyzed into a suitable buffer system using Slide-A-Lyzer Cassettes (Thermo Fisher Scientific, Waltham, MA). This buffer system (*e.g.*, 5 mM phosphate/5 mM citrate was also used as a reference/blank for the DSC measurement. The antibody was analyzed at 1-2 mg/ml. An automated VP-Capillary DSC System (MicroCal, Piscataway, NJ) was used. Unfolding of the molecules was studied by applying a 1°C/minute scan rate over a 25°C - 95°C temperature range. Other measurement parameters were as follows: fitting period: 16 seconds; pre-scan wait: 10 minutes; feedback mode: none.

*Air/Liquid Interface Shaking Studies*

Shaking studies were conducted at a concentration of 1 mg/ml in 6R glass vials on an HS 260 IKA shaker (Wilmington, NC) at a speed of 150 rpms (revolutions per minute). The optical density of samples was evaluated following shaking for various periods. Similarly, SEC was also done for samples pulled at various time points.

*PEG Solubility*

PEG 3000 was used for solubility studies. A 50 % w/v solution of PEG 3000 was prepared in water. Small aliquots of this solution were added to a stock solution of protein in buffer at 0.5 mg/ml concentration. The total volume required at the time first signs of precipitation originated was noted down.

*Real Solubility*

For real solubility evaluations, the DVD-Ig protein was concentrated and stored overnight at 5°C. The solution was visually inspected for precipitates, phase separation, turbidity, etc. The supernatant (or both phases) was checked for dissolved concentration.

*Near UV-CD*

The near UV-CD scans were taken at 1 mg/ml concentrations using 2 ml vial fill on a Jasco spectrometer (JASCO Analytical Instruments, Easton, MD) between 250 and 320 nm. The scan rate was 50 nm/minute and an average of 3 scans was taken. The spectrometer was allowed to equilibrate by turning on the lamp before data acquisition.

*ATR-FTIR Spectrometry*

FTIR scans were taken at 1 mg/ml concentrations using 10 $\mu$l solutions on a Bruker ATR-FTIR (Bruker Optics, Billerica, MA). The scans were collected between 400-4000 cm $^{-1}$ and area normalized and second derivatized before being curve fitted using Origin software (OriginLab, Northampton, MA).

*Light Scattering*

Light scattering studies were done on a Malvern zetasizer (Malvern Instruments Ltd., Worcestershire, UK) using a backscattering angle of 173°. Toluene was used as a standard and a buffer (*e.g.*, acetate, histidine, and Tris) was used as a blank. An automatic mode was used.

*Dynamic Scanning Fluorimetry (DSF)*

DSF was employed to assess the propensity of a protein to unfold. The technique involved the addition of dye Sypro Orange to the protein samples. This fluorescent dye is sensitive to hydrophobic surfaces and shows increased fluorescence in such environments. The sample with dye was then heated and the fluorescence signal as a function of temperature was monitored. As the temperature increased, the protein started to unfold and exposed its hydrophobic interior. This lead to dye binding to this region and a greater fluorescence signal. The temperature at which the signal begins to increase is the onset temperature ($T_{on}$). Proteins that have less intrinsic stability are more prone to unfold and have lower $T_{on}$ values than proteins with greater intrinsic stability. DSF also provided a high throughput tool for rapid screening of clones in a 96 well format and eliminated potential limitations of larger quantities of samples and longer run times in DSC. 6 $\mu$l of the 0.4X SYPRO Orange dye (Invitrogen, Carlsbad, CA) was added to 27 $\mu$l of the DVD-Ig protein solution. The scan rate was 1°C/minute and scans were taken from 25-75°C.

*Lyophilization Methods*

The DVD-Ig proteins were lyophilized according to standard methods and the stability of the resulting lyophilisates were investigated.

*Sample Preparation and Lyophilization*

The vials were stoppered with autoclaved and dried lyo stoppers. Afterwards the vials were lyophilized with a freeze dryer. A typical cycle is shown below in Table 4. The samples were reconstituted to a 100 mg/ml DVD-Ig protein solution.

Table 4: Typical Freeze-Dry Cycle Used To Lyophilize DVD-Ig Proteins

| Step | | Shelf temperature [°C] | Time / Step [hh:min] | Step time [min] | Pressure [mbar] |
|---|---|---|---|---|---|
| 0 | Start | 20 | 0:00:00 | 0 | 1000 |
| 1 | Loading | 20 | 0:00:00 | 0 | 1000 |
| 2 | Freezing I (ramp) | 0 | 0:20:00 | 20 | 1000 |
| 3 | Freezing I | 0 | 2:10:00 | 130 | 1000 |
| 4 | Freezing II (ramp) | -45 | 1:20:00 | 80 | 1000 |
| 5 | Freezing II | -45 | 3:00:00 | 180 | 1000 |
| 6 | Adjust vacuum | -45 | 1:00:00 | 60 | 0,066 |
| 7 | Primary drying I (ramp) | -25 | 1:00:00 | 60 | 0,066 |
| 8 | Primary drying I | -25 | 70:00:00 | 4200 | 0,066 |
| 9 | Secondary drying II (ramp) | 25 | 2:00:00 | 120 | 0,066 |
| 10 | Secondary drying II | 25 | 0:15:00 | 15 | 0,036 |
| 11 | Secondary drying II | 25 | 8:00:00 | 480 | 0,036 |
| 12 | Holding step (ramp) | 5 | 0:30:00 | 30 | 0,036 |
| 13 | Holding step | 5 | 0:00:00 | 0 | 0,036 |
| 14 | Venting N2 atmosphere | 5 | 0:00:00 | 0 | 500 |
| | Total | | | 5375 | |

## I. STABILITY OF DVD-IG PROTEINS

Examples 1-3 demonstrate that DVD-Ig proteins are less stable, *e.g.*, aggregating more easily and having a lower melting temperature, than antibodies due to the increased structural complexity of DVD-Ig proteins.

## EXAMPLE 1. Thermodynamic Comparison of DVD-Ig Proteins and Antibodies

An experiment was performed to determine the stability of a DVD-Ig protein in comparison to an antibody. Differential scanning calorimetry (DSC) of an IgG1 molecule (a monoclonal antibody, mAb) and a DVD-Ig was performed to determine the differences in the thermodynamic properties of the two molecules. Specifically, DSC was performed to compare the thermodynamic properties of an exemplary antibody (Briakinumab, an anti-IL12 monoclonal antibody) to those of a DVD-Ig protein (TNF/PGE2; DVD-B). Formulation information and DSC conditions are provided below in Example 2. Comparison of the DSC profiles of Briakinumab to those of TNF/PGE2 (DVD-B) shows the differences in three versus four domain unfolding (Figure 1). It is also clear from Figure 1 that the thermal unfolding of the DVD-Ig protein started earlier than that of the antibody, which indicates that the overall thermodynamic stability (or the intrinsic stability) of the DVD-Ig molecule is lower than that of the antibody. It should be noted that while the DVD-Ig protein in Figure 1 (DVD-B) had a DSC profile indicating an initial unfolding at about 50 °C, this same DVD-Ig protein was characterized as an LS-DVD-Ig protein given results from further stability testing.

The thermodynamic stability (*e.g.*, onset of unfolding as determined by dynamic scanning calorimetry) of mAbs is in general approximately about 5 °C higher compared to the panel of DVD-Ig proteins listed in Table 1. Although there is generally no direct correlation between the temperature of unfolding and the aggregation propensity of a group of DVD-Ig proteins, the values can be used as a screen to assess the stability of a given group of DVD-Ig proteins. Therefore, the onset temperature of unfolding can be utilized to identify AS-DVD-Ig proteins or LS-DVD-Ig proteins. As described in Figure 4, the overall distribution shows that DVD-Ig proteins generally have a lower unfolding temperature in comparison to antibodies. Figure 4 provides the compiled results from 16 different DVD-Ig proteins and 14 different antibodies. The assays performed in the studies described in Figure 4 are similar to those described above and in Figure 1.

**EXAMPLE 2. Impact of pH on the Stability of DVD-Ig proteins in Solution**

The thermodynamic stability (intrinsic stability) of DVD-Ig proteins formulated in solutions having a pH of 4, 6, or 8 was evaluated using differential scanning calorimetry (DSC). All formulations had a DVD-Ig protein concentration of 1 mg/ml in 5 mM citrate/5mM phosphate buffer. Heating was performed at a scan rate of 1 °C/minute. Results showing the impact of pH on the stability of multiple DVD-Ig proteins are provided in Table 5 below. Tm1-Tm4 described in Table 5 represent the thermal melting/unfolding temperatures of various domains, *e.g.,* CH1, CH2 and CH3 domains, etc. The stabilities of two antibodies (Adalimumab and Briakinumab) are also described for comparison.

Table 5: Impact of pH on Stability of DVD-Ig Protein Formulations with pH 4,6, or 8. as Assessed by Differential Scanning Calorimetry (DSC)

| DVD-Ig | pH | Tm1 °C | Tm2°C | Tm3°C | Tm4°C | Onset °C |
|---|---|---|---|---|---|---|
| 5 | 4 | 62.3 | 63.9 | 73.9 | 80.7 | 45 |
| 6 | 4 | 61.5 | 69.5 | 75.4 | 82.2 | 45 |
| 37 | 4 | 61.4 | 66.7 | 77.1 | 82.7 | 52 |
| 38 | 4 | 68.4 | 70.4 | 75.8 | 81.6 | 56 |
| 53 | 4 | 58.6 | 67.9 | 76.5 | 82.7 | 46 |
| 54 | 4 | 66 | 67.9 | 75.2 | 82.6 | 52.5 |
| 65 | 4 | 61.3 | 69 | 73.9 | 81.3 | 52.5 |
| 66 | 4 | 65.5 | 67.6 | 74.7 | 81.9 | 55 |
| 165 | 4 | 63 | 67.4 | 75.4 | 82.1 | 47.5 |
| 166 | 4 | 67.3 | 71.7 | 74.9 | 82.4 | 55 |
| 257 | 4 | 66.2 | 69.2 | 76.6 | 83.5 | 52.5 |
| 258 | 4 | 68.2 | 69.6 | 78.8 | 83.7 | 55 |
| 277 | 4 | 61.4 | 64.8 | 75.3 | 82.6 | 50 |
| 278 | 4 | 55.8 | 67.5 | 76.1 | 82.8 | 45 |
| 281 | 4 | 65.8 | 68.4 | 79.1 | 83.1 | 55 |
| 282 | 4 | 73.1 | 75 | 77.4 | 83.8 | 57.5 |
| 5 | 6 | 61.9 | 65.1 | 76 | 81.8 | 50 |
| 6 | 6 | 61.6 | 69.8 | 76.2 | 81.9 | 48 |
| 37 | 6 | 60.4 | 67.5 | 77.2 | 83.1 | 51 |
| 38 | 6 | 69.07 | 70.3 | 75.9 | 82 | 57.5 |
| 53 | 6 | 58.3 | 67.05 | 76.4 | 82 | 49 |
| 54 | 6 | 65.8 | 67.6 | 74.9 | 82 | 56 |
| 65 | 6 | 61.2 | 67.3 | 73.4 | 82.4 | 51.5 |
| 66 | 6 | 65.3 | 67.5 | 74.8 | 81.9 | 55 |
| 165 | 6 | 62.6 | 67.3 | 75.7 | 82.1 | 50 |
| 166 | 6 | 67.3 | 71 | 74 | 82 | 54 |
| 257 | 6 | 66.6 | 69.2 | 75.8 | 82.9 | 57 |
| 258 | 6 | 67.8 | 70 | 77.5 | 79.5 | 55 |
| 277 | 6 | 61.4 | 65.8 | 74.7 | 82.23 | 52.5 |
| 278 | 6 | 56.6 | 66.7 | 75.8 | 82.3 | 45 |
| 281 | 6 | 65.8 | 67.8 | 78.6 | 81.7 | 53 |

(continued)

| DVD-Ig | pH | Tm1 °C | Tm2°C | Tm3°C | Tm4°C | Onset °C |
|--------|----|--------|-------|-------|-------|----------|
| 282 | 6 | 69.8 | 75 | 77.9 | 83.4 | 57 |
| 5 | 8 | 65.16 | 68.35 | 77 | 82.48 | 45 |
| 6 | 8 | 62 | 69.07 | 73.08 | 82.61 | 45 |
| 38 | 8 | 68.4 | 70.7 | 74.8 | 82.8 | 50 |
| 53 | 8 | 57.6 | 68.7 | 75 | 83 | 40 |
| 54 | 8 | 65.5 | 67.6 | 74.5 | 82.8 | 52.5 |
| 65 | 8 | 61 | 69.6 | 72.5 | 82.8 | 48 |
| 66 | 8 | 64.8 | 67.1 | 72.4 | 82.9 | 52.5 |
| 165 | 8 | 63.9 | 68.6 | 74.1 | 82.6 | 47.5 |
| 166 | 8 | 64.3 | 69.3 | 74 | 82.7 | 45 |
| 257 | 8 | 67.2 | 69.3 | 76.7 | 83.6 | 55 |
| 258 | 8 | 69.5 | 71.4 | 78.1 | 83.9 | 53 |
| 277 | 8 | 60.18 | 68.98 | 74.5 | 83 | 50 |
| 278 | 8 | 52.8 | 68.8 | 75.8 | 83.02 | 44 |
| 281 | 8 | 65.2 | 67.1 | 78.3 | 83.2 | 51 |
| 282 | 8 | 71.9 | 74.5 | 77.4 | 83.9 | 55 |
| mAbs | | | | | | |
| Adalimumab | 6 | 72 | 76 | 84 | NA | 62 |
| Briakinumab | 6 | 69 | 76 | 83 | NA | 59 |

As shown in Table 5, DVD-Ig proteins in general have unfolding onset temperatures of greater than about 50 °C, and the melting temperatures are therefore slightly lower than those of antibodies and other stable proteins. Example 2 shows that the onset temperature for Briakinumab and Adalimumab is around 60 °C at pH 6, whereas, for DVD-Ig proteins, the average is around 53 °C.

The data also shows that the melting temperatures of DVD-Ig proteins are higher at pH 6 and pH 8 than at pH 4. Thus, pH affects the physico-chemical stability of DVD-Ig proteins, and stability appears best at approximately pH 6.

To further assess the impact of solution pH on the stability of DVD-Ig protein formulations during long-term storage, DVD-Ig protein formulations were analyzed using SEC before being subjected to storage (TO) or after being subjected to 3 months of accelerated storage (T3m). Storage stability of the DVD-Ig proteins in solutions (1 mg/ml DVD-Ig protein in 5 mM citrate/5mM phosphate buffer with the presence of 80 mg/ml sucrose) formulated at pH of 4, 6, or 8 was evaluated. For accelerated storage, samples were filled into sterile vials (approx. 500 μl each) and stored under controlled conditions (in temperature chambers and in the absence of light) at 40°C. The percentage of DVD-Ig protein monomers (Mon) aggregates (Agg), and fragments (Frag) was determined using SEC and the results are presented in Table 6.

Table 6: Impact of pH on the Stability of 1 mg/ml DVD-Ig Protein Solutions Before and After Accelerated Storage

| DVD-I | pH | Mon/T0 | Mon/T3m | Agg/T0 | Agg/T3m | Frag/T0 | Frag/T3m |
|-------|----|--------|---------|--------|---------|---------|----------|
| 5 | 4 | 91.14 | 57.51 | 5 | 0.79 | 3.85 | 41.6 |
| 6 | 4 | 97.38 | 62.46 | 2.29 | 4.11 | 0.31 | 33.4 |
| 38 | 4 | 94.9 | 56.52 | 3.58 | 22.02 | 1.5 | 21.44 |
| 53 | 4 | 97.61 | 28.11 | 0.99 | 53.94 | 1.38 | 17.93 |
| 54 | 4 | 96.57 | 53.89 | 1.93 | 15.2 | 1.48 | 30.89 |
| 65 | 4 | 94.46 | 50.35 | 1.33 | 26.21 | 4.21 | 23.42 |

(continued)

| DVD-I | pH | Mon/T0 | Mon/T3m | Agg/T0 | Agg/T3m | Frag/T0 | Frag/T3m |
|---|---|---|---|---|---|---|---|
| 66 | 4 | 97.99 | 58.39 | 0.9 | 17.72 | 1.1 | 23.87 |
| 165 | 4 | 96.2 | 68.38 | 2.25 | 3.18 | 1.53 | 28.42 |
| 166 | 4 | 97.09 | 66.3 | 0.76 | 0.66 | 2.14 | 30.55 |
| 258 | 4 | 98.61 | 45.42 | 0.46 | 30.56 | 0.91 | 24 |
| 277 | 4 | 97.05 | 61.26 | 1.55 | 11.48 | 1.38 | 27.23 |
| 278 | 4 | 98.33 | 45.58 | 0.9 | 28.13 | 0.76 | 26.27 |
| 5 | 6 | 90.46 | 81.75 | 7.02 | 3.04 | 2.51 | 12.77 |
| 6 | 6 | 97.02 | 85.44 | 2.78 | 2.71 | 0.18 | 11.82 |
| 38 | 6 | 95.31 | 87.89 | 3.48 | 5.48 | 1.2 | 6.61 |
| 53 | 6 | 97.75 | 86.68 | 1.02 | 6.21 | 1.22 | 7.08 |
| 54 | 6 | 96.48 | 88.03 | 2.07 | 5.37 | 1.43 | 6.59 |
| 65 | 6 | 94.89 | 87.13 | 0.89 | 5.78 | 4.21 | 7.06 |
| 66 | 6 | 97.99 | 88.93 | 0.9 | 5.13 | 1.09 | 5.91 |
| 165 | 6 | 96.21 | 86.31 | 2.24 | 5.84 | 1.53 | 7.82 |
| 166 | 6 | 98.6 | 89.37 | 1.39 | 0.92 | 0 | 7.38 |
| 258 | 6 | 98.98 | 84.76 | 0.2 | 9.41 | 0.81 | 5.81 |
| 277 | 6 | 97.48 | 83.96 | 1.48 | 5.11 | 1.03 | 10.91 |
| 278 | 6 | 98.65 | 79.81 | 0.78 | 9.52 | 0.56 | 10.65 |
| 5 | 8 | 90.21 | 67.86 | 7.26 | 4.51 | 2.51 | 22.75 |
| 6 | 8 | 97.36 | 76.33 | 2.44 | 8.89 | 0.18 | 14.76 |
| 38 | 8 | 95.09 | 80.11 | 3.47 | 9.37 | 1.42 | 10.5 |
| 53 | 8 | 97.81 | 79.63 | 0.96 | 9.82 | 1.22 | 10.53 |
| 54 | 8 | 96.69 | 81.13 | 1.93 | 9.44 | 1.37 | 9.41 |
| 65 | 8 | 93.12 | 79.5 | 1.27 | 9.88 | 5.59 | 10.59 |
| 66 | 8 | 97.69 | 81.37 | 0.99 | 9.17 | 1.31 | 9.44 |
| 165 | 8 | 96.57 | 71.02 | 2.03 | 18.85 | 1.39 | 10.11 |
| 166 | 8 | 97.23 | 79.76 | 0.76 | 1.92 | 1.99 | 11.3 |
| 258 | 8 | 98.67 | 84.22 | 0.24 | 5.91 | 1.07 | 9.84 |
| 277 | 8 | 97.06 | 70.08 | 1.72 | 16.13 | 1.2 | 13.78 |
| 278 | 8 | 98.18 | 43.34 | 0.98 | 45.59 | 0.83 | 11.04 |

The results in Table 6 showed that DVD-Ig proteins can be initially formulated in a pH range from pH 4 to pH 8 and that the initial values of the freshly prepared samples at T0 do not show major differences. However, following accelerated storage, the DVD-Ig proteins at pH 4 and pH 8 showed significant loss in monomer and a corresponding increase in aggregates or fragmentation.

The stability of the tested DVD-Ig proteins was highest at around pH 6. All of the DVD-Ig proteins tested (including DVD 5, DVD 6, DVD 38, DVD 53, DVD 54, DVD 65, DVD 66, DVD 165, DVD 166, DVD 258, DVD 277, and DVD 278) had a greater percentage of monomers and a lower percentage of fragments at pH 6 than at pH 4 or at pH 8.

Nine of the twelve DVD-Ig proteins tested showed a lower percentage of aggregates at pH 6 than at pH 4, and for the three DVD-Ig proteins that showed the reverse pattern, the difference in the percentage of aggregates at pH 6 and pH 4 was very small (difference of less than 2.7%). Also, eleven of the twelve DVD-Ig proteins tested showed a

37

lower percentage of aggregates at pH 6 than at pH 8. Thus, accelerated storage resulted in increased aggregate formation. However, the increase was less than anticipated, particularly at pH 6.

*Impact of Solution pH on the Storage Stability of IL12IL18 DVD-Ig Protein Formulations*

To assess the impact of solution pH on the stability of DVD-Ig protein formulations during storage, DVD-Ig protein formulations with solution pH of 4, 6, or 8 were analyzed using SEC and IEC before being subjected to storage (TO) or after being subjected to 4 days (4d), 7 days (7d), or 21 days (21d) of storage at 5°C, 40°C, or 50°C (See Tables 6, 7 and 8). The solutions evaluated had an IL12-IL18 DVD-Ig concentration of 2 mg/ml and were in a buffer of 10 mM citrate and 10 mM phosphate. Samples were filled into sterile vials (approx. 500 (j.1 each) and stored under controlled conditions (in temperature chambers and in the absence of light). The percentage of DVD-Ig protein monomers, aggregates, and fragments was determined using SEC (see Table 7) and the numbers of main, acidic and basic species were assessed using IEC (see Table 8).

Table 7: Storage Stability of IL12-IL18 DVD-Ig Protein Formulations with pH 4. 6. or 8 as Measured Using SEC

| | Monomer | | | Aggregates | | | Fragments | | |
|---|---|---|---|---|---|---|---|---|---|
| | pH 4 | pH 6 | pH 8 | pH 4 | pH 6 | pH 8 | pH 4 | pH 6 | pH 8 |
| T0 | 97.64 | 97.61 | 97.84 | 1.35 | 1.36 | 1.23 | 1.01 | 1.03 | 0.94 |
| Storage at 5°C | | | | | | | | | |
| 4d, 5°C | 97.98 | 97.64 | 97.95 | 1.07 | 1.31 | 1.19 | 0.95 | 1.06 | 0.87 |
| 7d, 5°C | 97.68 | 98.02 | 97.8 | 1.25 | 1.17 | 1.08 | 1.07 | 0.81 | 1.11 |
| 21d, 5°C | 98.13 | 97.81 | 97.64 | 1.23 | 1.17 | 1.08 | 0.88 | 0.96 | 1.28 |
| Accelerated Storage at 40°C | | | | | | | | | |
| 4d, 40°C | 97.21 | 97.32 | 96 | 0.56 | 1.4 | 1.68 | 2.24 | 1.28 | 2.32 |
| 7d, 40°C | 96.15 | 97.32 | 94.57 | 0.71 | 1.35 | 1.96 | 3.15 | 1.33 | 3.48 |
| 21d, 40°C | 91.99 | 96.39 | 91.31 | 1.16 | 1.49 | 3.15 | 6.85 | 2.12 | 5.54 |
| Accelerated Storage at 50°C | | | | | | | | | |
| 4d, 50°C | 90.2 | 97.07 | 91.73 | 5.54 | 1.46 | 2.78 | 4.26 | 1.47 | 5.49 |
| 7d, 50°C | 83.19 | 96.35 | 81.32 | 9.66 | 1.42 | 10.68 | 7.15 | 2.24 | 8.2 |
| 21d, 50°C | 52.98 | 91.36 | 46.57 | 33.1 | 4.17 | 33.35 | 13.91 | 4.47 | 17.18 |

The SEC data show that the IL12IL18 DVD-Ig protein formulations were most stable at pH 6. At pH 6, the stored IL12IL18 DVD-Ig protein formulations generally showed >95% monomers and <2% aggregates. Even under accelerated storage conditions of 50°C for 21 days, the formulation retained >90% monomers and <5% aggregates. IL12IL18 DVD-Ig formulations were more stable at pH 6 than pH 4 and pH 8, particularly in the longer duration and higher temperature storage conditions (*e.g*., in the 21 day, 50°C condition). According to these results the IL12/IL18 DVD-Ig protein would be considered an AS-DVD-Ig protein given the stability results following storage at 50 °C.

Table 8: Storage Stability of TT.12IL1R DVD-Ig Protein Formulations with pH 4, 6, or 8 as Measured Using IEC

| | Main Species | | | Acidic | | | Basic | | |
|---|---|---|---|---|---|---|---|---|---|
| | pH 4 | pH 6 | pH 8 | pH 4 | pH 6 | pH 8 | pH 4 | pH 6 | pH 8 |
| T0 | 69.98 | 71.6 | 69.13 | 14.96 | 15.21 | 18.12 | 15.06 | 13.19 | 12.75 |
| 4d,5°C | 70.32 | 71 | 68.46 | 15.08 | 15.54 | 18.84 | 14.6 | 13.46 | 12.75 |
| 7d,5°C | 69.74 | 70.69 | 67.14 | 15.43 | 15.91 | 19.71 | 14.83 | 13.41 | 13.15 |
| 21d, 5°C | 69.67 | 71.32 | 66.78 | 15.65 | 16.26 | 20.95 | 14.68 | 12.42 | 12.26 |
| 4d, 40°C | 56.43 | 68.09 | 43.52 | 21.14 | 18.73 | 45.51 | 22.33 | 13.19 | 10.97 |
| 7d, 40°C | 49.1 | 65.11 | 26.55 | 26.08 | 22.03 | 59.93 | 24.83 | 12.86 | 13.53 |

(continued)

| | Main Species | | | Acidic | | | Basic | | |
|---|---|---|---|---|---|---|---|---|---|
| | pH 4 | pH 6 | pH 8 | pH 4 | pH 6 | pH 8 | pH 4 | pH 6 | pH 8 |
| 4d, 50°C | 36.58 | 57.09 | 25.88 | 29.47 | 29.48 | 48.23 | 33.95 | 13.43 | 25.89 |

The IEC data for the IL12IL18 DVD-Ig protein formulations at pH 4, 6 and 8 show that the chemical degradation is dependent on the pH and the storage temperature. The highest chemical stability was observed for samples stored at 5°C. The minor changes in stability detected after 21 days indicated that the IL12IL18 DVD-Ig protein was stable at this storage temperature, which is considered common for commercial biotherapeutics. Although the differences in stability were small, the highest chemical stability was observed for formulations at pH 6. The lack of chemical stability was even more pronounced at the elevated storage temperatures of 40 °C and 50 °C.

**EXAMPLE 3. DVD-Ig Proteins Aggregate More Easily Than Monoclonal Antibodies**

The impact of shaking on the aggregation of antibodies versus DVD-Ig proteins was examined. Shaking is a stress that can lead to the aggregation of molecules. The susceptibility of a DVD-Ig protein, TNF/PGE2 (DVD-B), to aggregation following shaking was compared with a monoclonal antibody, Briakinumab, using solutions having a protein concentration of 1 mg/ml (solutions at pH 6, 10 mM citrate/10 mM phosphate) in 6R vials. The 6R vials were filled with samples of 5 ml of the protein solution and shaken on an HS 260 IKA shaker (Wilmington, NC) at a speed of 150 revolutions per minute (rpm) for various lengths of time (0, 5, 24, 48, or 96 hours). The samples were checked for optical density at 500 nm, which provides a measurement of the turbidity of the solutions. Higher turbidity indicates greater aggregation and less stability. The results are shown in Table 9, revealing that the DVD-Ig protein aggregates more readily than the monoclonal antibody.

Table 9: Impact of Shaking on the $OD_{500}$ of 1 mg/ml Solutions of TNF / PGE2 DVD-Ig Protein and Briakinumab

| | Optical Density (OD) at 500nM | |
|---|---|---|
| Time (h) | Briakinumab | TNF/PGE2 (DVD-B) |
| 0 | 0 | -0.0095 |
| 5 | 0.001 | 0.01175 |
| 24 | 0.01 | 0.0949 |
| 48 | 0.025 | 0.295 |
| 96 | 0.045 | 0.58 |

As indicated by $OD_{500}$ measurements which show turbidity of the solution, shaking caused the DVD-Ig protein to form more sub-visible and visible aggregates than the monoclonal antibody. Thus, after 48 hours of shaking, the DVD-Ig protein was more prone to colloidal instability and, therefore, less stable than the monoclonal antibody. The greater formation of visible aggregates of DVD-Ig protein compared with monoclonal antibody indicates that the DVD-Ig protein is generally less stable than the monoclonal antibody against shear stress in solution. Also, these results suggest that not all DVD-Ig proteins are as stable at pH 6 as a monoclonal antibody.

**II. ASSAY FOR IDENTIFYING AN AQUEOUS STABLE DVD-IG PROTEIN (AS-DVD-IG)**

**EXAMPLE 4. DVD-Ig Proteins Can be Characterized as "Aqueous Stable" or "Aqueous Non-Stable"**

As discussed above, the inventors found that formulations wherein the DVD-Ig protein was in the aqueous state suffered from certain problems such as aggregation and/or fragmentation of the DVD-Ig protein monomer. The inventors conducted experiments and discovered, surprisingly and unpredictably, that a subset of DVD-Ig proteins can be stably formulated in the aqueous state even at high concentrations. The following example describes an SEC study showing that, surprisingly, DVD-Ig proteins can be characterized as either aqueous stable, *e.g.,* the DVD-Ig protein shows low change in percent rel. peak area in monomers or aqueous non-stable, *e.g.,* the DVD-Ig protein is prone to aggregation and or fragmentation. Notably, many of the DVD-Ig proteins tested were found to be aqueous non-stable or lyophilized non-stable. Due to the structural complexity of DVD-Ig proteins and the prominence of hydrophobic interactions at high concentrations, it was not expected that DVD-Ig proteins would be stable in formulations at high concentrations.

To assess the impact of storage temperature during accelerated or long-term storage of protein liquid formulations on protein stability, various DVD-Ig proteins were exposed to short-term storage at elevated temperatures in order to quickly gain insight in the formulation feasibility for long-term storage at lower temperatures (*e.g.*, 2-8°C).

*Stability Screen at α High Concentration for 14 Days*

DVD-Ig protein formulations with concentrations of 60 mg/ml were analyzed using SEC before being subjected to storage (T=0) or after being subjected to 14 days of accelerated storage (T=14days) (Table 10). Storage stability of the DVD-Ig proteins in solution (60 mg/ml, 10 mM citrate/10mM phosphate buffer with 80 mg/ml sucrose) was evaluated at 40 °C. After defined storage periods, samples were pulled and the impact of storage time on DVD-Ig protein stability was evaluated. Briefly, samples were filled into sterile vials (approx. 500 μL each) and stored under controlled conditions (in temperature chambers and in the absence of light) at 40 °C. At predefined points of time, samples of prepared solutions were pulled for analysis according to the sample pull scheme. The percentages of DVD-Ig protein monomers (Mon), aggregates (Agg), and fragments (Frag) were determined using SEC, and the results are presented in Table 10.

Table 10: Impact of High Concentration on the Storage Stability of DVD-Ig Protein Solutions

| DVD-Ig | Mon/T0 | Mon/T14d | Agg/T0 | Agg/T14d | Frag/T0 | Frag/T14d |
|---|---|---|---|---|---|---|
| 5 | 79.1 | 73.28 | 19.12 | 24.26 | 1.77 | 2.44 |
| 6 | 84.01 | 89.46 | 12.69 | 9.81 | 3.29 | 0.71 |
| 37 | 92.85 | 74.35 | 6.34 | 23.42 | 0.8 | 2.21 |
| 65 | 95.4 | 92.79 | 1.31 | 5.39 | 3.28 | 1.79 |
| 66 | 96.56 | 94.49 | 1.08 | 3.88 | 2.35 | 1.6 |
| 165 | 90.17 | 64.09 | 8.99 | 33.44 | 0.83 | 2.45 |
| 166 | 98.17 | 94.93 | 1.09 | 3.11 | 0.72 | 1.93 |
| 257 | 94.83 | 76.47 | 4.77 | 21.78 | 0.39 | 1.72 |
| 277 | 97.46 | 85.06 | 1.77 | 13.19 | 0.76 | 1.73 |
| 278 | 98.45 | 73.01 | 1.06 | 25.69 | 0.48 | 1.27 |
| 281 | 93.92 | 68.01 | 2.78 | 30.5 | 3.29 | 1.46 |
| 282 | 98.34 | 95.39 | 1.03 | 3.05 | 0.62 | 1.54 |

Surprisingly, as shown in Table 10, the inventors discovered that a subset of the DVD-Ig proteins tested was stable when formulated in the aqueous state. Ten of the twelve DVD-Ig proteins tested (DVD 5, DVD 6, DVD 37, DVD 65, DVD 66, DVD 166, DVD 257, DVD 277, DVD 278, and DVD 282) showed less than 26% aggregate formation and had greater than 73% monomers following 14 days of accelerated storage. Five of the DVD-Ig proteins tested (DVD 6, DVD 65, DVD 66, DVD 166, and DVD 282) showed aggregate formation of less than 10%, and three of these (DVD 66, DVD 166, and DVD 282) showed aggregate formation of less than 5%.

As described above, certain DVD-Ig proteins ("Aqueous Stable DVD-Ig" proteins or "AS-DVD-Ig" proteins) remain stable (*e.g.*, less than 6 % relative (rel.) peak area change in monomers or less than 10% relative (rel.) peak area change in monomers as determined by SEC) following accelerated storage in 14 days at 40 °C, even when formulated at high concentration (*e.g.,* concentrations of 60 mg/ml or higher). The majority of DVD-Ig proteins tended to aggregate during accelerated storage (non-AS-DVD-Ig proteins), as would be expected based on the general structure of DVD-Ig proteins and the stability studies described in Examples 1-3. Thus, in certain embodiments, the cutoff for separating the AS-DVD-Ig proteins from the non-AS-DVD-Ig proteins was taken as the formation of 10 % net aggregates or less in 14 days at 40 °C when stored at > 50 mg/ml at pH 6, as seen in the above non-limiting example where five of the twelve DVD-Ig proteins tested showed aggregate formation at this level. In certain embodiments, the cutoff for separating the AS-DVD-Ig proteins and the non-AS-DVD-Ig proteins was taken as the formation of 6 % net aggregates or less in 14 days at 40 °C when stored at > 50 mg/ml at pH 6, as seen in the above non-limiting example where four of the twelve DVD-Ig proteins tested showed aggregate formation at this level.

Many of DVD-Ig proteins do not show low aggregation, *e.g.*, 1% or less aggregation at 5 °C after 21 days or 10% or less aggregation at 40 °C following 21 days of storage. For example, in an assay which examined monomer loss after 7 days in a solution having a TNF/IL13 DVD-Ig protein concentration of 50 mg/ml at either 4 °C or 40 °C, many of DVD-Ig proteins showed an increase in monomer loss as determined by SEC. In some cases the amount of monomer loss was negative as the monomer level increased in these cases (*e.g.,* some of the aggregates dissociated and formed back monomer and hence the apparent decrease in loss). A third experiment tested TNF/SOST DVD-Ig proteins in a solution having a DVD-Ig protein concentration of 50 mg/ml at 4 °C. As in the experiment relating to TNF/IL13 DVD-Ig proteins, many of the DVD-Ig proteins showed an increase in monomer loss (determined by SEC).

Notably, the above assays can also be used to distinguish Lyophilized-Stable DVD-Immunoglubulin (LS-DVD-Ig) proteins. The cutoff for separating the LS-DVD-Ig proteins and the non-LS-DVD-Ig proteins was taken as the formation of 15 % net or less aggregates in 14 days at 40 °C when stored at > 50 mg/ml at pH 6. Thus, DVD-Ig proteins tested in the above assay that result in less than 10% or less than 6% aggregation or less than 10 % or less than 6 % relative (rel.) peak area change in monomers are considered both AS-DVD-Ig protein and LS-DVD-Ig proteins, and DVD-Ig proteins resulting in less than 15% aggregation or less than 15 % relative (rel.) peak area change in monomers are considered LS-DVD-Ig proteins. Both AS-DVD-Ig protein and LS-DVD-Ig proteins represent only a percentage of the overall DVD-Ig proteins tested.

### III. STABILITY OF NON-AS-DVD-IG PROTEINS IN FORMULATIONS

The following examples provide data showing the stability of non-AS-DVD-Ig proteins (which fail the aggregation test, *i.e.*, show more than, for example, 6% aggregation, described in Example 4 above), in formulations, in comparison to AS-DVD-Ig proteins (described in Sections IV to VIII).

### EXAMPLE 5: Impact of Concentration on the Storage Stability of an Exemplary Non-AS-DVD-Ig Protein

To assess the impact of protein concentration on long term storage stability, formulations of an exemplary non-AS-DVD-Ig protein with concentrations of 1, 2, 5, 10, 25, 50, and 75 mg/ml were subjected to storage for 14 days at 40°C. The formulations had a pH of 6 and were in 15mM histidine buffer alone. The samples were filled into sterile vials (approx. 500 $\mu$l each) and stored under controlled conditions (in temperature chambers and in the absence of light). The samples were analyzed using SEC to determine the percentage of aggregates following storage. The resulting data is provided in Table 11.

Table 11: The Total Percent Aggregates in the DVD-B protein as Measured Using SEC Following Storage at 40°C for 14 Days

| Concentration of DVD-B | Aggregates % |
| --- | --- |
| 1 mg/ml | 0 |
| 2 mg/ml | 0.2 |
| 5 mg/ml | 3.64 |
| 10 mg/ml | 6.76 |
| 25 mg/ml | 18.02 |
| 50 mg/ml | 32.82 |
| 75 mg/ml | 48.28 |

The data in Table 11 indicate that under the tested conditions (*i.e.*, pH 6, 15mM histidine buffer) DVD-B becomes unstable, namely, a high proportion of aggregates form after 14 days of storage at 40°C when high concentrations are reached. The percentage of aggregates formed exceeded 18% at a concentration of 25 mg/ml or more. Thus, DVD-B, a non-AS-DVD-Ig protein, was not stable in histidine buffer as evidenced by increased aggregation during storage.

### EXAMPLE 6: Impact of pH, Ionic Strength, and Concentration on the Storage Stability of an Exemplary Non-AS-DVD-Ig Protein

To assess the impact of pH, ionic strength, and concentration on the storage stability of a DVD-Ig protein in solution, various formulations of DVD-B (5 mg/ml and 100 mg/ml) were evaluated at 40°C and 5°C. After defined storage periods, samples were pulled and the impact of storage time on DVD-Ig protein stability was evaluated. The following buffers were used: acetate for pH 4.5, histidine for pH 6 and Tris for pH 8. A 2 mM concentration of buffer was used for 1 mM ionic strength solutions and a 10 mM concentration of buffer for 20 and 100 mM ionic strength solutions (sodium chloride was used to further maintain ionic strength). Samples were filled into sterile vials, approx. 500 µl each, and stored under controlled conditions in a temperature chamber and in the absence of light. After 3 months at 5°C (5C, 3m) or 21 days at 40°C (40C, 21d), samples of prepared solutions were analyzed using SEC. The numbers of net aggregates measured using SEC are presented in Table 12. Tables 13 and 14 further show that the addition of different stabilizers/excipients (e.g., sucrose and Tween80) did not result in significant improvement in percent monomer remaining after defined time points (results were obtained using the methodology described above). The negative values of net aggregate values at the initial time points for the low concentration samples indicate also the initial formation of reversible aggregates.

Table 12: Impact of Storage of DVD-B Protein Under Various Formulation Conditions on the Amount of Aggregates Formed as Measured By SEC

| DVD-Ig Protein Concentration and Storage Condition | pH | Added Ionic strength | Aggregate (Net) |
|---|---|---|---|
| DVD-B, 100 mg/ml, 5°C, 3m | 4.5 | 1 | 49.51 |
| | | 20 | 54.79 |
| | | 100 | 56.64 |
| | 6 | 0 | 11.68 |
| | | 1 | 6.6 |
| | | 20 | 9.4 |
| | | 100 | 7.85 |
| | 8 | 1 | 5.66 |
| | | 20 | 8.08 |
| | | 100 | 7.87 |
| DVD-B, 5 mg/ml, 40°C, 21d | 6 | 0 | -6.56 |
| | | 1 | -3.3 |
| | | 20 | 10.79 |
| | | 100 | 9.99 |
| DVD-B, 100 mg/ml, 40°C, 21d | 6 | 0 | 73.37 |
| | | 1 | 70.39 |
| | | 20 | 77.39 |
| | | 100 | 65.4 |

Table 13: Polyol and Polysorbate Has Little to No Effect on Stability of DVD-B at 1 mg/ml in Histidine Buffer, pH 6

| | Sample No. | Monomer | Aggregate | Fragment | AUC |
|---|---|---|---|---|---|
| | | **Histidine Buffer** | | | |
| T0 | | 98.97 | 0.34 | 0.67 | 79787 |
| T4, 40°C | 1 | 98.13 | 0.82 | 1.04 | 78552 |
| | 2 | 97.98 | 0.87 | 1.13 | 78622 |
| | Average | 98.055 | 0.845 | 1.085 | 78587 |
| T7, 40°C | 1 | 97.45 | 1.15 | 1.39 | 77836 |
| | 2 | 97.42 | 1.2 | 1.36 | 78137 |
| | Average | 97.435 | 1.175 | 1.375 | 77986.5 |
| T21, 40°C | 1 | 92.21 | 4.34 | 3.44 | 170875 |

(continued)

| | Sample No. | **Monomer** | **Aggregate** | **Fragment** | **AUC** |
|---|---|---|---|---|---|
| | Histidine Buffer | | | | |
| | 2 | 93.19 | 3.7 | 3.1 | 72149 |
| | Average | 92.7 | 4.02 | 3.27 | 121512 |
| T4, 50°C | 1 | 94.06 | 4.15 | 1.77 | 39698 |
| | 2 | 93.37 | 4.44 | 2.18 | 43002 |
| | Average | 93.715 | 4.295 | 1.975 | 41350 |
| T7, 50°C | 1 | 93.09 | 3.9 | 2.99 | 26451 |
| | 2 | 91.95 | 4.81 | 3.22 | 30158 |
| | Average | 92.52 | 4.355 | 3.105 | 28304.5 |
| 30 mM Hist, 80 mg/ml Sucrose, 0.02 % Tween 80 | | | | | |
| T0 | | 97.5 | 1.61 | 0.88 | 62902 |
| T21, 40°C | 1 | 95.19 | 1.47 | 2.95 | 79362 |
| | 2 | 95.56 | 1.67 | 3.13 | 79445 |
| | Average | 95.375 | 1.57 | 3.04 | 79403.5 |
| T4, 50°C | 1 | 94.94 | 3.38 | 1.66 | 80742 |
| | 2 | 94.98 | 3.3 | 1.62 | 79436 |
| | Averape | 94.96 | 3.34 | 1.64 | 80089 |
| T7, 50°C | 1 | 91.06 | 6.71 | 2.21 | 79672 |
| | 2 | 91.01 | 6.6 | 2.37 | 79820 |
| | Average | 91.035 | 6.655 | 2.29 | 79746 |

The addition of polyol to the formulation resulted in a slight improvement in monomer content and a decrease in the levels of aggregates of DVD-B at Img/ml.

Table 14: Polysorbate Has Little to No Effect on Stability of DVD-B at 100 mg/ml in Histidine Buffer, pH 6

| Buffer: 15 mM Histidine | | | | |
|---|---|---|---|---|
| | **Monomer** | **Aggregate** | **Fragment** | **AUC** |
| T0 | 96.26 | 2.43 | 1.3 | 73681 |
| T7 (Vial1) | 41.4 | 56.1 | 2.34 | 64692 |
| T7 (Vial2) | 42.5 | 55.2 | 2.14 | 63246 |
| T7 (Avg.) | 41.95 | 55.65 | 2.24 | 63969 |
| T21 (Vial1) | 37.2 | 60.03 | 2.76 | 52389 |
| T21(Vial2) | 38.8 | 58.05 | 3.13 | 50722 |
| T21(Avg.) | 38 | 59.04 | 2.945 | 51555.5 |
| **Buffer: 15 mM Histidine + 0.02 % Tween 80** | | | | |
| | **Monomer** | **Aggregate** | **Fragment** | **AUC** |
| T0 | 96.22 | 2.43 | 1.33 | 72007 |
| T7 (Vial1) | 42.9 | 54.8 | 2.2 | 65403 |
| T7 (Vial2) | 47 | 50.8 | 2.09 | 58048 |

(continued)

| Buffer: 15 mM Histidine + 0.02 % Tween 80 | | | | |
|---|---|---|---|---|
| | Monomer | Aggregate | Fragment | AUC |
| T7 (Avg.) | 44.95 | 52.8 | 2.145 | 61725.5 |
| T21(Vial1) | 40.32 | 54.54 | 5.12 | 32321 |
| T21(Vial2) | 38.38 | 55.9 | 5.7 | 30927 |
| T21(Avg.) | 39.35 | 55.22 | 5.41 | 31624 |

The addition of polyol to the formulation resulted in a slight improvement in monomer content and a decrease in the levels of aggregates of DVD-B at 100mg/ml.

Table 15: Polyol or Surfactant Does Not Improve Stability (1 mg/ml) of DVD-B

| pH 6 Formulation | Monomer (%) | | | Aggregate (%) | | | Fragment (%) | | |
|---|---|---|---|---|---|---|---|---|---|
| | T0 | 1M | 3M | T0 | 1M | 3M | T0 | 1M | 3M |
| 15 mM Na Phos. | 96.45 | 90.03 | 80.83 | 1.41 | 5 | 9.09 | 2.12 | 4.95 | 10.06 |
| 15 mM Na Cit. | 96.51 | 90.72 | 85.3 | 1.44 | 5.3 | 6.57 | 2.04 | 3.97 | 8.11 |
| 15 mM Na Succ. | 96.06 | 87.41 | 78.17 | 1.53 | 5.46 | 8.24 | 2.39 | 7.11 | 13.57 |
| 15 mM Na Acet. | 96.14 | 89.62 | 81.8 | 1.48 | 4.76 | 7.52 | 2.36 | 5.6 | 10.66 |
| 15 mM Arg. | 96.12 | 92.48 | 85.72 | 1.65 | 3.39 | 5.59 | 2.21 | 4.11 | 8.68 |
| 15 mM Hist. | 96.42 | 91.82 | 81.6 | 1.29 | 3.85 | 6.39 | 2.28 | 4.32 | 12 |
| Self Buff. | 96.03 | 88.79 | 81.44 | 1.57 | 3.91 | 4.18 | 2.38 | 7.29 | 14.37 |
| UB 10 mg/ml Mannitol | 95.86 | 90.18 | 84.08 | 2.01 | 5.74 | 8.09 | 2.11 | 4.07 | 7.81 |
| UB 10 mg/ml Sorbitol | 96.49 | 89.36 | 84.09 | 1.38 | 6.56 | 7.68 | 2.11 | 4.07 | 8.21 |
| UB 10 mg/ml Sucrose | 96.12 | 90.03 | 84.26 | 1.58 | 5.92 | 7.62 | 2.28 | 4.03 | 8.11 |
| UB 10 mg/ml Trehalose | 96.34 | 89.97 | 84.31 | 1.5 | 5.98 | 7.59 | 2.14 | 4.03 | 8.09 |
| UB 2.5% Gly | 96.43 | 87.22 | | 1.42 | 8.59 | | 2.13 | 4.17 | |
| UB 15 mM (NH$_4$)$_2$SO$_4$ | 96.69 | 90.92 | 85.76 | 1.22 | 4.97 | 5.68 | 2 | 4.1 | 8.55 |
| UB 20 mM NaCl | 96.44 | 90.35 | 84.36 | 1.41 | 5.5 | 6.74 | 2.13 | 4.13 | 8.88 |
| UB 200 mM NaCl | 96.37 | 91.85 | 85.2 | 1.52 | 3.24 | 3.62 | 2.09 | 4.89 | 11.16 |
| UB = citrate / phosphate buffer | | | | | | | | | |

The data show that although various solution conditions were analyzed, DVD-B was not very stable even at pH 6 (see, for example Table 13). Also, at pH 6, the ionic strength did not show a consistent relationship with net aggregate formation. The poor stability is indicated by the formation of high amounts of aggregates even in the 5°C storage condition. Furthermore, the addition of a polyol and/or a surfactant did not improve aggregation of DVD-B (see Tables 13, 14, and 15).

As described above in Examples 1 to 3, many DVD-Ig proteins are intrinsically unstable. However, surprisingly, certain DVD-Ig proteins can be characterized as being stable, as described in Example 4. The experiments described in the Examples below demonstrate that AS-DVD-Ig proteins can unexpectedly be stably formulated, even at high concentrations, despite the differences in amino acid sequence. The below examples stand in contrast to Examples 5 and 6, which show the failure of non-AS-DVD-Ig proteins to be formulated.

## IV. AS-DVD-IG PROTEINS ARE STABLE IN FORMULATIONS CONTAINING A BUFFER AT PH RANGE OF 4.5-7.5

### EXAMPLE 7: Effect of Buffer Concentrations on the Stability of DVD-Ig Proteins

The concentration of a buffer, *e.g.*, histidine, is one of the important factors that may influence protein stability during accelerated/long-term storage of protein liquid formulations. To assess the impact, the protein was exposed to short-term storage at elevated and real time temperatures in order to quickly gain insight into stable formulations for long-term storage at lower temperatures (*e.g.*, 2-8°C).

Storage stability of DVD-Ig proteins in solution was evaluated at 40°C and 5°C. After defined storage periods, samples were pulled and the impact of storage time on DVD-Ig protein stability was evaluated. The concentrations of histidine that were evaluated include 0, 5, 15, 50, and 200 mM.

Samples were filled into sterile vials (approx. 500 µl each) and stored under controlled conditions (in temperature chambers and in the absence of light). After 7days and 21 days, samples of the prepared solutions were analyzed using SEC and IEC.

Table 16: Impact of Storage of Various DVD-Ig Proteins at Low and High Concentrations under Various Histidine Concentrations on SEC

| T0 | | | | | | | | | |
|---|---|---|---|---|---|---|---|---|---|
| DVD-A, pH 5.2, 1 mg/ml | Agg | Mon | Frag | Precipitation* | DVD-A, pH 5.2, 75 mg/ml | Agg | Mon | Frag | Precipitation* |
| 0 Histidine | 1.89 | 97.21 | 0.88 | N | 0 Histidine | 2.63 | 96.39 | 0.97 | N |
| 5 mM Histidine | 0.93 | 98.19 | 0.86 | N | 5 mM Histidine | 0.52 | 98.27 | 1.19 | N |
| 15 mM Histidine | 1.41 | 97.64 | 0.94 | N | 15 mM Histidine | 1.69 | 97.38 | 0.92 | N |
| 50 mM Histidine | 1.83 | 96.84 | 1.31 | N | 50 mM Histidine | 2.01 | 96.86 | 1.11 | N |
| 200 mM Histidine | 2.09 | 96.96 | 0.94 | N | 200 mM Histidine | 2.27 | 96.78 | 0.94 | N |
| 40C, 7d | | | | | | | | | |
| DVD-A, pH 5.2, 1 mg/ml | Agg r | Mon | Frag | | DVD-A, pH 5.2, 75 mg/ml | Agg | Mon | Frag | |
| 0 Histidine | 1.55 | 96.4 | 2.03 | N | 0 Histidine | 10.52 | 87.77 | 1.7 | N |
| 5 mM Histidine | 1.19 | 97.17 | 1.62 | N | 5 mM Histidine | 1.26 | 95.01 | 3.71 | N |
| 15 mM Histidine | 2.47 | 95.49 | 2.03 | N | 15 mM Histidine | 13.76 | 83.35 | 2.88 | N |
| 50 mM Histidine | 2.31 | 95.42 | 2.25 | N | 50 mM Histidine | 16.62 | 80.82 | 2.54 | N |
| 200 mM Histidine | 2.14 | 95.92 | 1.92 | N | 200 mM Histidine | 7.47 | 89.68 | 2.83 | Y |
| 40C, 21d | | | | | | | | | |
| DVD-A, pH 5.2, 1 mg/ml | Agg | Mon | Frag | Precipitation* | DVD-A, pH 5.2, 75 mg/ml | Agg | Mon | Frag | Precipitation* |
| 0 Histidine | | | | | 0 Histidine | 15.77 | 82.2 | 2.02 | Y |
| 5 mM Histidine | 2.52 | 95.3 | 2.16 | N | 5 mM Histidine | 14.07 | 83.8 | 2.12 | N |

(continued)

| 40C, 21d | | | | | | | | | |
|---|---|---|---|---|---|---|---|---|---|
| DVD-A, pH 5.2, 1 mg/ml | Agg | Mon | Frag | Precipitation* | DVD-A, pH 5.2, 75 mg/ml | Agg | Mon | Frag | Precipitation* |
| 15 mM Histidine | 3.39 | 93.85 | 2.74 | N | 15 mM Histidine | 18.07 | 79.49 | 2.42 | N |
| 50 mM Histidine | 2.74 | 94.4 | 2.84 | N | 50 mM Histidine | 13.41 | 83.71 | 2.86 | Y |
| 200 mM Histidine | 1.9 | 94.91 | 3.18 | N | 200 mM Histidine | 8.35 | 87.66 | 3.97 | Y |
| 5C, 21d | | | | | | | | | |
| DVD-A, pH 5.2, 1 mg/ml | Agg | Mon | Frag | Precipitation* | DVD-A, pH 5.2, 75 mg/ml | Agg | Mon | Frag | |
| 0 Histidine | 1.24 | 97.62 | 1.12 | N | 0 Histidine | 1.78 | 97.16 | 1.04 | N |
| 5 mM Histidine | 1 | 97.86 | 1.12 | N | 5 mM Histidine | 1.74 | 97.33 | 0.91 | N |
| 15 mM Histidine | 1.08 | 97.43 | 1.47 | N | 15 mM Histidine | 2.01 | 96.77 | 1.21 | N |
| 50 mM Histidine | 1.25 | 97.36 | 1.37 | N | 50 mM Histidine | 2.31 | 96.45 | 1.22 | N |
| 200 mM Histidine | 1.61 | 97.08 | 1.3 | N | 200 mM Histidine | | | | N |
| T0 | | | | | | | | | |
| DVD-C, pH 5.4, 1 mg/ml | Agg | Mon | Frag | Precipitation* | DVD-C, pH 5.4, 100 mg/ml | Agg | Mon | Frag | Precipitation* |
| 0 Histidine | 2.31 | 96.35 | 1.33 | N | 0 Histidine | 3.05 | 95.18 | 1.75 | N |
| 5 mM Histidine | 1.82 | 96.64 | 1.52 | N | 5 mM Histidine | 2.55 | 95.84 | 1.6 | N |
| 15 mM Histidine | 1.83 | 96.53 | 1.63 | N | 15 mM Histidine | 2.2 | 96.1 | 1.68 | N |
| 50 mM Histidine | 1.87 | 96.67 | 1.44 | N | 50 mM Histidine | 1.8 | 96.54 | 1.64 | N |
| 200 mM Histidine | 2.17 | 96.14 | 1.68 | N | 200 mM Histidine | 1.7 | 96.49 | 1.8 | N |
| 40C, 7d | | | | | | | | | |
| DVD-C, pH 5.4, 1 mg/ml | Agg | Mon | Frag | Precipitation* | DVD-C, pH 5.4, 100 mg/ml | Agg | Mon | Frag | Precipitation* |
| 0 Histidine | 2.41 | 92.11 | 5.47 | N | 0 Histidine | 3.18 | 94.49 | 2.31 | N |
| 5 mM Histidine | 1.62 | 95.93 | 2.43 | N | 5 mM Histidine | 2.65 | 95.2 | 2.13 | N |
| 15 mM Histidine | 1.49 | 95.9 | 2.59 | N | 15 mM Histidine | 2.46 | 94.96 | 2.57 | N |

(continued)

| 40C, 7d | | | | | | | | | |
|---|---|---|---|---|---|---|---|---|---|
| DVD-C, pH 5.4, 1 mg/ml | Agg | Mon | Frag | Precipitation* | DVD-C, pH 5.4, 100 mg/ml | Agg | Mon | Frag | Precipitation* |
| 50 mM Histidine | 1.38 | 96.16 | 2.45 | N | 50 mM Histidine | 2.33 | 95.16 | 2.49 | N |
| 200 mM Histidine | 1.47 | 96.12 | 2.39 | N | 200 mM Histidine | 1.82 | 95.19 | 2.97 | N |
| 40C, 21d | | | | | | | | | |
| DVD-C, pH 5.4, 1 mg/ml | Agg | Mon | Frag | Precipitation* | DVD-C, pH 5.4, 100 mg/ml | Agg | Mon | Frag | Precipitation* |
| 0 Histidine | 1.38 | 95.5 | 3.11 | N | 0 Histidine | 3.45 | 93.64 | 2.89 | N |
| 5 mM Histidine | 1.5 | 95.39 | 3.1 | N | 5 mM Histidine | 2.8 | 94.27 | 2.91 | N |
| 15 mM Histidine | 1.26 | 96.01 | 2.71 | N | 15 mM Histidine | 2.55 | 94.56 | 2.87 | N |
| 50mM Histidine | 1.34 | 95.83 | 2.82 | N | 50mM Histidine | 2.26 | 94.9 | 2.83 | N |
| 200 mM Histidine | 1.38 | 95.72 | 2.88 | N | 200 mM Histidine | 2.84 | 93.29 | 3.86 | N |
| 5C, 21d | | | | | | | | | |
| DVD-C, pH 5.4, 1 mg/ml | Agg | Mon | Frag | Precipitation* | DVD-C, pH 5.4, 100 mg/ml | Agg | Mon | Frag | Precipitation* |
| 0 Histidine | 2.08 | 96.12 | 1.78 | N | 0 Histidine | 2.77 | 94.77 | 2.44 | N |
| 5 mM Histidine | 1.56 | 96.72 | 1.71 | N | 5 mM Histidine | 2.35 | 95.43 | 2.2 | N |
| 15 mM Histidine | 1.27 | 97.08 | 1.63 | N | 15 mM Histidine | 2.18 | 95.42 | 2.38 | N |
| 50 mM Histidine | 1.4 | 96.67 | 1.91 | N | 50 mM Histidine | 1.95 | 96.07 | 1.96 | N |
| 200 mM Histidine | 1.59 | 96.62 | 1.77 | N | 200 mM Histidine | 1.95 | 96.1 | 1.94 | N |
| T0 | | | | | | | | | |
| IL12IL18, pH 5.4, 1 mg/ml | Age | Mon | Frag | Precipitation* | IL12IL18, pH 5.4, 150 mg/ml | Agg | Mon | Frag | Precipitation* |
| 0 Histidine | 2.95 | 95.23 | 1.8 | N | 0 Histidine | 3.48 | 94.22 | 2.29 | N |
| 5 mM Histidine | 1.92 | 96.28 | 1.79 | N | 5 mM Histidine | 4.64 | 93.26 | 2.09 | N |
| 15 mM Histidine | 2.16 | 95.71 | 2.12 | N | 15 mM Histidine | 4.33 | 93.53 | 2.12 | N |
| 50 mM Histidine | 2.07 | 96 | 1.91 | N | 50 mM Histidine | 3.93 | 94.2 | 1.88 | N |

(continued)

| T0 | | | | | | | | | |
|---|---|---|---|---|---|---|---|---|---|
| IL12IL18, pH 5.4, 1 mg/ml | Age | Mon | Frag | Precipitation* | IL12IL18, pH 5.4, 150 mg/ml | Agg | Mon | Frag | Precipitation* |
| 200 mM Histidine | 2.46 | 95.65 | 1.87 | N | 200 mM Histidine | 3.55 | 94.48 | 1.96 | N |
| **40C, 7d** | | | | | | | | | |
| IL12IL18, pH 5.4, 1 mg/ml | Agg | Mon | Frag | Precipitation* | IL12IL18, pH 5.4, 150 mg/ml | Agg | Mon | Frag | Precipitation* |
| 0 Histidine | 2.88 | 93.82 | 3.29 | N | 0 Histidine | 4.71 | 91.46 | 3.82 | N |
| 5 mM Histidine | 1.32 | 95.82 | 2.85 | N | 5 mM Histidine | 5.79 | 90.44 | 3.75 | N |
| 15 mM Histidine | 1.02 | 96.12 | 2.85 | N | 15 mM Histidine | 4.87 | 91.81 | 3.3 | N |
| 50 mM Histidine | 1.57 | 95.23 | 3.19 | N | 50 mM Histidine | 8.58 | 87.7 | 3.71 | N |
| 200 mM Histidine | 1.43 | 95.56 | 3 | N | 200 mM Histidine | 6.78 | 89.81 | 3.4 | N |
| **40C, 21d** | | | | | | | | | |
| IL12IL18, pH 5.4, 1 mg/ml | Agg | Mon | Frag | Precipitation* | IL121IL18, pH 5.4, 150 mg/ml | Agg | Mon | Frag | Precipitation* |
| 0 Histidine | 1.51 | 94.69 | 3.79 | N | 0 Histidine | 5.18 | 91.21 | 3.6 | N |
| 5 mM Histidine | 1.08 | 95.87 | 3.04 | N | 5 mM Histidine | 6.76 | 89.67 | 3.56 | N |
| 15 mM Histidine | 1.07 | 95.85 | 3.06 | N | 15 mM Histidine | 5.7 | 91.19 | 3.09 | N |
| 50 mM Histidine | 1.2 | 95.56 | 3.32 | N | 50 mM Histidine | 12.87 | 83.35 | 3.76 | N |
| 200 mM Histidine | 1.42 | 95.42 | 3.15 | N | 200 mM Histidine | 7.21 | 89.88 | 2.89 | N |
| **5C, 21d** | | | | | | | | | |
| IL12IL18, pH 5.4. 1 mg/ml | Agg | Mon | Frag | Precipitation* | IL12IL18, pH 5.4, 150 mg/ml | Agg | Mon | Frag | Precipitation* |
| 0 Histidine | 1.71 | 96.03 | 2.24 | N | 0 Histidine | 5.96 | 91.35 | 2.67 | N |
| 5 mM Histidine | 1.25 | 96.44 | 2.3 | N | 5 mM Histidine | 6.94 | 90.97 | 2.07 | N |
| 15 mM Histidine | 1.4 | 96.13 | 2.46 | N | 15 mM Histidine | 5.69 | 92.19 | 2.1 | N |
| 50 mM Histidine | 1.78 | 96.03 | 2.18 | N | 50 mM Histidine | 10.07 | 88.13 | 1.78 | N |

(continued)

| 5C, 21d | | | | | | | | | |
|---|---|---|---|---|---|---|---|---|---|
| IL12IL18, pH 5.4. 1 mg/ml | Agg | Mon | Frag | Precipitation* | IL12IL18, pH 5.4, 150 mg/ml | Agg | Mon | Frag | Precipitation* |
| 200 mM Histidine | 2.1 | 95.7 | 2.19 | N | 200 mM Histidine | 1.32 | 95.56 | 3.11 | N |
| * Precipitation indicates if insoluble visible aggregates were observed (Yes or No) | | | | | | | | | |

Table 17: Impact of Storage of Various DVD-Ig Proteins at Low and High Concentrations Under Various Histidine Concentrations on IEC

| T0 | | | | | | | |
|---|---|---|---|---|---|---|---|
| DVD-A, pH 5.2, 1 mg/ml | Acidic | Main | Basic | DVD-A, pH 5.2, 75 mg/ml | Acidic | Main | Basic |
| 0 Histidine | 15.66 | 49.3 | 35.02 | 0 Histidine | 19.71 | 50.72 | 29.55 |
| 5 mM Histidine | 13.15 | 50.9 | 35.93 | 5 mM Histidine | 19.11 | 50.8 | 30.08 |
| 15 mM Histidine | 15.11 | 46.74 | 38.13 | 15 mM Histidine | 18.58 | 44.52 | 36.88 |
| 50mM Histidine | 13.27 | 52.38 | 34.33 | 50mM Histidine | 15.69 | 52.06 | 32.24 |
| 200 mM Histidine | 14.55 | 52.01 | 33.42 | 200 mM Histidine | 16.79 | 45.36 | 37.84 |
| 40C, 7d | | | | | | | |
| DVD-A, pH 5.2, 1 mg/ml | Acidic | Main | Basic | DVD-A, pH 5.2, 75 mg/ml | Acidic | Main | Basic |
| 0 Histidine | 24.97 | 40.98 | 34.03 | 0 Histidine | 21.7 | 43.6 | 34.68 |
| 5 mM Histidine | 22.21 | 44.35 | 33.43 | 5 mM Histidine | 28.31 | 43.99 | 27.69 |
| 15 mM Histidine | 19.79 | 44.91 | 35.29 | 15 mM Histidine | 21.21 | 41.34 | 37.44 |
| 50mM Histidine | 18.19 | 46.11 | 35.68 | 50mM Histidine | 18.42 | 42.84 | 38.73 |
| 200 mM Histidine | 18.08 | 48.08 | 33.83 | 200 mM Histidine | 16.71 | 42.09 | 41.19 |
| 5C, 21d | | | | | | | |
| DVD-A, pH 5.2, 1 mg/ml | Acidic | Main | Basic | DVD-A, pH 5.2, 75 mg/ml | Acidic | Main | Basic |
| 0 Histidine | 15.66 | 46.47 | 37.86 | 0 Histidine | 16.41 | 48.16 | 35.41 |
| 5 mM Histidine | 18.76 | 49.37 | 31.85 | 5 mM Histidine | 16.19 | 49.44 | 34.36 |
| 15 mM Histidine | 14.34 | 52.8 | 32.85 | 15 mM Histidine | 16.91 | 45.77 | 37.3 |
| 50mM Histidine | 15.06 | 50.33 | 34.59 | 50mM Histidine | 17.17 | 47.45 | 35.37 |
| 200 mM Histidine | 14.49 | 48.8 | 36.7 | 200 mM Histidine | 15.77 | 46.18 | 38.03 |

(continued)

| T0 | | | | | | | |
|---|---|---|---|---|---|---|---|
| DVD-C, pH 5.4, 1 mg/ml | | | | DVD-C, pH 5.4, 100 mg/ml | | | |
| 0 Histidine | 26.28 | 59.4 | 14.3 | 0 Histidine | 24.53 | 66.44 | 9.01 |
| 5 mM Histidine | 23.01 | 69.42 | 7.56 | 5 mM Histidine | 24.17 | 65.96 | 9.86 |
| 15 mM Histidine | 22.05 | 67.79 | 10.14 | 15 mM Histidine | 23.67 | 67.77 | 8.55 |
| 50mM Histidine | 21.9 | 69.09 | 9 | 50mM Histidine | 22.71 | 67.65 | 9.63 |
| 200 mM Histidine | 20.99 | 70.74 | 8.26 | 200 mM Histidine | | | |
| 40C, 7d | | | | | | | |
| DVD-C, pH 5.4, 1 mg/ml | | | | DVD-C, pH 5.4, 100 mg/ml | | | |
| 0 Histidine | 34.05 | 55.74 | 10.2 | 0 Histidine | 28.68 | 61.69 | 9.61 |
| 5 mM Histidine | 31.26 | 59.07 | 9.65 | 5 mM Histidine | 29.05 | 61.57 | 9.37 |
| 15 mM Histidine | 27.8 | 61.86 | 10.33 | 15 mM Histidine | 29.2 | 61.25 | 9.54 |
| 50mM Histidine | 25.97 | 59.33 | 14.49 | 50mM Histidine | 27.43 | 61.86 | 10.69 |
| 200 mM Histidine | 26.35 | 62.28 | 11.36 | 200 mM Histidine | 28.04 | 59.58 | 12.37 |
| 5C, 21d | | | | | | | |
| DVD-C, pH 5.4, 1 mg/ml | | | | DVD-C, pH 5.4, 100 mg/ml | | | |
| 0 Histidine | 25.05 | 65.7 | 9.23 | 0 Histidine | 23.43 | 66.85 | 9.71 |
| 5 mM Histidine | 23.66 | 66.17 | 10.15 | 5 mM Histidine | 22.3 | 68.58 | 9.11 |
| 15 mM Histidine | 22.11 | 69.96 | 8.91 | 15 mM Histidine | 23.39 | 67.31 | 9.29 |
| 50mM Histidine | 21.79 | 68.96 | 9.23 | 50mM Histidine | 23.67 | 67.96 | 8.35 |
| 200 mM Histidine | 21.96 | 70.23 | 7.8 | 200 mM Histidine | 22.6 | 69.68 | 7.65 |
| T0 | | | | | | | |
| IL12IL18, pH 5.4, 1 mg/ml | | | | IL12IL18, pH 5.4, 150 mg/ml | | | |
| 0 Histidine | 30.24 | 51.83 | 17.92 | 0 Histidine | 31.05 | 50.74 | 18.2 |
| 5 mM Histidine | 29.11 | 54.71 | 16.16 | 5 mM Histidine | 30.3 | 53.53 | 16.16 |
| 15 mM Histidine | 28.36 | 57.23 | 14.39 | 15 mM Histidine | 30.02 | 53.9 | 16.07 |
| 50mM Histidine | 29.15 | 53.29 | 17.55 | 50mM Histidine | 28.42 | 51.31 | 20.26 |
| 200 mM Histidine | 35.59 | 52.31 | 12.09 | 200 mM Histidine | 28.85 | 55.24 | 15.9 |

(continued)

| 40C, 7d | | | | | | | |
|---|---|---|---|---|---|---|---|
| IL12IL18, pH 5.4, 1 mg/ml | | | | IL12IL18, pH 5.4, 150 mg/ml | | | |
| 0 Histidine | 37.24 | 45.3 | 17.45 | 0 Histidine | 34.95 | 44.85 | 20.19 |
| 5 mM Histidine | 36 | 47.57 | 16.42 | 5 mM Histidine | 31.94 | 45.7 | 22.34 |
| 15 mM Histidine | 36.17 | 50.01 | 13.81 | 15 mM Histidine | 32.39 | 45.54 | 22.05 |
| 50mM Histidine | 35.39 | 49.12 | 15.47 | 50mM Histidine | 37 | 41.76 | 21.23 |
| 200 mM Histidine | 38.48 | 45.03 | 16.47 | 200 mM Histidine | 30.86 | 47.34 | 21.78 |
| 5C, 21d | | | | | | | |
| IL12IL18, pH 5.4, 1 mg/ml | | | | IL12IL18, pH 5.4, 150 mg/ml | | | |
| 0 Histidine | 30.72 | 51.83 | 17.44 | 0 Histidine | 30.91 | 50.49 | 18.59 |
| 5 mM Histidine | 30.25 | 51 | 18.74 | 5 mM Histidine | 29.14 | 50.47 | 20.37 |
| 15 mM Histidine | 30.07 | 56.33 | 13.58 | 15 mM Histidine | 28.45 | 50.95 | 20.58 |
| 50mM Histidine | 30.51 | 55.5 | 13.97 | 50mM Histidine | 27.91 | 52.66 | 19.42 |
| 200 mM Histidine | 42.9 | 49 | 8.08 | 200 mM Histidine | 27.96 | 48.84 | 22.19 |

Tables 16 and 17 show that the amount of monomer remaining at different time points and at the formation of insoluble aggregates indicates that histidine concentrations in the range of about 5 to about 50 mM provided optimum stability. A concentration of about 200 mM histidine resulted in the formation of insoluble aggregates in some cases (see indication of precipitation in Table 16). 0 mM histidine formulations showed enhanced aggregation as indicated by formation of insoluble and soluble aggregates in some cases (in some cases soluble aggregates were higher at 0 than that at 5 mM histidine concentrations). Secondly, the pH is expected to be well maintained for longer storage times in formulations containing histidine. ,

The error in IEC measurements is usually higher (2-3 % variation) compared to SEC measurements with the same formulation. Hence taking that into account, no significant differences were observed within formulations as assayed by IEC indicating that the chemical stability was not as affected by the molarity of histidine, hence the chemical stability is independent of the buffer concentration (generally, in contrast to aggregation propensity).

**Example 8: Example of the Stability of an AS-DVD Ig Protein (DVD-CAnti-IL1 alpha/beta DVD-Ig Protein) in Solution**

An anti-ILI alpha/beta DVD-Ig protein (DVD-C) was assessed for stability over time at both 100 mg/ml and 1 mg/ml in different buffers, at different pHs and at different temperatures. Buffers that were tested at 100 mg/ml of DVD-C included 15 mM acetate pH 4; 15 mM acetate pH 5; 15 mM histidine pH 5.5; 15 mM succinate pH 5.5; 15 mM histidine pH 6.0; Water (no buffer) pH 6.0; 15 mM citrate pH 6.0; 15 mM histidine pH 6.5; and 15 mM Tris pH 8.0. Buffers that were tested at 1 mg/ml of DVD-C included 10 mM citrate + 10 mM phosphate buffer at pH 3, 4, 5, 6, 7, or 8.

The samples were stored at 50°C, 40°C, 25°C, and 5°C. At certain time points, samples were pulled and evaluated for stability. Physical stability was evaluated by size exclusion chromatography (SE-HPLC or SEC), including % aggregate, % monomer, % fragment, and total species recovered were quantitated. Chemical stability was evaluated by weak cation exchange chromatography (IEX-HPLC or IEC), including % acidic, % main, and % basic species quantitated.

Tables 18 and 19 describe stability of DVD-C at 100 mg/ml and 1 mg/ml, respectively, in various buffers and pH. In both Tables 17 and 18, size exclusion chromatography (SEC) data and ion-exchange chromatography (IEC) data

EP 3 466 973 A1

is displayed. Formulation and abbreviation keys are given below each table.

Table 18. Stability at Various Temperatures of DVD-C at 100 mg/ml in Different Buffers and at Different pHs.

| Time | Temp(°C) | Formulation | pH | SEC data | | | | IEX data | | |
|---|---|---|---|---|---|---|---|---|---|---|
| | | | | % HMW | % M | % LMW | AUC | % Acidic | % Main | % Basic |
| T0 | --- | ace | 4 | 0.88 | 98.03 | 1.09 | 45941 | 19.65 | 71.68 | 8.67 |
| T0 | --- | ace | 5 | 0.94 | 98.19 | 0.87 | 45789 | 19.79 | 71.60 | 8.61 |
| T0 | --- | his | 5.5 | 0.98 | 98.15 | 0.86 | 48085 | 20.40 | 71.90 | 7.70 |
| T0 | --- | succ | 5.5 | 1.04 | 97.96 | 1.00 | 49317 | 19.70 | 71.54 | 8.76 |
| T0 | --- | his | 6 | 1.15 | 97.93 | 0.92 | 48468 | 20.68 | 70.94 | 8.38 |
| T0 | --- | water | 6 | 1.87 | 97.27 | 0.86 | 48356 | 18.95 | 72.35 | 8.70 |
| T0 | --- | citrate | 6 | 1.38 | 97.54 | 1.09 | 44457 | 19.35 | 72.37 | 8.28 |
| T0 | --- | his | 6.5 | 1.16 | 97.93 | 0.91 | 47102 | 21.17 | 70.45 | 8.38 |
| T0 | --- | tris | 8 | 2.40 | 96.65 | 0.95 | 53889 | 21.71 | 69.12 | 9.17 |
| T7d | 40 | ace | 4 | 0.92 | 97.03 | 2.05 | 49124 | 19.16 | 71.61 | 9.23 |
| T7d | 40 | ace | 5 | 1.12 | 97.31 | 1.57 | 49077 | 21.62 | 71.09 | 7.29 |
| T7d | 40 | his | 5.5 | 1.17 | 97.27 | 1.56 | 49662 | 19.48 | 73.60 | 6.92 |
| T7d | 40 | succ | 5.5 | 1.46 | 97.06 | 1.48 | 48821 | 23.18 | 69.51 | 7.31 |
| T7d | 40 | his | 6 | 1.72 | 96.96 | 1.32 | 42872 | 20.99 | 73.36 | 5.64 |
| T7d | 40 | water | 6 | 2.48 | 96.22 | 1.30 | 37817 | 21.67 | 72.02 | 6.31 |
| T7d | 40 | citrate | 6 | 1.79 | 96.83 | 1.37 | 43022 | 21.70 | 71.52 | 6.78 |
| T7d | 40 | his | 6.5 | 2.08 | 96.65 | 1.27 | 48176 | 23.68 | 70.89 | 5.43 |
| T7d | 40 | tris | 8 | 4.58 | 93.87 | 1.55 | 48306 | 40.86 | 52.70 | 6.44 |
| T1mo | 40 | ace | 4 | 1.32 | 94.29 | 4.38 | 52518 | 34.78 | 32.07 | 33.16 |
| T1mo | 40 | ace | 5 | 1.64 | 95.36 | 3.00 | 53762 | 36.48 | 52.11 | 11.41 |
| T1mo | 40 | his | 5.5 | 1.61 | 95.38 | 3.01 | 52489 | 33.12 | 55.52 | 11.36 |
| T1mo | 40 | succ | 5.5 | 2.25 | 94.73 | 3.01 | 51508 | 40.94 | 46.88 | 12.19 |
| T1mo | 40 | his | 6 | 2.81 | 94.53 | 2.66 | 52229 | 34.46 | 56.00 | 9.54 |
| T1mo | 40 | water | 6 | 3.66 | 93.75 | 2.58 | 52285 | 33.46 | 56.53 | 10.01 |
| T1mo | 40 | citrate | 6 | 2.67 | 94.91 | 2.42 | 51968 | 36.54 | 52.80 | 10.67 |
| T1mo | 40 | his | 6.5 | 3.61 | 93.69 | 2.71 | 50276 | 39.06 | 51.20 | 9.74 |
| T1mo | 40 | tris | 8 | 7.49 | 85.24 | 7.28 | 53081 | 52.90 | 15.51 | 31.58 |
| T1mo | 25 | ace | 4 | 0.98 | 97.30 | 1.72 | 56026 | 23.39 | 59.02 | 17.59 |
| T1mo | 25 | ace | 5 | 1.15 | 97.31 | 1.54 | 55264 | 22.44 | 69.01 | 8.55 |
| T1mo | 25 | his | 5.5 | 1.16 | 97.41 | 1.43 | 54356 | 22.05 | 69.43 | 8.51 |
| T1mo | 25 | succ | 5.5 | 1.42 | 97.07 | 1.51 | 52417 | 23.00 | 67.63 | 9.37 |
| T1mo | 25 | his | 6 | 1.75 | 96.74 | 1.51 | 52220 | 23.80 | 67.67 | 8.52 |
| T1mo | 25 | water | 6 | 2.66 | 95.94 | 1.40 | 51198 | 23.68 | 67.35 | 8.98 |
| T1mo | 25 | citrate | 6 | 1.91 | 96.59 | 1.49 | 51137 | 22.41 | 68.14 | 9.45 |
| T1mo | 25 | his | 6.5 | 1.93 | 96.58 | 1.49 | 50594 | 25.93 | 65.50 | 8.56 |
| T1mo | 25 | tris | 8 | 4.35 | 93.87 | 1.78 | 51644 | 39.99 | 50.14 | 9.87 |

(continued)

| Time | Temp(°C) | Formulation | pH | SEC data | | | | IEX data | | |
|------|----------|-------------|-----|----------|------|-------|------|----------|--------|---------|
| | | | | % HMW | % M | % LMW | AUC | % Acidic | % Main | % Basic |
| T3mo | 40 | ace | 4 | 2.19 | 85.66 | 12.15 | 54546 | 45.24 | 29.61 | 25.15 |
| T3mo | 40 | ace | 5 | 2.69 | 89.70 | 7.61 | 63493 | 52.09 | 31.52 | 16.39 |
| T3mo | 40 | his | 5.5 | 2.57 | 89.79 | 7.64 | 64361 | 48.68 | 35.18 | 16.14 |
| T3mo | 40 | succ | 5.5 | 3.53 | 88.89 | 7.58 | 57855 | 60.00 | 24.73 | 15.28 |
| T3mo | 40 | his | 6 | 4.08 | 90.70 | 5.21 | 60062 | 50.85 | 37.10 | 12.04 |
| T3mo | 40 | water | 6 | 4.80 | 90.50 | 4.70 | 58908 | 47.55 | 43.39 | 9.06 |
| T3mo | 40 | citrate | 6 | 3.87 | 91.38 | 4.75 | 55505 | 56.39 | 30.85 | 12.76 |
| T3mo | 40 | his | 6.5 | 5.56 | 89.08 | 5.37 | 56243 | 58.77 | 32.28 | 8.95 |
| T3mo | 40 | tris | 8 | 14.20 | 75.16 | 10.64 | 59362 | 63.76 | 22.12 | 14.12 |
| T3mo | 25 | ace | 4 | 1.15 | 96.39 | 2.46 | 60821 | 25.24 | 61.82 | 12.94 |
| T3mo | 25 | ace | 5 | 1.29 | 96.85 | 1.86 | 53513 | 23.62 | 66.47 | 9.91 |
| T3mo | 25 | his | 5.5 | 1.34 | 96.84 | 1.82 | 56041 | 23.05 | 67.70 | 9.25 |
| T3mo | 25 | succ | 5.5 | 1.69 | 96.36 | 1.95 | 53657 | 27.27 | 61.25 | 11.48 |
| T3mo | 25 | his | 6 | 2.04 | 96.23 | 1.73 | 51684 | 26.17 | 63.16 | 10.67 |
| T3mo | 25 | water | 6 | 2.81 | 95.49 | 1.70 | 52442 | 25.01 | 64.07 | 10.91 |
| T3mo | 25 | citrate | 6 | 2.08 | 96.11 | 1.80 | 51993 | 24.48 | 64.34 | 11.18 |
| T3mo | 25 | his | 6.5 | 2.26 | 96.01 | 1.72 | 50988 | 30.52 | 59.06 | 10.42 |
| T3mo | 25 | tris | 8 | 5.61 | 91.87 | 2.51 | 52070 | 59.46 | 31.54 | 8.99 |
| T3mo | 5 | ace | 4 | 0.85 | 98.01 | 1.14 | 50571 | 18.16 | 71.76 | 10.09 |
| T3mo | 5 | ace | 5 | 1.06 | 97.91 | 1.04 | 49173 | 18.44 | 71.54 | 10.02 |
| T3mo | 5 | his | 5.5 | 1.33 | 97.65 | 1.02 | 51947 | 19.97 | 70.28 | 9.75 |
| T3mo | 5 | succ | 5.5 | 1.32 | 97.59 | 1.09 | 50358 | 19.12 | 70.88 | 10.00 |
| T3mo | 5 | his | 6 | 2.18 | 96.79 | 1.03 | 49875 | 22.34 | 67.47 | 10.19 |
| T3mo | 5 | water | 6 | 8.21 | 90.70 | 1.09 | 48448 | 18.89 | 67.56 | 13.55 |
| T3mo | 5 | citrate | 6 | 2.20 | 96.65 | 1.15 | 48907 | 19.08 | 70.77 | 10.16 |
| T3mo | 5 | his | 6.5 | 1.86 | 97.12 | 1.02 | 47895 | 22.91 | 67.10 | 9.99 |
| T3mo | 5 | tris | 8 | 5.93 | 93.02 | 1.05 | 49015 | 24.67 | 63.29 | 12.04 |

Formulation and abbreviation key:

ace = 15 mM acetate
his = 15 mM histidine succ = 15 mM succinate
water = formulated in water by dialysis
citrate = 15 mM citrate
tris = 15 mM TRIS
% HMW = percentage of high molecular weight species quantitated by SEC
% M = percentage of monomer quantitated by SEC
% LMW = percentage of low molecular weight species (fragments) quantitated by SEC
AUC = total integrated area of the SEC curve
% acidic = percentage of acidic species relative to the main species quantitated by IEC
% main = percentage of main species quantitated by IEC

% basic = percentage of basic species relative to the main species quantitated by IEX

T0 = time zero

T7d = 7 days of storage

T1mo = 1 month of storage

T3mo = 3 months of storage

Table 19. Stability at various temperatures of DVD-C at 1.0 mg/ml in 10 mM citrate + 10 mM phosphate buffer and at different pHs.

| Time | Temp(°C) | pH | SEC data | | | | IEX data | | |
|------|----------|-----|--------|------|-------|--------|----------|--------|---------|
| | | | % HMW | % M | % LMW | AUC | % Acidic | % Main | % Basic |
| 0 | --- | 3 | ND | ND | ND | ND | ND | ND | ND |
| 0 | --- | 4 | 0.78 | 97.97 | 1.25 | 106805 | 10.14 | 73.38 | 16.48 |
| 0 | --- | 5 | 1.07 | 97.71 | 1.21 | 104165 | 9.80 | 73.84 | 16.35 |
| 0 | --- | 6 | 1.23 | 97.62 | 1.15 | 99838 | 10.80 | 73.52 | 15.69 |
| 0 | --- | 7 | 1.37 | 97.43 | 1.21 | 98566 | 10.45 | 73.79 | 15.76 |
| 0 | --- | 8 | 1.48 | 97.30 | 1.22 | 93914 | 10.33 | 74.41 | 15.26 |
| 7d | 40 | 3 | ND | ND | ND | ND | ND | ND | ND |
| 7d | 40 | 4 | 0.70 | 94.51 | 4.79 | 99177 | 38.59 | 41.06 | 20.35 |
| 7d | 40 | 5 | 1.09 | 97.43 | 1.48 | 105735 | 24.92 | 57.07 | 18.01 |
| 7d | 40 | 6 | 1.30 | 97.16 | 1.54 | 101459 | 21.48 | 62.95 | 15.57 |
| 7d | 40 | 7 | 1.53 | 96.38 | 2.09 | 94903 | 16.60 | 69.48 | 13.93 |
| 7d | 40 | 8 | 2.00 | 95.74 | 2.25 | 95090 | 50.88 | 36.05 | 13.07 |
| 7d | 50 | 3 | ND | ND | ND | ND | ND | ND | ND |
| 7d | 50 | 4 | ND | ND | ND | ND | ND | ND | ND |
| 7d | 50 | 5 | 2.72 | 93.91 | 3.37 | 102195 | 49.52 | 32.87 | 17.61 |
| 7d | 50 | 6 | 2.23 | 95.55 | 2.22 | 99767 | 37.88 | 47.12 | 15.00 |
| 7d | 50 | 7 | 1.67 | 94.59 | 3.74 | 96751 | 54.67 | 32.09 | 13.24 |
| 7d | 50 | 8 | 1.82 | 94.13 | 4.05 | 85005 | 61.11 | 13.48 | 25.41 |
| 21d | 5 | 3 | ND | ND | ND | ND | ND | ND | ND |
| 21d | 5 | 4 | 0.01 | 96.54 | 3.45 | 105701 | 11.64 | 73.47 | 14.89 |
| 21d | 5 | 5 | 0.00 | 96.29 | 3.71 | 102947 | 11.40 | 73.19 | 15.41 |
| 21d | 5 | 6 | 0.01 | 96.33 | 3.66 | 98166 | 11.56 | 73.70 | 14.74 |
| 21d | 5 | 7 | 0.01 | 96.24 | 3.75 | 96077 | 17.02 | 67.39 | 15.60 |
| 21d | 5 | 8 | 0.01 | 95.63 | 4.36 | 91507 | 17.87 | 68.99 | 13.15 |
| 21d | 25 | 3 | ND | ND | ND | ND | ND | ND | ND |
| 21d | 25 | 4 | 0.08 | 95.89 | 4.02 | 107054 | 32.05 | 49.45 | 18.51 |
| 21d | 25 | 5 | 0.07 | 96.85 | 3.08 | 104609 | 20.27 | 62.85 | 16.88 |
| 21d | 25 | 6 | 0.05 | 96.86 | 3.09 | 100308 | 18.61 | 66.21 | 15.18 |
| 21d | 25 | 7 | 0.08 | 96.40 | 3.52 | 97790 | 18.83 | 67.03 | 14.14 |
| 21d | 25 | 8 | 0.07 | 95.82 | 4.12 | 92666 | 41.03 | 45.81 | 13.16 |
| 21d | 40 | 3 | ND | ND | ND | ND | ND | ND | ND |

(continued)

| Time | Temp(°C) | pH | SEC data | | | | IEX data | | |
|---|---|---|---|---|---|---|---|---|---|
| | | | % HMW | % M | % LMW | AUC | % Acidic | % Main | % Basic |
| 21d | 40 | 4 | 0.09 | 88.58 | 11.33 | 79690 | 61.27 | 18.98 | 19.74 |
| 21d | 40 | 5 | 0.20 | 95.13 | 4.67 | 105902 | 46.17 | 37.31 | 16.52 |
| 21d | 40 | 6 | 0.23 | 95.96 | 3.81 | 101455 | 31.13 | 54.02 | 14.85 |
| 21d | 40 | 7 | 0.33 | 94.32 | 5.35 | 98972 | 53.39 | 34.34 | 12.27 |
| 21d | 40 | 8 | 0.37 | 93.05 | 6.57 | 91576 | 59.60 | 15.00 | 25.40 |
| 21d | 50 | 3 | ND | ND | ND | ND | ND | ND | ND |
| 21d | 50 | 4 | ND | ND | ND | ND | ND | ND | ND |
| 21d | 50 | 5 | 0.20 | 91.14 | 8.66 | 90993 | 56.65 | 10.23 | 33.13 |
| 21d | 50 | 6 | 0.34 | 93.57 | 6.09 | 100229 | 66.40 | 21.90 | 11.70 |
| 21d | 50 | 7 | 0.54 | 89.92 | 9.54 | 91695 | 50.18 | 26.86 | 22.97 |
| 21d | 50 | 8 | 0.47 | 87.11 | 12.41 | 67323 | 47.09 | 17.82 | 35.09 |
| 3mo | 40 | 3 | ND | ND | ND | ND | ND | ND | ND |
| 3mo | 40 | 4 | 27.59 | 40.51 | 31.91 | 61759 | NR | NR | NR |
| 3mo | 40 | 5 | 5.12 | 85.68 | 9.19 | 119476 | NR | NR | NR |
| 3mo | 40 | 6 | 2.79 | 91.55 | 5.67 | 113431 | NR | NR | NR |
| 3mo | 40 | 7 | 3.40 | 88.33 | 8.27 | 106409 | NR | NR | NR |
| 3mo | 40 | 8 | 5.41 | 80.86 | 13.73 | 98142 | NR | NR | NR |
| 3mo | 25 | 3 | ND | ND | ND | ND | ND | ND | ND |
| 3mo | 25 | 4 | 1.10 | 91.64 | 7.26 | 109681 | 53.69 | 23.99 | 22.32 |
| 3mo | 25 | 5 | 1.24 | 96.19 | 2.57 | 106230 | 37.33 | 41.02 | 21.65 |
| 3mo | 25 | 6 | 1.56 | 96.65 | 1.79 | 102674 | 27.46 | 53.25 | 19.29 |
| 3mo | 25 | 7 | 1.85 | 95.59 | 2.56 | 100526 | 17.69 | 65.85 | 16.46 |
| 3mo | 25 | 8 | 2.37 | 94.80 | 2.83 | 95500 | 65.32 | 20.08 | 14.59 |
| 3mo | 5 | 3 | ND | ND | ND | ND | ND | ND | ND |
| 3mo | 5 | 4 | 0.83 | 97.90 | 1.28 | 104383 | 18.59 | 60.63 | 20.78 |
| 3mo | 5 | 5 | 1.14 | 97.86 | 1.00 | 101554 | 16.91 | 58.78 | 24.30 |
| 3mo | 5 | 6 | 1.31 | 97.75 | 0.95 | 97897 | 17.38 | 63.16 | 19.46 |
| 3mo | 5 | 7 | 1.54 | 97.43 | 1.03 | 96067 | 18.48 | 63.33 | 18.19 |
| 3mo | 5 | 8 | 1.67 | 97.27 | 1.06 | 92532 | 20.44 | 61.36 | 18.21 |
| 6.5mo | 25 | 3 | ND | ND | ND | ND | ND | ND | ND |
| 6.5mo | 25 | 4 | 1.67 | 85.69 | 12.64 | 7753 | 70.60 | 10.11 | 19.28 |
| 6.5mo | 25 | 5 | 1.35 | 94.61 | 4.04 | 7865 | 50.36 | 28.97 | 20.67 |
| 6.5mo | 25 | 6 | 1.69 | 95.80 | 2.51 | 7719 | 36.64 | 46.73 | 16.63 |
| 6.5mo | 25 | 7 | 2.12 | 94.15 | 3.74 | 7523 | 55.68 | 28.89 | 15.44 |
| 6.5mo | 25 | 8 | 2.71 | 93.02 | 4.26 | 7155 | 68.76 | 16.22 | 15.01 |
| 6.5mo | 5 | 3 | ND | ND | ND | ND | ND | ND | ND |
| 6.5mo | 5 | 4 | 0.76 | 97.78 | 1.46 | 7508 | 19.30 | 59.53 | 21.17 |

(continued)

| Time | Temp(°C) | pH | SEC data | | | | IEX data | | |
|---|---|---|---|---|---|---|---|---|---|
| | | | % HMW | % M | % LMW | AUC | % Acidic | % Main | % Basic |
| 6.5mo | 5 | 5 | 1.07 | 97.92 | 1.00 | 7315 | 17.96 | 60.34 | 21.70 |
| 6.5mo | 5 | 6 | 1.38 | 97.75 | 0.87 | 6969 | 19.99 | 60.32 | 19.69 |
| 6.5mo | 5 | 7 | 1.61 | 97.38 | 1.02 | 6859 | 19.46 | 62.30 | 18.24 |
| 6.5mo | 5 | 8 | 1.71 | 97.17 | 1.11 | 6501 | 21.06 | 59.64 | 19.30 |

Abbreviation key:

% HMW = percentage of high molecular weight species quantitated by SEC
% M = percentage of monomer quantitated by SEC
% LMW = percentage of low molecular weight species (fragments) quantitated by SEC
AUC = total integrated area of the SEC curve
% acidic = percentage of acidic species relative to the main species quantitated by IEX
% main = percentage of main species quantitated by IEX
% basic = percentage of basic species relative to the main species quantitated by IEX
ND = no species detected
NR = assay not performed
0 = time zero
7d = 7 days of storage
21d = 1 month of storage
3mo = 3 months of storage
6.5mo = 6.5 months of storage

The molecule completely degraded during dialysis at pH 3. No species were detected by SEC or IEC after dialysis at this condition at time zero. Also, storage at 50°C at pH 4 yielded the same result. Both results are indicated by ND.

In addition, the thermal stability of DVD-C was assessed by differential scanning calorimetry (DSC). The thermal stability was evaluated at 1.0 mg/ml of the molecule formulated in 10 mM citrate + 10 mM phosphate buffer at pH 4, 5, 6, 7, or 8. A higher onset temperature of unfolding or higher domain midpoint temperature of unfolding means greater thermal stability. The thermal stability at different pHs is often correlated with the long-term stability of the molecule formulated at those pHs. Therefore, the DSC data can help identify the pHs at which the molecule is most and least stable.

Table 20. Differential Scanning Calorimetry Data of DVD-C at 1.0 mg/ml in 10 mM Citrate + 10 mM Phosphate Buffer and at Different pHs*

| pH | Onset (°C) | Tm1 (°C) | Tm2 (°C) | Tm3 (°C) | Tm4 (°C) |
|---|---|---|---|---|---|
| 4 | 49.2 | 64.4 | 67.3 | 74.2 | 78.8 |
| 5 | 55.0 | 68.1 | 69.9 | 76.2 | 82.0 |
| 6 | 54.8 | 67.5 | 69.4 | 75.7 | 83.1 |
| 7 | 55.0 | 67.2 | 69.0 | 74.4 | 82.7 |
| 8 | 54.4 | 67.1 | 68.8 | 73.8 | 82.5 |
| * Numbered Tm values indicate the midpoint of the unfolding transitions. | | | | | |

As described above, the stability of DVD-C was tested in a number of different buffers and pHs, when stored at three different temperatures (40°C, 25°C, or 5°C). The concentration of DVD-C ranged from 1 mg/ml to 100 mg/ml. At 100 mg/ml, buffers included acetate, histidine, succinate, citrate, and tris. DVD-C was also formulated in plain water. The pH of the formulations ranged from 4 to 8. At 1 mg/ml, the protein was formulated in citrate-phosphate buffer with the pH ranging from 3 to 8. Once formulated, the samples of the DVD-Ig proteins were stored at the aforementioned temperatures. At specific time points, aliquots were taken and assessed for physical stability by SEC and chemical stability by weak cation exchange (WCX).

Overall, the data indicated DVD-C is stable except at pH 3 and pH 4. Specifically, the data suggest that physical stability of DVD-C is greatest at a pH near 5.5 and that chemical stability is highest at a pH near 6.0. Histidine and succinate were determined to be appropriate buffers for these pHs.

In addition, the thermal stability of DVD-C was assessed by differential scanning calorimetry (DSC), as described in Table 18. The thermal stability was evaluated at 1.0 mg/ml of DVD-C formulated in citrate-phosphate buffer at pHs 4 to 8. A higher onset temperature of unfolding (Ton) or higher domain midpoint temperature of unfolding (Tm) means greater thermal stability. Thermal stability is likely correlated with the long-term stability of the DVD-Ig protein. The data indicate similar thermal stability in the pH range of 5 to 8.

## EXAMPLE 9: Effect of a Polyol on the Stability of AS-DVD-Ig Proteins

Dynamic scanning fluorescence (DSF) was employed to assess the propensity of a protein to unfold. The impact of polyols, *e.g.*, sucrose and sorbitol, was investigated in order to assess the effect of these polyols on the stability of the protein in solution. DVD-A, DVD-C and an IL12/IL18 DVD-Ig protein were used as exemplary AS-DVD-Ig proteins. As shown in Table 21 below, in general, AS-DVD-Ig proteins at various concentrations (1, 100, 150 mg/ml) are stable with the presence of sucrose (*e.g.*, 40-160 mg/ml) and sorbitol (*e.g.*, 20-80 mg/ml). Moreover, an increase in the concentration of sorbitol and sucrose provided resulted in a slight increased stability. Hence addition of sugars to a buffer (*e.g.*, 15 mM histidine) containing protein formulation enhances the stability of AS-DVD-Ig proteins slightly

Table 21: Impact Of Polyols On The Thermodynamic Stability Of Various AS-DVD-Ig Proteins As Assessed By Dynamic Scanning Fluorescence At pH 6

| IL12IL18 DVD-Ig Protein Conc. | Onset of Unfolding (°C) |
|---|---|
| 1mg/ml, No Sorbitol | 62.8 |
| 1mg/ml, 20 mg/ml Sorbitol | 63.2 |
| 1mg/ml, 40 mg/ml Sorbitol | 63.1 |
| 1mg/ml, 80 mg/ml Sorbitol | 63.5 |
| 150 mg/ml, No Sorbitol | 57.5 |
| 150 mg/ml, 20 mg/ml Sorbitol | 57.6 |
| 150 mg/ml, 40 mg/ml Sorbitol | 57.6 |
| 150 mg/ml, 80 mg/ml Sorbitol | 58.5 |
| 1mg/ml, No Sucrose | 62.8 |
| 1mg/ml, 40 mg/ml Sucrose | 63.1 |
| 1m/ml, 80 mg/ml Sucrose | 64.1 |
| 1mg/ml, 160 mg/ml Sucrose | 64.3 |
| 150 mg/ml, No Sucrose | 57.5 |
| 150 mg/ml, 40 mg/ml Sucrose | 58.2 |
| 150 mg/ml, 80 mg/ml Sucrose | 58.3 |
| 150 mg/ml, 160 mg/ml Sucrose | 58.3 |
| DVD-C | |
| 1mg/ml, No Sorbitol | 62 |
| 1mg/ml, 20 mg/ml Sorbitol | 62.3 |
| 1mg/ml, 40 mg/ml Sorbitol | 62 |
| 1mg/ml, 80 mg/ml Sorbitol | 62.1 |
| 100 mg/ml, No Sorbitol | 47 |
| 100 mg/ml, 20 mg/ml Sorbitol | 47.5 |
| 100 mg/ml, 40 mg/ml Sorbitol | 47 |

(continued)

| DVD-C | |
|---|---|
| 100 mg/ml, 80 mg/ml Sorbitol | 48.1 |
| 1mg/ml, No Sucrose | 62 |
| 1mg/ml, 40 mg/ml Sucrose | 62.1 |
| 1mg/ml, 80 mg/ml Sucrose | 62 |
| 1mg/ml, 160 mg/ml Sucrose | 62.2 |
| 100 mg/ml, No Sucrose | 47 |
| 100 mg/ml, 40 mg/ml Sucrose | 46.9 |
| 100 mg/ml, 80 mg/ml Sucrose | 48.1 |
| 100 mg/ml, 160 mg/ml Sucrose | 48 |
| DVD-A | |
| 1mg/ml, No Sorbitol | 55.8 |
| 1mg/ml, 20 mg/ml Sorbitol | 56 |
| 1mg/ml, 40 mg/ml Sorbitol | 56 |
| 1mg/ml, 80 mg/ml Sorbitol | 56.1 |
| 75 mg/ml, No Sorbitol | 49.5 |
| 75 mg/ml, 20 mg/ml Sorbitol | 50 |
| 75 mg/ml, 40 mg/ml Sorbitol | 50.5 |
| 75 mg/ml, 80 mg/ml Sorbitol | 50.2 |
| 1mg/ml, No Sucrose | 55.8 |
| 1mg/nfl, 40 mg/ml Sucrose | 56 |
| 1mg/ml, 80 mg/ml Sucrose | 56.4 |
| 1mg/ml, 160 mg/ml Sucrose | 56.1 |
| 75 mg/ml, No Sucrose | 49.5 |
| 75 mg/ml, 40 mg/ml Sucrose | 49.5 |
| 75 mg/ml, 80 mg/ml Sucrose | 50 |
| 75 mg/ml, 160 mg/ml Sucrose | 51.1 |

As evidenced by the above data in Table 21, polyols (*e.g.*, sorbitol and sucrose) can improve stability of AS-DVD-Ig proteins in solution in a broad range (20 to 160 mg/ml sucrose and 20 to 80/ mg/ml sorbitol). Furthermore, the data reveal a decrease in the onset of unfolding, hence a decrease in the thermodynamic stability, with increasing DVD-Ig protein concentration. These data correlate with the observed instability, such as aggregation observed at high concentration liquid formulations.

## V. AS-DVD-IG PROTEINS ARE STABLE IN FORMULATIONS CONTAINING A BUFFER AND A POLYOL AT PH RANGE OF 4.5-7.5

### EXAMPLE 10: Effect of A Buffer on the Storage Stability of DVD-A in A Formulation Containing A Polyol

To assess the effect of a buffer on the storage stability of DVD-A in different buffers, the stability of the formulations was assessed before storage (T0) or after 7 days (7d) or 21 days (21d) of storage at 40°C (accelerated storage). DVD-A (85 mg/ml), an AS-DVD-Ig protein, was formulated in various buffers (15 mM citrate, 15 mM histidine, 15 mM arginine, 15 mM acetate, or water) at a pH of 5.2. Samples were filled into sterile vials (approx. 500 $\mu$l each) and stored under controlled conditions in temperature chambers and in the absence of light. The samples were analyzed using SEC and the results are provided in Tables 22 and 23.

Table 22: Effect of Buffer Type on Storage Stability of DVD-A As Measured By SEC

| Formulation | Time Point | Mon | Agg | Frag |
|---|---|---|---|---|
| 15 mM acetate, 80 mg/ml sucrose, pH 4.0 | T0 | 95.96 | 2.75 | 1.28 |
| | 7d, 40C | 71.84 | 24.93 | 3.21 |
| | 21d, 40C | 66.62 | 26.91 | 6.45 |
| 15 mM acetate, 80 mg/ml sucrose, pH 5.2 | T0 | 96.06 | 2.78 | 1.14 |
| | 7d, 40C | 89.19 | 8.76 | 2.03 |
| | 21d, 40C | 90.93 | 4.41 | 4.65 |
| 15 mM arginine, 80 mg/ml sucrose, pH 5.2 | T0 | 95.84 | 3.07 | 1.08 |
| | 7d, 40C | 89.62 | 8.54 | 1.82 |
| | 21d, 40C | 81.03 | 12.41 | 6.54 |
| 15 mM citrate, 80 mg/ml sucrose, pH 5.2 | T0 | 95.72 | 3.19 | 1.08 |
| | 7d, 40C | 90.54 | 6.96 | 2.48 |
| | 21d, 40C | 87.39 | 7.74 | 4.86 |
| 15 mM histidine, 80 mg/ml sucrose, pH 5.2 | T0 | 96 | 2.9 | 1.09 |
| | 7d, 40C | 91.04 | 6.91 | 2.03 |
| | 21d, 40C | 87.58 | 8.63 | 3.78 |
| Water, 80 mg/ml sucrose, pH 5.2 | T0 | 94.89 | 4.04 | 1.05 |
| | 7d, 40C | 88.4 | 9.76 | 1.82 |
| | 21d, 40C | 81.41 | 14.39 | 4.18 |

Table 23: Effect of Buffer Type on Storage Stability of DVD-A As Measured By IEC

| Formulation | Time Point | Main | Acidic | Basic |
|---|---|---|---|---|
| | T0 | 56.88 | 9.43 | 33.68 |
| 15 mM acetate, 80 mg/ml sucrose, pH 4.0 | 7d, 40C | 46.91 | 18.74 | 34.33 |
| | 21d, 40C | 34.62 | 24.82 | 40.54 |
| 15 mM acetate, 80 mg/ml sucrose, pH 5.2 | T0 | 57.2 | 9.45 | 33.34 |
| | 7d, 40C | 47.61 | 18.74 | 33.64 |
| | 21d, 40C | 35.29 | 33.35 | 31.35 |
| 15 mM arginine, 80 mg/ml sucrose, pH 5.2 | T0 | 63.44 | 9.93 | 26.62 |
| | 7d, 40C | 49.22 | 19.57 | 31.2 |
| | 21d, 40C | 37.87 | 25.3 | 36.81 |
| 15 mM citrate, 80 mg/ml sucrose, pH 5.2 | T0 | 59.59 | 7.88 | 35.51 |
| | 7d, 40C | 45.92 | 20.82 | 33.24 |
| | 21d, 40C | 31.97 | 31.81 | 36.21 |
| 15 mM histidine, 80 mg/ml sucrose, pH 5.2 | T0 | 58.29 | 8.18 | 33.52 |
| | 7d, 40C | 44.97 | 16.84 | 38.17 |
| | 21d, 40C | 36.33 | 24.88 | 38.77 |

(continued)

| Formulation | Time Point | Main | Acidic | Basic |
|---|---|---|---|---|
| Water, 80 mg/ml sucrose, pH 5.2 | T0 | 57.05 | 9.53 | 33.41 |
| | 7d, 40C | 48.23 | 19.96 | 37.15 |
| | 21d, 40C | 37.69 | 25.15 | 37.15 |

SEC results provided in Table 22 show that a pH 5.2 histidine formulation compared to the pH 4 histidine formulation had the lowest level of aggregates, although citrate and acetate buffers showed low levels of aggregates as well. Although citrate and acetate showed less soluble aggregates as measured by SEC, the solutions were visibly turbid indicating formation of insoluble aggregates. The visual turbidity was not significant, however, given the SEC measurements. Overall, citrate and acetate formulations were only slightly less stable than the histidine formulations. Given that the noise/error in IEC measurement is usually higher, no significant differences in the chemical stability were observed within the formulations presented in Table 23. The polyol only formulation was also slightly less stable as compared to the histidine formulation. Given the overall stability characteristics of proteins identified as AS-DVD-Ig proteins, the slight differences observed in the SEC and IEC analysis of Tables 22 and 23 showed that each of the tested buffers at pH 5.2 was stable. Thus, the differences observed between the tested buffers at pH 5.2 indicated that each could be used to provide stable formulations for AS-DVD-Ig proteins, including those at high concentrations.

## VI. AS-DVD-IG PROTEINS ARE STABLE IN FORMULATIONS CONTAINING A BUFFER, A POLYOL, AND A SURFACTANT AT PH RANGE OF 4.5-7.5

### EXAMPLE 11: AS-DVD-Ig Proteins Are Stable in A Range Of Histidine Formulations Containing Surfactants and Sugars

The following example describes the impact of pH, buffers, and excipients (including surfactants and polyols) on the physico-chemical stability of DVD-Ig proteins at low and high concentration formulations during accelerated / real time stability testing. In order to demonstrate that DVD-Ig proteins have distinctly different protein properties compared to monoclonal antibodies, five different formulation conditions that are widely known and used to maintain the stability of monoclonal antibodies were tested.

Using size exclusion chromatography (SEC) and ion exchange chromatography (IEC), the storage stability of low concentration (1 mg/ml) and higher concentration (100 mg/ml) AS-DVD-Ig protein formulations was evaluated following three storage conditions: no storage (T0), 1 month at a controlled temperature of 5 °C (1m, 5C), and 1 month at a controlled temperature of 40 °C (1m, 40C). Formulations with varying pH (a range of 5.25 to 7.2 was selected), buffers, and excipients were tested, according to the following conditions:

1) pH 5.25 and pH 6, 15 mM histidine, 80 mg/ml sucrose, 0.01% Tween 80;
2) pH 6, 15 mM histidine, 40 mg/ml sorbitol, 0.01% Tween 80;
3) PBS (10 mM phosphate, 125 mM NaCl) at pH 6 and 7.2;
4) 20 mM glycine, 26 mg/ml glycerol pH 6; and
5) Water, 0.01% Tween 80 pH 5 and 6.

The results are presented in Tables 24-27 below. The data show that not all DVD-Ig proteins are stable in all tested pH and formulation conditions. DVD-B formed high amounts of aggregates under all the solution conditions tested and were classified as non-AS-DVD-Ig proteins (in accordance with the assay presented in Example 4). All the other DVD-Ig proteins (previously selected as being AS-DVD-Ig proteins) behaved well and were stable.

Sucrose, sorbitol, glycerol, and glycine were used to evaluate the effect of these excipients. Tween 80 (polysorbate 80), a surfactant that provides stabilization against shaking stress, was also used to evaluate its impact on the stability of high concentration solutions. The impact of salt concentration was evaluated by varying the ionic strength using sodium chloride.

In general, a formulation at pH 6 or pH 5.2 in a histidine buffer was effective for all AS-DVD-Ig proteins. Both sorbitol and sucrose each improved stability. Sucrose resulted in the formation of slightly less monomer after defined time points. The presence of salt resulted in increased instability (defined as less monomer remaining), especially in the case of the IL12/IL18 DVD-Ig protein of Table 24 at 100 mg/mL and to a lesser extent at 1 mg/mL, as shown in Table 27. The presence of glycerol and glycine resulted in less monomer remaining following shelf stability as compared to other formulations. For example, according to Table 24, the IL12IL18 DVD-Ig protein was slightly more stable at pH 6 in the presence of sucrose than sorbitol. The data also demonstrate that the formulations with either none or very little ionic strength showed comparable stability over time.

SEC data showed the physical stability of the DVD-Ig proteins, wherein the rate of formation of aggregates and/or fragments was evaluated (see Table 24 & 26). As shown in Table 26 all molecules showed no significant tendency to aggregation at low concentrations. IEC data is an indicator of the chemical stability of a DVD-Ig protein. Deamidation, for example, results in formation of acidic species (conversion of the main species to acidic species). Generation of positively charged variants would lead to an increase in the basic species. Formation of any of the two acidic or basic species indicates instability, as the formation of these two species results in an overall decrease in the % of main species (see Table 25 & 27).

The results in Tables 24 to 27 suggest that an AS-DVD-Ig protein is stable in formulations comprising a surfactant alone, *e.g.,* 0.01% Tween 80 at pH 5 - 6.

Table 24: 100 mg/ml SEC Data for Various Stored Formulations

| DVD-Ig | Formulation | Time Point, temp | Mon | Agg | Frag |
|---|---|---|---|---|---|
| IL12IL18 | 15 mM Histidine, 80 mg/ml Sucrose, 0.01 % Tween, pH 5.25 | T0 | 96.53 | 1.1 | 2.36 |
| | | 1m, 5C | 95.97 | 1.75 | 2.26 |
| | | 1m, 40C | 92.58 | 2.86 | 4.55 |
| IL12IL18 | 15 mM Histidine, 80 mg/ml Sucrose, 0.01 % Tween, pH 6 | T0 | 96.31 | 1.15 | 2.52 |
| | | 1m, 5C | 96 | 1.71 | 2.27 |
| | | 1m, 40C | 92.11 | 3.5 | 4.38 |
| IL12IL18 | 15 mM Histidine, 40 mg/ml Sorbitol, 0.01 % Tween, pH 6 | T0 | 96.61 | 1.25 | 2.13 |
| | | 1m, 5C | 95.71 | 1.78 | 2.5 |
| | | 1m, 40C | 91.62 | 4.04 | 4.33 |
| 6 | 15 mM Histidine, 80 mg/ml Sucrose, 0.01 % Tween, pH 5.25 | T0 | 89.61 | 10.38 | 0 |
| | | 1m, 5C | 89.48 | 10.51 | 0 |
| | | 1m, 40C | 85.85 | 9.96 | 4.18 |
| 6 | 15 mM Histidine, 80 mg/ml Sucrose, 0.01 % Tween, pH 6 | T0 | 88.58 | 11.41 | 0 |
| | | 1m, 5C | 86.8 | 13.19 | 0 |
| | | 1m, 40C | 83.09 | 12.68 | 4.21 |
| 65 | 15 mM Histidine, 80 mg/ml Sucrose, 0.01 % Tween, pH 5.25 | T0 | 93.08 | 0.93 | 5.98 |
| | | 1m, 5C | 92.59 | 1.67 | 5.72 |
| | | 1m, 40C | 85.25 | 11.47 | 3.27 |
| 65 | 15 mM Histidine, 80 mg/ml Sucrose, 0.01 % Tween, pH 6 | T0 | 93.34 | 0.85 | 5.79 |
| | | 1m, 5C | 92.45 | 1.71 | 5.82 |
| | | 1m, 40C | 86.15 | 10.69 | 3.14 |
| 66 | 15 mM Histidine, 80 mg/ml Sucrose, 0.01 % Tween, pH 5.25 | T0 | 97.57 | 0.62 | 1.8 |
| | | 1m, 5C | 97.6 | 0.92 | 1.47 |
| | | 1m, 40C | 91.65 | 5 | 3.34 |
| 66 | 15 mM Histidine, 80 mg/ml Sucrose, 0.01 % Tween, pH 6 | T0 | 97.41 | 0.71 | 1.87 |
| | | 1m, 5C | 97.69 | 1.07 | 1.22 |
| | | 1m, 40C | 92.68 | 4.3 | 3.01 |
| 66 | 15 mM Histidine, 40 mg/ml Sorbitol, 0.01 % Tween, pH 6 | T0 | 97.65 | 0.61 | 1.73 |
| | | 1m, 5C | 97.52 | 1.04 | 1.42 |
| | | 1m, 40C | 91.53 | 3.98 | 4.47 |

(continued)

| DVD-Ig | Formulation | Time Point, temp | Mon | Agg | Frag |
|---|---|---|---|---|---|
| IL12IL18 | 10 mM Phosphate, 125 mM NaCl, pH 6 | T0 | 96.13 | 1.57 | 2.28 |
| | | 1m, 5C | 94.2 | 3.34 | 2.44 |
| | | 1m, 40C | 88.48 | 7.3 | 4.2 |
| IL12IL18 | 10 mM Phosphate, 125 mM NaCl, pH 7.2 | T0 | 96.07 | 1.66 | 2.26 |
| | | 1m, 5C | 94.64 | 2.91 | 2.43 |
| | | 1m, 40C | 87.71 | 8.03 | 4.25 |
| DVD-B | 10 mM Phosphate, 125 mM NaCl, pH 6 | T0 | 96.24 | 2.11 | 1.64 |
| | | 1m, 5C | 94.66 | 3.85 | 1.48 |
| | | 1m, 40C | 41.53 | 53.68 | 4.77 |
| DVD-B | 10 mM Phosphate, 125 mM NaCl, pH 7.2 | T0 | 95.75 | 2.45 | 1.79 |
| | | 1m, 5C | 95.11 | 3.62 | 1.26 |
| | | 1m, 40C | 33.96 | 60.56 | 5.46 |
| IL12IL18 | 20 mM Glycine, 26 mg/ml Glycerol, pH 6.0 | T0 | 96.48 | 1.27 | 2.24 |
| | | 1m, 5C | 95.06 | 2.07 | 2.86 |
| | | 1m, 40C | 90.33 | 4.7 | 4.95 |
| DVD-B | 20 mM Glycine, 26 mg/ml Glycerol, pH 6.0 | T0 | 96.29 | 1.88 | 1.81 |
| | | 1m, 5C | 95.93 | 2.62 | 1.43 |
| | | 1m, 40C | 23.32 | 73.53 | 3.14 |
| IL12IL18 | Water, 0.01% Tween 80, pH 5.0 | T0 | 95.06 | 2.55 | 2.37 |
| | | 1m, 5C | 94.12 | 2.97 | 2.89 |
| | | 1m, 40C | 90.99 | 4.93 | 4.07 |
| IL12IL18 | Water, 0.01% Tween 80, pH 6.0 | T0 | 94.59 | 2.88 | 2.52 |
| | | 1m, 5C | 94.22 | 3.28 | 2.48 |
| | | 1m, 40C | 90.9 | 4.82 | 4.26 |
| 5 | Water, 0.01% Tween 80, pH 5.0 | T0 | 65.33 | 31.34 | 3.31 |
| | | 1m, 5C | 17.02 | 79.12 | 3.84 |
| | | 1m, 40C | 82.54 | 13.41 | 4.03 |
| 5 | Water, 0.01% Tween 80, pH 6.0 | T0 | 43.43 | 53.33 | 3.23 |
| | | 1m, 5C | 17.53 | 79.15 | 3.3 |
| | | 1m, 40C | 74.6 | 21.38 | 4.03 |

Table 25: 100 mg/ml IEC Data for Various Stored Formulations

| DVD-Ig | Formulation | Time Point, temp | Main | Acidic | Basic |
|---|---|---|---|---|---|
| IL12IL18 | 15 mM Histidine, 80 mg/ml Sucrose, 0.01 % Tween, pH 5.25 | T0 | 58.32 | 27.98 | 13.68 |
| | | 1m, 5C | 57.54 | 26.72 | 15.73 |
| | | 1m, 40C | 44.91 | 37.65 | 17.43 |

(continued)

| DVD-Ig | Formulation | Time Point, temp | Main | Acidic | Basic |
|---|---|---|---|---|---|
| IL12IL18 | 15 mM Histidine, 80 mg/ml Sucrose, 0.01 % Tween, pH 6 | T0 | 58.63 | 27.84 | 13.51 |
| | | 1m, 5C | 56.89 | 29.15 | 13.94 |
| | | 1m, 40C | 41.47 | 44.38 | 14.14 |
| IL12IL18 | 15 mM Histidine, 40 mg/ml Sorbitol, 0.01 % Tween, pH 6 | T0 | 58.1 | 28.09 | 13.79 |
| | | 1m, 5C | 60.53 | 27.48 | 11.98 |
| | | 1m, 40C | 41.02 | 44.69 | 14.29 |
| 6 | 15 mM Histidine, 80 mg/ml Sucrose, 0.01 % Tween, pH 5.25 | T0 | 42.99 | 11.66 | 45.34 |
| | | 1m, 5C | 42.53 | 10.26 | 47.19 |
| | | 1m, 40C | 34.99 | 25.55 | 39.45 |
| 6 | 15 mM Histidine, 80 mg/ml Sucrose, 0.01 % Tween, pH 6 | T0 | 41.03 | 11.84 | 47.12 |
| | | 1m, 5C | 41.73 | 10.91 | 47.34 |
| | | 1m, 40C | 36.18 | 25.94 | 37.86 |
| 65 | 15 mM Histidine, 80 mg/ml Sucrose, 0.01 % Tween, pH 5.25 | T0 | 49.64 | 39.02 | 11.33 |
| | | 1m, 5C | 45.48 | 38.77 | 15.73 |
| | | 1m, 40C | 51.17 | 30.64 | 18.18 |
| 65 | 15 mM Histidine, 80 mg/ml Sucrose, 0.01 % Tween, pH 6 | T0 | 50 | 39.1 | 10.88 |
| | | 1m, 5C | 49.31 | 38.95 | 11.72 |
| | | 1m, 40C | 29.83 | 52.65 | 17.5 |
| 66 | 15 mM Histidine, 80 mg/ml Sucrose, 0.01 % Tween, pH 5.25 | T0 | 63.78 | 24.78 | 11.43 |
| | | 1m, 5C | 60.03 | 25.27 | 14.69 |
| | | 1m, 40C | 42.13 | 41.93 | 15.93 |
| 66 | 15 mM Histidine, 80 mg/ml Sucrose, 0.01 % Tween, pH 6 | T0 | 61.5 | 26.21 | 12.27 |
| | | 1m, 5C | 59.06 | 25.85 | 15.07 |
| | | 1m, 40C | 42.31 | 43.47 | 41.2 |
| 66 | 15 mM Histidine, 40 mg/ml Sorbitol, 0.01 % Tween, pH 6 | T0 | 57.18 | 24.54 | 18.26 |
| | | 1m, 5C | 61.9 | 26.5 | 11.59 |
| | | 1m, 40C | 41.24 | 45.29 | 13.46 |
| IL12IL18 | 10 mM Phosphate, 125 mM NaCl, pH 6 | T0 | 58.2 | 28.1 | 13.69 |
| | | 1m, 5C | 57.43 | 27.35 | 15.21 |
| | | 1m, 40C | 40.68 | 42.04 | 17.27 |
| IL12IL18 | 10 mM Phosphate, 125 mM NaCl, pH 7.2 | T0 | 54.51 | 27.99 | 17.48 |
| | | 1m, 5C | 53.79 | 28.08 | 18.11 |
| | | 1m, 40C | 27.08 | 58.25 | 14.65 |
| DVD-B | 10 mM Phosphate, 125 mM NaCl, pH 6 | T0 | 52.94 | 27.32 | 19.72 |
| | | 1m, 5C | 54.38 | 27.19 | 18.42 |
| | | 1m, 40C | 29.14 | 38.63 | 32.21 |

(continued)

| DVD-Ig | Formulation | Time Point, temp | Main | Acidic | Basic |
|---|---|---|---|---|---|
| DVD-B | 10 mM Phosphate, 125 mM NaCl, pH 7.2 | T0 | 27.93 | 52.59 | 19.46 |
| | | 1m, 5C | 54.07 | 26.57 | 19.35 |
| | | 1m, 40C | 19.79 | 48.61 | 31.59 |
| IL12IL18 | 20 mM Glycine, 26 mg/ml Glycerol, pH 6.0 | T0 | 57.95 | 28.99 | 13.05 |
| | | 1m, 5C | 57.85 | 29.95 | 12.18 |
| | | 1m, 40C | 37.55 | 47.49 | 14.94 |
| DVD-B | 20 mM Glycine, 26 mg/ml Glycerol, pH 6.0 | T0 | 30.77 | 51 | 18.22 |
| | | 1m, 5C | 50 | 33.32 | 16.67 |
| | | 1m, 40C | 27.41 | 58.52 | 14.05 |
| IL12IL18 | Water, 0.01% Tween 80, pH 5.0 | T0 | 56.03 | 27.31 | 16.64 |
| | | 1m, 5C | 57.67 | 28.06 | 14.26 |
| | | 1m, 40C | 44.97 | 41.34 | 13.68 |
| IL12IL18 | Water, 0.01% Tween 80, pH 6.0 | T0 | 58.4 | 28.57 | 13.02 |
| | | 1m, 5C | 58.93 | 26.36 | 14.7 |
| | | 1m, 40C | 45.37 | 39.4 | 15.21 |
| 5 | Water, 0.01% Tween 80, pH 5.0 | T0 | 46.39 | 19.48 | 34.12 |
| | | 1m, 5C | 16.75 | 8.54 | 74.69 |
| | | 1m, 40C | 44.57 | 36.64 | 18.78 |
| 5 | Water, 0.01% Tween 80, pH 6.0 | T0 | 32.31 | 14.89 | 52.78 |
| | | 1m, 5C | 16.32 | 8.27 | 75.4 |
| | | 1m, 40C | 39.7 | 35.23 | 25.06 |

Table 26: 1 mg/ml SEC Data for Various Stored Formulations

| DVD-Ig | Formulation | Time Point, temp | Mon | Agg | Frag |
|---|---|---|---|---|---|
| IL12IL18 | 15 mM Histidine, 80 mg/ml Sucrose, 0.01 % Tween, pH 5.25 | T0 | 97.87 | 0.73 | 1.39 |
| | | 1m, 5C | 97.83 | 0.57 | 1.59 |
| | | 1m, 40C | 95.55 | 0.74 | 3.7 |
| IL12IL18 | 15 mM Histidine, 80 mg/ml Sucrose, 0.01 % Tween, pH 6 | T0 | 98.04 | 0.32 | 1.62 |
| | | 1m, 5C | 97.95 | 0.24 | 1.79 |
| | | 1m, 40C | 95.83 | 0.58 | 3.58 |
| IL12IL18 | 15 mM Histidine, 40 mg/ml Sorbitol, 0.01 % Tween, pH 6 | T0 | 96.2 | 0.56 | 3.22 |
| | | 1m, 5C | 96.41 | 0.47 | 3.1 |
| | | 1m, 40C | 94.21 | 0.66 | 5.12 |
| 6 | 15 mM Histidine, 80 mg/ml Sucrose, 0.01 % Tween, pH 5.25 | T0 | 92.05 | 7.94 | 0 |
| | | 1m, 5C | 99.23 | 0.76 | 0 |
| | | 1m, 40C | 95.52 | 0.53 | 3.94 |

(continued)

| DVD-Ig | Formulation | Time Point, temp | Mon | Agg | Frag |
|---|---|---|---|---|---|
| 6 | 15 mM Histidine, 80 mg/ml Sucrose, 0.01 % Tween, pH 6 | T0 | 93.59 | 6.4 | 0 |
| | | 1m, 5C | 99.33 | 0.66 | 0 |
| | | 1m, 40C | 95.3 | 0.59 | 4.1 |
| 65 | 15 mM Histidine, 80 mg/ml Sucrose, 0.01 % Tween, pH 5.25 | T0 | 94.74 | 0.51 | 4.73 |
| | | 1m, 5C | 94.34 | 0.49 | 5.15 |
| | | 1m, 40C | 93.06 | 0.91 | 6.02 |
| 65 | 15 mM Histidine, 80 mg/ml Sucrose, 0.01 % Tween, pH 6 | T0 | 94.33 | 0.37 | 5.29 |
| | | 1m, 5C | 93.98 | 0.36 | 5.65 |
| | | 1m, 40C | 92.71 | 0.87 | 6.41 |
| 66 | 15 mM Histidine, 80 mg/ml Sucrose, 0.01 % Tween, pH 5.25 | T0 | 98.76 | 0.51 | 0.72 |
| | | 1m, 5C | 98.59 | 0.51 | 0.89 |
| | | 1m, 40C | 96.37 | 1.02 | 2.59 |
| 66 | 15 mM Histidine, 80 mg/ml Sucrose, 0.01 % Tween, pH 6 | T0 | 98.82 | 0.2 | 0.96 |
| | | 1m, 5C | 98.9 | 0.16 | 0.92 |
| | | 1m, 40C | 96.82 | 0.66 | 2.51 |
| 66 | 15 mM Histidine, 40 mg/ml Sorbitol, 0.01 % Tween, pH 6 | T0 | 97.87 | 0.29 | 1.83 |
| | | 1m, 5C | 97.3 | 0.23 | 2.45 |
| | | 1m, 40C | 95.5 | 1.02 | 3.48 |
| IL12IL18 | 10 mM Phosphate, 125 mM NaCl, pH 6 | T0 | 95.38 | 1.54 | 3.07 |
| | | 1m, 5C | 95.29 | 1.54 | 3.15 |
| | | 1m, 40C | 91.87 | 1.95 | 6.17 |
| IL12IL18 | 10 mM Phosphate, 125 mM NaCl, pH 7.2 | T0 | 94.36 | 2.73 | 2.89 |
| | | 1m, 5C | 93.78 | 2.99 | 3.22 |
| | | 1m, 40C | 86.45 | 3.64 | 9.9 |
| DVD-B | 10 mM Phosphate, 125 mM NaCl, pH 6 | T0 | 95.82 | 1.66 | 2.5 |
| | | 1m, 5C | 95.54 | 1.53 | 2.91 |
| | | 1m, 40C | 91.39 | 2.31 | 6.29 |
| DVD-B | 10 mM Phosphate, 125 mM NaCl, pH 7.2 | T0 | 94.87 | 2.43 | 2.68 |
| | | 1m, 5C | 95.01 | 2.3 | 2.68 |
| | | 1m, 40C | 88.42 | 3.24 | 8.32 |
| IL12IL18 | 20 mM Glycine, 26 mg/ml Glycerol, pH 6.0 | T0 | 96.48 | 0.36 | 3.14 |
| | | 1m, 5C | 96.51 | 0.29 | 3.18 |
| | | 1m, 40C | 93.82 | 0.4 | 5.77 |
| DVD-B | 20 mM Glycine, 26 mg/ml Glycerol, pH 6.0 | TO | 96.05 | 1.33 | 2.6 |
| | | 1m, 5C | 95.47 | 1.15 | 3.37 |
| | | 1m, 40C | 94.14 | 1.22 | 4.63 |

(continued)

| DVD-Ig | Formulation | Time Point, temp | Mon | Agg | Frag |
|--------|-------------|------------------|-----|-----|------|
| IL12IL18 | Water, 0.01% Tween 80, pH 5.0 | T0 | 89.16 | 2.42 | 8.41 |
| | | 1m, 5C | 95.1 | 1.86 | 3.02 |
| | | 1m, 40C | 89.69 | 4.11 | 6.18 |
| IL12IL18 | Water, 0.01% Tween 80, pH 6.0 | T0 | 94.24 | 2.88 | 2.86 |
| | | 1m, 5C | 94.89 | 2.6 | 2.49 |
| | | 1m, 40C | 90.66 | 3.38 | 5.94 |
| 5 | Water, pH 5.0 | T0 | 69.49 | 26.36 | 4.13 |
| | | 1m, 5C | 68.87 | 24.88 | 6.23 |
| | | 1m, 40C | 91.35 | 3.6 | 5.08 |
| 5 | Water, pH 6.0 | T0 | 49.15 | 46.65 | 4.19 |
| | | 1m, 5C | 51.96 | 43.27 | 4.76 |
| | | 1m, 40C | 92.56 | 2.45 | 4.97 |

Table 27: 1 mg/ml IEC Data for the Various Stored Formulations

| DVD-Ig | Formulation | Time Point, temp | Main | Acidic | Basic |
|--------|-------------|------------------|------|--------|-------|
| IL12IL18 | 15 mM Histidine, 80 mg/ml Sucrose, 0.01 % Tween, pH 5.25 | T0 | 61.83 | 24 | 14.14 |
| | | 1m, 5C | 61.76 | 26.77 | 11.46 |
| | | 1m, 40C | 41.58 | 39.69 | 18.72 |
| IL12IL18 | 15 mM Histidine, 80 mg/ml Sucrose, 0.01 % Tween, pH 6 | T0 | 61.31 | 26.12 | 12.55 |
| | | 1m, 5C | 59.67 | 25.3 | 15.02 |
| | | 1m, 40C | 42.98 | 45.85 | 11.15 |
| IL12IL18 | 15 mM Histidine, 40 mg/ml Sorbitol, 0.01 % Tween, pH 6 | T0 | 60.81 | 25.86 | 13.31 |
| | | 1m, 5C | 59.07 | 27.12 | 13.8 |
| | | 1m, 40C | 38.22 | 47.55 | 14.21 |
| 6 | 15 mM Histidine, 80 mg/ml Sucrose, 0.01 % Tween, pH 5.25 | T0 | 41.13 | 10.3 | 48.56 |
| | | 1m, 5C | 46.96 | 10.38 | 42.64 |
| | | 1m, 40C | 36 | 24.57 | 39.42 |
| 6 | 15 mM Histidine, 80 mg/ml Sucrose, 0.01 % Tween, pH 6 | T0 | 44.43 | 9.92 | 45.63 |
| | | 1m, 5C | 46.33 | 11.55 | 42.11 |
| | | 1m, 40C | 37.38 | 26.09 | 36.52 |
| 65 | 15 mM Histidine, 80 mg/ml Sucrose, 0.01 % Tween, pH 5.25 | T0 | 50.6 | 39.58 | 9.81 |
| | | 1m, 5C | 49.57 | 37.75 | 11.66 |
| | | 1m, 40C | 33.38 | 50.27 | 16.33 |
| 65 | 15 mM Histidine, 80 mg/ml Sucrose, 0.01 % Tween, pH 6 | T0 | 51.74 | 38.73 | 9.52 |
| | | 1m, 5C | 53.21 | 36.58 | 10.2 |
| | | 1m, 40C | 35.11 | 49.96 | 14.92 |

(continued)

| DVD-Ig | Formulation | Time Point, temp | Main | Acidic | Basic |
|---|---|---|---|---|---|
| 66 | 15 mM Histidine, 80 mg/ml Sucrose, 0.01 % Tween, pH 5.25 | T0 | 59.89 | 25.96 | 14.14 |
| | | 1m, 5C | 58.1 | 25.05 | 16.84 |
| | | 1m, 40C | 45.43 | 37.21 | 17.34 |
| 66 | 15 mM Histidine, 80 mg/ml Sucrose, 0.01 % Tween, pH 6 | T0 | 58.18 | 24.86 | 16.94 |
| | | 1m, 5C | 61.69 | 24.94 | 13.36 |
| | | 1m, 40C | 44.83 | 41.39 | 13.77 |
| 66 | 15 mM Histidine, 40 mg/ml Sorbitol, 0.01 % Tween, pH 6 | T0 | 61.49 | 24.96 | 13.53 |
| | | 1m, 5C | 60.34 | 24.96 | 16.69 |
| | | 1m, 40C | 38.32 | 50.92 | 10.75 |
| IL12IL18 | 10 mM Phosphate, 125 mM NaCl, pH 6 | T0 | 58.52 | 26.18 | 15.29 |
| | | 1m, 5C | 58.98 | 27.17 | 13.84 |
| | | 1m, 40C | 37.54 | 47.49 | 14.96 |
| IL12IL18 | 10 mM Phosphate, 125 mM NaCl, pH 7.2 | T0 | 63.15 | 23.13 | 13.71 |
| | | 1m, 5C | 58.58 | 26.07 | 15.33 |
| | | 1m, 40C | 16.92 | 73.33 | 9.74 |
| DVD-B | 10 mM Phosphate, 125 mM NaCl, pH 6 | T0 | 54.38 | 27.35 | 18.25 |
| | | 1m, 5C | 56.48 | 27.42 | 16.09 |
| | | 1m, 40C | 30.99 | 50.68 | 18.32 |
| DVD-B | 10 mM Phosphate, 125 mM NaCl, pH 7.2 | T0 | 54.86 | 30 | 15.12 |
| | | 1m, 5C | 52.66 | 31.63 | 15.69 |
| | | 1m, 40C | 8.64 | 60.51 | 30.84 |
| IL12IL18 | 20 mM Glycine, 26 mg/ml Glycerol, pH 6.0 | T0 | 57.58 | 27.8 | 14.6 |
| | | 1m, 5C | 58.02 | 29.5 | 12.46 |
| | | 1m, 40C | 28.8 | 60.9 | 10.29 |
| TNFPGE 2 | 20 mM Glycine, 26 mg/ml Glycerol, pH 6.0 | T0 | 53.05 | 30.46 | 16.48 |
| | | 1m, 5C | 44.21 | 33.97 | 21.8 |
| | | 1m, 40C | 5.52 | 81.17 | 13.3 |
| IL12IL18 | Water, 0.01% Tween 80, pH 5.0 | T0 | 57.76 | 26.51 | 15.71 |
| | | 1m, 5C | 58.44 | 26.13 | 15.41 |
| | | 1m, 40C | 36.16 | 44.81 | 19.02 |
| IL12IL18 | Water, 0.01% Tween 80, pH 6.0 | T0 | 59.24 | 26.09 | 14.66 |
| | | 1m, 5C | 58.66 | 26.96 | 14.36 |
| | | 1m, 40C | 35.97 | 44.16 | 19.85 |
| 5 | Water, 0.01% Tween 80, pH 5.0 | T0 | 51.73 | 19.4 | 28.85 |
| | | 1m, 5C | 48.63 | 20.14 | 31.21 |
| | | 1m, 40C | 44.52 | 41.73 | 13.73 |

(continued)

| DVD-Ig | Formulation | Time Point, temp | Main | Acidic | Basic |
|---|---|---|---|---|---|
| 5 | Water, 0.01% Tween 80, pH 6.0 | T0 | 34.34 | 14 | 51.62 |
| | | 1m, 5C | 32.27 | 15.38 | 47.33 |
| | | 1m, 40C | 42.03 | 44.28 | 13.67 |

**EXAMPLE 12. Impact of Storage on the Stability of an AS-DVD-Ig Protein, (DVD-C), In Various Formulations**

The pH and the storage temperature of a protein formulation are two important factors influencing protein stability during accelerated/long-term storage. To assess the impact of these factors, the DVD-Ig protein was exposed to short-term storage at elevated and real time temperatures in order to gain insight into the formulation feasibility of long-term storage at lower temperatures (e.g., 2-8°C).

The storage stability of DVD-C in solution (100 mg/ml) was evaluated in formulations at 40°C. After defined storage periods, samples were pulled and the impact of storage time on DVD-Ig protein stability was evaluated. Samples were filled into sterile vials (approx. 500 μl each) and stored under controlled conditions, in a temperature chamber and in the absence of light. At predefined points of time, samples of prepared solutions were pulled for analysis according to the sample pull scheme. The resulting data is provided in Tables 28 and 29.

Table 28: Impact of Storage of DVD-C at 100 mg/ml Concentrations in Various Conditions As Measured By SEC

| Formulation | Time Point | Mon | Agg | Frag |
|---|---|---|---|---|
| 15 mM acetate 80 mg/ml sucrose 0.02 % Tween 80 pH 5 | T0 | 97.66 | 1.09 | 1.20 |
| | 7d, 40°C | 96.32 | 1.70 | 1.98 |
| | 1m, 40°C | 94.18 | 2.43 | 3.39 |
| 15 mM histidine 80 mg/ml sucrose 0.02 % Tween 80 pH 6 | T0 | 97.66 | 1.091 | 1.20 |
| | 7d, 40°C | 95.92 | 2.09 | 1.99 |
| | 1m, 40°C | 93.64 | 2.72 | 3.64 |

Table 29: Impact of Storage of DVD-C at 100 mg/ml Concentrations in Various Conditions As Measured By IEC

| Formulation | Time Point | Main | Acidic | Basic |
|---|---|---|---|---|
| 15 mM acetate 80 mg/ml sucrose 0.02 % Tween 80 pH 5 | T0 | 73.84 | 9.80 | 16.35 |
| | T2m, 5°C | 69.18 | 10.88 | 19.95 |
| 15 mM histidine 80 mg/ml sucrose 0.02 % Tween 80 pH 6 | T0 | 73.84 | 9.80 | 16.35 |
| | T2m, 5°C | 67.99 | 10.59 | 21.42 |

The data provided in Tables 28 and 29 show that DVD-C was very stable (compared to some unstable DVD-Ig proteins, for example, in Example 5) in that only minimal loss in monomer levels occurred during the test storage conditions and hence would be classified as an AS-DVD-Ig protein.

**EXAMPLE 13: Effect of a Surfactant on the Stability of AS-DVD-Ig Proteins in Buffer and Polyol Containing Formulations**

It is generally beneficial to set a formulation pH more than 1 unit from the protein's isoelectric point (pI). The more a formulation pH approximates the pI, generally, the more the overall surface of the protein is regarded as uncharged, thus contributing to protein-protein attraction of non-polar groups, and thus enhancing non-covalent aggregation and instability. Shaking and stirring foster physical instability, creating hydrophobic air/water interfaces, which result in alignment of protein molecules at these interfaces, and eventually result in aggregation. Given that air is more hydrophobic than water, the interface between air and liquid is deemed to be a denaturing surface at which aggregation, especially of (partially) unfolded proteins can originate. The effective air-water interface can be increased by shaking or stirring.

In the following example, the effect of various concentrations of a surfactant (e.g., Tween 80) on the instability of exemplary DVD-Ig proteins was evaluated. The study was done in the absence or presence of the polyol sucrose. The data presented in Tables 30 and 31 compare various surfactant concentrations (0 to 2 mg/ml) in a histidine buffer at pH 5.2 or 5.4 with and without sucrose (80 mg/ml). Results of turbidity measurements show that a surfactant (Tween 80) in a concentration range of 0.05 mg/ml - 2 mg/ml provided stability against shear/ denaturation stress to AS-DVD-Ig proteins in general. The turbidity increased upon lowering the surfactant concentration to 0.01 mg/ml. Similar observations were made for AS-DVD-Ig proteins in the presence of sucrose. All studies were conducted at 15 mM histidine at a DVD-Ig protein concentration of 1 mg/ml. The corresponding SEC analysis showed that, in general, a significant loss in rel. % monomer for samples that contained 0.05 - 0.1 mg/mL Tween80, indicating that the amount of Tween 80 that yielded the most stable formulations is between 0.5 mg/mL and 2 mg/mL. Turbidity measurements at 500 nm using UV are listed in Table 30 and the SEC analysis of the samples is listed in Table 31. Ranges of pH were also tested in the formulations described below.

Table 30: Effect of Tween on the Stability of Various DVD-Ig Proteins as Assessed by Air/Liquid Interface Denaturation Study/Shaking Study* by measurements at 500 nm using UV

| DVD Form: | DVD-A, 15 mM Histidine, pH 5.2, 80 mg/ml Sucrose | | | DVD-C, 15 mM Histidine, pH 5.4, 80 mg/ml Sucrose | | | IL12IL18, 15 mM Histidine, pH 5.4, 80 mg/ml Sucrose | | |
|---|---|---|---|---|---|---|---|---|---|
| Tween 80 | T0 | T24h | T96h | T0 | T24h | T96h | T0 | T24h | T96h |
| 0 | 0.11 | 0.07 | 0.55 | 0.085 | 0.08 | 0.107 | 0.051 | 0.051 | 0.051 |
| 2 mg/ml | 0.069 | 0.018 | 0.004 | 0.085 | 0.038 | 0.003 | 0.069 | 0.072 | 0.017 |
| 0.5 mg/ml | 0.014 | 0.014 | 0.002 | 0.025 | 0.036 | 0.004 | 0.09 | 0.006 | 0.008 |
| 0.1 mg/ml | 0.061 | 0.02 | 0.001 | 0.043 | 0.007 | 0.002 | 0.055 | 0.011 | 0.0109 |
| 0.05 mg/ml | 0.018 | 0.006 | 0.001 | 0.019 | 0.008 | 0.002 | 0.022 | 0.008 | 0.0208 |
| 0.01 mg/ml | 0.007 | 0.007 | 1.03 | 0.03 | 0.033 | 0.22 | 0.057 | 0.081 | 0.082 |
| | | | | | | | | | |
| | DVD-A, 15 mM Histidine, pH 5.2 | | | DVD-C, 15 mM Histidine, pH 5.4 | | | IL12IL18, 15 mM Histidine, pH 5.4 | | |
| Tween 80 | T0 | T24h | T96h | T0 | T24h | T96h | T0 | T24h | T96h |
| 0 | 0.045 | 0.038 | 0.29 | 0.015 | 0.045 | 0.038 | 0.046 | 0.046 | 0.038 |
| 2 mg/ml | 0.049 | 0.024 | 0.004 | 0.015 | 0.02 | 0.003 | 0.097 | 0.03 | 0.018 |
| 0.5 mg/ml | 0.016 | 0.058 | 0.004 | 0.03 | 0.019 | 0.003 | 0.049 | 0.031 | 0.013 |
| 0.1 mg/ml | 0.005 | 0.005 | 0.002 | 0.004 | 0.005 | 0.002 | 0.012 | 0.021 | 0.017 |
| 0.05 mg/ml | 0.004 | 0.011 | 0.002 | 0.005 | 0.001 | 0.009 | 0.005 | 0.015 | 0.017 |
| 0.01 mg/ml | 0.005 | 0.03 | 0.66 | 0.01 | 0.015 | 0.022 | 0.05 | 0.022 | 0.041 |
| * The OD 500 was measured using UV | | | | | | | | | |

Table 31: Effect of Tween on the Stability of Various DVD-Ig Proteins As Assessed By Air/Liquid Interface Denaturation Study/Shaking Study*

| | DVD Form: DVD-A, 15 mM Histidine, pH 5.2, 80 mg/ml Sucrose | | | | | | | | DVD Form: DVD-C, 15 mM Histidine, pH 5.4, 80 mg/ml Sucrose | | | | | | | |
|---|---|---|---|---|---|---|---|---|---|---|---|---|---|---|---|---|
| Tween 80 mg/ml | T0 | | | | T96h | | | | T0 | | | | T96h | | | |
| | Agg | Mon | Frag | AUC | Agg | Mon | Frag | AUC | Agg | Mon | Frag | AUC | Agg | Mon | Frag | AUC |
| 0 | 1.7 | 97.25 | 1.03 | 73550 | 1.35 | 97.55 | 1.08 | 39881 | 1.96 | 96.79 | 1.23 | 64166 | 1.35 | 97.55 | 1.08 | 39881 |
| 2 | 1.67 | 97.58 | 0.74 | 759057 | 2.33 | 96.67 | 0.99 | 74778 | 2.16 | 96.4 | 1.43 | 72649 | 2.33 | 96.67 | 0.99 | 74778 |
| 0.5 | 1.76 | 97.43 | 0.8 | 75635 | 2.35 | 96.42 | 1.21 | 74782 | 2 | 96.66 | 1.33 | 71044 | 2.35 | 96.42 | 1.21 | 74782 |
| 0.1 | 1.65 | 97.66 | 0.68 | 73158 | 3.85 | 95.06 | 1.07 | 72816 | 2.09 | 96.43 | 1.47 | 70277 | 3.85 | 95.06 | 1.07 | 72816 |
| 0.05 | 1.66 | 97.61 | 0.71 | 77474 | 18.86 | 80.36 | 0.76 | 76431 | 2.01 | 96.56 | 1.41 | 700701 | 18.86 | 80.36 | 0.76 | 76431 |
| 0.01 | 1.68 | 97.5 | 0.81 | 77410 | 9.32 | 83.25 | 7.41 | 11267 | 2.11 | 96.54 | 1.33 | 70397 | 9.32 | 83.25 | 7.41 | 11267 |

| | DVD Form: IL12IL18, 15 mM Histidine, pH 5.4, 80 mg/ml Sucrose | | | | | | | | DVD Form: DVD-A, 15 mM Histidine, pH 5.2 | | | | | | | |
|---|---|---|---|---|---|---|---|---|---|---|---|---|---|---|---|---|
| Tween 80 mg/ml | T0 | | | | T96h | | | | T0 | | | | T96h | | | |
| | Agg | Mon | Frag | AUC | Agg | Mon | Frag | AUC | Agg | Mon | Frag | AUC | Agg | Mon | Frag | AUC |
| | 7.15 | 91.18 | 1.66 | 76420 | 6.11 | 92.04 | 1.83 | 72538 | 1.59 | 97.58 | 0.81 | 76481 | 1.09 | 98.02 | 0.87 | 60575 |
| 0 | 7.29 | 90.64 | 2.05 | 77867 | 6.09 | 91.94 | 1.96 | 76447 | 1.55 | 97.65 | 0.79 | 85152 | 1.63 | 97.49 | 0.87 | 84633 |
| 2 | 7.04 | 91.4 | 1.54 | 77164 | 6.14 | 91.98 | 1.87 | 75844 | 1.65 | 97.64 | 0.7 | 88543 | na | na | na | na |
| 0.5 | 7.08 | 91.23 | 1.68 | 72875 | 6.4 | 90.9 | 2.68 | 71645 | 1.69 | 97.57 | 0.73 | 85244 | 1.61 | 97.66 | 0.72 | 87583 |
| 0.1 | 7.03 | 91.3 | 1.65 | 69962 | 8.8 | 86.44 | 4.75 | 72941 | 1.72 | 97.57 | 0.7 | 86031 | 2.22 | 96.67 | 1.09 | 86215 |
| 0.05 | 6.81 | 91.57 | 1.61 | 77682 | 12.77 | 85.19 | 2.03 | 70341 | 1.63 | 97.63 | 0.72 | 81730 | 28.99 | 69.37 | 1.63 | 15513 |

| | DVD Form: DVD-C, 15 mM Histidine, pH 5.4 | | | | | | | | DVD Form: IL12IL18, 15 mM Histidine, pH 5.4 | | | | | | | |
|---|---|---|---|---|---|---|---|---|---|---|---|---|---|---|---|---|
| Tween 80 mg/ml | T0 | | | | T96h | | | | T0 | | | | T96h | | | |
| | Agg | Mon | Frag | AUC | Agg | Mon | Frag | AUC | Agg | Mon | Frag | AUC | Agg | Mon | Frag | AUC |
| | 2.05 | 96.56 | 1.37 | 71752 | 1.71 | 97.3 | 0.98 | 67869 | 6.9 | 91.47 | 1.61 | 75651 | 6.34 | 91.6 | 2.05 | 73693 |
| 0 | 2.08 | 96.63 | 1.28 | 75988 | 2.03 | 96.58 | 1.37 | 75354 | 6.77 | 91.76 | 1.46 | 75316 | 6.43 | 91.61 | 1.95 | 75143 |
| 2 | 2.1 | 96.44 | 1.45 | 77324 | 2 | 96.49 | 1.5 | 76488 | 6.88 | 91.57 | 1.53 | 75439 | 6.12 | 91.84 | 2.03 | 74397 |

| Tween 80 mg/ml | DVD Form: DVD-C, 15 mM Histidine, pH 5.4 | | | | | | | | DVD Form: IL12IL18, 15 mM Histidine, pH 5.4 | | | | | | | |
|---|---|---|---|---|---|---|---|---|---|---|---|---|---|---|---|---|
| | T0 | | | | T96h | | | | T0 | | | | T96h | | | |
| | Agg | Mon | Frag | AUC | Agg | Mon | Frag | AUC | Agg | Mon | Frag | AUC | Agg | Mon | Frag | AUC |
| 0.5 | 2.11 | 96.33 | 1.55 | 77859 | 1.86 | 96.59 | 1.54 | 76809 | 7.02 | 91.18 | 1.78 | 74981 | 7.06 | 90.81 | 2.11 | 74698 |
| 0.1 | 2.21 | 96.28 | 1.5 | 79134 | 2.38 | 96.09 | 1.52 | 77153 | 7.04 | 91.37 | 1.57 | 76385 | 5.61 | 92.44 | 1.93 | 75375 |
| 0.05 | 2.08 | 96.44 | 1.47 | 76726 | 2.95 | 95.52 | 1.51 | 74056 | 6.66 | 91.69 | 1.64 | 65218 | 10.76 | 87.29 | 1.93 | 62074 |

* The SEC data corresponds to 0 and 96 h shaking samples of Table 30.

EP 3 466 973 A1

**Example 14: Effect of Surfactant Concentration on the Stability of IL12IL18 DVD-Ig Protein as Measured in Shaking Studies**

The following example describes the effect of different concentrations (a range of concentration) of polysorbate 80 and poloxamer on the shaking stability of an IL12IL18 DVD-Ig protein at concentrations of 1 mg/mL at pH 6 (15 mM histidine + 80 mg/mL sucrose) as measured using optical density at two different wavelengths of 350 and 500 nm. Samples were taken after 0, 24, 48, 120, and 240 hours.

Table 32: Effect of Surfactants on Formulation with Buffer and Polyol

| | | IL12IL18 1mg/ml; 15 mM Histidine + 80 mg/mL sucrose pH 6 | | | | | | | | | |
|---|---|---|---|---|---|---|---|---|---|---|---|
| Surfactant | | OD500 nm | | | | | OD350 nm | | | | |
| | Shake Time (H) | 0 | 24 | 48 | 120 | 240 | 0 | 24 | 48 | 120 | 240 |
| | | | | | | | | | | | |
| Tween 80 (mg/ml) | 0 | 0.003 | 0.06 | 0.065 | 0.137 | 0.387 | 0.01 | 0.097 | 0.135 | 0.22 | 0.574 |
| | 0.01 | 0.0036 | 0.029 | 0.011 | 0.065 | 0.108 | 0.01 | 0.01 | 0.03 | 0.167 | 0.227 |
| | 0.05 | 00056 | 0.003 | 0.01 | 0.087 | 0.113 | 0.013 | 0.01 | 0.027 | 0.17 | 0.22 |
| | 0.1 | 0.0036 | 0.002 | 0.0022 | 0.067 | 0.004 | 0.01 | 0.01 | 0.009 | 0.135 | 0.011 |
| | 0.5 | 0.003 | 0.002 | 0.002 | 0.04 | 0.001 | 0.01 | 0.009 | 0.009 | 0.086 | 0.009 |
| | 2 | 0.035 | 0.002 | 0.0025 | 0.0025 | 0.002 | 0.062 | 0.012 | 0.013 | 0.014 | 0.013 |
| | | | | | | | | | | | |
| | | IL12IL18 1mg/ml; 15 mM Histidine + 80 mg/mL sucrose pH 6 | | | | | | | | | |
| | | 500 nM | | | | | 350 nM | | | | |
| | Shake Time (H) | 0 | 24 | 48 | 120 | 240 | 0 | 24 | 48 | 120 | 240 |
| | | | | | | | | | | | |
| Poloxamer (% w/v) | 0 | 0.003 | 0.035 | 0.044 | 0.236 | 0.226 | 0.01 | 0.06 | 0.08 | 0.4 | 0.374 |
| | 0.01 | 0.004 | 0.004 | 0.007 | 0.013 | 0.0126 | 0.01 | 0.012 | 0.019 | 0.031 | 0.034 |
| | 0.05 | 0.003 | 0.014 | 0.002 | 0.027 | 0.002 | 0.01 | 0.02 | 0.011 | 0.009 | 0.011 |
| | 0.1 | 0.003 | 0.035 | 0.0037 | 0.0038 | 0.003 | 0.01 | 0.01 | 0.012 | 0.012 | 0.012 |
| | 0.5 | 0.003 | 0.003 | 0.003 | 0.003 | 0.004 | 0.01 | 0.01 | 0.011 | 0.011 | 0.013 |
| | 2 | 0.0036 | 0.003 | 0.003 | 0.005 | 0.004 | 0.011 | 0.01 | 0.01 | 0.013 | 0.013 |

As described in Table 32, the addition of surfactants increased the shaking stability of the IL12/IL18 DVD-Ig protein. Polysorbate concentration in the range of 0.05-2 mg/mL was determined to be the most effective for stability of the IL12/IL18 DVD-Ig protein, and poloxamer was determined to be most effective in the range of 0.1-2 % w/v. DVD-B was also tested and showed similar stability when tested in this particular shaking assay.

**Example 15: Effect of Polyol (Sucrose and Sorbitol) Concentration on the Stability of IL12IL18 and DVD-B DVD-Ig Protein**

The following example shows the effect of polyols sucrose and sorbitol in the presence of polysorbate 80 on the stability of the IL12IL18 DVD-Ig protein as measured at 3mg/mL, at pH 6 by intrinsic fluorescence using an automated high throughput instrument Optim-1000 from Avacta (York, UK) as described in the methods section. 9 μl MCA's were used for the study. Thermal scans were obtained using a scan rate of 1°C/minute and scans were taken from 25-75°C. All samples were freshly prepared from stock solutions for the sucrose and sorbitol excipients.

Table 33: Effect of polyols on 3 mg/ml IL12IL18 and DVD-B DVD-Ig protein formulations with 15 mM His buffer at pH 6 and polysorbate

| Sucrose (mg/ml) | Sorbitol (mg/ml) | Tween | DSF (°C) IL12IL18 | DSF (°C) DVD-B |
|---|---|---|---|---|
| 0 | | 0.01% | 60 | 54 |
| 10 | | 0.01% | 61 | 55 |
| 40 | | 0.01% | 61 | 55 |
| 70 | | 0.01% | 60 | 56 |
| 100 | | 0.01% | 60 | 57 |
| | 0 | 0.01% | 61 | 53 |
| | 10 | 0.01% | 62 | 54 |
| | 20 | 0.01% | 61 | 55 |
| | 40 | 0.01% | 62 | 55 |
| | 60 | 0.01% | 62 | 56 |

Based on the data in Table 33, the stability of the DVD-B Ig protein increased with an increase in the concentration of either polyol (sucrose or sorbitol). It was determined that 100 mg/mL for sucrose and 60 mg/mL for sorbitol was about the maximum concentrations that would achieve osmolality and hence were investigated. Sucrose in the range of 60-100 mg/mL was determined to be effective, while sorbitol in the range of 20-60 mg/mL was effective for stability. In contrast, the results shown in Table 33 for the IL12/IL18 DVD-Ig protein did not show an improvement in the stability as measured by DSF.

**Example 16: Impact of pH and Histidine Concentration (at pH 6) on the Shelf Stability of DVD-B and IL12IL18 DVD-Ig Protein at 100mg/ml**

The following examples show the effect of histidine concentration (ranging from 0 - 200 mM) on the shelf stability of the DVD-B and the IL12IL18 DVD-Ig protein as measured using size exclusion chromatography (SEC). Samples were prepared and storage stability of the DVD-Ig proteins in solution at 100 mg/mL was evaluated at 5°C and 40 °C. After defined storage periods, samples were pulled and the impact of storage time on DVD-Ig protein stability was evaluated. Briefly, samples were filled into sterile vials (approx. 500 μL each) and stored under controlled conditions (in temperature chambers and in the absence of light) at 40 °C. At predefined points of time, samples of prepared solutions were pulled for analysis according to the sample pull scheme.

Table 34: Effect of pH on Stability of IL12IL18 DVD-Ig Protein and DVD-B DVD-Ig Protein at 100 mg/mL in Solution

| °C | Sample | Time (D) | Formulation | Agg | Mon | Frag |
|---|---|---|---|---|---|---|
| N/A | IL12-18 | 0 | 0 mM His pH 6+ tween+ sucrose | 3.08 | 95.12 | 1.8 |
| N/A | IL12-18 | 0 | 5 mM His pH 6+ tween+ sucrose | 2.8 | 95.18 | 2.02 |

(continued)

| °C | Sample | Time (D) | Formulation | Agg | Mon | Frag |
|---|---|---|---|---|---|---|
| N/A | IL12-18 | 0 | 10 mM His pH 6+ tween+ sucrose | 2.92 | 95.06 | 2.02 |
| N/A | IL12-18 | 0 | 50 mM His pH 6+ tween+ sucrose | 2.94 | 94.93 | 2.13 |
| N/A | IL12-18 | 0 | 200 mM His pH 6+ tween+ sucrose | 3.11 | 94.63 | 2.25 |
| N/A | IL12-18 | 0 | 15 mM Ace pH 4.50+ tween+ sucrose | 2.88 | 95.06 | 2.06 |
| N/A | IL12-18 | 0 | 15 mM His pH 6+ tween+ sucrose | 2.54 | 95.4 | 2.06 |
| N/A | IL12-18 | 0 | 15 mM Pho pH 7.40+ tween+ sucrose | 3.16 | 95.53 | 1.31 |
| N/A | DVDB | 0 | 0 mM His pH 6+ tween+ sucrose | 3.96 | 94.2 | 1.84 |
| N/A | DVDB | 0 | 5 mM His pH 6+ tween+ sucrose | 4.55 | 93.66 | 1.79 |
| N/A | DVDB | 0 | 10 mM His pH 6+ tween+ sucrose | 4.61 | 93.64 | 1.75 |
| N/A | DVDB | 0 | 50 mM His pH 6+ tween+ sucrose | 5.52 | 92.6 | 1.88 |
| N/A | DVDB | 0 | 200 mM His pH 6+ tween+ sucrose | 4.94 | 93.24 | 1.83 |
| N/A | DVDB | 0 | 15 mM Ace pH 4.50+ tween+ sucrose | 11.19 | 86.86 | 1.95 |
| N/A | DVDB | 0 | 15 mM His pH 6+ tween+ sucrose | 5.92 | 92.9 | 1.18 |
| N/A | DVDB | 0 | 15 mM Pho pH 7.40+ tween+ sucrose | 3.26 | 94.9 | 1.73 |
| 5 | IL12-18 | 7 | 0 mM His pH 6+ tween+ sucrose | 2.82 | 94.98 | 2.19 |
| 5 | IL12-18 | 7 | 5 mM His pH 6+ tween+ sucrose | 2.53 | 95.36 | 2.1 |
| 5 | IL12-18 | 7 | 10 mM His pH 6+ tween+ sucrose | 2.63 | 95.9 | 1.48 |
| 5 | IL12-18 | 7 | 50 mM His pH 6+ tween+ sucrose | 2.7 | 95.57 | 1.73 |
| 5 | IL12-18 | 7 | 200 mM His pH 6+ tween+ sucrose | 3.03 | 95.08 | 1.9 |
| 5 | IL12-18 | 7 | 15 mM Ace pH 4.50+ tween+ sucrose | 2.23 | 95.73 | 2.05 |
| 5 | IL12-18 | 7 | 15 mM His pH 6+ tween+ sucrose | 2.38 | 95.46 | 2.16 |
| 5 | IL12-18 | 7 | 15 mM Pho pH 7.40+ tween+ sucrose | 2.76 | 94.66 | 2.56 |
| 5 | DVDB | 7 | 0 mM His pH 6+ tween+ sucrose | 4.76 | 93.43 | 1.82 |
| 5 | DVDB | 7 | 5 mM His pH 6+ tween+ sucrose | 5.41 | 92.75 | 1.85 |
| 5 | DVDB | 7 | 10 mM His pH 6+ tween+ sucrose | 5.15 | 93.11 | 1.74 |
| 5 | DVDB | 7 | 50 mM His pH 6+ tween+ sucrose | 6.44 | 91.64 | 1.92 |
| 5 | DVDB | 7 | 200 mM His pH 6+ tween+ sucrose | 5.99 | 92.27 | 1.74 |
| 5 | DVDB | 7 | 15 mM Ace pH 4.50+ tween+ sucrose | 14.69 | 83.4 | 1.91 |
| 5 | DVDB | 7 | 15 mM His pH 6+ tween+ sucrose | 7.97 | 90.15 | 1.87 |

(continued)

| °C | Sample | Time (D) | Formulation | Agg | Mon | Frag |
|---|---|---|---|---|---|---|
| 5 | DVDB | 7 | 15 mM Pho pH 7.40+ tween+ sucrose | 4.06 | 94.24 | 1.69 |
| 5 | IL12-18 | 21 | 0 mM His pH 6+ tween+ sucrose | 4.01 | 91.56 | 4.43 |
| 5 | IL12-18 | 21 | 5 mM His pH 6+ tween+ sucrose | 3.08 | 92.37 | 4.55 |
| 5 | IL12-18 | 21 | 10 mM His pH 6+ tween+ sucrose | 2.95 | 92.69 | 4.35 |
| 5 | IL12-18 | 21 | 50 mM His pH 6+ tween+ sucrose | 2.86 | 92.71 | 4.43 |
| 5 | IL12-18 | 21 | 200 mM His pH 6+ tween+ sucrose | 3.28 | 92.22 | 4.5 |
| 5 | IL12-18 | 21 | 15 mM Ace pH 4.50+ tween+ sucrose | 2.18 | 93.18 | 4.65 |
| 5 | IL12-18 | 21 | 15 mM His pH 6+ tween+ sucrose | 2.42 | 92.36 | 5.22 |
| 5 | IL12-18 | 21 | 15 mM Pho pH 7.40+ tween+ sucrose | 3.95 | 91.84 | 4.2 |
| 5 | DVDB | 21 | 0 mM His pH 6+ tween+ sucrose | 5.21 | 91.17 | 3.62 |
| 5 | DVDB | 21 | 5 mM His pH 6+ tween+ sucrose | 6.68 | 89.81 | 3.51 |
| 5 | DVDB | 21 | 10 mM His pH 6+ tween+ sucrose | 6.26 | 89.85 | 3.89 |
| 5 | DVDB | 21 | 50 mM His pH 6+ tween+ sucrose | 8.13 | 87.88 | 3.99 |
| 5 | DVDB | 21 | 200 mM His pH 6+ tween+ sucrose | 8.29 | 87.66 | 4.06 |
| 5 | DVDB | 21 | 15 mM Ace pH 4.50+ tween+ sucrose | 19.23 | 77.03 | 3.73 |
| 5 | DVDB | 21 | 15 mM His pH 6+ tween+ sucrose | 9.54 | 86.75 | 3.71 |
| 5 | DVDB | 21 | 15 mM Pho pH 7.40+ tween+ sucrose | 6.25 | 89.65 | 4.09 |
| 40 | IL12-18 | 7 | 0 mM His pH 6+ tween+ sucrose | 2.56 | 95.01 | 2.44 |
| 40 | IL12-18 | 7 | 5 mM His pH 6+ tween+ sucrose | 2.06 | 95.45 | 2.5 |
| 40 | IL12-18 | 7 | 10 mM His pH 6+ tween+ sucrose | 1.93 | 95.59 | 2.48 |
| 40 | IL12-18 | 7 | 50 mM His pH 6+ tween+ sucrose | 1.82 | 95.3 | 2.92 |
| 40 | IL12-18 | 7 | 200 mM His pH 6+ tween+ sucrose | 2.18 | 95.04 | 2.78 |
| 40 | IL12-18 | 7 | 15 mM Ace pH 4.50+ tween+ sucrose | 1.97 | 95.27 | 2.76 |
| 40 | IL12-18 | 7 | 15 mM His pH 6+ tween+ sucrose | 1.85 | 95.61 | 2.54 |
| 40 | IL12-18 | 7 | 15 mM Pho pH 7.40+ tween+ sucrose | 4.35 | 92.57 | 3.08 |
| 40 | DVDB | 7 | 0 mM His pH 6+ tween+ sucrose | 44.35 | 54.38 | 1.28 |
| 40 | DVDB | 7 | 5 mM His pH 6+ tween+ sucrose | 51.66 | 46.62 | 1.72 |
| 40 | DVDB | 7 | 10 mM His pH 6+ tween+ sucrose | 50.76 | 47.45 | 1.79 |
| 40 | DVDB | 7 | 50 mM His pH 6+ tween+ sucrose | 53.53 | 44.78 | 1.69 |
| 40 | DVDB | 7 | 200 mM His pH 6+ tween+ sucrose | 43.21 | 54.95 | 1.84 |

(continued)

| °C | Sample | Time (D) | Formulation | Agg | Mon | Frag |
|---|---|---|---|---|---|---|
| 40 | DVDB | 7 | 15 mM Ace pH 4.50+ tween+ sucrose | 64.17 | 33.34 | 2.49 |
| 40 | DVDB | 7 | 15 mM His pH 6+ tween+ sucrose | 50.51 | 46.87 | 2.5 |
| 40 | DVDB | 7 | 15 mM Pho pH 7.40+ tween+ sucrose | 36.56 | 60.79 | 2.65 |
| 40 | IL12-18 | 21 | 0 mM His pH 6+ tween+ sucrose | 4.09 | 90.71 | 5.19 |
| 40 | IL12-18 | 21 | 5 mM His pH 6+ tween+ sucrose | 3.16 | 91.26 | 5.58 |
| 40 | IL12-18 | 21 | 10 mM His pH 6+ tween+ sucrose | 3.15 | 91.78 | 5.07 |
| 40 | IL12-18 | 21 | 50 mM His pH 6+ tween+ sucrose | 2.53 | 91.99 | 5.48 |
| 40 | IL12-18 | 21 | 200 mM His pH 6+ tween+ sucrose | 2.88 | 91.52 | 5.61 |
| 40 | IL12-18 | 21 | 15 mM Ace pH 4.50+ tween+ sucrose | 2.52 | 91.59 | 5.89 |
| 40 | IL12-18 | 21 | 15 mM His pH 6+ tween+ sucrose | 2.29 | 91.97 | 5.74 |
| 40 | IL12-18 | 21 | 15 mM Pho pH 7.40+ tween+ sucrose | 5.35 | 88.56 | 6.09 |
| 40 | DVDB | 21 | 0 mM His pH 6+ tween+ sucrose | 55.03 | 39.46 | 5.51 |
| 40 | DVDB | 21 | 5 mM His pH 6+ tween+ sucrose | 57.67 | 37.17 | 5.16 |
| 40 | DVDB | 21 | 10 mM His pH 6+ tween+ sucrose | 55.56 | 39.33 | 5.11 |
| 40 | DVDB | 21 | 50 mM His pH 6+ tween+ sucrose | 58.62 | 36.27 | 5.11 |
| 40 | DVDB | 21 | 200 mM His pH 6+ tween+ sucrose | 56.6 | 37.74 | 5.66 |
| 40 | DVDB | 21 | 15 mM Ace pH 4.50+ tween+ sucrose | 67.47 | 26.81 | 5.71 |
| 40 | DVDB | 21 | 15 mM His pH 6+ tween+ sucrose | 63.14 | 31.52 | 5.34 |
| 40 | DVDB | 21 | 15 mM Pho pH 7.40+ tween+ sucrose | 49.52 | 44.7 | 5.78 |

Table 34 also shows the effect of pH range from 4.5-7.4 (pH 4.5 is 15 mM Acetate, pH 6 is 15 mM Histidine and pH 7.4 is 15 mM Phosphate) on the stability of the two DVD-Ig proteins. The data indicate that the stability of IL1IL18 DVD-Ig protein, an AS-DVD-Ig protein, is maintained between the pH of about 4.5 to about 7.4, while the non-AS DVD-Ig protein DVD-B is unstable over pH 4.5 as indicated by the much higher level of aggregate formation over time. The amount of the buffering agent histidine seems to have almost no effect on stability, indicating that the stability issues for non-AS DVD-Ig proteins can be mitigated by formulation parameters, as might have been the case for monoclonal antibodies.

The data indicates that the stability of IL12IL18 DVD-Ig protein is maintained between the pH range of 4.5-7.4, while DVD-B is unstable at either pH 4.5 or pH 7.4 in solution. The stability of the two DVD-Ig proteins show similar stability profiles, however, between the 0-200 mM histidine concentration range at pH 6 given the above conditions in solution.

**Example 17: Effect of Citrate Concentration at pH 6 on the Shelf Stability of DVD-B and IL12IL18 DVD-Ig Proteins at 100 mg/ml**

The following example describes the impact of citrate buffer concentration (ranging from 0 - 100 mM) on the shelf stability of the DVD-B and IL12/IL18 DVD-Ig protein. Samples were prepared and storage stability of the DVD-Ig proteins in solution at 100 mg/mL was evaluated at 5°C and 40 °C. After defined storage periods, samples were pulled and the impact of storage time on DVD-Ig protein stability was evaluated. Briefly, samples were filled into sterile vials (approx. 500 μL each) and stored under controlled conditions (in temperature chambers and in the absence of light) at 40 °C. At predefined points of time, samples of prepared solutions were pulled for analysis according to the sample pull scheme.

Table 35: Effect of pH on Stability of DVD-B and IL12IL18 DVD-Ig Proteins

| °C | Sample | Time (D) | Formulation | Agg | Mon | Frag |
|----|--------|----------|-------------|-----|-----|------|
|    | IL12/18 | 0 | 0 mM Cit pH 6 | 3.2 | 94.98 | 1.82 |
|    | IL12/18 | 0 | 5 mM Cit pH 6 | 3.3 | 94.68 | 2.2 |
|    | IL12/18 | 0 | 10 mM Cit pH 6 | 3.06 | 94.76 | 2.18 |
|    | IL12/18 | 0 | 50 mM Cit pH 6 | 2.52 | 95.28 | 2.19 |
|    | IL12/18 | 0 | 100 mM Cit pH 6 | 2.82 | 95.05 | 2.13 |
|    | IL12/18 | 0 | 0 mM Cit pH 6+ Sucrose+tween | 2.73 | 95.11 | 2.16 |
|    | BL12/18 | 0 | 5 mM Cit pH 6+ Sucrose+tween | 2.47 | 95.55 | 1.97 |
|    | IL12/18 | 0 | 10 mM Cit pH 6+ Sucrose+tween | 2.67 | 95.1 | 2.23 |
|    | IL12/18 | 0 | 50 mM Cit pH 6+ Sucrose+tween | 2.57 | 95.37 | 2.05 |
|    | IL12/18 | 0 | 1 00 mM Cit pH 6+ Sucrose+tween | 2.55 | 95.24 | 2.21 |
| 5 | IL12/18 | 7 | 0 mM Cit pH 6 | 2.91 | 94.51 | 2.58 |
| 5 | IL12/18 | 7 | 5 mM Cit pH 6 | 3.16 | 94.76 | 2.08 |
| 5 | IL12/18 | 7 | 10 mM Cit pH 6 | 2.77 | 94.77 | 2.46 |
| 5 | IL12/18 | 7 | 50 mM Cit pH 6 | 3.16 | 94.67 | 2.17 |
| 5 | IL12/18 | 7 | 100 mM Cit pH 6 | 2.84 | 95.81 | 1.36 |
| 5 | IL12/18 | 7 | 0 mM Cit pH 6+ Sucrose+tween | 2.58 | 95.21 | 2.21 |
| 5 | IL12/18 | 7 | 5 mM Cit pH 6+ Sucrose+tween | 2.98 | 94.84 | 2.18 |
| 5 | IL12/18 | 7 | 10 mM Cit pH 6+ Sucrose+tween | 2.78 | 94.82 | 2.4 |
| 5 | IL12/18 | 7 | 50 mM Cit pH 6+ Sucrose+tween | 2.87 | 94.79 | 2.35 |
| 5 | IL12/18 | 7 | 100 mM Cit pH 6+ Sucrose+tween | 2.77 | 94.97 | 2.25 |
| 5 | IL12/18 | 21 | 0 mM Cit pH 6 | 3.22 | 94.21 | 2.57 |
| 5 | IL12/18 | 21 | 5 mM Cit pH 6 | 3.64 | 95.31 | 1.05 |
| 5 | IL12/18 | 21 | 10 mM Cit pH 6 | 3.4 | 93.9 | 2.7 |
| 5 | IL12/18 | 21 | 50 mM Cit pH 6 | 3.66 | 94.53 | 1.81 |
| 5 | IL12/18 | 21 | 100 mM Cit pH 6 | 3.3 | 94.47 | 2.23 |
| 5 | IL12/18 | 21 | 0 mM Cit pH 6+ Sucrose+tween | 2.93 | 94.44 | 2.68 |
| 5 | IL12/18 | 21 | 5 mM Cit pH 6+ Sucrose+tween | 3.19 | 94.71 | 2.1 |
| 5 | IL12/18 | 21 | 10 mM Cit pH 6+ Sucrose+tween | 3.2 | 94.54 | 2.26 |
| 5 | IL12/18 | 21 | 50 mM Cit pH 6+ Sucrose+tween | 3.12 | 94.51 | 2.37 |
| 5 | IL12/18 | 21 | 100 mM Cit pH 6+ Sucrose+tween | 2.88 | 94.94 | 2.19 |
| 40 | IL12/18 | 7 | 0 mM Cit pH 6 | 3.06 | 94.81 | 2.12 |
| 40 | IL12/18 | 7 | 5 mM Cit pH 6 | 3.4 | 94.33 | 2.27 |
| 40 | DL12/18 | 7 | 10 mM Cit pH 6 | 3.28 | 94.47 | 2.24 |
| 40 | IL12/18 | 7 | 50 mM Cit pH 6 | 3.81 | 94.13 | 2.06 |
| 40 | IL12/18 | 7 | 100 mM Cit pH 6 | 3.55 | 94.07 | 2.38 |

(continued)

| °C | Sample | Time (D) | Formulation | Agg | Mon | Frag |
|----|--------|----------|-------------|-----|-----|------|
| 40 | IL12/18 | 7 | 0 mM Cit pH 6+ Sucrose+tween | 2.7 | 96.87 | 0.44 |
| 40 | IL12/18 | 7 | 5 mM Cit pH 6+ Sucrose+tween | 2.84 | 94.61 | 2.55 |
| 40 | IL12/18 | 7 | 10 mM Cit pH 6+ Sucrose+tween | 2.98 | 94.61 | 2.4 |
| 40 | IL12/18 | 7 | 50 mM Cit pH 6+ Sucrose+tween | 2.88 | 94.84 | 2.27 |
| 40 | IL12/18 | 7 | 100 mM Cit pH 6+ Sucrose+tween | 2.96 | 94.64 | 2.4 |
| 40 | IL12/18 | 21 | 0 mM Cit pH 6 | 4.48 | 91.27 | 4.26 |
| 40 | IL12/18 | 21 | 5 mM Cit pH 6 | 4.92 | 90.99 | 4.1 |
| 40 | IL12/18 | 21 | 10 mM Cit pH 6 | 4.62 | 91.2 | 4.18 |
| 40 | IL12/18 | 21 | 50 mM Cit pH 6 | 4.69 | 91.53 | 3.78 |
| 40 | IL12/18 | 21 | 100 mM Cit pH 6 | 4.24 | 92.23 | 3.52 |
| 40 | IL12/18 | 21 | 0 mM Cit pH 6+ Sucrose+tween | 3.92 | 91.87 | 4.21 |
| 40 | IL12/18 | 21 | 5 mM Cit pH 6+ Sucrose+tween | 3.92 | 92.05 | 4.03 |
| 40 | IL12/18 | 21 | 10 mM Cit pH 6+ Sucrose+tween | 4.01 | 91.63 | 4.36 |
| 40 | IL12/18 | 21 | 50 mM Cit pH 6+ Sucrose+tween | 3.66 | 92.52 | 3.81 |
| 40 | IL12/18 | 21 | 100 mM Cit pH 6+ Sucrose+tween | 3.78 | 91.89 | 4.33 |
| | | | | | | |
| 5 | | 3 Month | 0 mM Cit pH 6 | 2.53 | 94.36 | 3.11 |
| 5 | | 3 Month | 5 mM Cit pH 6 | 2.89 | 94.33 | 2.77 |
| 5 | IL12/18 | 3 Month | 10 mM Cit pH 6 | 3 | 94.35 | 2.64 |
| 5 | IL12/18 | 3 Month | 50 mM Cit pH 6 | 3.78 | 93.83 | 2.39 |
| 5 | IL12/18 | 3 Month | 100 mM Cit pH 6 | 3.58 | 94.45 | 1.98 |
| 5 | IL12/18 | 3 Month | 0 mM Cit pH 6+ Sucrose+tween | 2.94 | 94.71 | 2.35 |
| 5 | IL12/18 | 3 Month | 5 mM Cit pH 6+ Sucrose+tween | 3.58 | 94.29 | 2.13 |
| 5 | IL12/18 | 3 Month | 10 mM Cit pH 6+ Sucrose+tween | 2.95 | 95.16 | 1.89 |
| 5 | IL12/18 | 3 Month | 50 mM Cit pH 6+ Sucrose+tween | 3.44 | 94.58 | 1.98 |
| 5 | IL12/18 | 3 Month | 100 mM Cit pH 6+ Sucrose+tween | 3.24 | 94.54 | 2.21 |
| | | | | | | |
| 5 | IL12/18 | 6 Month | 0 mM Cit pH 6 | 6.4 | 90.99 | 2.61 |
| 5 | IL12/18 | 6 Month | 5 mM Cit pH 6 | 6.18 | 91.58 | 2.24 |
| 5 | IL12/18 | 6 Month | 10 mM Cit pH 6 | 6.04 | 92.12 | 1.84 |
| 5 | IL12/18 | 6 Month | 50 mM Cit pH 6 | 5.72 | 92.9 | 1.38 |
| 5 | IL12/18 | 6 Month | 100 mM Cit pH 6 | 5.98 | 92.86 | 1.16 |
| 5 | IL12/18 | 6 Month | 0 mM Cit pH 6+ Sucrose+tween | 4.98 | 94.02 | 1 |
| 5 | IL12/18 | 6 Month | 5 mM Cit pH 6+ Sucrose+tween | 5.47 | 92.96 | 1.5 |
| 5 | IL12/18 | 6 Month | 10 mM Cit pH 6+ Sucrose+tween | 4.51 | 94.56 | 0.9 |
| 5 | IL12/18 | 6 Month | 50 mM Cit pH 6+ Sucrose+tween | 4.58 | 93.44 | 1.9 |
| 5 | IL12/18 | 6 Month | 100 mM Cit pH 6+ Sucrose+tween | 4.63 | 93.54 | 1.82 |
| 5 | IL12/18 | 6 Month | 0 mM Cit pH 6 | 6.4 | 90.99 | 2.61 |

(continued)

| °C | Sample | Time (D) | Formulation | Agg | Mon | Frag |
|----|--------|----------|-------------|-----|-----|------|
|    |        |          |             |     |     |      |
|    | DVD-B  | 0        | 0 mM Cit pH 6 | 2.6 | 95.63 | 1.77 |
|    |        | 0        | 5 mM Cit pH 6 | 2.6 | 95.49 | 1.87 |
|    | DVD-B  | 0        | 10 mM Cit pH 6 | 2.41 | 95.88 | 1.72 |
|    | DVD-B  | 0        | 50 mM Cit pH 6 | 1.76 | 96.62 | 1.62 |
|    | DVD-B  | 0        | 100 mM Cit pH 6 | 1.34 | 97 | 1.66 |
|    | DVD-B  | 0        | 0 mM Cit pH 6+ Sucrose+tween | 1.54 | 97.31 | 1.15 |
|    | DVD-B  | 0        | 5 mM Cit pH 6+ Sucrose+tween | 1.52 | 96.74 | 1.73 |
|    | DVD-B  | 0        | 10 mM Cit pH 6+ Sucrose+tween | 1.5 | 96.8 | 1.71 |
|    | DVD-B  | 0        | 50 mM Cit pH 6+ Sucrose+tween | 1.5 | 96.74 | 1.75 |
|    | DVD-B  | 0        | 100 mM Cit pH 6+ Sucrose+tween | 1.11 | 97.16 | 1.73 |
| 5  | DVD-B  | 7        | 0 mM Cit pH 6 | 2.61 | 95.54 | 1.85 |
| 5  | DVD-B  | 7        | 5 mM Cit pH 6 | 3.01 | 95.37 | 1.62 |
| 5  | DVD-B  | 7        | 10 mM Cit pH 6 | 3.2 | 95.12 | 1.68 |
| 5  | DVD-B  | 7        | 50 mM Cit pH 6 | 2.85 | 95.5 | 1.64 |
| 5  | DVD-B  | 7        | 100 mM Cit pH 6 | 2.14 | 96.22 | 1.64 |
| 5  | DVD-B  | 7        | 0 mM Cit pH 6+ Sucrose+tween | 2.32 | 95.95 | 1.73 |
| 5  | DVD-B  | 7        | 5 mM Cit pH 6+ Sucrose+tween | 2.86 | 95.47 | 1.66 |
| 5  | DVD-B  | 7        | 10 mM Cit pH 6+ Sucrose+tween | 2.71 | 95.61 | 1.68 |
| 5  | DVD-B  | 7        | 50 mM Cit pH 6+ Sucrose+tween | 2.75 | 95.62 | 1.64 |
| 5  | DVD-B  | 7        | 100 mM Cit pH 6+ Sucrose+tween | 1.97 | 96.35 | 1.68 |
| 5  | DVD-B  | 21       | 0 mM Cit pH 6 | 3.33 | 94.44 | 2.23 |
| 5  | DVD-B  | 21       | 5 mM Cit pH 6 | 4.97 | 92.72 | 2.31 |
| 5  | DVD-B  | 21       | 10 mM Cit pH 6 | 5.52 | 92.56 | 1.92 |
| 5  | DVD-B  | 21       | 50 mM Cit pH 6 | 4.88 | 93.82 | 1.3 |
| 5  | DVD-B  | 21       | 100 mM Cit pH 6 | 3.7 | 94.76 | 1.54 |
| 5  | DVD-B  | 21       | 0 mM Cit pH 6+ Sucrose+tween | 3 | 95.28 | 1.72 |
| 5  | DVD-B  | 21       | 5 mM Cit pH 6+ Sucrose+tween | 4.88 | 93.06 | 2.06 |
| 5  | DVD-B  | 21       | 10 mM Cit pH 6+ Sucrose+tween | 5 | 93.41 | 1.59 |
| 5  | DVD-B  | 21       | 50 mM Cit pH 6+ Sucrose+tween | 5.01 | 93.14 | 1.84 |
| 5  | DVD-B  | 21       | 100 mM Cit pH 6+ Sucrose+tween | 3.77 | 94.38 | 1.85 |
| 40 | DVD-B  | 7        | 0 mM Cit pH 6 | 51.75 | 46.63 | 1.62 |
| 40 | DVD-B  | 7        | 5 mM Cit pH 6 | 58.47 | 39.22 | 2.31 |
| 40 | DVD-B  | 7        | 10 mM Cit pH 6 | 58.39 | 38.8 | 2.8 |
| 40 | DVD-B  | 7        | 50 mM Cit pH 6 | 47.99 | 49.69 | 2.32 |
| 40 | DVD-B  | 7        | 100 mM Cit pH 6 | 39.78 | 58.14 | 2.09 |
| 40 | DVD-B  | 7        | 0 mM Cit pH 6+ Sucrose+tween | 47.61 | 49.83 | 2.56 |
| 40 | DVD-B  | 7        | 5 mM Cit pH 6+ Sucrose+tween | 56.88 | 40.48 | 2.64 |

(continued)

| °C | Sample | Time (D) | Formulation | Agg | Mon | Frag |
|----|--------|----------|-------------|-----|-----|------|
| 40 | DVD-B | 7 | 10 mM Cit pH 6+ Sucrose+tween | 46.46 | 50.97 | 2.57 |
| 40 | DVD-B | 7 | 50 mM Cit pH 6+ Sucrose+tween | 45.57 | 52.03 | 2.4 |
| 40 | DVD-B | 7 | 100 mM Cit pH 6+ Sucrose+tween | 34.2 | 63.53 | 2.27 |
| 40 | DVD-B | 21 | 0 mM Cit pH 6 | 60.28 | 36.18 | 3.54 |
| 40 | DVD-B | 21 | 5 mM Cit pH 6 | n/a | n/a | n/a |
| 40 | DVD-B | 21 | 10 mM Cit pH 6 | n/a | n/a | n/a |
| 40 | DVD-B | 21 | 50 mM Cit pH 6 | n/a | n/a | n/a |
| 40 | DVD-B | 21 | 100 mM Cit pH 6 | n/a | n/a | n/a |
| 40 | DVD-B | 21 | 0 mM Cit pH 6+ Sucrose+tween | 56.73 | 39.59 | 3.67 |
| 40 | DVD-B | 21 | 5 mM Cit pH 6+ Sucrose+tween | 59.87 | 36.82 | 3.3 |
| 40 | DVD-B | 21 | 10 mM Cit pH 6+ Sucrose+tween | 53.36 | 43.59 | 3.05 |
| 40 | DVD-B | 21 | 50 mM Cit pH 6+ Sucrose+tween | 51.27 | 45.72 | 3.01 |
| 40 | DVD-B | 21 | 100 mM Cit pH 6+ Sucrose+tween | 44.11 | 52.84 | 3.05 |
| 5 | DVD-B | 3 Month | 0 mM Cit pH 6 | 7.25 | 90.86 | 1.9 |
| 5 | DVD-B | 3 Month | 5 mM Cit pH 6 | 13.89 | 84.29 | 1.82 |
| 5 | DVD-B | 3 Month | 10 mM Cit pH 6 | 18.99 | 79.33 | 1.68 |
| 5 | DVD-B | 3 Month | 50 mM Cit pH 6 | 16.07 | 82.2 | 1.73 |
| 5 | DVD-B | 3 Month | 100 mM Cit pH 6 | 12.87 | 85.6 | 1.52 |
| 5 | DVD-B | 3 Month | 0 mM Cit pH 6+ Sucrose+tween | 7.43 | 90.74 | 1.83 |
| 5 | DVD-B | 3 Month | 5 mM Cit pH 6+ Sucrose+tween | 15.08 | 83.23 | 1.69 |
| 5 | DVD-B | 3 Month | 10 mM Cit pH 6+ Sucrose+tween | 16.03 | 82.02 | 1.95 |
| 5 | DVD-B | 3 Month | 50 mM Cit pH 6+ Sucrose+tween | 17.09 | 81.17 | 1.74 |
| 5 | DVD-B | 3 Month | 100 mM Cit pH 6+ Sucrose+tween | 10.89 | 87.64 | 1.46 |
| 5 | DVD-B | 6 Month | 0 mM Cit pH 6 | 15.5 | 82.31 | 2.2 |
| 5 | DVD-B | 6 Month | 5 mM Cit pH 6 | 27.45 | 71.13 | 1.43 |
| 5 | DVD-B | 6 Month | 10 mM Cit pH 6 | 34.37 | 63.99 | 1.63 |
| 5 | DVD-B | 6 Month | 50 mM Cit pH 6 | 29.11 | 69.6 | 1.29 |
| 5 | DVD-B | 6 Month | 100 mM Cit pH 6 | | | |
| 5 | DVD-B | 6 Month | 0 mM Cit pH 6+ Sucrose+tween | 15.24 | 83.22 | 1.54 |
| 5 | DVD-B | 6 Month | 5 mM Cit pH 6+ Sucrose+tween | 27.28 | 71.19 | 1.53 |
| 5 | DVD-B | 6 Month | 10 mM Cit pH 6+ Sucrose+tween | 27.69 | 70.8 | 1.51 |
| 5 | DVD-B | 6 Month | 50 mM Cit pH 6+ Sucrose+tween | 27.18 | 70.78 | 2.04 |
| 5 | DVD-B | 6 Month | 100 mM Cit pH 6+ Sucrose+tween | 21.8 | 76.46 | 1.74 |

The two DVD-Ig proteins in Table 35 showed different stability profiles between the 0-100 mM citrate concentrations at pH 6. While AS-DVD-Ig protein IL12IL18 showed a high stability with only a minor increase in aggregates overtime, especially at 5 °C, the opposite was true for the non AS-DVD-Ig protein DVD-B. This suggests that a high concentration liquid formulation would be feasible for the IL12IL18 DVD-Ig protein but not for DVD-B under those conditions.

## VIII. STABLE LYOPHILIZED DVD-IG (LS-DVD-IG) PROTEIN FORMULATIONS

Examples 18 and 19 describe the stability of LS-DVD-Ig proteins in the lyophilized form. Example 18 describes surprising results that demonstrate freeze/ thaw (F/T) stability of LS-DVD-Ig proteins. Example 19 describes studies showing stable lyophilized formulations containing LS-DVD-Ig proteins. Freezing is the first step in lyophilization and hence molecules that do not have freeze thaw stability are susceptible to instability during lyophilization.

## EXAMPLE 18: Impact of Solution pH on the Stability of DVD-Ig Proteins Subjected to Repeated Freeze/Thaw Cycles

The freeze thaw behavior of DVD-Ig proteins at a protein concentration of 1 mg/ml in 5 mM citrate/5mM phosphate buffer was evaluated by cycling the protein solution up to 2 times between the frozen state and the liquid state at pH 4, pH 6, and pH 8. Freezing was performed using temperature controlled -80°C freezer, and thawing was performed using a 30°C temperature controlled water bath. Samples were pulled after the second freeze/thaw (F/T) cycle and analyzed by SEC. Table 36 shows the effect of freeze/thaw processing on the amount of monomer (Mon) of remaining and the amount of fragments (Frag) and aggregates (Agg) formed in the samples formulated at these pH levels.

Table 36: Numbers Of DVD-Ig Protein Monomers. Aggregates. And Fragments as Determined by SEC Before and After Repeated Freeze/Thaw Cycles of DVD-Ig Protein Formulations with Solution pH Values of 4. 6. or 8

| DVD-Ig | pH | Mon/T0 | Agg/T0 | Frag/T0 | Mon/T2 | Agg/T2 | Frag/T2 |
|---|---|---|---|---|---|---|---|
| 5 | 4 | 95.06 | 0.44 | 4.49 | 93.57 | 1.52 | 4.89 |
| 6 | 4 | 95.26 | 1.21 | 3.52 | 94.94 | 1.72 | 3.33 |
| 37 | 4 | 97.32 | 1.89 | 0.78 | 96.64 | 2.48 | 0.86 |
| 38 | 4 | 94.46 | 3.81 | 1.72 | 94.91 | 3.55 | 1.52 |
| 53 | 4 | 97.47 | 1 | 1.52 | 97.41 | 1.06 | 1.52 |
|  | 4 | 96.06 | 1.87 | 2.06 | 95.87 | 1.85 | 2.26 |
| 65 | 4 | 94.44 | 1.2 | 4.35 | 93.82 | 1.15 | 5.01 |
| 66 | 4 | 98.01 | 0.91 | 1.07 | 97.78 | 1.03 | 1.18 |
| 165 | 4 | 96.62 | 2.17 | 1.2 | 95.45 | 2.34 | 2.19 |
| 166 | 4 | 97.74 | 0.91 | 1.34 | 97.53 | 0.91 | 1.54 |
| 257 | 4 | 97.63 | 1.8 | 0.56 | 96.73 | 2.73 | 0.53 |
| 258 | 4 | 98.84 | 0.19 | 0.95 | 96.73 | 2.28 | 0.97 |
| 277 | 4 | 95.53 | 1.43 | 3.03 | 95.45 | 1.6 | 2.93 |
| 278 | 4 | 95.75 | 1.56 | 2.68 | 95.4 | 1.99 | 2.59 |
| 281 | 4 | 98.72 | 0.63 | 0.63 | 97.47 | 1.83 | 0.68 |
| 282 | 4 | 98.63 | 0.61 | 0.74 | 97.88 | 1.38 | 0.72 |
| 5 | 6 | 94.8 | 3.13 | 2.06 | 94.96 | 3.37 | 1.67 |
| 6 | 6 | 95.79 | 1.99 | 2.2 | 93.71 | 4.02 | 2.26 |
| 37 | 6 | 96.73 | 2.52 | 0.74 | 94.88 | 4.42 | 0.68 |
| 38 | 6 | 95.01 | 3.74 | 1.23 | 95.5 | 3.29 | 1.2 |
| 53 | 6 | 97.56 | 1 | 1.43 | 97.51 | 1.08 | 1.4 |
| 54 | 6 | 95.86 | 2.07 | 2.06 | 95.68 | 2.18 | 2.13 |
| 65 | 6 | 94.17 | 1.09 | 4.72 | 94.13 | 1.16 | 4.7 |
| 66 | 6 | 97.99 | 0.85 | 1.15 | 98.03 | 0.83 | 1.13 |
| 165 | 6 | 96.86 | 2.12 | 1.01 | 96.03 | 2.38 | 1.57 |

(continued)

| DVD-Ig | pH | Mon/T0 | Agg/T0 | Frag/T0 | Mon/T2 | Agg/T2 | Frag/T2 |
|---|---|---|---|---|---|---|---|
| 166 | 6 | 97.75 | 0.91 | 1.33 | 97.82 | 0.9 | 1.26 |
| 257 | 6 | 97.51 | 1.97 | 0.51 | 97.31 | 2.19 | 0.49 |
| 258 | 6 | 99.07 | 0.18 | 0.73 | 98.52 | 0.76 | 0.7 |
| 277 | 6 | 96.74 | 1.79 | 1.46 | 96.64 | 1.92 | 1.43 |
| 278 | 6 | 97.65 | 1.22 | 1.12 | 97.62 | 1.32 | 1.04 |
| 281 | 6 | 95.67 | 0.92 | 3.4 | 95.7 | 0.98 | 3.3 |
| 282 | 6 | 98.55 | 0.83 | 0.61 | 98.49 | 0.89 | 0.6 |
| 5 | 8 | 93.23 | 3.86 | 2.89 | 93.7 | 3.56 | 2.73 |
| 6 | 8 | 95.35 | 1.93 | 2.7 | 94.3 | 2.94 | 2.74 |
| 37 | 8 | 94.88 | 4.27 | 0.84 | 95.64 | 3.54 | 0.8 |
| 38 | 8 | 95.52 | 3.14 | 1.32 | 96.09 | 2.61 | 1.28 |
| 53 | 8 | 97.56 | 1.04 | 1.39 | 97.66 | 1.02 | 1.31 |
| 54 | 8 | 95.77 | 1.91 | 2.3 | 95.91 | 1.9 | 2.17 |
| 65 | 8 | 94.1 | 1.14 | 4.74 | 94.29 | 1.14 | 4.56 |
| 66 | 8 | 97.84 | 0.95 | 1.2 | 97.74 | 0.95 | 1.3 |
| 165 | 8 | 96.63 | 2.23 | 1.12 | 96.07 | 2.32 | 1.59 |
| 166 | 8 | 97.55 | 0.9 | 1.54 | 97.77 | 0.85 | 1.37 |
| 257 | 8 | 97.12 | 2.19 | 0.67 | 96.62 | 2.7 | 0.67 |
| 258 | 8 | 98.81 | 0.28 | 0.9 | 98.71 | 0.39 | 0.89 |
| 277 | 8 | 95.48 | 2.23 | 2.27 | 95.52 | 2.24 | 2.22 |
| 278 | 8 | 95.94 | 1.83 | 2.21 | 96.08 | 1.67 | 2.23 |
| 281 | 8 | 95.61 | 1 | 3.38 | 95.94 | 0.98 | 3.06 |
| 282 | 8 | 98.28 | 1.04 | 0.67 | 98.26 | 1.05 | 0.67 |

DVD 5, DVD 6, DVD 37, DVD 38, DVD 53, DVD 54, DVD 65, DVD 66, DVD 165, DVD 166, DVD 257, DVD 258, DVD 277, DVD 278, DVD 281, and DVD 282 demonstrated stability after being subjected to repeated freeze thaw cycles. These data indicate that DVD-Ig proteins that are formulated in a pH range of about 4 to about 8 remain stable after repeated F/T processing. The high stability of the DVD-Ig protein formulations tested (all showed greater than 93% monomer content and 11/16 formulations showed greater than 95% monomer content) was unexpected, because DVD-Ig proteins are much more complex than IgGs. Complex molecules such as DVD-Ig proteins would be expected to aggregate and fragment easily when exposed to freezing and thawing.

*Impact of Solution pH On the Stability of DVD-B Subjected to Repeated Freeze/Thaw Cycles*

The freeze thaw behavior of DVD-B at a protein concentration of 2 mg/ml in 10 mM citrate/10mM phosphate buffer was evaluated by cycling the protein solution up to 4 times between the frozen state and the liquid state at pH 4-9. Freezing was performed by means of a temperature controlled -80°C freezer, and thawing was performed by means of a 30°C temperature controlled water bath. Samples were pulled after each freeze/thaw (F/T) cycle and analyzed by light obscuration and SEC. Table 37 shows the effect of freeze/thaw processing on the number of sub-visible particles formed as determined using light obscuration measurements at various pH values.

Table 37: Numbers of Subvisible Particles Per mL as Determined by Light Obscuration Assays After 0-4 Freeze/
Thaw (F/T) Cycles of DVD-B Formulations with a pH Value of 4, 5. 6, 7, 8, Or 9

| (A) Numbers of particles $\geq$ 1 micron in size. | | | | | | |
|---|---|---|---|---|---|---|
| Number of F/T cycles | pH = 4 | pH = 5 | pH = 6 | pH = 7 | pH = 8 | pH = 9 |
| 0 | 39.16 | 55 | 44 | 41 | 55 | 46 |
| 1 | 334.5 | 1291 | 38127 | 31896 | 28444 | 25970 |
| 2 | 6728.6 | 7935 | 80064 | 61592 | 58562 | 46863 |
| 3 | 13658 | 18733 | 128448 | 95775 | 89934 | 67225 |
| 4 | 5702 | 37930 | 175024 | 132768 | 120339 | 89389 |
| (B) Numbers of particles $\geq$ 10 microns in size. | | | | | | |
| Number of F/T cycles | pH = 4 | pH = 5 | pH = 6 | pH = 7 | pH = 8 | pH = 9 |
| 0 | 1.33 | 1.5 | 2.6 | 2.6 | 5.5 | 1.83 |
| 1 | 15.16 | 3.16 | 28 | 45 | 27.16 | 16.83 |
| 2 | 207.5 | 62 | 142 | 165 | 148 | 134 |
| 3 | 342 | 136 | 440 | 740 | 773 | 375 |
| 4 | 572 | 269 | 2418 | 4099 | 4537 | 1425 |
| (C) Numbers of particles $\geq$ 25 microns in size. | | | | | | |
| Number of F/T cycles | pH = 4 | pH = 5 | pH = 6 | pH = 7 | pH = 8 | pH = 9 |
| 0 | 0.33 | 0.33 | 0.16 | 0.5 | 1.16 | 0.16 |
| 1 | 3.3 | 0.5 | 3.83 | 2 | 4.33 | 1.33 |
| 2 | 40.16 | 10.83 | 2.33 | 6.33 | 1.16 | 3.16 |
| 3 | 71.16 | 6.67 | 13.5 | 39.83 | 39.33 | 5.33 |
| 4 | 118.83 | 67.63 | 198.67 | 476.16 | 641.5 | 142.83 |

The results of the light obscuration assays show that the numbers of particles formed by DVD-B formulations with a pH of 4 to 9 was low. The numbers of particles formed increased with increasing pH and were at a maximum at around the pI of the molecule (pI 8.5). However, with only one exception, the protein solutions tested satisfied the requirements of the International Conference on Harmonization of Technical Requirements for Registration of Pharmaceuticals for Human Use (ICH) guidelines, which requires less than 600 particles of size 25 microns or higher per ml.
Table 38 shows SEC measurements of the stability of DVD-B after freeze/thaw processing. These measurements include the percentage of monomers, aggregates, and fragments, as well as the area under the curve (AUC).

Table 38: Stability Of DVD-B as Determined by SEC After 0-4 Freeze/Thaw Cycles of DVD-B Formulations with
Solution pH Values of 4, 5, 6, 7, 8, Or 9

| (A) SEC measurements of solutions not subjected to F/T cycles | | | | |
|---|---|---|---|---|
|  | Mon | Agg | Frag | AUC |
| pH 4 Vial 1 | 96.38 | 1.44 | 2.16 | 76861 |
| pH 4 Vial 2 | 95.61 | 1.56 | 2.82 | 76123 |
| pH 4 Mean | 95.995 | 1.5 | 2.49 | 76492 |
| pH 5 Vial 1 | 96.1 | 1.57 | 2.32 | 73704 |
| pH 5 Vial 2 | 96.16 | 1.55 | 2.28 | 74196 |

(continued)

| (A) SEC measurements of solutions not subjected to F/T cycles | | | | |
|---|---|---|---|---|
| | Mon | Agg | Frag | AUC |
| pH 5 Mean | 96.13 | 1.56 | 2.3 | 73950 |
| pH 6 Vial 2 | 95.09 | 1.69 | 3.2 | 77475 |
| pH 7 Vial 2 | 95.61 | 1.81 | 2.56 | 75863 |
| pH 8 Vial 2 | 95.84 | 1.87 | 2.27 | 74943 |
| pH 9 Vial 1 | 95.66 | 2.11 | 2.21 | 82103 |
| pH 9 Vial 2 | 95.43 | 2.14 | 2.41 | 81843 |
| pH 9 Mean | 95.545 | 2.125 | 2.31 | 81973 |
| (B) SEC measurements after one F/T cycle | | | | |
| pH 4 Vial 1 | 96.37 | 1.68 | 1.94 | 74694 |
| pH 4 Vial 2 | 95.39 | 1.74 | 2.86 | 76213 |
| pH 4 Mean | 95.88 | 1.71 | 2.4 | 75453.5 |
| pH 5 Vial 1 | 96.18 | 1.82 | 1.98 | 71511 |
| pH 5 Vial 2 | 96.08 | 1.6 | 2.31 | 76345 |
| pH 5 Mean | 96.13 | 1.71 | 2.145 | 73928 |
| pH 6 Vial 1 | 96.37 | 1.6 | 2.02 | 75448 |
| pH 6 Vial 2 | 95.24 | 1.6 | 3.14 | 77009 |
| pH 6 Mean | 95.805 | 1.6 | 2.58 | 76228.5 |
| pH 7 Vial 1 | 95.88 | 1.86 | 2.24 | 75821 |
| pH 7 Vial 2 | 95.28 | 1.96 | 2.74 | 76470 |
| pH 7 Mean | 95.58 | 1.91 | 2.49 | 76145.5 |
| pH 8 Vial 1 | 95.64 | 2.01 | 2.34 | 74757 |
| pH 8 Vial 2 | 95.59 | 2.08 | 2.31 | 74270 |
| pH 8 Mean | 95.81536 | 1. 777143 | 2.3925 | 75306.68 |
| pH 9 Vial 1 | 95.62 | 2.15 | 2.22 | 81981 |
| pH 9 Vial 2 | 95.57 | 2.13 | 2.29 | 81516 |
| pH 9 Mean | 95.595 | 2.14 | 2.255 | 81748.5 |
| (C) SEC measurements after two F/T | | | | |
| pH 4 Vial 1 | 95.75 | 1.98 | 2.25 | 75402 |
| pH 4 Vial 2 | 95.29 | 1.98 | 2.71 | 74567 |
| pH 4 Mean | 95.52 | 1.98 | 2.48 | 74984.5 |
| pH 5 Vial 1 | 96.07 | 1.85 | 2.06 | 71454 |
| pH 5 Vial 2 | 95.92 | 1.88 | 2.19 | 71525 |
| pH 5 Mean | 95.995 | 1.865 | 2.125 | 71489.5 |
| pH 6 Vial 1 | 96.33 | 1.58 | 2.08 | 74666 |
| pH 6 Vial 2 | 95.43 | 1.62 | 2.93 | 75080 |
| pH 6 Mean | 95.88 | 1.6 | 2.505 | 74873 |
| pH 7 Vial 1 | 95.77 | 1.87 | 2.34 | 74813 |

(continued)

| (C) SEC measurements after two F/T | | | | |
|---|---|---|---|---|
| pH 7 Vial 2 | 95.72 | 1.84 | 2.42 | 74584 |
| pH 7 Mean | 95.745 | 1.855 | 2.38 | 74698.5 |
| pH 8 Vial 1 | 95.72 | 2.02 | 2.24 | 73460 |
| pH 8 Vial 2 | 95.78 | 2 | 2.21 | 68769 |
| pH 8 Mean | 95.75 | 2.01 | 2.225 | 71114.5 |
| pH 9 Vial 1 | 95.58 | 2.1 | 2.3 | 73356 |
| pH 9 Vial 2 | 95.57 | 2.12 | 2.3 | 77337 |
| pH 9 Mean | 95.575 | 2.11 | 2.3 | 75346.5 |
| (D) SEC measurements after three F/T cycles | | | | |
| pH 4 Vial 1 | 95.62 | 2.14 | 2.22 | 72539 |
| pH 4 Vial 2 | 95.01 | 2.23 | 2.75 | 72480 |
| pH 4 Mean | 95.315 | 2.185 | 2.485 | 72509.5 |
| pH 5 Vial 1 | 95.9 | 1.99 | 2.09 | 68689 |
| pH 5 Vial 2 | 95.8 | 1.98 | 2.21 | 65381 |
| pH 5 Mean | 95.85 | 1.985 | 2.15 | 67035 |
| pH 6 Vial 2 | 95.55 | 1.63 | 2.81 | 71195 |
| pH 7 Vial 1 | 96.03 | 1.84 | 2.12 | 70617 |
| pH 7 Vial 2 | 95.77 | 1.86 | 2.35 | 71405 |
| pH 7 Mean | 95.9 | 1.85 | 2.235 | 71011 |
| pH 8 Vial 1 | 95.86 | 2.04 | 2.08 | 69364 |
| pH 8 Vial 2 | 95.59 | 2.12 | 2.28 | 69308 |
| pH 8 Mean | 95.725 | 2.08 | 2.18 | 69336 |
| pH 9 Vial 1 | 95.03 | 2.42 | 2.54 | 77679 |
| pH 9 Vial 2 | 95.59 | 2.22 | 2.18 | 76113 |
| pH 9 Mean | 95.31 | 2.32 | 2.36 | 76896 |
| (E) SEC measurements after four F/T cycles | | | | |
| pH 4 Vial 1 | 94.38 | 2.53 | 3.08 | 53902 |
| pH 4 Vial 2 | 94.08 | 2.4 | 3.5 | 72943 |
| pH 4 Mean | 94.23 | 2.465 | 3.29 | 63422.5 |
| pH 5 Vial 1 | 96.01 | 1.88 | 2.1 | 66962 |
| pH 5 Vial 2 | 95.85 | 1.96 | 2.18 | 67786 |
| pH 5 Mean | 95.93 | 1.92 | 2.14 | 67374 |
| pH 6 Vial 1 | 96.26 | 1.62 | 2.11 | 70371 |
| pH 6 Vial 2 | 95.41 | 1.67 | 2.9 | 71086 |
| pH 6 Mean | 95.835 | 1.645 | 2.505 | 70728.5 |
| pH 7 Vial 1 | 95.87 | 1.82 | 2.29 | 67317 |
| pH 7 Vial 2 | 96.03 | 1.81 | 2.15 | 67869 |
| pH 7 Mean | 95.95 | 1.815 | 2.22 | 67593 |

(continued)

| (E) SEC measurements after four F/T cycles | | | | |
|---|---|---|---|---|
| pH 8 Vial 1 | 95.62 | 2.07 | 2.3 | 69629 |
| pH 8 Vial 2 | 95.31 | 2.15 | 2.52 | 69401 |
| pH 8 Mean | 95.465 | 2.11 | 2.41 | 69515 |
| pH 9 Vial 1 | 95.44 | 2.27 | 2.28 | 77495 |
| pH 9 Vial 2 | 95.58 | 2.18 | 2.22 | 73373 |
| pH 9 Mean | 95.51 | 2.225 | 2.25 | 75434 |

The results in Table 38 indicate that the DVD-Ig protein does not form significant amounts of aggregates even after 4 F/T cycles. The good F/T stability is surprising as it was anticipated that due to their higher structural complexity DVD-Ig proteins would be more prone to undergo degradation at the liquid / ice interface or even cold denaturation upon multiple freeze /thaw cycles. This is a common observation for proteins and many other DVD-Ig proteins show a significant amount of aggregation upon freeze/thawing. Since freezing is the initial, and a critical, step of the lyophilizing process, the data suggests that DVD-B can be lyophilized and hence converted into a stable biotherapeutic dosage form.

The results in Table 37 indicate that the DVD-Ig protein does not form significant aggregates even after 4 F/T cycles. The good F/T stability was surprising as it was anticipated that the stability might not be as good as was observed given the intrinsic lack of stability and increased structural complexity relative to an antibody.

**EXAMPLE 19: Impact of Lyophilization on the Stability of DVD-Ig Proteins**

Aggregates may form during the process of lyophilization as well as later on during shelf stability of the solid protein. The aggregates formed during lyophilization are generally measured following immediate reconstitution.

Storage stability of DVD-Ig proteins was evaluated for prolonged periods of time at controlled temperature conditions. After defined storage periods, samples were pulled and the impact of storage time and storage temperature on the stability of lyophilized DVD-Ig proteins was evaluated by size exclusion chromatography (SEC) and ion exchange chromatography (IEC). Three DVD-Ig proteins were studied: DVD-B (TNF-PGE2), DVD-A (TNF-IL17), and DVD-C (IL1$\alpha$-IL1$\beta$). Of the three, DVD-A and DVD-C are AS-DVD-Ig proteins. DVD-B is a non-AS-DVD-Ig protein, but was identified as an LS-DVD-Ig protein as it (as well as LS-DVD-Ig proteins DVD-A and DVD-C) was found to be stable in a lyophilized formulation. The formulations were lyophilized in solutions as shown in Table 39 in a formulation containing a buffer, a polyol, and a surfactant.

Table 39: Composition of Lyophilized Formulations

| Component | DVD-A 55 mg/ml | DVD-B 55 mg/ml | DVD-C 48 mg/ml |
|---|---|---|---|
| Histidine | 2.33 (15mM) | 2.33 (15mM) | 2.33 (15mM) |
| Sucrose | 46 (4.6%) | 46 (4.6%) | 46 (4.6%) |
| Polysorbate 80 | 0.2 (0.02%) | 0.2 (0.02%) | 0.1 (0.01%) |
| 0.1 M HCl | q.s. (pH 5.25) | q.s. (pH 5.25) | q.s. (pH 6.0) |

Table 40 describes the percentages of monomers, aggregates, and fragments that were measured using SEC before storage (T0) or following storage of the lyophilized formulations at either 5°C or 40°C for the given storage periods.

Table 40: Stability of Stored Lyophilized Formulations as Assessed Using SEC

| DVD-Ig | Storage Condition | Monomer | Aggregate | Fragment |
|---|---|---|---|---|
| DVD-A | T0 | 94.22 | 5.09 | 0.67 |
| | 1 Week 40°C | 94.12 | 5.23 | 0.64 |
| | 4 Week 40°C | 94.41 | 5.14 | 0.43 |
| | 3m 40C | 93.31 | 6.24 | 0.43 |

(continued)

| DVD-Ig | Storage Condition | Monomer | Aggregate | Fragment |
|---|---|---|---|---|
| | 3m 40C + 3h RT | 93.17 | 6.38 | 0.43 |
| DVD-B | T0 | 96.59 | 2.11 | 1.28 |
| | 1 Week 40°C | 96.11 | 2.59 | 1.28 |
| | 4 Week 40°C | 95.44 | 3.19 | 1.36 |
| | 4 Week 5°C | 96.47 | 2.2 | 1.31 |
| | 4 Week 40°C + 3h RT | 95.35 | 3.29 | 1.34 |
| DVD-C | T0 | 96.55 | 2.94 | 0.5 |
| | 1 Week 40°C | 97.165 | 1.59 | 1.23 |
| | 1m 40°C | 96.88 | 1.93 | 1.18 |
| | 3m 5°C | 97.42 | 1.36 | 1.21 |

The SEC data in Table 40 shows that lyophilized LS-DVD-Ig proteins remain stable for periods of up to 3 months of storage because they show high percentages of monomers and low percentages of aggregates and fragments. After accelerated storage for 3 months at 40°C and 3 hours of reconstitution time, lyophilized DVD-A had more than 93% monomers, less than 6.4% aggregates, and only about 0.4% fragments. After 4 weeks of accelerated storage at 40°C, lyophilized DVD-B had more than 95% monomers and only about 3.2% aggregates and about 1.4% fragments. After 1 month of accelerated storage at 40°C, lyophilized DVD-C had approximately 97% monomers, 2% aggregates, and 1% fragments. Thus, lyophilized formulations of DVD-A, DVD-B, and DVD-C showed long term stability, as assessed by SEC.

Table 41 below provides data regarding the stability of stored formulations measured using IEC. The impact of chemical stability was not significant as observed from formation of acidic and basic species with time. Therefore, all tested molecules do not show significant chemical degradation in the lyophilized state. Storage stability of lyophilized DVD-Ig proteins was evaluated at 40°C and 5°C. After defined storage periods, the lyophilized samples were pulled and the impact of storage time on DVD-Ig protein stability was evaluated. Samples were reconstituted with water for injection and upon complete reconstitution analyzed using SEC and IEC.

Table 41: Stability of Stored Lyophilized Formulations as Assessed Using IEC

| DVD-Ig | Storage condition | Main | Acidic | Basic | X |
|---|---|---|---|---|---|
| DVD-A | T0 | 73.28 | 3.6 | 20.78 | 2.32 |
| | 1 Week 40°C | 72.59 | 3.38 | 21.91 | 2.1 |
| | 4 Week 40°C | 70.73 | 3.6 | 23.44 | 2.21 |
| | 3m 40°C | 71.75 | 3.84 | 22 | 2.39 |
| | 3m 40°C + 3h RT | 71.8 | 3.71 | 22.03 | 2.44 |
| DVD-B | T0 | 48.84 | 38.54 | 12.6 | |
| | 1 Week 40°C | 48.18 | 38.72 | 13.09 | |
| | 4 Week 40°C | 45.87 | 37.6 | 16.53 | |
| | 4 Week 5°C | 48.17 | 38.16 | 13.56 | |
| | 4 Week 40°C + 3h RT | 45.7 | 37.79 | 16.5 | |
| DVD-C | 1 Week 40°C | 54.82 | 28.82 | 16.35 | |
| | 1m 40°C | 53.61 | 28.84 | 17.53 | |
| | 3m 5°C | 55.43 | 28.57 | 15.99 | |

The impact of chemical stability seen above in Table 40 was not significant as observed from formation of acidic and basic species with time.

Table 42 below provides the reconstitution times of the stored lyophilized formulations. One basic criterion for characterizing the feasibility of a lyophilized dosage form is the time it takes for a lyophilized sample to be converted in a homogenous, clear liquid after reconstitution with a solvent, usually water for injection. Ideally, the reconstitution procedure takes less than 10 minutes. As shown in Table 41 all tested samples showed complete reconstitution after a maximum of 5 minutes. This indicates that AS-DVD-Ig proteins (*e.g.*, DVD-Ig proteins A & C) can be formulated as stable lyophilized formulations. Moreover, the above examples show that LS-DVD-Ig proteins - which are not stable in liquid formulations - can be stabilized using lyophilization (*e.g.*, DVD-B).

Table 42: Reconstitution Time (RT) Of the Lyophilized Formulations (With Water for Injection)

| DVD-Ig | Storage condition | RT |
|---|---|---|
| DVD-A | T0 | 30s |
| | T1 week 40°C | 26s |
| | T4 week 40°C | 48s |
| | T3 month 40°C | 300s |
| DVD-B | T0 | 48s |
| | T1 week 40°C | 60s |
| | T4 week 5°C | 69s |
| | T4 week 40°C | 89s |
| DVD-C | T0 | 47s |
| | T1 week 40°C | 76s |
| | 3m 5°C | 62s |
| | 1m 40°C | 53s |

## IX. CHARACTERIZATION OF DVD-IG PROTEINS

Examples 20-23 provide further characterization of DVD-Ig proteins generally.

**Example 20: Solubility Assessment of DVD-Ig Proteins Using PEG 3000**

Polyethylene glycols (PEG) are often used as "crowders," which force the protein in a smaller amount of water, because some of the available solvent is bound to or occupied by the crowder, typically a polymer. PEGs are used to assess the true solubility of a protein by utilizing micro amounts of the protein material available at early stages of development. In general, the greater the amount of PEG is required to induce precipitation, the greater is the anticipated true solubility of the protein in solution.

The following studies were carried out using small aliquots of PEG solution (50% w/v) added to a stock solution of protein (0.5 mg/ml) in a buffer (5 mM citrate and 5 mM phosphate) at pH 6. Table 43 shows the data for various DVD-Ig proteins.

Table 43. Amount of PEG 3000 Required To Induce Precipitation in a 0.5 mg/ml Protein Solution of DVD-Ig Proteins

| DVD-Ig | % PEG 3000 required to induce precipitation |
|---|---|
| 5 | 10 |
| 6 | 9.37 |
| 37 | 10 |
| 38 | 9.33 |
| 53 | 8.12 |
| 54 | 9.37 |
| 65 | 7.5 |

(continued)

| DVD-Ig | % PEG 3000 required to induce precipitation |
|---|---|
| 66 | 7.5 |
| 165 | 10.625 |
| 166 | 11.875 |
| 257 | 9.37 |
| 258 | 10.62 |
| 277 | 12.5 |
| 278 | 13.75 |
| 281 | 8.12 |
| 282 | 7.5 |

These data show that the amount of PEG 3000 required to induce precipitation of DVD-Ig proteins is typical of highly soluble proteins (*i.e.*, those proteins with a true solubility that exceeds about 100 mg/ml). Although the PEG precipitation assay is a standard assay in antibody formulation assessment to provide information about its solubility, it would not be sufficient to predict whether a DVD-Ig protein would be classified as AS or LS or even non LS, indicating the complexity of DVD-Ig proteins and the challenging formulation efforts compared to monoclonal antibodies.

**Example 21: Assessment of the Tertiary Structure of DVD-Ig Proteins Using Near UV CD Scans**

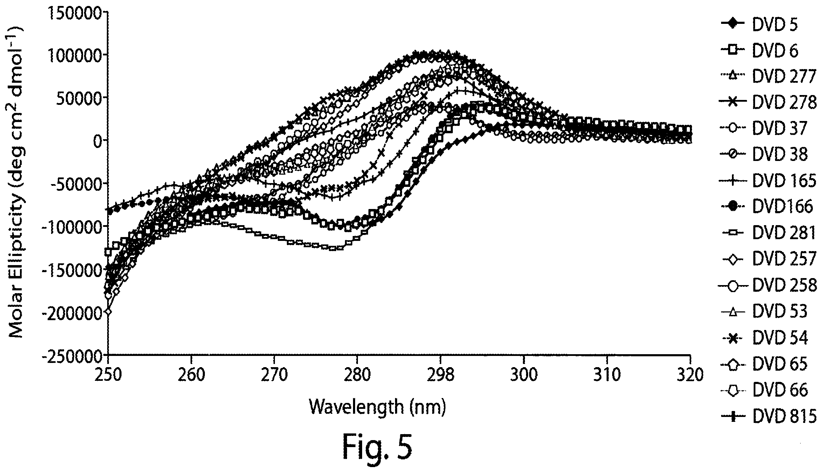

The structure of a protein is one of the important factors influencing protein stability during accelerated/long-term storage of protein liquid and lyophilizate formulations. To assess the tertiary structure of the DVD-Ig proteins, near UV CD scan between 250-320 nm was taken on a Jasco Spectrometer with a scan rate of 50 nm/minute at a concentration of 1mg/ml. An average of 3 scans/ DVD-Ig protein was taken. The pH used in the study was 6 in 5 mM citrate and 5 mM phosphate conditions. UV CD scans of monoclonal antibodies were also obtained for comparison. The data presented in Figure 5 indicate that the DVD-Ig proteins have folded structures as indicated by the presence of featured ellipticity profiles in the 250-320 nm region.
The DVD-Ig ellipticity values presented in Figure 5 are similar to those observed for known stable monoclonal antibodies. Therefore, both the DVD-Ig proteins and the mAbs are expected to show similar storage stability. However, LS-DVD-Ig proteins are actually less stable than the mAbs. Therefore, UV-CD data alone cannot be used to accurately predict the stability issues observed for LS-DVD-Ig proteins.

**Example 22: Assessment of the Secondary Structure of DVD-Ig Proteins Using ATR-FTIR**

To assess the secondary structure of DVD-Ig proteins, the second derivative, area normalized FTIR scans taken on an ATR-FTIR instrument from Bruker (Tensor 27) in the region 1600-1700 cm$^{-1}$ were curve fitted and the various peaks were analyzed and added up to get total % beta sheet structure in the molecule. Especially, peaks such as that at 1638 cm$^{-1}$, which are an indicator of the beta sheet arrangement, were taken into account. This is because beta sheets are known to comprise the majority of the secondary structure of mAbs. Therefore it is expected that DVD-Ig proteins which are derived from mAbs should also contain mostly beta sheets as their secondary structure composition. Any deviation from this may indicate that the overall structural integrity of the molecule is compromised. The studies were done in 5 mM citrate/5 mM phosphate buffer at a pH of 4, 6, or 8. The concentration of the DVD-Ig protein was 1mg/ml. The total percentage of beta structure was assessed for 16 DVD-Ig proteins (see Table 44).

Table 44: The Total % Beta Structure in DVD-Ig Proteins Measured Using ATR-FTIR

| DVD-Ig | pH 6 | pH 4 | pH 8 |
|---|---|---|---|
| 5 | 89.8 | 86.5 | 95.8 |
| 6 | 94.1 | 78.2 | 92.3 |
| 37 | 89.6 | 85.9 | 90.9 |
| 38 | 96.5 | 92 | 95.6 |

(continued)

| DVD-Ig | pH 6 | pH 4 | pH 8 |
|--------|------|------|------|
| 53 | NA | NA | 96.4 |
| 54 | 97.4 | 87.7 | 96.8 |
| 65 | 95.2 | 95.8 | 94.8 |
| 66 | 94.6 | 94.2 | 94.2 |
| 165 | NA | NA | 95.2 |
| 166 | 95.1 | 90 | 95.2 |
| 257 | 95.8 | 89.6 | 90.8 |
| 258 | 95.6 | 88.3 | 96.6 |
| 277 | 93.6 | 85.3 | 95 |
| 278 | 93.8 | 77.4 | 94.3 |
| 281 | 94.4 | 84.4 | 85 |
| 282 | 93 | 84.7 | 84.6 |

The results presented in Table 45 show that all of the 16 DVD-Ig proteins studied have a folded secondary structure that is composed primarily of beta elements. The proportion of beta elements ranged from about 85% to about 97% which is similar to that observed for standard antibodies. This was unexpected since DVD-Ig proteins are engineered molecules and are assumed to have a lower percentage of beta sheets as compared to mAbs (although they still are expected to contain beta sheets as their predominant secondary structure). These findings suggest that secondary structure composition is not a factor for the overall lower storage stability of DVD-Ig proteins as compared to antibodies. The proportion of beta elements ranged from about 85% to about 97%.

**EXAMPLE 23: Impact of Ionic Strength and pH on the Second Virial Coefficient of DVD-B Formulations**

Second virial coefficient ($B_{22}$) is a thermodynamic parameter and an indicator of the protein-protein attractive or repulsive interactions in solutions. A positive value indicates repulsive interactions and a negative value indicates attractive interactions. Repulsive interactions usually translate into better long term storage. The scattered light intensity is related to the molecular weight and $B_{22}$ by the following equation.

$$\frac{Kc}{R_\theta} = \frac{1}{M_w} + 2B_{22}c + B_{222}c^2 + \ldots$$

Where K is optical constant and is given by

$$K = \frac{\left[2\pi n \left(\frac{dn}{dc}\right)\right]^2}{N_A \, \lambda^4}$$

$R_\theta$ is the excess Rayleigh ratio, a measure of light scattered by the solute, n is the solvent refractive index, dn/dc is the refractive index increment of the solute, $N_A$ is the Avogadro's number, and $\lambda$ is the wavelength of the incident light. Since for most dilute solutions, higher order virial coefficients have negligible values, the following equation (Debye) is used to obtain the second virial coefficients.

$$\frac{Kc}{R_\theta} = \frac{1}{M_w} + 2B_{22}c$$

The scattered intensities were measured on a Malvern Zetasizer Nano. The second virial coefficient values were all positive and indicate that DVD-Ig proteins behave as typical protein molecules with respect to this calculation at least under dilute conditions. The buffers used were acetate for pH 4.5, histidine for pH 6 and Tris for pH 8. 2 mM concentration of buffer was used for 1 mM ionic strength solutions and 10 mM for 20 and 100 mM ionic strength solutions. The rest of the ionic strength was maintained by sodium chloride. The results are shown in Tables 33 and 34. The values of the second virial coefficients were higher on average at pH 4.5 and pH 6.0 than at pH 8.0, suggesting that DVD-B would store better at pH 4.5 or pH 6.0 than at pH 8.0. Also, the values of the second virial coefficients were higher at lower ionic strength, suggesting that lower ionic strength may also be associated with stability of TNF-PGE2 DVD-Ig protein.

$D_s$ is the self-diffusion coefficient of the molecule at infinite dilution. $k_d$ is a parameter describing the interaction between the molecules in solution. A positive value for $k_d$ indicates intermolecular repulsion and vice versa.

Table 45: Virial Coefficient Values for DVD-B protein at 0 mm Ionic Strength

| pH | $B_{22}$ X $10^{-4}$ (mol mL/gm$^2$) | $B_{222}$ X $10^{-2}$ (mol mL$^2$/gm$^3$) | $B_{2222}$ X $10^{-1}$ (mol mL$^3$/gm$^4$) | $B_{22222}$ X $10^2$ (mol mL$^4$/gm$^5$) | $M_w$ (X 1000) |
|---|---|---|---|---|---|
| 6.0 | 136 ± 5.77 | -273.8 ± 16.6 | 3376.1± 339 | -221 ± 35.8 | 189 ± 14 |

Table 46: Second Virial Coefficients Under Various Conditions for DVD-B Protein

| pH | Ionic Strength (mM) | $D_s$ X $10^{-7}$ (cm$^2$/s)* | $k_d$ (mL/gm)† | $B_{22}$ X $10^{-4}$ (mol mL/gm$^2$)‡ | $M_w$ (X 1000)§ |
|---|---|---|---|---|---|
| 4.5 | 1 | 3.42 ± 0.04 | 434.90 ± 15.31 | 26.09 ± 0.82 | 195 ± 1 |
| | 20 | 3.66 ± 0.004 | 5.96 ± 0.32 | 5.08 ± 0.03 | 168 ± 2.89 |
| | 100 | 3.60 ± 0.03 | -13.33 ±0.88 | 3.09 ± 0.07 | 160 ±3.38 |
| 6 | 1 | 3.41 ± 0.06 | 379.28 ± 51.34 | 14.71 ± 1.09 | 183 ± 0.57 |
| | 20 | 3.75 ± 0.02 | -11.04 ± 1.37 | 3.37 ± 0.06 | 163 ± 2.35 |
| | 100 | 3.70 ± .002 | -23.05 ± 0.02 | 2.51 ± 0.19 | 170 ± 3.40 |
| 8 | 1 | 3.81 ± 0.01 | -4.74 ± 0.06 | 3.50 ± 0.05 | 155 ± 0.78 |
| | 20 | 3.73 ± 0.02 | -27.31 ± 0.29 | 2.14 ± 0.03 | 162 ± 5.08 |
| | 100 | 3.66 ± 0.03 | -25.33 ± 0.90 | 2.34 ± 0.07 | 164 ± 4.59 |

As mentioned, if $B_{22}$ and kD are positive in sign and have a large magnitude, it is an indication of strong repulsive interactions among the DVD-Ig proteins in solution. This is an ideal condition for the long term storage stability of a DVD-Ig protein liquid formulation because there is a lowered probability of DVD-Ig proteins encountering each other in solution to form aggregates. The above data suggest that low ionic strength and low pH as part of a formulation will result in greater long term DVD-Ig protein stability than a formulation with high ionic strength and high pH. Also, since ionic strength and pH contribute highly to the $B_{22}$ value, it indicates that electrostatic repulsion in general comprises a significant portion of the $B_{22}$ value.

With respect to comparing DVD-Ig proteins and antibodies, it may be that a more positive $B_{22}$ value is required for a DVD-Ig protein formulation to have a similar long term stability profile as that of an antibody formulation. This is because DVD-Ig proteins are larger than antibodies and the hard-sphere contribution to repulsion (and $B_{22}$) is greater for DVD-Ig proteins. Therefore, if both DVD-Ig proteins and antibodies have the same electrostatic repulsions contributing to $B_{22}$, the $B_{22}$ term for DVD-Ig proteins will be more positive to reflect the same degree of protein-protein repulsion in solution.

## EXAMPLE 24: Pharmacokinetic Study of DVD-Ig Proteins

The following example describes pharmacokinetic studies of various DVD-Ig proteins.

*Variable Domain Combination and Orientation Impacts Serum Stability*

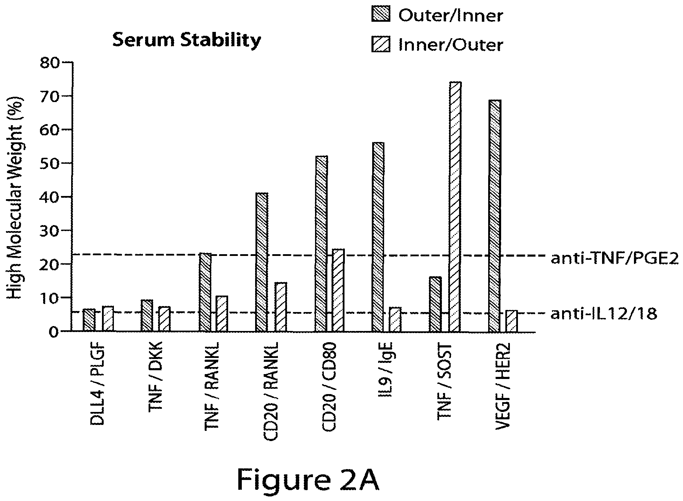

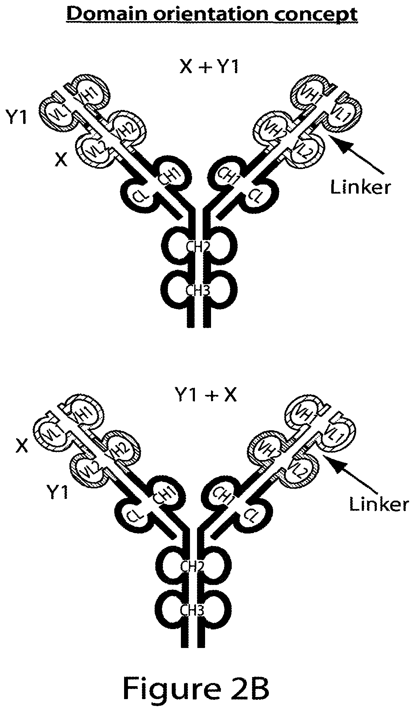

As described in Figure 2, certain variable domain combinations provide a more stable DVD-Ig protein than other combinations. Figure 2A describes the serum stability for certain DVD-Ig proteins in two different domain orientations, *i.e.*, "outer / inner" and "inner / outer". For example, the DVD-Ig protein TNF/SOST has a high molecular weight (HMW) % of about 16% in the "outer / inner" orientation, but has about a 75% HMW in the opposite orientation. The domain orientation concepts are set forth in Figure 2B. The DVD-Ig proteins in Figure 2 include short/short linker combinations and are huIgG1 isotypes.

*In Vitro Pharmacokinetic Study*

The pharmacokinetic (PK) properties of various biologic therapeutics were assessed following 4 mg/kg single intravenous doses in male Sprague-Dawley rats. Blood samples were collected throughout the 28 day studies. Serum samples were analyzed using an MSD assay employing anti-human Ig capture and Sulfo-Tag labeled goat anti-human IgG antibody for chemiluminescent detection. Pharmacokinetic parameters for each animal were calculated using WinNonlin software by non-compartmental analysis.

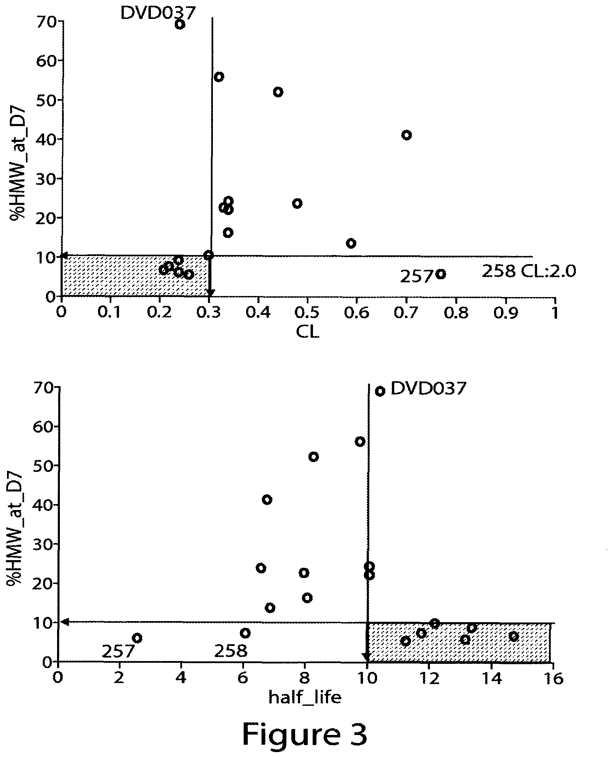

Figure 3 describes results from a pharmacokinetic study using rats. The study examined the correlation between clearance (CL) and T ½ with high molecular weight (HMW) aggregate formation *in vitro* in rat serum. As shown in Figure 3, DVD-Ig proteins with less than 10% HMW aggregate in vitro are more likely to have low (< 0.3 ml / hour / kg) clearance and long (> 10 days) half life. The outliers included DVD 257 and DVD 258. The DLL4/VEGF DVD-Ig protein recognized the rat target, and the PK was affected by TMD. DVD 037 (VEGF/HER2; SEQ ID NOs: 34 and 35) showed bad in vitro properties, but good PK in vivo. The amino acid sequences for the heavy and light chains of DLL4/VEGF DVD-Ig protein are provided in SEQ ID NOs: 68 and 69 of Table 65.

## EXAMPLE 25: Viscosity Study of DVD-Ig Proteins

The following example describes viscosity studies for an exemplary AS-DVD-Ig protein (DVD-A).

Viscosity was measured on m-VROC low volume viscometer from Rheosense (Redwood, CA). m-VROC measure viscosity from the pressure drop of a test liquid as it flows through a rectangular slit. As the test liquid is pumped to flow through the flow channel, pressure is measured at increasing distance from the inlet. Plot of the straight line in the pressure vs. position of the sensor is proportional to the viscosity.

The instrument was evacuated beforehand to minimize the usage of material and subsequently recover the material. Air was hence used to clean the instrument before a sample measurement was made. An initial flow rate of 40 $\mu$l/minute - 200 $\mu$l/minute was used to obtain the required pressure differential. Saturation of the pressure chamber quickly stabilizes the viscosity reading, and twenty microliters of sample achieved stabilization. Once the instrument has been primed with the sample, less than 5 microliters of additional sample was enough to give a stable second reading. A total of less than thirty microliters (< 35 microliters) of sample was thus enough to give readings in triplicate.

Viscosity of all samples was also measured on a rolling ball viscometer from Anton Paar (X, X). 1.8 mm capillary was used for samples of viscosity range 2-70 cP and 1.6 mm capillary was used for samples of minimal viscosity (less than 2 cP). The instrument was pre-calibrated and run at any of the various possible angles (70°, 50° and/or 40°).

Viscosity of DVDA was determined in histidine formulations having different molarity (*i.e.*, 0 mM to 30 mM histidine) and pH (*i.e.*, pH 4.8 to pH 8.3) in various DVD-A concentrations. Results from the measurements are provided below in Tables 47 to 49.

Table 47: Viscosity Measurements of 34 mg/ml DVD-A at Various pH and Histidine Molarity

| DVDA 34mg/ml | | | | | |
|---|---|---|---|---|---|
| | | | | | |
| Anton Paar Viscosity readings (mPa.s.) | | | | | |
| | | | | | |
| | pH 4.8 | pH 5.4 | pH 6 | pH 6.6 | pH 8.3 |
| 0 Mm Histidine | 1.83 | 2.9 | 5.22 | Na | na |
| 5 Mm Histidine | 1.59 | 1.81 | 2.46 | 3.86 | 1.83 |
| 30 Mm Histidine | 1.33 | 1.71 | 2.03 | 3.5 | 1.97 |
| | | | | | |
| | | | | | |
| | | | | | |

(continued)

| Rheosense Viscosity measurments | | | | | |
|---|---|---|---|---|---|
| | pH 4.8 | pH 5.4 | pH 6 | pH 6.6 | pH 8.3 |
| 0 Mm Histidine | | | | | |
| 1 | 3.07 | 4.11 | 6.03 | Na | 2.35 |
| 2 | 3.04 | 4.04 | 5.84 | Na | 2.36 |
| 3 | 2.93 | 4.15 | 6.08 | Na | 2.40 |
| **Average Viscosity** | **3.01** | **4.10** | **5.98** | **Na** | **2.37** |
| | | | | | |
| 5 Mm Histidine | | | | | |
| 1 | 1.58 | 1.73 | 2.56 | 3.87 | 2.22 |
| 2 | 1.52 | 1.71 | 2.55 | 3.87 | 2.12 |
| 3 | 1.59 | 1.72 | 2.53 | 3.86 | 2.13 |
| **Average Viscosity** | **1.56** | **1.72** | **2.55** | **3.86** | **2.16** |
| | | | | | |
| 30 Mm Histidine | | | | | |
| 1 | 1.37 | 1.57 | 1.88 | 3.34 | 2.84 |
| 2 | 1.39 | 1.58 | 1.91 | 3.37 | 2.93 |
| 3 | 1.33 | 1.58 | 1.86 | 3.50 | 2.73 |
| **Average Viscosity** | **1.36** | **1.58** | **1.88** | **3.40** | **2.83** |

Table 48: Viscosity Measurements of 15 mg/ml DVD-A at 0 Mm Histidine at pH 4.8 To 8.3

| DVDA 15mg/ml 0mM | | | | | |
|---|---|---|---|---|---|
| | pH 4.8 | pH 5.4 | pH 6 | pH 6.6 | pH 8.3 |
| 0 Mm Histidine | | | | | |
| 1 | 1.66 | 1.30 | 1.28 | Na | 1.85 |
| 2 | 1.64 | 1.34 | 1.31 | Na | 1.82 |
| 3 | 1.64 | 1.30 | 1.29 | Na | 1.80 |
| **Average Viscosity** | **1.65** | **1.31** | **1.29** | **Na** | **1.82** |

Table 49: Viscosity Measurements of Various Concentrations of DVD-A at Various pH And Histidine Molarity

| DVDA | | | | | |
|---|---|---|---|---|---|
| | | 5mM | | 30mM | |
| | | Anton Paar | Rheosense | Anton Paar | Rheosense |
| | | | | | |
| pH4.8 (95mg/ml) | 1 | 4.63 | 6.924 | 5.29 | 4.179 |
| | 2 | 4.67 | 6.925 | 5.3 | 4.241 |
| | Average | 4.65 | 6.9245 | 5.295 | 4.21 |

(continued)

| DVDA | | | | | | |
|---|---|---|---|---|---|---|
| | | 5mM | | 30mM | | |
| | | Anton Paar | Rheosense | Anton Paar | Rheosense |
| Ph5.4 (77mg/ml) | 1 | 10.45 | 10.09 | 6.3 | 6.027 |
| | 2 | 10.5 | 10.1 | 6.29 | 6.016 |
| | Average | 10.475 | 10.095 | 6.295 | 6.0215 |
| pH6 (47.8mp/ml) | 1 | 6.23 | 5.721 | 3.643 | 3.34 |
| | 2 | 6.3 | 5.695 | 3.648 | 3.36 |
| | Average | 6.265 | 5.708 | 3.6455 | 3.35 |
| | | | | | |
| | 5mM | | 30mM | | |

The results described above in Tables 47-49 show the impact of ionic strength, pH and protein concentration on the viscosity of the DVD-Ig protein solutions. DVD-A (SEQ ID NOs: 62 and 63) is an AS-DVD-Ig protein and the results above show that the viscosity values can be modulated by formulation means to accommodate a syringeable liquid formulation at high concentrations that would be appropriate for pharmaceutical compositions and in-vivo use. Since DVD-Ig proteins have not only a much higher molecular weight of about 200kD compared to standard monoclonal antibodies of about 150 kD, their structure is also more complex. Therefore, it was surprising that the viscosity of mAbs and DVD-Ig is relatively similar and is even at high concentration below about 10 cPs.

**EXAMPLE 26: Thermal Stability of DVD-Ig Proteins**

The following example describes results from three different tests examining thermal stability of DVD-Ig proteins, including an exemplary AS-DVD-Ig protein and an exemplary LS-DVD-Ig protein, versus monoclonal antibodies, such as Adalimumab.

*Dynamic Scanning Fluorescence*

An automated high throughput instrument Optim-1000 from Avacta (York, UK) was used for the study. 9 microliter micro cubic arrays (MCAs) were used for the study. For preparation of stock samples, 3 microliter Sypro orange (Invitrogen, Cambridge, MA) was added to 27 microliter sample solution in order to obtain a final 1X concentration of the dye. The dye is available as 5000X commercial product, although any dye would be suitable. Thermal scans were obtained from 26°C to 95°C at a scan rate of 60°C/hour. Baseline was fitted for linearity and the first point (the temperature) whose inclusion decreased the $R^2$ below 0.95 was taken as the onset temperature. Repeat studies confirmed that the variation in onset temperatures was less than 5 %.

*Intrinsic Fluorescence*

Tryptophan fluorescence was used to evaluate the unfolding temperatures. Hitachi FL-4500 instrument from Hitachi (Tokyo, Japan) was used for the study. The temperatures were maintained using a water bath. The temperature in the cuvette was monitored using a thermocouple and a temperature monitor CSi32 from Omega Inc. (Stamford, CT). A front surface triangular quartz cuvette from VWR (MA) was used as this minimized the inner filter effects and hence resulted in strong emission signals. An excitation wavelength of 295 nm was used. Emission was monitored between 328-338 nm. Although the $\lambda_{max}$ was observed at 332 nm, the intensity was monitored at 335 nm for comparison. The thermal scans were obtained from 30°C to 70°C at a scan rate of 78°C/hour. Baseline was fitted for linearity and the first point (the temperature) whose inclusion decreased the $R^2$ below 0.95 was taken as the onset temperature. Repeat studies confirmed that the variation in onset temperatures was less than 5 %. The increased scan rate did not significantly affect the onset temperatures.

*Differential Scanning Calorimetry (DSC)*

DSC was used to characterize the thermodynamic stability of the proteins under various solution conditions. An automated cap DSC instrument from Microcal (Northampton, MA) was used. The thermal scans were obtained from 25°C to 65°C at a scan rate of 60°C/hour. Since, aggregation and precipitation that follows unfolding in high concentration samples can lead to blocking of the cap DSC cells which than become rather difficult to clean, the scans were obtained only until ≈ 5°C beyond the onset temperature to prevent any such occurrence. A prescan equilibration thermostat of 10 minutes was applied before each scan. A corresponding buffer scan was taken immediately following the sample scan. The difference in onset was less than 2°C between repeat scans. Baseline was fitted for linearity and the first point (the temperature) whose inclusion decreased the $R^2$ below 0.95 was taken as the onset temperature. Repeat studies confirmed that the variation in onset temperatures was less than 5 %.

Results from the study are provided in Table 50.

Table 50: Results from Thermal Stability Studies Comparing DVD-Ig Proteins to mAbs

|  | Intrinsic fluorescence | | Extrinsic fluorescence | | DSC | |
|---|---|---|---|---|---|---|
|  | 1 mg/mL | 75 mg/mL | 1 mg/mL | 75 mg/mL | 1 mg/mL | 75 mg/mL |
| **mAb1** | 66 | 64 | 63 | 57 | 59 | 57 |
| **mAb2** | 74 | 69 | 73 | 68 | 63 | 59 |
| **mAb3** | 65 | 62 | 65 | 63 | 59 | 55 |
| **mAb4** | 72 | 67 | 64 | 58 | 61 | 59 |
| **mAb5** | 71 | 64 | 66 | 61 | 59 | 56 |
| **IL12IL18** | 69 | 67 | 64 | 61 | 62 | 59 |
| **DVD-B TNF/PGE2** | 56 | 53 | 53 | 49 | 54 | 50 |

The results described in Table 50 show the impact of protein concentration on the thermal stability of the protein solution. DVD1 (IL12IL18), an AS DVD and DVD2 (also referred to herein as DVD-B), an LS-DVD-Ig protein, and other mAbs all show that protein concentration only has a slight impact on the thermal stability of the protein. So the feasibility of a liquid high concentration formulation may be independent of the impact of protein concentration on the thermal stability of the protein; however, high concentration liquid formulations present other well-known types of instabilities, *e.g.*, shelf instabilities. Generally, as described in Examples 1-3, DVD-Ig proteins have a lower melting temperature than antibodies. In some instances, *e.g.*, DVD1, similar melting temperatures are observed, but generally this is not the case.

## X. ANTI-DLL4 / ANTI-VEGF DVD-IG FORMULATIONS (AQUEOUS AND LYOPHILIZED)

**EXAMPLE 27: Preformulation Characterization of Anti-DLL4/Anti-VEGF DVD-Ig Protein h1A11.1SL-Av**

The storage stability (5°C) and accelerated stability (40°C) of an anti-DLL4/anti-VEGF DVD (h1A11.1-SL-Av, Table 41) was evaluated in the formulations and protein concentrations listed below. Stability was evaluated by size exclusion chromatography (SEC) and % aggregate, % monomer, % fragment, and total species recovered were quantitated. Overall, the formulations cover a pH range of 5 to 7 and a protein concentration range of 1.0 to 118 mg/ml.

At 5°C and 40°C temperatures and at protein concentrations of 50, 30, and 10 mg/ml, formulations were: 15 mM acetate pH 5; 15 mM phosphate pH 7; 30 mM acetate, 80 mg/ml sucrose, 0.02% Tween 80 at pH 5; 30 mM histidine, 80 mg/ml sucrose, 0.02% Tween 80 at pH 6; PBS (phosphate buffered saline). All formulations contained 0.02% sodium azide to prevent microbial growth during storage. At 5°C and 40°C temperatures and at protein concentrations of 60, 50, 30, and 10 mg/ml, the formulation was 15 mM histidine pH 6 (also containing 0.02% sodium azide to prevent microbial growth during storage). At 5°C and at a protein concentration of 118 mg/ml, the formulation was 15 mM histidine pH 6 (also containing 0.02% sodium azide to prevent microbial growth during storage). At 40°C and at a protein concentration of 1.0 mg/ml, the formulations were 10 mM citrate and 10 mM phosphate at pHs 5, 6, and 7. Formulations with protein were filtered to remove possible microbes.

Freeze-thaw stability was performed by subjecting the protein in formulation to four cycles of freezing at -80°C for at least 20 hours and thawing in a 30°C water bath. The formulations that were tested for freeze-thaw stability are listed below. Stability was evaluated by SE-HPLC and % aggregate, % monomer, % fragment, and total species recovered were quantitated. The formulations were 15 mM histidine pH 6 at 60 mg/ml protein (also containing 0.02% sodium azide to prevent microbial growth) and 10 mM citrate and 10 mM phosphate at pHs 5, 6, and 7 and 1.0 mg/ml protein (filtered to remove possible microbes).

Finally, differential scanning calorimetry to measure thermal stability was performed on the protein in 10 mM citrate and 10 mM phosphate buffer at pHs 5, 6, and 7 and 1.0 mg/ml protein. The onset temperature of unfolding and the midpoint temperatures of unfolding (Tm) of each protein domain were quantitated.

Table 51: Accelerated Stability at 40°C of H1a11.1-SL-Av at Different Concentrations and In Different Buffers, Excipients, and pHs

| Protein Concentration (mg/ml) | time | temp (°C) | buffer | pH | % Aggregate | % Monomer | % Fragment | Total Area |
|---|---|---|---|---|---|---|---|---|
| --- | pre-dialysis | --- | --- | --- | 2.71 | 96.31 | 0.98 | 53058 |
| 50, 30, 10 | T0 | --- | ace | 5 | 2.89 | 96.08 | 1.03 | 48033 |
| 50, 30, 10 | T0 | --- | his | 6 | 2.81 | 96.23 | 0.96 | 46995 |
| 50, 30, 10 | T0 | --- | phos | 7 | 2.91 | 96.09 | 1.00 | 52571 |
| 50, 30, 10 | T0 | --- | ace-suc-tw | 5 | 2.54 | 96.50 | 0.96 | 50185 |
| 50, 30, 10 | T0 | --- | his-suc-tw | 6 | 2.37 | 96.62 | 1.01 | 50771 |
| 50, 30, 10 | T0 | --- | PBS | 7 | 2.90 | 96.08 | 1.01 | 49170 |
| 50 | T7d | 40 | ace | 5 | 5.19 | 93.32 | 1.49 | 49028 |
| 30 | T7d | 40 | ace | 5 | 3.86 | 94.68 | 1.47 | 48171 |
| 10 | T7d | 40 | ace | 5 | 2.60 | 95.97 | 1.43 | 48379 |
| 50 | T7d | 40 | his | 6 | 5.25 | 93.46 | 1.29 | 47731 |
| 30 | T7d | 40 | his | 6 | 4.13 | 94.58 | 1.29 | 46684 |
| 10 | T7d | 40 | his | 6 | 2.73 | 95.84 | 1.42 | 46877 |
| 50 | T7d | 40 | phos | 7 | 9.02 | 89.52 | 1.46 | 53429 |
| 30 | T7d | 40 | phos | 7 | 6.11 | 92.40 | 1.49 | 51923 |
| 10 | T7d | 40 | phos | 7 | 3.94 | 94.57 | 1.49 | 53098 |
| 50 | T7d | 40 | ace-suc-tw | 5 | 5.42 | 92.85 | 1.73 | 50373 |
| 30 | T7d | 40 | ace-suc-tw | 5 | 4.07 | 94.06 | 1.87 | 48768 |
| 10 | T7d | 40 | ace-suc-tw | 5 | 2.66 | 95.20 | 2.14 | 49396 |
| 50 | T7d | 40 | his-suc-tw | 6 | 3.44 | 95.02 | 1.54 | 50040 |
| 30 | T7d | 40 | his-suc-tw | 6 | 4.16 | 94.14 | 1.70 | 48715 |
| 10 | T7d | 40 | his-suc-tw | 6 | 2.86 | 95.24 | 1.90 | 49871 |
| 50 | T7d | 40 | PBS | 7 | 8.13 | 90.28 | 1.60 | 49207 |
| 30 | T7d | 40 | PBS | 7 | 5.82 | 92.55 | 1.63 | 48853 |
| 10 | T7d | 40 | PBS | 7 | 3.62 | 94.82 | 1.56 | 48166 |
| 50 | T21d | 40 | ace | 5 | 6.65 | 90.83 | 2.51 | 48536 |

(continued)

| Protein Concentration (mg/ml) | time | temp (°C) | buffer | pH | % Aggregate | % Monomer | % Fragment | Total Area |
|---|---|---|---|---|---|---|---|---|
| 30 | T21d | 40 | ace | 5 | 4.55 | 92.91 | 2.54 | 48520 |
| 10 | T21d | 40 | ace | 5 | 2.71 | 94.70 | 2.59 | 48395 |
| 50 | T21d | 40 | his | 6 | 7.01 | 90.71 | 2.27 | 46729 |
| 30 | T21d | 40 | his | 6 | 4.69 | 93.10 | 2.21 | 46687 |
| 10 | T21d | 40 | his | 6 | 2.77 | 94.93 | 2.30 | 46866 |
| 50 | T21d | 40 | phos | 7 | 13.39 | 83.83 | 2.78 | 52244 |
| 30 | T21d | 40 | phos | 7 | 9.38 | 87.76 | 2.86 | 53556 |
| 10 | T21d | 40 | phos | 7 | 4.77 | 92.32 | 2.91 | 52536 |
| 50 | T21d | 40 | ace-suc-tw | 5 | 6.37 | 90.34 | 3.30 | 48268 |
| 30 | T21d | 40 | ace-suc-tw | 5 | 4.27 | 91.91 | 3.82 | 47211 |
| 10 | T21d | 40 | ace-suc-tw | 5 | 2.26 | 93.02 | 4.72 | 46322 |
| 50 | T21d | 40 | his-suc-tw | 6 | 6.84 | 89.82 | 3.34 | 47140 |
| 30 | T21d | 40 | his-suc-tw | 6 | 4.60 | 91.90 | 3.50 | 47416 |
| 10 | T21d | 40 | his-suc-tw | 6 | 2.67 | 93.66 | 3.67 | 48166 |
| 50 | T21d | 40 | PBS | 7 | 12.13 | 84.81 | 3.06 | 49845 |
| 30 | T21d | 40 | PBS | 7 | 8.09 | 88.78 | 3.13 | 48108 |
| 10 | T21d | 40 | PBS | 7 | 4.20 | 92.63 | 3.17 | 48803 |

Buffer key (all buffers contain 0.02% sodium azide to prevent microbial growth): **ace** = 15 mM acetate pH 5; **his** = 15 mM histidine pH 6; **phos** = 15 mM phosphate pH 7 **ace-suc-tw** = 30 mM acetate, 80 mg/ml sucrose, 0.02% Tw80 **his-suc-tw** = 30 mM histidine, 80 mg/ml sucrose, 0.02% Tw80 **PBS** = phosphate buffered saline

Table 52. Storage Stability at 5°C of H1a11.1-SL-Av at Different Concentrations and In Different Buffers, Excipients, and pHs (Buffer Key Same as In Table 53)

| Protein Concentration (mg/ml) | time | temp (°C) | buffer | pH | % Aggregate | % Monomer | % Fragment | Total Area |
|---|---|---|---|---|---|---|---|---|
| --- | pre-dialysis | --- | --- | --- | 2.71 | 96.31 | 0.98 | 53058 |
| 50, 30, 10 | T0 | --- | ace | 5 | 2.89 | 96.08 | 1.03 | 48033 |
| 50, 30, 10 | T0 | --- | his | 6 | 2.81 | 96.23 | 0.96 | 46995 |
| 50, 30, 10 | T0 | --- | phos | 7 | 2.91 | 96.09 | 1.00 | 52571 |
| 50, 30, 10 | T0 | --- | ace-suc-tw | 5 | 2.54 | 96.50 | 0.96 | 50185 |

(continued)

| Protein Concentration (mg/ml) | time | temp (°C) | buffer | pH | % Aggregate | % Monomer | % Fragment | Total Area |
|---|---|---|---|---|---|---|---|---|
| 50, 30, 10 | T0 | --- | his-suc-tw | 6 | 2.37 | 96.62 | 1.01 | 50771 |
| 50, 30, 10 | T0 | --- | PBS | 7 | 2.90 | 96.08 | 1.01 | 49170 |
| 50 | T7d | 5 | ace | 5 | 2.96 | 95.99 | 1.05 | 49118 |
| 30 | T7d | 5 | ace | 5 | 2.74 | 96.21 | 1.06 | 48434 |
| 10 | T7d | 5 | ace | 5 | 2.62 | 96.23 | 1.15 | 48915 |
| 50 | T7d | 5 | his | 6 | 2.93 | 95.87 | 1.20 | 47967 |
| 30 | T7d | 5 | his | 6 | 2.75 | 96.06 | 1.19 | 47182 |
| 10 | T7d | 5 | his | 6 | 2.55 | 96.31 | 1.13 | 47395 |
| 50 | T7d | 5 | phos | 7 | 3.15 | 95.64 | 1.21 | 53843 |
| 30 | T7d | 5 | phos | 7 | 3.10 | 95.76 | 1.14 | 53372 |
| 10 | T7d | 5 | phos | 7 | 2.91 | 95.96 | 1.13 | 53269 |
| 50 | T7d | 5 | ace-suc-tw | 5 | 2.75 | 96.13 | 1.12 | 50236 |
| 30 | T7d | 5 | ace-suc-tw | 5 | 2.62 | 96.11 | 1.27 | 50026 |
| 10 | T7d | 5 | ace-suc-tw | 5 | 2.56 | 96.18 | 1.26 | 49290 |
| 50 | T7d | 5 | his-suc-tw | 6 | 2.84 | 96.10 | 1.07 | 50129 |
| 30 | T7d | 5 | his-suc-tw | 6 | 2.58 | 96.19 | 1.23 | 49272 |
| 10 | T7d | 5 | his-suc-tw | 6 | 2.64 | 96.08 | 1.28 | 50926 |
| 50 | T7d | 5 | PBS | 7 | 3.26 | 95.59 | 1.15 | 49502 |
| 30 | T7d | 5 | PBS | 7 | 3.07 | 95.64 | 1.29 | 49724 |
| 10 | T7d | 5 | PBS | 7 | 2.83 | 95.87 | 1.29 | 49563 |
| 50 | T21d | 5 | ace | 5 | 2.57 | 95.76 | 1.67 | 49722 |
| 30 | T21d | 5 | ace | 5 | 2.37 | 96.03 | 1.60 | 48882 |
| 10 | T21d | 5 | ace | 5 | 2.22 | 96.09 | 1.69 | 49255 |
| 50 | T21d | 5 | his | 6 | 2.63 | 95.63 | 1.74 | 44884 |
| 30 | T21d | 5 | his | 6 | 2.42 | 95.95 | 1.62 | 47510 |
| 10 | T21d | 5 | his | 6 | 2.19 | 96.08 | 1.73 | 47015 |
| 50 | T21d | 5 | phos | 7 | 3.06 | 94.96 | 1.98 | 53449 |
| 30 | T21d | 5 | phos | 7 | 2.69 | 95.46 | 1.85 | 52938 |
| 10 | T21d | 5 | phos | 7 | 2.35 | 95.84 | 1.81 | 52703 |
| 50 | T21d | 5 | ace-suc-tw | 5 | 2.25 | 95.76 | 1.99 | 50960 |

(continued)

| Protein Concentration (mg/ml) | time | temp (°C) | buffer | pH | % Aggregate | % Monomer | % Fragment | Total Area |
|---|---|---|---|---|---|---|---|---|
| 30 | T21d | 5 | ace-suc-tw | 5 | 2.08 | 95.90 | 2.02 | 49042 |
| 10 | T21d | 5 | ace-suc-tw | 5 | 1.97 | 95.84 | 2.19 | 49851 |
| 50 | T21d | 5 | his-suc-tw | 6 | 2.24 | 95.62 | 2.14 | 49983 |
| 30 | T21d | 5 | his-suc-tw | 6 | 2.09 | 95.86 | 2.05 | 48813 |
| 10 | T21d | 5 | his-suc-tw | 6 | 1.97 | 95.83 | 2.19 | 49984 |
| 50 | T21d | 5 | PBS | 7 | 2.84 | 95.07 | 2.09 | 50641 |
| 30 | T21d | 5 | PBS | 7 | 2.27 | 95.62 | 2.12 | 48441 |
| 10 | T21d | 5 | PBS | 7 | 1.99 | 95.94 | 2.07 | 48978 |
| 50 | T10mo | 5 | his | 6 | 8.05 | 91.04 | 0.91 | 45552 |
| 30 | T10mo | 5 | his | 6 | 5.81 | 93.29 | 0.90 | 46607 |
| 10 | T10mo | 5 | his | 6 | 3.62 | 95.46 | 0.92 | 46207 |
| 50 | T10mo | 5 | his-suc-tw | 6 | 8.08 | 90.26 | 1.67 | 45430 |
| 30 | T10mo | 5 | his-suc-tw | 6 | 5.98 | 92.43 | 1.58 | 42967 |
| 10 | T10mo | 5 | his-suc-tw | 6 | 3.95 | 94.25 | 1.80 | 42567 |

Table 53. Storage Stability at 5°C, Accelerated Stability at 40°C, and Freeze-Thaw Stability Of H1a11.1-SL-Av at Different Concentrations and In Different Buffers and pHs

| Protein Concentration (mg/ml) | time/FT | temp (°C) | buffer | pH | % Aggregate | % Monomer | % Fragment | Total Area |
|---|---|---|---|---|---|---|---|---|
| 1 | T0 | --- | cit-phos | 5 | 7.07 | 92.14 | 0.80 | 46824 |
| 1 | T8d | 40 | cit-phos | 5 | 2.23 | 96.39 | 1.38 | 47090 |
| 1 | T22d | 40 | cit-phos | 5 | 7.10 | 89.62 | 3.28 | 47956 |
| 1 | FT2 | --- | cit-phos | 5 | 7.91 | 90.75 | 1.34 | 46502 |
| 1 | FT4 | --- | cit-phos | 5 | 7.41 | 92.18 | 0.41 | 52181 |
|  |  |  |  |  |  |  |  |  |
| 1 | T0 | --- | cit-phos | 6 | 7.17 | 92.33 | 0.50 | 45809 |

(continued)

| Protein Concentration (mg/ml) | time/FT | temp (°C) | buffer | pH | % Aggregate | % Monomer | % Fragment | Total Area |
|---|---|---|---|---|---|---|---|---|
| 1 | T8d | 40 | cit-phos | 6 | 2.56 | 96.03 | 1.42 | 46783 |
| 1 | T22d | 40 | cit-phos | 6 | 5.79 | 91.73 | 2.48 | 47401 |
| 1 | FT2 | --- | cit-phos | 6 | 7.14 | 91.48 | 1.38 | 45256 |
| 1 | FT4 | --- | cit-phos | 6 | 7.09 | 92.56 | 0.34 | 45004 |
| | | | | | | | | |
| 1 | T0 | --- | cit-phos | 7 | 6.82 | 92.67 | 0.51 | 47025 |
| 1 | T8d | 40 | cit-phos | 7 | 2.52 | 95.95 | 1.53 | 48080 |
| 1 | T22d | 40 | cit-phos | 7 | 5.52 | 91.58 | 2.90 | 48706 |
| 1 | FT2 | --- | cit-phos | 7 | 7.23 | 91.52 | 1.25 | 46732 |
| 1 | FT4 | --- | cit-phos | 7 | 7.15 | 92.49 | 0.36 | 46561 |
| | | | | | | | | |
| 60 and 118 | T0 | --- | his | 6 | 8.03 | 91.15 | 0.82 | 43528 |
| 60 | T7d | 40 | his | 6 | 7.17 | 91.76 | 1.07 | 45333 |
| 60 | T21d | 40 | his | 6 | 15.77 | 82.13 | 2.10 | 44729 |
| 60 | T7d | 5 | his | 6 | 3.83 | 95.32 | 0.86 | 46774 |
| 60 | T26d | 5 | his | 6 | 7.14 | 92.56 | 0.30 | 63982 |
| 118 | T5mo | 5 | his | 6 | 12.82 | 86.65 | 0.53 | 55869 |
| 60 | T5mo | 5 | his | 6 | 9.46 | 90.03 | 0.51 | 64573 |
| 60 | FT2 | --- | his | 6 | 6.71 | 92.59 | 0.70 | 42259 |
| 60 | FT4 | --- | his | 6 | 6.33 | 93.62 | 0.05 | 41054 |

Key: **FT** = freeze thaw **FT2** = analysis after two cycles of freeze and thaw; freezing at -80°C and thawing in a 30°C water bath **FT4** = analysis after four cycles of freeze and thaw; freezing at -80°C and thawing in a 30°C water bath **cit-phos** = 10 mM citrate and 10 mM phosphate **his** = 15 mM histidine and 0.02% sodium azide (azide for preventing microbial growth)

Table 54. Differential Scanning Calorimetry Data of H1a11.1-SL-Av at 1 mg/ml in 10 mm Citrate + 10 Mm Phosphate at Different pHs

| pH | Onset (°C) | Tm1 (°C) | Tm2 (°C) | Tm3 (°C) | Tm4 (°C) |
|---|---|---|---|---|---|
| 5 | 55 | 68.2 | 68.86 | 75.56 | 81.18 |
| 6 | 58 | 69.04 | 70.47 | 75.24 | 82.04 |
| 7 | 59 | 69.52 | 70.94 | 74.44 | 82.06 |

Overall, the data in Tables 51 to 54 suggest that h1A11.1-SL-Av has a degradation profile typically observed for stable monoclonal antibodies. The aggregation and fragmentation is greater at elevated temperatures (40°C) than at lower temperatures (5°C).

At 5°C, there is no apparent increase in aggregation or fragmentation after 21 days of storage. At 40°C, the amount of degradation was higher than those values typically observed for stable mAbs. However, it was not so great as to preclude it from further development. Overall, this DVD-Ig protein qualified as an AS-DVD-Ig protein based on its stability.

**EXAMPLE 28: Formulation Selection for Anti-DLL4/Anti-VEGF DVD-Ig Protein**

Materials and Methods. The stability of anti-DLL4/anti-VEGF DVD-Ig h1A11.1 -SL-Av protein was evaluated in the five formulations listed in Table 55. All formulations were prepared in 15 mM histidine buffer. Formulations F1 to F4 were prepared at 50 mg/ml protein concentration. In these formulations, the pH ranged from 5.5 to 6.0, polysorbate 80 concentration ranged from 0 to 0.05% w/v, sucrose concentration ranged from 0 to 7.5% w/v, and arginine concentration ranged from 0 to 1% w/v. Formulation F4 was prepared in 15 mM histidine buffer at pH 6.0 without any stabilizers and served as a study control for the 50 mg/ml liquid formulation stability assessment. In addition, one formulation was prepared at 25 mg/ml protein concentration at pH 6.0 (Formulation F5). In this formulation, the polysorbate 80 concentration was 0.025% w/v and sucrose concentration was 3.8% w/v.

Table 55. Formulation Composition Description

| Formulation Identifier | anti-DLL4/anti-VEGF DVD-Ig Concentration (mg/mL) | Buffer | pH | Polysorbate 80 (Tween 80) (% w/v) | Sucrose (% w/v) | Arginine (% w/v) |
|---|---|---|---|---|---|---|
| F1 | 50 | Histidine | 6.0 | 0.05 | 7.5 | 0 |
| F2 | 50 | 15 mM Histidine | 5.5 | 0.05 | 7.5 | 0 |
| F3 | 50 | 15 mM Histidine | 6.0 | 0.05 | 7.5 | 1 |
| F4 | 50 | 15 mM Histidine | 6.0 | 0 | 0 | 0 |
| F5 | 25 | Histidine | 6.0 | 0.025 | 3.8 | 0 |

In the above formulations, 15 mM histidine buffer was selected because it provides adequate buffering capacity to maintain the target formulation pH. Sucrose was evaluated as a stabilizer against freeze-thaw stress (cryoprotectant) and lyophilization process-induced stress (lyoprotectant). Polysorbate 80 (surfactant) and arginine were added to potentially stabilize the formulation against aggregates and particulates formation.

Freeze-thaw and Liquid Formulation Stability Testing. The stability of all liquid formulations was evaluated after three cycles of freeze/thaw (F/T) stress, and after 1 month storage at -80, 5, 25 and 40° C. Stability was tested by a broad panel of analytical assays including Visual appearance, % Aggregates by Size Exclusion Chromatography (SE-HPLC), Charge heterogeneity by Cation Exchange Chromatography (CEX-HPLC), Fragmentation by reduced SDS-Capillary Electrophoresis (CE-SDS), Sub-visible particles by Micro Flow Imaging (MFI) and DLL4/ VEGF binding potency using ELISA.

The freeze/thaw and liquid stability testing results are provided in Table 56.

Table 56. Freeze-Thaw and Liquid Formulation Stability Results

| Formulation Identifier | Time Points | Visual Appearance | % Aggregates by SEC-HPLC | CEX-HPLC | | | % Purity | Sub-visible Particles by MFI | | | Binding Potency by ELISA | |
|---|---|---|---|---|---|---|---|---|---|---|---|---|
| | | | | % Acidic region | % Main peak | % Basic region | | ≥ 2 μm/mL | ≥ 10 μm/mL | ≥ 25 μm/mL | DLL4 | VEGF |
| F1 | T0 | EFVP | 1.0 | 21.6 | 61.7 | 16.7 | 97.7 | 3333 | 5 | 0 | 93 | 113 |
| | 3FT | EFVP | 1.1 | 21.5 | 61.8 | 16.6 | 97.7 | 2388 | 50 | 5 | NP | NP |
| | 1M at -80°C | EFVP | 1.1 | 21.0 | 62.1 | 16.8 | 97.8 | 1364 | 15 | 5 | NP | NP |
| | 1M at 5°C | EFVP | 2.0 | 21.2 | 62.4 | 16.3 | 97.8 | 1064 | 0 | 0 | NP | NP |
| | 1M at 25°C | EFVP | 4.3 | 23.5 | 62.2 | 14.2 | 97.4 | 1559 | 5 | 5 | NP | NP |
| | 1M at 40°C | EFVP | 7.2 | 35.8 | 43.0 | 21.2 | 95.0 | 1219 | 15 | 0 | 94 | 104 |
| F2 | T0 | EFVP | 1.3 | 21.4 | 61.8 | 16.8 | 97.5 | 1589 | 15 | 0 | 93 | 113 |
| | 3FT | EFVP | 1.3 | 21.4 | 61.8 | 16.9 | 97.6 | 435 | 5 | 5 | NP | NP |
| | 1M at -80°C | EFVP | 1.4 | 21.0 | 62.0 | 17.0 | 97.8 | 315 | 0 | 0 | NP | NP |
| | 1M at 5°C | EFVP | 2.1 | 20.9 | 62.3 | 16.8 | 97.8 | 230 | 5 | 5 | NP | NP |
| | 1M at 25°C | EFVP | 4.4 | 22.8 | 61.6 | 15.6 | 97.4 | 1149 | 5 | 0 | NP | NP |
| | 1M at 40°C | EFVP | 7.9 | 33.8 | 40.9 | 25.3 | 95.1 | 655 | 5 | 0 | 95 | 97 |

(continued)

| Formulation Identifier | Time Points | Visual Appearance | % Aggregates by SEC-HPLC | CEX-HPLC | | | % Purity | Sub-visible Particles by MFI | | | Binding Potency by ELISA | |
|---|---|---|---|---|---|---|---|---|---|---|---|---|
| | | | | % Acidic region | % Main peak | % Basic region | | ≥ 2 μm/mL | ≥ 10 μm/mL | ≥ 25 μm/mL | DLL4 | VEGF |
| F3 | T0 | EFVP | 1.1 | 21.5 | 61.7 | 16.7 | 97.6 | 784 | 0 | 0 | 93 | 113 |
| | 3FT | EFVP | 1.1 | 21.3 | 61.8 | 16.9 | 97.5 | 490 | 10 | 0 | NP | NP |
| | 1M at -80°C | EFVP | 1.1 | 21.0 | 61.9 | 17.1 | 97.9 | 250 | 0 | 0 | NP | NP |
| | 1M at 5°C | EFVP | 2.0 | 20.8 | 62.0 | 17.2 | 97.8 | 225 | 5 | 0 | NP | NP |
| | 1M at 25°C | EFVP | 4.9 | 21.5 | 58.3 | 20.2 | 97.3 | 834 | 10 | 0 | NP | NP |
| | 1M at 40°C | EFVP | 11.3 | 32.1 | 42.3 | 25.6 | 95.0 | 1464 | 5 | 0 | 97 | 101 |
| F4 | T0 | TMTC | 1.2 | 21.6 | 61.8 | 16.6 | 97.4 | 23707 | 370 | 5 | 93 | 113 |
| | 3FT | TMTC | 1.5 | 21.4 | 61.8 | 16.8 | 97.4 | 105467 | 5906 | 30 | NP | NP |
| | 1M at -80°C | TMTC | 1.2 | 21.1 | 62.0 | 16.9 | 97.8 | 42024 | 1329 | 60 | NP | NP |
| | 1M at 5°C | EFVP | 2.0 | 21.3 | 62.3 | 16.5 | 97.8 | 1189 | 0 | 0 | NP | NP |
| | 1M at 25°C | EFVP | 4.5 | 23.5 | 61.8 | 14.7 | 97.2 | 89299 | 3053 | 165 | NP | NP |
| | 1M at 40°C | EFVP | 7.7 | 34.9 | 44.2 | 20.9 | 94.8 | 61754 | 5051 | 670 | 101 | 97 |

(continued)

| Formulation Identifier | Time Points | Visual Appearance | % Aggregates by SEC-HPLC | CEX-HPLC | | | % Purity | Sub-visible Particles by MFI | | | Binding Potency by ELISA | |
|---|---|---|---|---|---|---|---|---|---|---|---|---|
| | | | | % Acidic region | % Main peak | % Basic region | | ≥ 2 μm/mL | ≥ 10 μm/mL | ≥ 25 μm/mL | DLL4 | VEGF |
| F5 | T0 | EFVP | 0.9 | 21.6 | 61.6 | 16.7 | 97.7 | 2808 | 5 | 5 | 93 | 113 |
| | 3FT | EFVP | 1.2 | 21.5 | 61.8 | 16.8 | 97.6 | 1949 | 0 | 0 | NP | NP |
| | 1M at -80°C | EFVP | 1.1 | 21.0 | 62.2 | 16.8 | 97.8 | 270 | 5 | 0 | NP | NP |
| | 1M at 5°C | EFVP | 1.5 | 21.2 | 61.9 | 16.8 | 97.8 | 709 | 10 | 0 | NP | NP |
| | 1M at 25°C | EFVP | 2.6 | 23.5 | 62.0 | 14.6 | 97.2 | 944 | 15 | 0 | NP | NP |
| | 1M at 40°C | EFVP | 3.9 | 37.0 | 44.4 | 18.6 | 95.0 | 974 | 20 | 0 | 92 | 95 |
| Key. EFVP: Essentially Free of Visible Particles, TMTC: Too Many To Count, NP: Not Performed | | | | | | | | | | | | |

As seen in the above table, Freeze-thaw stress resulted in the formation of visible particles and significantly higher sub-visible particle counts in Formulation F4 that was formulated without any stabilizers (e.g., polysorbate 80, sucrose, and arginine). Relative to the other formulations, this formulation also showed a trend of higher sub-visible particle counts after 1 month storage at 25 and 40°C. Formulation F5 with 25 mg/mL protein concentration showed significantly lower aggregation relative to the 50 mg/mL formulations over 1 month storage at 5, 25 and 40°C.

Lyophilized Formulation Stability Testing. The stability of select formulations was also evaluated after the formulations were lyophilized. The lyophilized drug product stability was assessed for all sucrose-containing formulations (F1, F2, F3, and F5). Stability was assessed after 2 weeks storage at 55°C. Stability was tested by a broad panel of analytical assays including Visual appearance (before and after reconstitution), Reconstitution time, % Aggregates by Size Exclusion Chromatography (SE-HPLC), Charge heterogeneity by Cation Exchange Chromatography (CEX-HPLC), Fragmentation by reduced SDS-Capillary Electrophoresis (CE-SDS), Sub-visible particles by Micro Flow Imaging (MFI), and Water Content by Karl Fischer titration.

The lyophilized formulation stability testing results are provided in Table 57. Reconstitution time for all evaluated formulations was approximately 1 minute. A slight increase in aggregation by SEC and % basic region by CEX was observed for all formulations under the stressed storage condition of 55°C. Minimal changes were observed in all other measured product stability attributes.

Table 57. Lyophilized Formulation Stability Results

| Formulati on Identifier | Time | Visual Appearance | | % Aggreg by SEC-HPLC | CEX-HPLC | | | % Purity by R CE-SDS | Sub-visible Particles by MFI | | |
|---|---|---|---|---|---|---|---|---|---|---|---|
| | | Before Recon | After Recon | | % Acidic region | % Main peak | % Basic region | | $\geq 2$ $\mu$m/ mL | $\geq 10\,\mu$m/ mL | $\geq 25\,\mu$m/ ML |
| F1 | T0 | White to off-white cake | EFVP | 1.1 | 21.3 | 61.9 | 16.8 | 97.6 | 749 | 20 | 10 |
| | 2 week s at 55°C | White to off-white cake | EFVP | 1.6 | 20.6 | 58.1 | 21.3 | 97.5 | 1639 | 15 | 0 |
| F2 | T0 | White to off-white cake | EFVP | 1.3 | 21.2 | 61.8 | 17.0 | 97.6 | 1254 | 15 | 0 |
| | 2 week s at 55°C | White to off-white cake | EFVP | 2.0 | 20.4 | 57.8 | 21.8 | 97.6 | 1609 | 10 | 0 |
| F3 | T0 | White to off-white cake | EFVP | 1.1 | 21.2 | 61.9 | 16.9 | 97.6 | 719 | 5 | 0 |
| | 2 week s at 55°C | White to off-white cake | EFVP | 1.4 | 20.8 | 59.5 | 19.8 | 97.5 | 475 | 10 | 5 |
| F5 | T0 | White to off-white cake | EFVP | 1.0 | 21.3 | 61.8 | 16.9 | 97.5 | 844 | 35 | 5 |
| | 2 week s at 55°C | White to off-white cake | EFVP | 1.5 | 20.5 | 58.0 | 21.5 | 97.7 | 270 | 5 | 0 |
| Key. EFVP = Essentially Free of Visible Particles; NP = Not Performed | | | | | | | | | | | |

The sequence of the anti-DLL4 / anti-VEGF DVD-Ig protein H1A11.1-SL-Av is set forth in Table 58 (DVD-Ig protein described in Examples 28 and 29).

Table 58: Full Length Sequence For DVD h1A11.1-SL-Av

| Name | Sequence |
|---|---|
| | 123456789012345678901234567890123456789012345678901234567890 |
| h1A11.1-SL-Av light chain | DIQMTQSPSSLSASVGDRVTITCRASEDIYSNLAWYQQKPGKAPKLLIYDTNNLADGVPS RFSGSGSGTDFTLTISSLQPEDFATYYCQQYNNYPPTFGQGTKLEIKRTVAAPSVFIFPP DIQMTQSPSSLSASVGDRVTITCSASQDISNYLNWYQQKPGKAPKVLIYFTSSLHSGVPS RFSGSGSGTDFTLTISSLQPEDFATYYCQQYSTVPWTFGQGTKVEIKRTVAAPSVFIFPP SDEQLKSGTASVVCLLNNFYPREAKVQWKVDNALQSGNSQESVTEQDSKDSTYSLSSTLT LSKADYEKHKVYACEVTHQGLSSPVTKSFNRGEC (SEQ ID NO: 28) |
| h1A11.1-SL-Av heavy chain | EVQLVESGGGLVQPGGSLRLSCAASGFTFSNFPMAWVRQAPGKGLEWVATISSSDGTTYY RDSVKGRFTISRDNAKNSLYLQMNSLRAEDTAVYYCARGYYNSPFAYWGQGTLVTVSSAS TKGPEVQLVESGGGLVQPGGSLRLSCAASGYTFTNYGMNWVRQAPGKGLEWVGWINTYTG EPTYAADFKRRFTFSLDTSKSTAYLQMNSLRAEDTAVYYCAKYPHYYGSSHWYFDVWGQG TLVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPCP APEAAGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYT LPPSREEMTKNQVSLTCLVKGFYPSDIAVEWESNGQPENNYKTTPPVLDSDGSFFLYSKL TVDKSRWQQGNVFSCSVMHEALHNHYTQKSLSLSPGK (SEQ ID NO: 29) |

Table 58 provides the full-length heavy and light chain sequences for DVD h1A11.1-SL-Av. Linker sequences are underlined, while CDRs and constant region sequences are in bold.

**EXAMPLE 29: Extended Preformulation Characterization of Second Anti-DLL4/Anti-VEGF DVD-Ig Protein (h1A11.1-LS-Av)**

Extended preformulation characterization on anti-DLL4/-antiVEGF DVD-Ig proteins was performed to explore how different formulations conditions impact the stability of the DVD-Ig proteins. Data for h1A11.1-LS-Av is presented in Tables 59 and 60. The storage stability (5°C) and accelerated stability (40°C) of the DVD-Ig protein was evaluated in the formulations and protein concentrations listed below. Stability was evaluated by SEC and % aggregate, % monomer, % fragment, and total species recovered were quantitated. Overall, the formulations cover a pH range of 5 to 7 and a protein concentration range of 10 to 50 mg/ml.

At 5°C and 40°C temperatures and at concentrations of 50, 30, and 10 mg/ml the following formulations were evaluated: 15 mM acetate pH 5, 15 mM histidine pH 6, 15 mM phosphate pH 7, 30 mM acetate, 80 mg/ml sucrose, 0.02% Tween 80 at pH 5, 30 mM histidine, 80 mg/ml sucrose, 0.02% Tween 80 at pH 6, and PBS (phosphate buffered saline). All formulations contained 0.02% sodium azide to prevent microbial growth during storage.

Table 59. Accelerated Stability at 40°C of h1A11.1-LS-Av

| Protein Concentration (mg/ml) | Time | temp (°C) | buffer | pH | % Aggregate | % Monomer | % Fragment | Total Area |
|---|---|---|---|---|---|---|---|---|
| --- | pre-dialysis | --- | --- | --- | 0.21 | 98.42 | 1.36 | 56054 |
| 50, 30, 10 | T0 | --- | ace | 5 | 0.28 | 98.41 | 1.31 | 56381 |
| 50, 30, 10 | T0 | --- | his | 6 | 0.46 | 98.23 | 1.31 | 54316 |
| 50, 30, 10 | T0 | --- | phos | 7 | 0.74 | 97.86 | 1.40 | 53212 |
| 50,30, 10 | T0 | --- | ace-suc-tw | 5 | 0.24 | 98.16 | 1.60 | 56244 |
| 50, 30, 10 | T0 | --- | his-suc-tw | 6 | 0.30 | 98.11 | 1.59 | 54076 |
| 50, 30, 10 | T0 | --- | PBS | 7 | 0.52 | 98.05 | 1.43 | 50085 |
| 50 | T7d | 40 | ace | 5 | 1.63 | 96.74 | 1.63 | 55563 |
| 30 | T7d | 40 | ace | 5 | 1.13 | 97.24 | 1.62 | 55194 |
| 10 | T7d | 40 | ace | 5 | 0.84 | 97.49 | 1.67 | 55029 |
| 50 | T7d | 40 | his | 6 | 2.00 | 96.62 | 1.38 | 53566 |
| 30 | T7d | 40 | his | 6 | 1.17 | 97.46 | 1.38 | 52443 |
| 10 | T7d | 40 | his | 6 | 0.60 | 98.00 | 1.40 | 53812 |
| 50 | T7d | 40 | phos | 7 | 4.31 | 94.02 | 1.67 | 52934 |
| 30 | T7d | 40 | phos | 7 | 2.85 | 95.46 | 1.69 | 52663 |
| 10 | T7d | 40 | phos | 7 | 1.20 | 97.11 | 1.69 | 52411 |
| 50 | T7d | 40 | ace-suc-tw | 5 | 1.10 | 96.23 | 2.66 | 54837 |
| 30 | T7d | 40 | ace-suc-tw | 5 | 0.77 | 96.40 | 2.83 | 52474 |
| 10 | T7d | 40 | ace-suc-tw | 5 | 0.43 | 96.39 | 3.17 | 50855 |
| 50 | T7d | 40 | his-suc-tw | 6 | 1.69 | 96.27 | 2.05 | 53017 |

(continued)

| Protein Concentration (mg/ml) | Time | temp (°C) | buffer | pH | % Aggregate | % Monomer | % Fragment | Total Area |
|---|---|---|---|---|---|---|---|---|
| 30 | T7d | 40 | his-suc-tw | 6 | 1.14 | 96.84 | 2.02 | 52153 |
| 10 | T7d | 40 | his-suc-tw | 6 | 0.59 | 97.30 | 2.11 | 52208 |
| 50 | T7d | 40 | PBS | 7 | 2.77 | 95.30 | 1.93 | 51623 |
| 30 | T7d | 40 | PBS | 7 | 1.73 | 96.28 | 1.99 | 49973 |
| 10 | T7d | 40 | PBS | 7 | 0.78 | 97.25 | 1.97 | 50851 |
| 50 | T21d | 40 | ace | 5 | 3.66 | 94.30 | 2.04 | 55920 |
| 30 | T21d | 40 | ace | 5 | 2.56 | 95.33 | 2.10 | 54188 |
| 10 | T21d | 40 | ace | 5 | 1.85 | 96.00 | 2.15 | 55213 |
| 50 | T21d | 40 | his | 6 | 4.14 | 94.28 | 1.58 | 54807 |
| 30 | T21d | 40 | his | 6 | 2.67 | 95.79 | 1.54 | 53071 |
| 10 | T21d | 40 | his | 6 | 1.59 | 96.82 | 1.58 | 54053 |
| 50 | T21d | 40 | phos | 7 | 8.52 | 89.32 | 2.16 | 53273 |
| 30 | T21d | 40 | phos | 7 | 5.58 | 92.54 | 1.89 | 53162 |
| 10 | T21d | 40 | phos | 7 | 3.01 | 94.89 | 2.10 | 52747 |
| 50 | T21d | 40 | ace-suc-tw | 5 | 4.12 | 93.78 | 2.10 | 56278 |
| 30 | T21d | 40 | ace-suc-tw | 5 | 2.93 | 94.94 | 2.13 | 55481 |
| 10 | T21d | 40 | ace-suc-tw | 5 | 1.99 | 95.75 | 2.26 | 54696 |
| 50 | T21d | 40 | his-suc-tw | 6 | 4.94 | 93.21 | 1.85 | 54034 |
| 30 | T21d | 40 | his-suc-tw | 6 | n/a | n/a | n/a | n/a |
| 10 | T21d | 40 | his-suc-tw | 6 | 2.00 | 96.30 | 1.70 | 52686 |
| 50 | T21d | 40 | PBS | 7 | 8.44 | 89.65 | 1.90 | 51697 |
| 30 | T21d | 40 | PBS | 7 | 5.54 | 92.43 | 2.03 | 50282 |
| 10 | T21d | 40 | PBS | 7 | 2.89 | 95.05 | 2.06 | 51580 |

Buffer key (all buffers contain 0.02% sodium azide to prevent microbial growth):**ace** = 15 mM acetate pH 5; **his** = 15 mM histidine pH 6; **phos** = 15 mM phosphate pH 7; **ace-suc-tw** = 30 mM acetate, 80 mg/ml sucrose, 0.02% Tween80; **his-suc-tw** = 30 mM histidine, 80 mg/ml sucrose, 0.02% Tween80; **PBS** = phosphate buffered saline

Table 60. Storage Stability At 5°C of h1A11.1-LS-Av

| Protein Concentration (mg/ml) | time | temp (°C) | buffer | pH | % Aggregate | % Monomer | % Fragment | Total Area |
|---|---|---|---|---|---|---|---|---|
| --- | pre-dialysis | --- | --- | --- | 0.21 | 98.42 | 1.36 | 56054 |
| 50, 30, 10 | T0 | --- | ace | 5 | 0.28 | 98.41 | 1.31 | 56381 |
| 50, 30, 10 | T0 | --- | his | 6 | 0.46 | 98.23 | 1.31 | 54316 |
| 50, 30, 10 | T0 | --- | phos | 7 | 0.74 | 97.86 | 1.40 | 53212 |
| 50, 30, 10 | T0 | --- | ace-suc-tw | 5 | 0.24 | 98.16 | 1.60 | 56244 |
| 50, 30, 10 | T0 | --- | his-suc-tw | 6 | 0.30 | 98.11 | 1.59 | 54076 |
| 50, 30, 10 | T0 | --- | PBS | 7 | 0.52 | 98.05 | 1.43 | 50085 |
| 50 | T7d | 5 | ace | 5 | 0.18 | 98.17 | 1.64 | 57599 |
| 30 | T7d | 5 | ace | 5 | 0.16 | 98.21 | 1.64 | 55889 |
| 10 | T7d | 5 | ace | 5 | 0.13 | 98.17 | 1.70 | 53289 |
| 50 | T7d | 5 | his | 6 | 0.18 | 98.14 | 1.68 | 55742 |
| 30 | T7d | 5 | his | 6 | 0.12 | 98.06 | 1.82 | 53603 |
| 10 | T7d | 5 | his | 6 | 0.13 | 98.07 | 1.80 | 53505 |
| 50 | T7d | 5 | phos | 7 | 0.23 | 97.72 | 2.05 | 54355 |
| 30 | T7d | 5 | phos | 7 | 0.18 | 97.77 | 2.04 | 53561 |
| 10 | T7d | 5 | phos | 7 | 0.13 | 97.72 | 2.15 | 53151 |
| 50 | T7d | 5 | ace-suc-tw | 5 | 0.09 | 97.40 | 2.51 | 57158 |
| 30 | T7d | 5 | ace-suc-tw | 5 | 0.08 | 97.43 | 2.49 | 55025 |
| 10 | T7d | 5 | ace-suc-tw | 5 | 0.08 | 97.34 | 2.58 | 53882 |
| 50 | T7d | 5 | his-suc-tw | 6 | 0.10 | 97.48 | 2.43 | 55272 |
| 30 | T7d | 5 | his-suc-tw | 6 | 0.08 | 97.63 | 2.29 | 52763 |
| 10 | T7d | 5 | his-suc-tw | 6 | 0.05 | 97.41 | 2.53 | 52903 |
| 50 | T7d | 5 | PBS | 7 | 0.12 | 97.31 | 2.58 | 51698 |
| 30 | T7d | 5 | PBS | 7 | 0.09 | 97.24 | 2.67 | 50144 |
| 10 | T7d | 5 | PBS | 7 | 0.08 | 97.28 | 2.64 | 50428 |
| 50 | T21d | 5 | ace | 5 | 0.87 | 98.45 | 0.68 | 57706 |
| 30 | T21d | 5 | ace | 5 | 0.80 | 98.55 | 0.65 | 56566 |
| 10 | T21d | 5 | ace | 5 | 0.83 | 98.47 | 0.70 | 54226 |
| 50 | T21d | 5 | his | 6 | 1.05 | 98.29 | 0.66 | 55911 |
| 30 | T21d | 5 | his | 6 | 0.92 | 98.40 | 0.68 | 54225 |

(continued)

| Protein Concentration (mg/ml) | time | temp (°C) | buffer | pH | % Aggregate | % Monomer | % Fragment | Total Area |
|---|---|---|---|---|---|---|---|---|
| 10 | T21d | 5 | his | 6 | 0.90 | 98.41 | 0.70 | 54128 |
| 50 | T21d | 5 | phos | 7 | 1.25 | 98.09 | 0.66 | 54980 |
| 30 | T21d | 5 | phos | 7 | 1.20 | 98.11 | 0.69 | 53903 |
| 10 | T21d | 5 | phos | 7 | 1.01 | 98.29 | 0.69 | 53271 |
| 50 | T21d | 5 | ace-suc-tw | 5 | 0.92 | 98.36 | 0.72 | 61574 |
| 30 | T21d | 5 | ace-suc-tw | 5 | 0.89 | 98.39 | 0.72 | 55532 |
| 10 | T21d | 5 | ace-suc-tw | 5 | 0.83 | 98.46 | 0.71 | 55841 |
| 50 | T21d | 5 | his-suc-tw | 6 | 1.00 | 98.27 | 0.73 | 55484 |
| 30 | T21d | 5 | his-suc-tw | 6 | 0.92 | 98.37 | 0.70 | 53335 |
| 10 | T21d | 5 | his-suc-tw | 6 | 0.82 | 98.49 | 0.69 | 53736 |
| 50 | T21d | 5 | PBS | 7 | 1.49 | 97.79 | 0.71 | 52405 |
| 30 | T21d | 5 | PBS | 7 | 1.29 | 98.02 | 0.70 | 51284 |
| 10 | T21d | 5 | PBS | 7 | 1.12 | 98.18 | 0.70 | 51377 |

The buffer key for Table 59 is the same as in Table 60.

**Example 30: Formulation Studies of Additional DLL4-VEGF DVD-Ig Proteins h1A11.1.A6-LS-Av and h1A11.1.A6-SL-Av**

Extended preformulation characterization on additional DLL4/VEGF DVD-Ig proteins was performed to explore how different formulations conditions impact the stability. Data for h1A11.1.A6-LS-Av and h1A11.1.A6-SL-Av DLL4/VEGF DVD-Ig proteins are presented below. These DVD-Ig proteins proved to be unstable and failed the screening criteria to be considered AS-DVD-Ig proteins.

The storage stability (5°C) and accelerated stability (40°C) of h1A11.1.A6-LS-Av and h1A11.1.A6-SL-Av were evaluated in the formulations and protein concentrations listed below. Stability was evaluated by SEC and % aggregate, % monomer, % fragment, and total species recovered were quantitated. Overall, the formulations cover a pH range of 5 to 7 and a protein concentration range of 10 to 50 mg/ml.

At 5°C and 40°C temperatures and at 50, 30, and 10 mg/ml, the following conditions were tested: 15 mM acetate pH 5; 15 mM histidine pH 6; 15 mM phosphate pH 7; 30 mM acetate, 80 mg/ml sucrose, 0.02% Tween 80 at pH 5; 30 mM histidine, 80 mg/ml sucrose, 0.02% Tween 80 at pH 6; and PBS (phosphate buffered saline). All formulations contained 0.02% sodium azide to prevent microbial growth during storage

Overall, the data provided in Tables 61-63 suggests the two DVD-Ig proteins have an atypical degradation profile not observed for stable monoclonal antibodies. The aggregation rate was actually greater at 5°C than at 40°C.

At 5°C, there is a rapid increase in aggregation after 21 days of storage. At 40°C, the amount of degradation also increases, but not at the rate observed at 5°C. In both cases, the aggregation is concentration dependent.

Overall, these DVD-Ig proteins failed the screen based on their 5°C instability and are examples of non-AS-DVD-Ig proteins.

Table 61. Accelerated Stability at 40°C of H1a11.1A6-LS-Av at Different Concentrations and In Different Buffers, Excipients, and pHs

| Protein Concentration (mg/ml) | Time | temp (°C) | buffer | pH | % Aggregate | % Monomer | % Fragment | Total Area |
|---|---|---|---|---|---|---|---|---|
| --- | pre-dialysis | --- | --- | --- | 0.66 | 98.64 | 0.70 | 48425 |
| 50, 30, 10 | T0 | --- | ace | 5 | 1.24 | 98.05 | 0.72 | 50944 |
| 50, 30, 10 | T0 | --- | his | 6 | 1.49 | 97.74 | 0.76 | 46462 |
| 50, 30, 10 | T0 | --- | phos | 7 | 1.76 | 97.67 | 0.58 | 43322 |
| 50, 30, 10 | T0 | --- | ace-suc-tw | 5 | 1.66 | 97.69 | 0.65 | 57038 |
| 50, 30, 10 | T0 | --- | his-suc-tw | 6 | 1.62 | 97.87 | 0.52 | 54299 |
| 50, 30, 10 | T0 | --- | PBS | 7 | 2.22 | 97.25 | 0.53 | 48116 |
| 50 | T7d | 40 | ace | 5 | 4.40 | 94.52 | 1.08 | 43246 |
| 30 | T7d | 40 | ace | 5 | 2.76 | 96.17 | 1.08 | 46266 |
| 10 | T7d | 40 | ace | 5 | 1.54 | 97.41 | 1.05 | 52178 |
| 50 | T7d | 40 | his | 6 | 5.11 | 94.02 | 0.87 | 50471 |
| 30 | T7d | 40 | his | 6 | 3.17 | 95.97 | 0.87 | 40875 |
| 10 | T7d | 40 | his | 6 | 1.60 | 97.52 | 0.88 | 48759 |
| 50 | T7d | 40 | phos | 7 | 12.24 | 86.69 | 1.07 | 44327 |
| 30 | T7d | 40 | phos | 7 | 7.81 | 91.09 | 1.11 | 42284 |
| 10 | T7d | 40 | phos | 7 | 3.46 | 95.42 | 1.12 | 44023 |
| 50 | T7d | 40 | ace-suc-tw | 5 | 5.46 | 93.48 | 1.07 | 63100 |
| 30 | T7d | 40 | ace-suc-tw | 5 | 3.42 | 95.55 | 1.03 | 59243 |
| 10 | T7d | 40 | ace-suc-tw | 5 | 1.83 | 97.03 | 1.14 | 60361 |
| 50 | T7d | 40 | his-suc-tw | 6 | 4.90 | 94.14 | 0.96 | 56706 |
| 30 | T7d | 40 | his-suc-tw | 6 | 3.65 | 95.50 | 0.84 | 53538 |
| 10 | T7d | 40 | his-suc-tw | 6 | 1.78 | 97.31 | 0.91 | 55447 |
| 50 | T7d | 40 | PBS | 7 | 14.78 | 84.08 | 1.14 | 58511 |
| 30 | T7d | 40 | PBS | 7 | 9.20 | 89.56 | 1.24 | 46917 |
| 10 | T7d | 40 | PBS | 7 | 4.08 | 94.73 | 1.19 | 49978 |
| 50 | T21d | 40 | ace | 5 | 5.58 | 92.75 | 1.67 | 53248 |
| 30 | T21d | 40 | ace | 5 | 3.59 | 94.67 | 1.74 | 51038 |
| 10 | T21d | 40 | ace | 5 | 1.90 | 96.38 | 1.72 | 50617 |
| 50 | T21d | 40 | his | 6 | 6.25 | 92.37 | 1.37 | 46908 |
| 30 | T21d | 40 | his | 6 | 3.82 | 94.77 | 1.41 | 46075 |

(continued)

| Protein Concentration (mg/ml) | Time | temp (°C) | buffer | pH | % Aggregate | % Monomer | % Fragment | Total Area |
|---|---|---|---|---|---|---|---|---|
| 10 | T21d | 40 | his | 6 | 1.93 | 96.61 | 1.47 | 47827 |
| 50 | T21d | 40 | phos | 7 | 13.12 | 84.98 | 1.90 | 40311 |
| 30 | T21d | 40 | phos | 7 | 9.11 | 88.98 | 1.91 | 42037 |
| 10 | T21d | 40 | phos | 7 | 4.05 | 93.96 | 1.99 | 43009 |
| 50 | T21d | 40 | ace-suc-tw | 5 | 6.45 | 91.73 | 1.82 | 56752 |
| 30 | T21d | 40 | ace-suc-tw | 5 | 4.21 | 93.92 | 1.87 | 56330 |
| 10 | T21d | 40 | ace-suc-tw | 5 | 2.11 | 95.88 | 2.00 | 57757 |
| 50 | T21d | 40 | his-suc-tw | 6 | 6.69 | 91.83 | 1.49 | 54091 |
| 30 | T21d | 40 | his-suc-tw | 6 | 4.22 | 94.30 | 1.48 | 52932 |
| 10 | T21d | 40 | his-suc-tw | 6 | 2.09 | 96.45 | 1.47 | 54118 |
| 50 | T21d | 40 | PBS | 7 | 15.74 | 82.23 | 2.03 | 52080 |
| 30 | T21d | 40 | PBS | 7 | 9.98 | 87.82 | 2.19 | 47188 |
| 10 | T21d | 40 | PBS | 7 | 4.71 | 93.04 | 2.25 | 47400 |

Buffer key (all buffers contain 0.02% sodium azide to prevent microbial growth): **ace** = 15 mM acetate pH 5; **his** = 15 mM histidine pH 6; **phos** = 15 mM phosphate pH 7 **ace-suc-tw** = 30 mM acetate, 80 mg/ml sucrose, 0.02% Tw80 **his-suc-tw** = 30 mM histidine, 80 mg/ml sucrose, 0.02% Tw80 **PBS** = phosphate buffered saline

Table 62. Storage Stability at 5°C of H1a11.1.A6-LS-Av at Different Concentrations and In Different Buffers, Excipients, and pHs (Buffer Key Same as In Table 63)

| Protein Concentration (mg/ml) | Time | temp (°C) | buffer | pH | % Aggregate | % Monomer | % Fragment | Total Area |
|---|---|---|---|---|---|---|---|---|
| --- | pre-dialysis | --- | --- | --- | 0.66 | 98.64 | 0.70 | 48425 |
| 50, 30, 10 | T0 | --- | ace | 5 | 1.24 | 98.05 | 0.72 | 50944 |
| 50, 30, 10 | T0 | --- | his | 6 | 1.49 | 97.74 | 0.76 | 46462 |
| 50, 30, 10 | T0 | --- | phos | 7 | 1.76 | 97.67 | 0.58 | 43322 |
| 50, 30, 10 | T0 | --- | ace-suc-tw | 5 | 1.66 | 97.69 | 0.65 | 57038 |
| 50, 30, 10 | T0 | --- | his-suc-tw | 6 | 1.62 | 97.87 | 0.52 | 54299 |
| 50, 30, 10 | T0 | --- | PBS | 7 | 2.22 | 97.25 | 0.53 | 48116 |
| 50 | T7d | 5 | ace | 5 | 4.68 | 94.74 | 0.58 | 54446 |
| 30 | T7d | 5 | ace | 5 | 2.77 | 96.62 | 0.62 | 51286 |
| 10 | T7d | 5 | ace | 5 | 1.75 | 97.62 | 0.63 | 51444 |

(continued)

| Protein Concentration (mg/ml) | Time | temp (°C) | buffer | pH | % Aggregate | % Monomer | % Fragment | Total Area |
|---|---|---|---|---|---|---|---|---|
| 50 | T7d | 5 | his | 6 | 7.46 | 91.88 | 0.66 | 46683 |
| 30 | T7d | 5 | his | 6 | 4.83 | 94.49 | 0.68 | 46282 |
| 10 | T7d | 5 | his | 6 | 2.36 | 96.95 | 0.69 | 47844 |
| 50 | T7d | 5 | phos | 7 | 1.84 | 97.46 | 0.71 | 34008 |
| 30 | T7d | 5 | phos | 7 | 1.89 | 97.48 | 0.63 | 36992 |
| 10 | T7d | 5 | phos | 7 | 2.05 | 97.30 | 0.65 | 41962 |
| 50 | T7d | 5 | ace-suc-tw | 5 | 4.74 | 94.65 | 0.61 | 57937 |
| 30 | T7d | 5 | ace-suc-tw | 5 | N/A | N/A | N/A | N/A |
| 10 | T7d | 5 | ace-suc-tw | 5 | 1.95 | 97.41 | 0.64 | 58618 |
| 50 | T7d | 5 | his-suc-tw | 6 | 7.97 | 91.45 | 0.59 | 54735 |
| 30 | T7d | 5 | his-suc-tw | 6 | 4.85 | 94.49 | 0.66 | 53379 |
| 10 | T7d | 5 | his-suc-tw | 6 | 2.34 | 97.01 | 0.65 | 54187 |
| 50 | T7d | 5 | PBS | 7 | 5.68 | 93.65 | 0.67 | 46544 |
| 30 | T7d | 5 | PBS | 7 | 5.20 | 94.13 | 0.67 | 45219 |
| 10 | T7d | 5 | PBS | 7 | 3.56 | 95.76 | 0.68 | 47653 |
| 50 | T21d | 5 | ace | 5 | 9.33 | 89.97 | 0.70 | 52020 |
| 30 | T21d | 5 | ace | 5 | 4.25 | 95.04 | 0.70 | 51223 |
| 10 | T21d | 5 | ace | 5 | 1.95 | 97.36 | 0.69 | 50950 |
| 50 | T21d | 5 | his | 6 | 19.35 | 79.71 | 0.94 | 44105 |
| 30 | T21d | 5 | his | 6 | 9.91 | 89.29 | 0.80 | 46096 |
| 10 | T21d | 5 | his | 6 | 3.11 | 96.15 | 0.73 | 47777 |
| 50 | T21d | 5 | phos | 7 | 1.65 | 97.49 | 0.86 | 25059 |
| 30 | T21d | 5 | phos | 7 | 1.49 | 97.75 | 0.76 | 29723 |
| 10 | T21d | 5 | phos | 7 | 2.00 | 97.09 | 0.90 | 38517 |
| 50 | T21d | 5 | ace-suc-tw | 5 | 9.41 | 89.79 | 0.79 | 56438 |
| 30 | T21d | 5 | ace-suc-tw | 5 | 4.72 | 94.50 | 0.79 | 56230 |
| 10 | T21d | 5 | ace-suc-tw | 5 | 2.13 | 97.01 | 0.86 | 58579 |
| 50 | T21d | 5 | his-suc-tw | 6 | 20.57 | 78.32 | 1.11 | 53114 |
| 30 | T21d | 5 | his-suc-tw | 6 | 10.17 | 88.99 | 0.85 | 53155 |

(continued)

| Protein Concentration (mg/ml) | Time | temp (°C) | buffer | pH | % Aggregate | % Monomer | % Fragment | Total Area |
|---|---|---|---|---|---|---|---|---|
| 10 | T21d | 5 | his-suc-tw | 6 | 3.20 | 95.94 | 0.86 | 54028 |
| 50 | T21d | 5 | PBS | 7 | 5.65 | 93.38 | 0.98 | 34294 |
| 30 | T21d | 5 | PBS | 7 | 2.99 | 96.04 | 0.96 | 36457 |
| 10 | T21d | 5 | PBS | 7 | 5.14 | 93.95 | 0.91 | 46566 |

Table 63. Accelerated Stability at 40°C of H1a11.1.A6-SL-Av at Different Concentrations and In Different Buffers, Excipients, and pHs

| Protein Concentration (mg/ml) | time | temp (°C) | buffer | pH | % Aggregate | % Monomer | % Fragment | Total Area |
|---|---|---|---|---|---|---|---|---|
| --- | pre-dialysis | --- | --- | --- | 1.19 | 97.75 | 1.06 | 58988 |
| 50, 30, 10 | T0 | --- | ace | 5 | 1.23 | 97.85 | 0.92 | 47138 |
| 50, 30, 10 | T0 | --- | his | 6 | 1.30 | 97.84 | 0.87 | 44711 |
| 3.4 | T0 | --- | phos | 7 | 0.86 | 98.00 | 1.14 | 57373 |
| 50, 30, 10 | T0 | --- | ace-suc-tw | 5 | 1.30 | 97.85 | 0.85 | 52129 |
| 50, 30, 10 | T0 | --- | his-suc-tw | 6 | 1.28 | 97.85 | 0.87 | 47563 |
| 6.6 | T0 | --- | PBS | 7 | 1.35 | 97.76 | 0.89 | 71146 |
| 50 | T7d | 40 | ace | 5 | 7.66 | 90.88 | 1.46 | 48331 |
| 30 | T7d | 40 | ace | 5 | 4.05 | 94.43 | 1.52 | 45731 |
| 10 | T7d | 40 | ace | 5 | 1.45 | 97.05 | 1.51 | 47455 |
| 50 | T7d | 40 | his | 6 | 8.68 | 90.23 | 1.09 | 45897 |
| 30 | T7d | 40 | his | 6 | 4.74 | 94.15 | 1.11 | 44807 |
| 10 | T7d | 40 | his | 6 | 1.62 | 97.07 | 1.31 | 45567 |
| 3.4 | T7d | 40 | phos | 7 | 2.04 | 96.51 | 1.45 | 56002 |
| 50 | T7d | 40 | ace-suc-tw | 5 | 9.10 | 89.27 | 1.62 | 52696 |
| 30 | T7d | 40 | ace-suc-tw | 5 | 5.23 | 93.14 | 1.64 | 50591 |
| 10 | T7d | 40 | ace-suc-tw | 5 | 1.91 | 96.09 | 2.00 | 51790 |
| 50 | T7d | 40 | his-suc-tw | 6 | 8.55 | 90.18 | 1.27 | 48458 |
| 30 | T7d | 40 | his-suc-tw | 6 | 4.82 | 93.19 | 1.99 | 46963 |
| 10 | T7d | 40 | his-suc-tw | 6 | 1.78 | 96.78 | 1.45 | 46676 |
| 6.6 | T7d | 40 | PBS | 7 | 4.53 | 93.83 | 1.64 | 70277 |

(continued)

| Protein Concentration (mg/ml) | time | temp (°C) | buffer | pH | % Aggregate | % Monomer | % Fragment | Total Area |
|---|---|---|---|---|---|---|---|---|
| 50 | T21d | 40 | ace | 5 | 8.27 | 89.20 | 2.53 | 47139 |
| 30 | T21d | 40 | ace | 5 | 4.41 | 93.03 | 2.56 | 45779 |
| 10 | T21d | 40 | ace | 5 | 1.60 | 95.68 | 2.72 | 46794 |
| 50 | T21d | 40 | his | 6 | 9.26 | 88.56 | 2.18 | 44423 |
| 30 | T21d | 40 | his | 6 | 5.19 | 92.86 | 1.96 | 43874 |
| 10 | T21d | 40 | his | 6 | 1.74 | 95.71 | 2.55 | 45249 |
| 3.4 | T21d | 40 | phos | 7 | 2.43 | 95.14 | 2.42 | 54476 |
| 50 | T21d | 40 | ace-suc-tw | 5 | 9.45 | 87.66 | 2.89 | 51846 |
| 30 | T21d | 40 | ace-suc-tw | 5 | 5.35 | 91.28 | 3.37 | 50456 |
| 10 | T21d | 40 | ace-suc-tw | 5 | 1.94 | 94.29 | 3.78 | 50414 |
| 50 | T21d | 40 | his-suc-tw | 6 | 8.72 | 89.02 | 2.26 | 46410 |
| 30 | T21d | 40 | his-suc-tw | 6 | 5.08 | 92.63 | 2.29 | 43210 |
| 10 | T21d | 40 | his-suc-tw | 6 | 1.95 | 95.68 | 2.37 | 46539 |
| 6.6 | T21d | 40 | PBS | 7 | 4.71 | 92.43 | 2.86 | 68811 |

Buffer key (all buffers contain 0.02% sodium azide to prevent microbial growth): **ace** = 15 mM acetate pH 5; **his** = 15 mM histidine pH 6; **phos** = 15 mM phosphate pH 7 **ace-suc-tw** = 30 mM acetate, 80 mg/ml sucrose, 0.02% Tw80 **his-suc-tw** = 30 mM histidine, 80 mg/ml sucrose, 0.02% Tw80 **PBS** = phosphate buffered saline

Table 64. Storage Stability at 5°C of H1a11.1.A6-SL-Av at Different Concentrations and In Different Buffers, Excipients, and pHs

| Protein Concentration (mg/ml) | Time | temp (°C) | buffer | pH | % Aggregate | % Monomer | % Fragment | Total Area |
|---|---|---|---|---|---|---|---|---|
| --- | pre-dialysis | --- | --- | --- | 1.19 | 97.75 | 1.06 | 58988 |
| 50, 30, 10 | T0 | --- | ace | 5 | 1.23 | 97.85 | 0.92 | 47138 |
| 50, 30, 10 | T0 | --- | his | 6 | 1.30 | 97.84 | 0.87 | 44711 |
| 3.4 | T0 | --- | phos | 7 | 0.86 | 98.00 | 1.14 | 57373 |
| 50, 30, 10 | T0 | --- | ace-suc-tw | 5 | 1.30 | 97.85 | 0.85 | 52129 |
| 50, 30, 10 | T0 | --- | his-suc-tw | 6 | 1.28 | 97.85 | 0.87 | 47563 |
| 6.6 | T0 | --- | PBS | 7 | 1.35 | 97.76 | 0.89 | 71146 |
| 50 | T7d | 5 | ace | 5 | 3.52 | 95.40 | 1.08 | 46507 |
| 30 | T7d | 5 | ace | 5 | 2.35 | 96.55 | 1.10 | 40267 |
| 10 | T7d | 5 | ace | 5 | 1.46 | 97.48 | 1.07 | 47761 |

(continued)

| Protein Concentration (mg/ml) | Time | temp (°C) | buffer | pH | % Aggregate | % Monomer | % Fragment | Total Area |
|---|---|---|---|---|---|---|---|---|
| 50 | T7d | 5 | his | 6 | 3.48 | 95.24 | 1.27 | 46306 |
| 30 | T7d | 5 | his | 6 | 3.63 | 95.34 | 1.03 | 46581 |
| 10 | T7d | 5 | his | 6 | 2.01 | 97.01 | 0.98 | 45843 |
| 3.4 | T7d | 5 | phos | 7 | 1.42 | 97.41 | 1.17 | 52150 |
| 50 | T7d | 5 | ace-suc-tw | 5 | 3.97 | 94.99 | 1.04 | 53439 |
| 30 | T7d | 5 | ace-suc-tw | 5 | 2.65 | 96.35 | 1.00 | 52119 |
| 10 | T7d | 5 | ace-suc-tw | 5 | 1.60 | 97.23 | 1.17 | 52681 |
| 50 | T7d | 5 | his-suc-tw | 6 | 4.81 | 94.10 | 1.09 | 48136 |
| 30 | T7d | 5 | his-suc-tw | 6 | 3.38 | 95.49 | 1.13 | 48266 |
| 10 | T7d | 5 | his-suc-tw | 6 | 1.88 | 96.90 | 1.22 | 47966 |
| 6.6 | T7d | 5 | PBS | 7 | 2.25 | 96.78 | 0.97 | 68563 |
| 50 | T21d | 5 | ace | 5 | 7.82 | 92.06 | 0.11 | 47402 |
| 30 | T21d | 5 | ace | 5 | 4.22 | 95.68 | 0.10 | 45736 |
| 10 | T21d | 5 | ace | 5 | 1.77 | 98.13 | 0.09 | 47900 |
| 50 | T21d | 5 | his | 6 | 7.36 | 92.48 | 0.17 | 47111 |
| 30 | T21d | 5 | his | 6 | 6.51 | 93.34 | 0.15 | 43911 |
| 10 | T21d | 5 | his | 6 | 2.92 | 96.98 | 0.10 | 45446 |
| 3.4 | T21d | 5 | phos | 7 | 0.05 | 99.53 | 0.42 | 45856 |
| 50 | T21d | 5 | ace-suc-tw | 5 | 9.13 | 90.73 | 0.14 | 52424 |
| 30 | T21d | 5 | ace-suc-tw | 5 | 4.79 | 95.07 | 0.15 | 51575 |
| 10 | T21d | 5 | ace-suc-tw | 5 | 1.98 | 97.87 | 0.15 | 51870 |
| 50 | T21d | 5 | his-suc-tw | 6 | 9.50 | 90.30 | 0.20 | 46588 |
| 30 | T21d | 5 | his-suc-tw | 6 | 6.20 | 93.64 | 0.16 | 46023 |
| 10 | T21d | 5 | his-suc-tw | 6 | 2.61 | 97.27 | 0.12 | 47285 |
| 6.6 | T21d | 5 | PBS | 7 | 3.12 | 96.76 | 0.13 | 62856 |

Buffer key (all buffers contain 0.02% sodium azide to prevent microbial growth): **ace** = 15 mM acetate pH 5; **his** = 15 mM histidine pH 6; **phos** = 15 mM phosphate pH 7 **ace-suc-tw** = 30 mM acetate, 80 mg/ml sucrose, 0.02% Tw80 **his-suc-tw** = 30 mM histidine, 80 mg/ml sucrose, 0.02% Tw80 **PBS** = phosphate buffered saline

Overall, the data in Tables 61 to 64 suggest the two DVD-Ig proteins have an atypical degradation profile not observed for stable monoclonal antibodies. The aggregation rate is actually greater at 5°C than at 40°C. At 5°C, there is a rapid increase in aggregation after 21 days of storage. At 40°C, the amount of degradation also increases, but not at the rate observed at 5°C. In both cases, the aggregation is concentration dependent. Overall, these DVD-Ig proteins did not qualify as AS-DVD-Ig proteins based on their 5°C instability and would be considered non-aqueous stable.

**Sequences**

Amino acid sequences of the heavy and light chains for other DVD-Ig proteins described herein are provided below in Table 65. Linker sequences are underlined, while CDR sequences are in bold. "L" below represents the light chain, and "H" below represents the heavy chain.

Table 65: DVD-Ig protein sequences

| DVD-Ig Heavy or Light Chain Name | Outer Variable Domain Name | Inner Variable Domain Name | Sequence, with Line Break Between Variable and Constant Regions 12345678901234567890123456789012345678901234567890 |
|---|---|---|---|
| DVD005H CD20/CD80 | AB001VH | AB007VH | QVQLQQPGAELVKPGASVKMSCKASGYTFT**SYNMH**WVKQT PGRGLEWIG**AIYPGNGDTSYNQKFKG**KATLTADKSSSTAY MQLSSLTSEDSAVYYCAR**STYYGGDWYFNV**WGAGTTVTVS A**ASTKGP**QVQLQESGPGLVKPSETLSLTCAVSGGSIS**GGY GWG**WIRQPPGKGLEWIG**SFYSSSGNTYYNPSLKS**QVTIST DTSKNQFSLKLNSMTAADTAVYYCVR**DRLFSVVGMVYNNW FDV**WGPGVLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:30)** |
| DVD005L CD20/CD80 | AB001VL | AB007VL | QIVLSQSPAILSPSPGEKVTMTC**RASSSVSYIH**WFQQKPG SSPKPWIY**ATSNLASGVPV**RFSGSGSGTSYSLTISRVEAE DAATYYC**QQWTSNPPT**FGGGTKLEIKR**TVAAP**ESALTQPP SVSGAPGQKVTISC**TGSTSNIGGYDLH**WYQQLPGTAPKLL IY**DINKRPSGIS**DRFSGSKSGTAASLAITGLQTEDEADYY C**QSYDSSLNAQV**FGGGTRLTVLG<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:31)** |

| DVD-Ig Heavy or Light Chain Name | Outer Variable Domain Name | Inner Variable Domain Name | Sequence, with Line Break Between Variable and Constant Regions<br>12345678901234567890123456789001234567890 |
|---|---|---|---|
| DVD006H<br>CD80/CD20 | AB007VH | AB001VH | QVQLQESGPGLVKPSETLSLTCAVSGGSIS**GGYGWG**WIRQ PPGKGLEWIG**SFYSSSGNTYYNPSLKS**QVTISTDTSKNQF SLKLNSMTAADTAVYYCVR**DRLFSVVGMVYNNWF**DVWGPG VLVTVSS**ASTKGP**QVQLQQPGAELVKPGASVKMSCKASGY TFT**SYNMH**WVKQTPGRGLEWIG**AIYPGNGDTSYNQKFKG**K ATLTADKSSSTAYMQLSSLTSEDSAVYYCAR**STYYGGDWY FNV**WGAGTTVTVSA<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:32)** |
| DVD006L | AB007VL | AB001VL | ESALTQPPSVSGAPGQKVTISC**TGSTSNIGGYDLH**WYQQL PGTAPKLLIY**DINKRPSGIS**DRFSGSKSGTAASLAITGLQ |
| CD80/CD20 | | | TEDEADYYC**QSYDSSLNAQV**FGGGTRLTVLG**QPKAAP**QIV LSQSPAILSPSPGEKVTMTC**RASSSVSYIH**WFQQKPGSSP KPWIY**ATSNLASGVPV**RFSGSGSGTSYSLTISRVEAEDAA TYYC**QQWTSNPPT**FGGGTKLEIKR<br><br>QPKAAPSVTLFPPSSEELQANKATLVCLISDFYPGAVTVA WKADSSPVKAGVETTTPSKQSNNKYAASSYLSLTPEQWKS HRSYSCQVTHEGSTVEKTVAPTECS**(SEQ ID NO:33)** |

122

EP 3 466 973 A1

| DVD-Ig Heavy or Light Chain Name | Outer Variable Domain Name | Inner Variable Domain Name | Sequence, with Line Break Between Variable and Constant Regions<br>12345678901234567890123456789012345678 90 |
|---|---|---|---|
| DVD037H<br>VEGF/HER2 | AB014VH | AB004VH | EVQLVESGGGLVQPGGSLRLSCAASGYTFT**NYGMN**WVRQA PGKGLEWVG**WINTYTGEPTYAADFKR**RFTFSLDTSKSTAY LQMNSLRAEDTAVYYCAK**YPHYYGSSHWYFDV**WGQGTLVT VSS<u>**ASTKGP**</u>EVQLVESGGGLVQPGGSLRLSCAASGFNIK**D TYIH**WVRQAPGKGLEWVA**RIYPTNGYTRYADSVKG**RFTIS ADTSKNTAYLQMNSLRAEDTAVYYCSR**WGGDGFYAMDY**WG QGTLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT OKSLSLSPGK**(SEQ ID NO:34)** |
| DVD037L<br>VEGF/HER2 | AB014VL | AB004VL | DIQMTQSPSSLSASVGDRVTITC**SASQDISNYLN**WYQQKP GKAPKVLIY**FTSSLHSGVPS**RFSGSGSGTDFTLTISSLQP EDFATYYC**QQYSTVPWT**FGQGTKVEIKR<u>**TVAAP**</u>DIQMTQS PSSLSASVGDRVTITC**RASQDVNTAVA**WYQQKPGKAPKLL IY**SASFLYSGVPS**RFSGSRSGTDFTLTISSLQPEDFATYY C**QQHYTTPPT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:35)** |

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD038H<br>Her2/VEGF | AB004VH | AB014VH | EVQLVESGGGLVQPGGSLRLSCAASGFNIK**DTYIH**WVRQA PGKGLEWVA**RIYPTNGYTRYADSVKG**RFTISADTSKNTAY LQMNSLRAEDTAVYYCSR**WGGDGFYAMDY**WGQGTLVTVSS **ASTKGP**EVQLVESGGGLVQPGGSLRLSCAASGYTFT**NYGM N**WVRQAPGKGLEWVG**WINTYTGEPTYAADFKR**RFTFSLDT SKSTAYLQMNSLRAEDTAVYYCAK**YPHYYGSSHWYFDV**WG QGTLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:36)** |
| DVD038L<br><br>Her2/VEGF | AB004VL | AB014VL | <u>DIQMTQSPSSLSASVGDRVTITC**RASQDVNTAVA**WYQQKP</u><br>GKAPKLLIY**SASFLYSGVPS**RFSGSRSGTDFTLTISSLQP EDFATYYC**QQHYTTPPT**FGQGTKVEIKR**TVAAP**DIQMTQS PSSLSASVGDRVTITC**SASQDISNYLN**WYQQKPGKAPKVL IY**FTSSLHSGVPS**RFSGSGSGTDFTLTISSLQPEDFATYY C**QQYSTVPWT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:37)** |

EP 3 466 973 A1

124

(continued)

| DVD-Ig Heavy or Light Chain Name | Outer Variable Domain Name | Inner Variable Domain Name | Sequence, with Line Break Between Variable and Constant Regions 12345678901234567890123456789001234567890 |
|---|---|---|---|
| DVD053H<br>TNF/RANKL | AB017VH | AB018VH | EVQLVESGGGLVQPGRSLRLSCAASGFTFD**DYAMH**WVRQA PGKGLEWVS**AITWNSGHIDYADSVEG**RFTISRDNAKNSLY LQMNSLRAEDTAVYYCAK**VSYLSTASSLDY**WGQGTLVTVS S<u>**ASTKGP**</u>EVQLLESGGGLVQPGGSLRLSCAASGFTFS**SYA MS**WVRQAPGKGLEWVS**GITGSGGSTYYADSVKG**RFTISRD NSKNTLYLQMNSLRAEDTAVYYCAK**DPGTTVIMSWFDP**WG QGTLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:38)** |
| DVD053L<br>TNF/RANKL | AB017VL | AB018VL | DIQMTQSPSSLSASVGDRVTITC**RASQGIRNYLA**WYQQKP GKAPKLLIY**AASTLQSGVPS**RFSGSGSGTDFTLTISSLQP EDVATYYC**QRYNRAPYT**FGQGTKVEIKR<u>**TVAAP**</u>EIVLTQS PGTLSLSPGERATLSC**RASQSVRGRYLA**WYQQKPGQAPRL LIY**GASSRATGIPD**RFSGSGSGTDFTLTISRLEPEDFAVF YC**QQYGSSPRT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:39)** |

125

EP 3 466 973 A1

(continued)

| DVD-Ig Heavy or Light Chain Name | Outer Variable Domain Name | Inner Variable Domain Name | Sequence, with Line Break Between Variable and Constant Regions<br>123456789012345678901234567890 |
|---|---|---|---|
| DVD054H<br>RANKL/TNF | AB018VH | AB017VH | EVQLLESGGGLVQPGGSLRLSCAASGFTFS**SYAMS**WVRQA PGKGLEWVS**GITGSGGSTYYADSVKG**RFTISRDNSKNTLY LQMNSLRAEDTAVYYCAK**DPGTTVIMSWFDP**WGQGTLVTV SS**ASTKGP**EVQLVESGGGLVQPGRSLRLSCAASGFTFDD**Y AMH**WVRQAPGKGLEWVS**AITWNSGHIDYADSVEG**RFTISR DNAKNSLYLQMNSLRAEDTAVYYCAK**VSYLSTASSLDY**WG QGTLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:40)** |
| DVD054L RANKL/TNF | AB018VL | AB017VL | EIVLTQSPGTLSLSPGERATLSC**RASQSVRGRYLA**WYQQK PGQAPRLLIY**GASSRATGIPD**RFSGSGSGTDFTLTISRLE PEDFAVFYC**QQYGSSPRT**FGQGTKVEIKR**TVAAP**DIQMTQ SPSSLSASVGDRVTITC**RASQGIRNYLA**WYQQKPGKAPKL LIY**AASTLQSGVPS**RFSGSGSGTDFTLTISSLQPEDVATY YC**QRYNRAPYT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:41)** |

126

| DVD-Ig Heavy or Light Chain Name | Outer Variable Domain Name | Inner Variable Domain Name | Sequence, with Line Break Between Variable and Constant Regions 123456789012345678901234567890 |
|---|---|---|---|
| DVD065H TNF/DKK | AB017VH | AB023VH | EVQLVESGGGLVQPGRSLRLSCAASGFTFD**DYAMH**WVRQA PGKGLEWVS**AITWNSGHIDYADSVEG**RFTISRDNAKNSLY LQMNSLRAEDTAVYYCAK**VSYLSTASSLDY**WGQGTLVTVS S<u>ASTKGP</u>EVQLVESGGGLVQPANSLKLSCAASGFTFS**DYA MA**WVRQSPKKGLEWVA**TIIYDGSSTYYRDSVKG**RFTISRD NAKSTLYLQMDSLRSEDTATYYCAT**GLGIATDYFDY**WGQG VLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:42)** |
| DVD065L TNF/DKK | AB017VL | AB023VL | DIQMTQSPSSLSASVGDRVTITC**RASQGIRNYLA**WYQQKP GKAPKLLIY**AASTLQSGVPS**RFSGSGSGTDFTLTISSLQP EDVATYYC**QRYNRAPYT**FGQGTKVEIKR<u>**TVAAP**</u>DIRMTQS PASLSASLGETVNIEC**LASEDIYSDLA**WYQQKPGKSPQLL IY**NANSLQNGVPS**RFSGSGSGTQYSLKINSLQSEDVATYF C**QQYNNYPPT**FGGGTKLELKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:43)** |

EP 3 466 973 A1

127

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD066H<br>DKK/TNF | AB023VH | AB017VH | EVQLVESGGGLVQPANSLKLSCAASGFTFS**DYAMA**WVRQS PKKGLEWVA**TIIYDGSSTYYRDSVKG**RFTISRDNAKSTLY LQMDSLRSEDTATYYCAT**GLGIATDYFDY**WGQGVLVTVSS <u>**ASTKGP**</u>EVQLVESGGGLVQPGRSLRLSCAASGFTFD**DYAM H**WVRQAPGKGLEWVS**AITWNSGHIDYADSVEG**RFTISRDN AKNSLYLQMNSLRAEDTAVYYCAK**VSYLSTASSLDY**WGQG TLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPE**AA**GG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT |
| | | | QKSLSLSPGK **(SEQ ID NO:44)** |
| DVD066L<br>DKK/TNF | AB023VL | AB017VL | DIRMTQSPASLSASLGETVNIEC**LASEDIYSDLA**WYQQKP GKSPQLLIY**NANSLQNGVPS**RFSGSGSGTQYSLKINSLQS EDVATYFC**QQYNNYPPT**FGGGTKLELKR<u>**TVAAP**</u>DIQMTQS PSSLSASVGDRVTITC**RASQGIRNYLA**WYQQKPGKAPKLL IY**AASTLQSGVPS**RFSGSGSGTDFTLTISSLQPEDVATYY C**QRYNRAPYT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC **(SEQ ID NO:45)** |

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD165H<br>CD20/RANKL | AB001VH | AB018VH | QVQLQQPGAELVKPGASVKMSCKASGYTFT**SYNMH**WVKQT<br>PGRGLEWIG**AIYPGNGDTSYNQKFKG**KATLTADKSSSTAY<br>MQLSSLTSEDSAVYYCAR**STYYGGDWYFNV**WGAGTTVTVS<br>**A**<u>**ASTKGP**</u>EVQLLESGGGLVQPGGSLRLSCAASGFTFS**SYA**<br>**MS**WVRQAPGKGLEWVS**GITGSGGSTYYADSVKG**RFTISRD<br>NSKNTLYLQMNSLRAEDTAVYYCAK**DPGTTVIMSWFDP**WG<br>QGTLVTVSS<br><br>ASTKGPSVFPLAPSSKSTSGGTAALGCLVKDYFPEPVTVS<br>WNSGALTSGVHTFPAVLQSSGLYSLSSVVTVPSSSLGTQT<br>YICNVNHKPSNTKVDKKVEPKSCDKTHTCPPCPAPEAAGG<br>PSVFLFPPKPKDTLMISRTPEVTCVVVDVSHEDPEVKFNW<br>YVDGVEVHNAKTKPREEQYNSTYRVVSVLTVLHQDWLNGK<br>EYKCKVSNKALPAPIEKTISKAKGQPREPQVYTLPPSREE<br>MTKNQVSLTCLVKGFYPSDIAVEWESNGQPENNYKTTPPV<br>LDSDGSFFLYSKLTVDKSRWQQGNVFSCSVMHEALHNHYT<br>QKSLSLSPGK**(SEQ ID NO:46)** |
| DVD165L<br>CD20/RANKL | AB001VL | AB018VL | QIVLSQSPAILSPSPGEKVTMTC**RASSSVSYIH**WFQQKPG<br>SSPKPWIY**ATSNLASGVPV**RFSGSGSGTSYSLTISRVEAE<br>DAATYYC**QQWTSNPPT**FGGGTKLEIKR<u>**TVAAP**</u>EIVLTQSP<br>GTLSLSPGERATLSC**RASQSVRGRYLA**WYQQKPGQAPRLL<br>IY**GASSRATGIPD**RFSGSGSGTDFTLTISRLEPEDFAVFY<br>C**QQYGSSPRT**FGQGTKVEIKR<br><br>TVAAPSVFIFPPSDEQLKSGTASVVCLLNNFYPREAKVQW<br>KVDNALQSGNSQESVTEQDSKDSTYSLSSTLTLSKADYEK<br>HKVYACEVTHQGLSSPVTKSFNRGEC**(SEQ ID NO:47)** |

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD166H<br>RANKL/CD20 | AB018VH | AB001VH | EVQLLESGGGLVQPGGSLRLSCAASGFTFS**SYAMS**WVRQA<br>PGKGLEWVS**GITGSGGSTYYADSVKG**RFTISRDNSKNTLY<br>LQMNSLRAEDTAVYYCAK**DPGTTVIMSWFDP**WGQGTLVTV<br>SS<u>**ASTKGP**</u>QVQLQQPGAELVKPGASVKMSCKASGYTFT**SY**<br>**NMH**WVKQTPGRGLEWIG**AIYPGNGDTSYNQKFKG**KATLTA<br>DKSSSTAYMQLSSLTSEDSAVYYCAR**STYYGGDWYFNV**WG<br>AGTTVTVSA<br><br>ASTKGPSVFPLAPSSKSTSGGTAALGCLVKDYFPEPVTVS<br>WNSGALTSGVHTFPAVLQSSGLYSLSSVVTVPSSSLGTQT<br>YICNVNHKPSNTKVDKKVEPKSCDKTHTCPPCPAPEAAGG<br>PSVFLFPPKPKDTLMISRTPEVTCVVVDVSHEDPEVKFNW<br>YVDGVEVHNAKTKPREEQYNSTYRVVSVLTVLHQDWLNGK<br>EYKCKVSNKALPAPIEKTISKAKGQPREPQVYTLPPSREE<br>MTKNQVSLTCLVKGFYPSDIAVEWESNGQPENNYKTTPPV |
| | | | LDSDGSFFLYSKLTVDKSRWQQGNVFSCSVMHEALHNHYT<br>QKSL<u>SLSPGK</u>**(SEQ ID NO:48)** |
| DVD166L<br>RANKL/CD20 | AB018VL | AB001VL | EIVLTQSPGTLSLSPGERATLSC**RASQSVRGRYLA**WYQQK<br>PGQAPRLLIY**GASSRATGIPD**RFSGSGSGTDFTLTISRLE<br>PEDFAVFYC**QQYGSSPRT**FGQGTKVEIKR<u>**TVAAP**</u>QIVLSQ<br>SPAILSPSPGEKVTMTC**RASSSVSYIH**WFQQKPGSSPKPW<br>IY**ATSNLASGVPV**RFSGSGSGTSYSLTISRVEAEDAATYY<br>C**QQWTSNPPT**FGGGTKLEIKR<br><br>TVAAPSVFIFPPSDEQLKSGTASVVCLLNNFYPREAKVQW<br>KVDNALQSGNSQESVTEQDSKDSTYSLSSTLTLSKADYEK<br>HKVYACEVTHQGLSSPVTKSFNRGEC**(SEQ ID NO:49)** |
| DVD257H<br>DLL4/PLGF | DLL4H | PIGFH | EVQLVESGGGLVQPGGSLRLSCAASGFTFT**DNWIS**WVRQA<br>PGKGLEWVG**YISPNSGFTYYADSVKG**RFTISADTSKNTAY<br>LQMNSLRAEDTAVYYCARD**NFGGYFDY**WGQGTLVTVSS<u>**AS**</u><br><u>**TKGP**</u>QVQLQQSGAELVKPGASVKISCKASGYTFT**DYYIN**W<br>VKLAPGQGLEWIG**WIYPGSGNTKYNEKFKG**KATLTIDTSS<br>STAYMQLSSLTSEDTAVYFCVRD**SPFFDY**WGQGTLLTVSS<br><br>**(SEQ ID NO:50)** |

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD257L<br>DLL4/PLGF | DLL4L | PIGFL | DIQMTQSPSSLSASVGDRVTITC**RASQDVSTAVA**WYQQKP GKAPKLLIY**SASFLYSGVPS**RFSGSGSGTDFTLTISSLQP EDFATTYYC**QQSYTGTVT**FGQGTKVEIKR<u>**TVAAP**</u>DIVLTQ SPDSLAVSLGERVTMNC**KSSQSLLNSGMRKSFLA**WYQQKP GQSPKLLIY**WASTRESGVPD**RFTGSGSGTDFTLTISSVQA EDVAVYYC**KQSYHLFT**FGSGTKLEIKR<br><br>(SEQ ID NO:51) |
| DVD258H<br>PLGF/DLL4 | PIGFH | DLL4H | QVQLQQSGAELVKPGASVKISCKASGYTFT**DYYIN**WVKLA PGQGLEWIG**WIYPGSGNTKYNEKFKG**KATLTIDTSSSTAY MQLSSLTSEDTAVYFCVRD**SPFFDY**WGQGTLLTVSS<u>**ASTK GP**</u>EVQLVESGGGLVQPGGSLRLSCAASGFTFT**DNWIS**WVR QAPGKGLEWVG**YISPNSGFTYYADSVKG**RFTISADTSKNT AYLQMNSLRAEDTAVYYCARD**NFGGYFDY**WGQGTLVTVSS<br><br>(SEQ ID NO:52) |
| DVD258L<br>PLGF/DLL4 | PIGFL | DLL4L | DIVLTQSPDSLAVSLGERVTMNC**KSSQSLLNSGMRKSFLA** WYQQKPGQSPKLLIY**WASTRESGVPD**RFTGSGSGTDFTLT ISSVQAEDVAVYYC**KQSYHLFT**FGSGTKLEIKR<u>**TVAAP**</u>DI QMTQSPSSLSASVGDRVTITC**RASQDVSTAVA**WYQQKPGK APKLLIY**SASFLYSGVPS**RFSGSGSGTDFTLTISSLQPED FATTYYC**QQSYTGTVT**FGQGTKVEIKR<br><br>(SEQ ID NO:53) |
| DVD277H<br>TNF/SOST(S 2) | AB017VH | AB050VH | EVQLVESGGGLVQPGRSLRLSCAASGFTFD**DYAMH**WVRQA PGKGLEWVS**AITWNSGHIDYADSVEG**RFTISRDNAKNSLY LQMNSLRAEDTAVYYCAK**VSYLSTASSLDY**WGQGTLVTVS S<u>**ASTKGP**</u>EVQLQQSGPELMKPGASVKMSCKASGYTFT**DYN** |

EP 3 466 973 A1

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| | | | **MH**WMKQNQGKSLEWIG**EINPNSGGSGYNQKFKG**KATLTVD KSSSTAYMELRSLTSEDSAVYYCAR**LGYYGNYEDWYFD**VW GAGTTVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:54)** |
| DVD277L<br>TNF/SOST(S 2) | AB017VL | AB050VL | DIQMTQSPSSLSASVGDRVTITC**RASQGIRNYLA**WYQQKP GKAPKLLIY**AASTLQSGVPS**RFSGSGSGTDFTLTISSLQP EDVATYYC**QRYNRAPYT**FGQGTKVEIKR<u>**TVAAP**</u>DLQMTQT TSSLSASLGDRVTISC**RASQDISNYLN**WYQQKPDGTVKLL IF**YTSTLQSGVPS**RFSGSGSGTNYSLTITNLEQDDAATYF C**QQGDTLPYT**FGGGTKLEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:55)** |

EP 3 466 973 A1

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD278H<br>SOST(S2)/T NF | AB050VH | AB017VH | EVQLQQSGPELMKPGASVKMSCKASGYTFT**DYNMH**WMKQN<br>QGKSLEWIG**EINPNSGGSGYNQKFKG**KATLTVDKSSSTAY<br>MELRSLTSEDSAVYYCAR**LGYYGNYEDWYFDV**WGAGTTVT<br>VSS<u>**ASTKGP**</u>EVQLVESGGGLVQPGRSLRLSCAASGFTFD**D**<br>**YAMH**WVRQAPGKGLEWVS**AITWNSGHIDYADSVEG**RFTIS<br>RDNAKNSLYLQMNSLRAEDTAVYYCAK**VSYLSTASSLDY**W<br>GQGTLVTVSS<br><br>ASTKGPSVFPLAPSSKSTSGGTAALGCLVKDYFPEPVTVS<br>WNSGALTSGVHTFPAVLQSSGLYSLSSVVTVPSSSLGTQT<br>YICNVNHKPSNTKVDKKVEPKSCDKTHTCPPCPAPEAAGG<br>PSVFLFPPKPKDTLMISRTPEVTCVVVDVSHEDPEVKFNW<br>YVDGVEVHNAKTKPREEQYNSTYRVVSVLTVLHQDWLNGK<br>EYKCKVSNKALPAPIEKTISKAKGQPREPQVYTLPPSREE<br>MTKNQVSLTCLVKGFYPSDIAVEWESNGQPENNYKTTPPV<br>LDSDGSFFLYSKLTVDKSRWQQGNVFSCSVMHEALHNHYT<br>QKSLSLSPGK**(SEQ ID NO:56)** |
| DVD278L<br>SOST(S2)/T NF | AB050VL | AB017VL | DLQMTQTTSSLSASLGDRVTISC**RASQDISNYLN**WYQQKP<br>DGTVKLLIF**YTSTLQSGVPS**RFSGSGSGTNYSLTITNLEQ<br>DDAATYFC**QQGDTLPYT**FGGGTKLEIKR<u>**TVAAP**</u>DIQMTQS<br>PSSLSASVGDRVTITC**RASQGIRNYLA**WYQQKPGKAPKLL<br>IY**AASTLQSGVPS**RFSGSGSGTDFTLTISSLQPEDVATYY<br>C**QRYNRAPYT**FGQGTKVEIKR<br><br>TVAAPSVFIFPPSDEQLKSGTASVVCLLNNFYPREAKVQW<br>KVDNALQSGNSQESVTEQDSKDSTYSLSSTLTLSKADYEK<br>HKVYACEVTHQGLSSPVTKSFNRGEC**(SEQ ID NO:57)** |
| DVD281H<br>IL-9(S2)/IgE | AB056VH | AB053VH | QVQLQQSGAELMKPGASVKLSCKATGYTFT**GSWIE**WIKQR<br>PGHGLEWIG**QILPGSGSAYYNEKFKG**KATFTADTSSKTVY<br>IQLISLTTEDSAIYYCAR**EDNYGSSSLAY**WGQGTLLTVSA |

EP 3 466 973 A1

133

(continued)

| DVD-Ig Heavy or Light Chain Name | Outer Variable Domain Name | Inner Variable Domain Name | Sequence, with Line Break Between Variable and Constant Regions<br>12345678901234567890123456789 01234567890 |
|---|---|---|---|
| | | | **ASTKGP**EVQLVESGGGLVQPGGSLRLSCAVSGYSIT**SGYS WN**WIRQAPGKGLEWVA**SITYDGSTNYNPSVKG**RITISRDD SKNTFYLQMNSLRAEDTAVYYCAR**GSHYFGHWHFAV**WGQG TLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:58)** |
| DVD281L<br>IL-9(S2)/IgE | AB056VL | AB053VL | DILLTQSPAILSVSPGERVSFSC**RASQSIGTNIH**WYQQRT NGSPRLLIK**YASESISGIPS**RFSGGGSGTDFTLSINSVES EDIADYYC**QQSNNWPLT**FGAGTKLELKR<u>**TVAAP**</u>DIQLTQS PSSLSASVGDRVTITC**RASQSVDYDGDSYMN**WYQQKPGKA PKLLIY**AASYLESGVPS**RFSGSGSGTDFTLTISSLQPEDF ATYYC**QQSHEDPYT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:59)** |

EP 3 466 973 A1

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DVD282H<br>IgE/IL-9(S2) | AB053VH | AB056VH | EVQLVESGGGLVQPGGSLRLSCAVSGYSIT**SGYSWN**WIRQ APGKGLEWVA**SITYDGSTNYNPSVKG**RITISRDDSKNTFY LQMNSLRAEDTAVYYCAR**GSHYFGHWHFAV**WGQGTLVTVS SA**STKGP**QVQLQQSGAELMKPGASVKLSCKATGYTFT**GSW IEW**IKQRPGHGLEWIG**QILPGSGSAYYNEKFKG**KATFTAD TSSKTVYIQLISLTTEDSAIYYCAR**EDNYGSSSLAY**WGQG TLLTVSA<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:60)** |
| DVD282L<br>IgE/IL-9(S2) | AB053VL | AB056VL | DIQLTQSPSSLSASVGDRVTITC**RASQSVDYDGDSYMN**WY QQKPGKAPKLLIY**AASYLESGVPS**RFSGSGSGTDFTLTIS SLQPEDFATYYC**QQSHEDPYT**FGQGTKVEIKR**TVAAP**DIL LTQSPAILSVSPGERVSFSC**RASQSIGTNIH**WYQQRTNGS PRLLIK**YASESISGIP**SRFSGGGSGTDFTLSINSVESEDI ADYYC**QQSNNWPLT**FGAGTKLELKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:61)** |
| TNF/IL-17H (DVD-A) | TNFH | IL-17H | EVQLVESGGGLVQPGRSLRLSCAASGFTFD**DYAMH**WVRQA PGKGLEWVS**AITWNSGHIDYADSVEG**RFTISRDNAKNSLY |

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| | | | LQMNSLRAEDTAVYYCAK**VSYLSTASSLDY**WGQGTLVTVS S**GGGGSGGGGS**EVQLVQSGAEVKKPGSSVKVSCKASGGSF GG**YGIG**WVRQAPGQGLEWMG**GITPFFGFADYAQKFQG**RVT ITADESTTTAYMELSGLTSDDTAVYYCARD**PNEFWNGYYS THDFDS**WGQGTTVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSRDE LTKNQVSLTCLVKGFYPSDIAVEWESNGQPENNYKTTPPV LDSDGSFFLYSKLTVDKSRWQQGNVFSCSVMHEALHNHYT QKSLSLSPGK<br>**(SEQ ID NO:62)** |
| TNF/IL-17L (DVD-A) | TNFL | IL-17L | DIQMTQSPSSLSASVGDRVTITC**RASQGIRNYLA**WYQQKP GKAPKLLIY**AASTLQSGVPS**RFSGSGSGTDFTLTISSLQP EDVATYYC**QRYNRAPYT**FGQGTKVEIKR**GGSGGGGSGS**EI VLTQSPDFQSVTPKEKVTITC**RASQDIGSELH**WYQQKPDQ PPKLLIK**YASHSTSGVPS**RFSGSGSGTDFTLTINGLEAED AGTYYC**HQTDSLPYT**FGPGTKVDIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:63)** |

| DVD-Ig Heavy or Light Chain Name | Outer Variable Domain Name | Inner Variable Domain Name | Sequence, with Line Break Between Variable and Constant Regions<br>12345678901234567890123456789001234567890 |
|---|---|---|---|
| TNF/PGE2H (DVD-B) | TNFH | PGE2 | EVQLVESGGGLVQPGRSLRLSCAASGFTFD**DYAMH**WVRQA PGKGLEWVS**AITWNSGHIDYADSVEG**RFTISRDNAKNSLY LQMNSLRAEDTAVYYCAK**VSYLSTASSLDY**WGQGTLVTVS S**ASTKGP**EVQLVQSGAEVKKPGASVKVSCKASGYTFT**KYW LG**WVRQAPGQGLEWMG**DIYPGYDYTHYNEKFKD**RVTLTTD TSTSTAYMELRSLRSDDTAVYYCAR**SDGSSTY**WGQGTLVT VSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSRDE LTKNQVSLTCLVKGFYPSDIAVEWESNGQPENNYKTTPPV LDSDGSFFLYSKLTVDKSRWQQGNVFSCSVMHEALHNHYT QKSLSLSPGK<br>**(SEQ ID NO:64)** |
| TNF/PGE2L (DVD-B) | TNF | PGE2 | DIQMTQSPSSLSASVGDRVTITC**RASQGIRNYLA**WYQQKP GKAPKLLIY**AASTLQSGVPS**RFSGSGSGTDFTLTISSLQP EDVATYYC**QRYNRAPYT**FGQGTKVEIKR<u>**TVAAP**</u>DVLMTQT PLSLPVTPGEPASISC**TSSQNIVHSNGNTYLE**WYLQKPGQ SPQLLIY**KVSNRFSGVPD**RFSGSGSGTDFTLKISRVEAED VGVYYC**FQVSHVPYT**FGGGTKVEIKR |
|  |  |  | TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQ ID NO:65)** |

EP 3 466 973 A1

137

(continued)

| DVD-Ig Heavy or Light Chain Name | Outer Variable Domain Name | Inner Variable Domain Name | Sequence, with Line Break Between Variable and Constant Regions<br>12345678901234567890123456789001234567890 |
|---|---|---|---|
| IL-la/IL-1bH (DVD-C) | IL-1aH | IL-1bH | EVQLVESGGGVVQPGRSLRLSCSASGFIFS**RYDMS**WVRQA PGKGLEWVA**YISHGGAGTYYPDSVKG**RFTISRDNSKNTLF LQMDSLRPEDTGVYFCAR**GGVTKGYFDV**WGQGTPVTVSS<u>**A STKGP**</u>QVQLVESGGGVVQPGRSLRLSCTASGFTFS**MFGVH** WVRQAPGKGLEWVA**AVSYDGSNKYYAESVKG**RFTISRDNS KNILFLQMDSLRLEDTAVYYCAR**GRPKVVIPAPLAH**WGQG TLVTFSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK**(SEQ ID NO:66)** |
| IL-1a/IL-1bL (DVD-C) | IL-1aL | 1L-1bL | DIQMTQSPSSLSASVGDRVTITC**RASGNIHNYLT**WYQQTP GKAPKLLIY**NAKTLADGVPS**RFSGSGSGTDYTFTISSLQP EDIATYYC**QHFWSIPYT**FGQGTKLQITR<u>**TVAAP**</u>DIQMTQS PSSVSASVGDRVTITC**RASQGISSWLA**WYQQKPGKAPKLL IY**EASNLETGVP**SRFSGSGSGSDFTLTISSLQPEDFATYY C**QQTSSFLLS**FGGGTKVEHKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC**(SEQIDNO:67)** |

138

EP 3 466 973 A1

(continued)

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DLL4/VEGFH C(h1A11.1. A6-LS-Av) | | | EVQLVESGGGLVQPGGSLRLSCAASGFTFR**HFPMA**WVRQA<br>PGKGLEWVA**TISSSDAWPSYRDSVKG**RFTISRDNAKNSLY<br>LQMNSLRAEDTAVYYCSR**GYYNSPFAY**WGQGTLVTVSS<u>**AS**</u><br><u>**TKGPSVFPLAP**</u>EVQLVESGGGLVQPGGSLRLSCAASGYTF<br>T**NYGMN**WVRQAPGKGLEWVG**WINTYTGEPTYAADFKR**RFT<br>FSLDTSKSTAYLQMNSLRAEDTAVYYCAK**YPHYYGSSHWY**<br>**FDV**WGQGTLVTVSS<br><br>ASTKGPSVFPLAPSSKSTSGGTAALGCLVKDYFPEPVTVS<br>WNSGALTSGVHTFPAVLQSSGLYSLSSVVTVPSSSLGTQT<br>YICNVNHKPSNTKVDKKVEPKSCDKTHTCPPCPAPEAAGG<br>PSVFLFPPKPKDTLMISRTPEVTCVVVDVSHEDPEVKFNW<br>YVDGVEVHNAKTKPREEQYNSTYRVVSVLTVLHQDWLNGK<br>EYKCKVSNKALPAPIEKTISKAKGQPREPQVYTLPPSREE<br>MTKNQVSLTCLVKGFYPSDIAVEWESNGQPENNYKTTPPV<br>LDSDGSFFLYSKLTVDKSRWQQGNVFSCSVMHEALHNHYT<br>QKSLSLSPGK* **(SEQ ID NO:68)** |
| DLL4/VEGFL C(h1A11.1. A6-LS-Av) | | | DIQMTQSPSSLSASVGDRVTITC**RASEDIYSNLA**WYQQKP<br>GKAPKLLIY**DTNNLADGVPS**RFSGSGSGTDFTLTISSLQP<br>EDFATYYC**QQYNNYPPT**FGQGTKLEIKR<u>**TVAAP**</u>DIQMTQS<br>PSSLSASVGDRVTITC**SASQDISNYLN**WYQQKPGKAPKVL |
| | | | IY**FTSSLHSGVPS**RFSGSGSGTDFTLTISSLQPEDFATYY<br>C**QQYSTVPWT**FGQGTKVEIKR<br><br>TVAAPSVFIFPPSDEQLKSGTASVVCLLNNFYPREAKVQW<br>KVDNALQSGNSQESVTEQDSKDSTYSLSSTLTLSKADYEK<br>HKVYACEVTHQGLSSPVTKSFNRGEC* **(SEQIDNO:69)** |

(continued)

| DVD-Ig Heavy or Light Chain Name | Outer Variable Domain Name | Inner Variable Domain Name | Sequence, with Line Break Between Variable and Constant Regions 12345678901234567890123456789001234567890 |
|---|---|---|---|
| DLL4/VEGFH C(h1A11.1. A6-SL-Av) | | | EVQLVESGGGLVQPGGSLRLSCAASGFTFR**HFPMA**WVRQA PGKGLEWVA**TISSSDAWPSYRDSVKG**RFTISRDNAKNSLY LQMNSLRAEDTAVYYCSR**GYYNSPFAY**WGQGTLVTVSS**AS TKGP**EVQLVESGGGLVQPGGSLRLSCAASGYTFT**NYGMN**W VRQAPGKGLEWVG**WINTYTGEPTYAADFKR**RFTFSLDTSK STAYLQMNSLRAEDTAVYYCAK**YPHYYGSSHWYFDV**WGQG TLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK **(SEQ ID NO:74)** |
| DLL4/VEGFL C(h1A11.1. A6-SL-Av) | | | DIQMTQSPSSLSASVGDRVTITC**RASEDIYSNLA**WYQQKP GKAPKLLIY**DTNNLADGVPS**RFSGSGSGTDFTLTISSLQP EDFATYYC**QQYNNYPPT**FGQGTKLEIKR**TVAAPSVFIFPP** DIQMTQSPSSLSASVGDRVTITC**SASQDISNYLN**WYQQKP GKAPKVLIY**FTSSLHSGVPS**RFSGSGSGTDFTLTISSLQP EDFATYYC**QQYSTVPWT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC **(SEQ ID NO:75)** |

EP 3 466 973 A1

140

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| IL12/IL18H | | | EVTLRESGPALVKPTQTLTLTCTFSGFSLS**KSVMGVS**WIR QPPGKALEWLA**HIYWDDDKYYNPSLKS**RLTISKDTSKNQV VLTMTNMDPVDTATYYCAR**RGIRSAMDY**WGQGTTVTVSS**A STKGP**EVQLVQSGTEVKKPGESLKISCKGSGYTVT**SYWIG** WVRQMPGKGLEWMG**FIYPGDSETRYSPTFQG**QVTISADKS FNTAFLQWSSLKASDTAMYYCAR**VGSGWYPYTFDI**WGQGT MVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK **(SEQ ID NO:70)** |
| IL12/IL18 LC | | | DIVMTQSPDSLAVSLGERATINC**KASQSVSNDVA**WYQQKP GQPPKLLIY**YASNRYTGVPD**RFSGSGSGTDFTLTISSLQA |
| | | | EDVAVYYC**QQDYNSPWT**FGGGTKVEIKR<u>**TVAAP**</u>EIVMTQS PATLSVSPGERATLSC**RASESISSNLA**WYQQKPGQAPRLF IY**TASTRATDIPA**RFSGSGSGTEFTLTISSLQSEDFAVYY C**QQYNNWPSIT**FGQGTRLEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC **(SEQ ID NO:71)** |

EP 3 466 973 A1

(continued)

EP 3 466 973 A1

| DVD-Ig Heavy or Light Chain Name | Outer Variable Domain Name | Inner Variable Domain Name | Sequence, with Line Break Between Variable and Constant Regions<br>1234567890123456789012345678901234567890 |
|---|---|---|---|
| DLL4/VEGF HC h1A11.1-LS-AV | | | EVQLVESGGGLVQPGGSLRLSCAASGFTFS**NFPMA**WVRQA PGKGLEWVA**TISSSDGTTYYRDSVKG**RFTISRDNAKNSLY LQMNSLRAEDTAVYYCAR**GYYNSPFAY**WGQGTLVTVSS<u>**AS TKGPSVFPLAP**</u>EVQLVESGGGLVQPGGSLRLSCAASGYTF **TNYGMN**WVRQAPGKGLEWVG**WINTYTGEPTYAADFKR**RFT FSLDTSKSTAYLQMNSLRAEDTAVYYCAK**YPHYYGSSHWY FDV**WGQGTLVTVSS<br><br>ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSCDKTHTCPPCPAPEAAGG PSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREE MTKNQVSLTCLVKGFYPSDIAVEWESNGQPENNYKTTPPV LDSDGSFFLYSKLTVDKSRWQQGNVFSCSVMHEALHNHYT QKSLSLSPGK **(SEQ ID NO:72)** |
| DLL4/VEGF LC h1A11.1-LS-AV | | | DIQMTQSPSSLSASVGDRVTITC**RASEDIYSNLA**WYQQKP GKAPKLLIY**DTNNLADGVPS**RFSGSGSGTDFTLTISSLQP EDFATYYC**QQYNNYPPT**FGQGTKLEIKR<u>**TVAAP**</u>DIQMTQS PSSLSASVGDRVTITC**SASQDISNYLN**WYQQKPGKAPKVL IY**FTSSLHSGVPS**RFSGSGSGTDFTLTISSLQPEDFATYY C**QQYSTVPWT**FGQGTKVEIKR<br><br>TVAAPSVFIFPPSDEQLKSGTASVVCLLNNFYPREAKVQW KVDNALQSGNSQESVTEQDSKDSTYSLSSTLTLSKADYEK HKVYACEVTHQGLSSPVTKSFNRGEC **(SEQ ID NO:73)** |

142

**EP 3 466 973 A1**

**Incorporation by Reference**

The contents of all cited references (including literature references, patents, patent applications, and websites) that may be cited throughout this application are hereby expressly incorporated by reference in their entirety, as are the references cited therein. The practice of the present disclosure will employ, unless otherwise indicated, conventional techniques of immunology, molecular biology and cell biology, which are well known in the art.

**Equivalents**

The disclosure may be embodied in other specific forms without departing from the spirit or essential characteristics thereof. The foregoing embodiments are therefore to be considered in all respects illustrative rather than limiting of the disclosure described herein. Scope of the disclosure is thus indicated by the appended claims rather than by the foregoing description, and all changes that come within the meaning and range of equivalency of the claims are therefore intended to be embraced herein.

**Claims**

1. An aqueous pharmaceutical formulation comprising an Aqueous Stable DVD-Ig (AS-DVD-Ig) protein that is capable of binding to DLL4 and VEGF and a buffer having a molarity of about 5 to about 50 mM, wherein the formulation has a pH of about 4.5 to about 7.5.

2. The aqueous pharmaceutical formulation of claim 1, wherein the formulation has a pH of 5 to 7.

3. The aqueous pharmaceutical formulation of claim 2, wherein the formulation has a pH of 6.

4. The aqueous pharmaceutical formulation of any one of claims 1-3, wherein the buffer has a molarity of 10 mM, 15 mM. or 30 mM.

5. The aqueous pharmaceutical formulation of any one of claims 1-4, wherein the buffer is acetate, histidine, or phosphate.

6. The aqueous pharmaceutical formulation of any one of claims 1-5, further comprising polysorbate.

7. The aqueous pharmaceutical formulation of any one of claims 1-6, further comprising sucrose or arginine.

Fig. 1

Figure 2A

## Domain orientation concept

Figure 2B

Figure 3

Fig. 4

Fig. 5

Europäisches
Patentamt

European
Patent Office

Office européen
des brevets

**EUROPEAN SEARCH REPORT**

Application Number

EP 18 17 1273

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2011/039370 A1 (BOEHRINGER INGELHEIM INT [DE]; BORGES ERIC [DE]; GSCHWIND ANDREAS [DE]) 7 April 2011 (2011-04-07) * claims 1-35 * | 1-7 | INV.<br>C07K16/24<br>C07K16/28<br>A61K9/00<br>A61K9/19 |
| Y | US 2011/212094 A1 (ABBOTT LAB [US]; GHAYUR TARIQ [US]; SALFELD JOCHEN G [US]; MCPHERSON M) 1 September 2011 (2011-09-01) * paragraph [0165] - paragraph [0170] * * paragraph [0300] - paragraph [0304] * * paragraph [0292] * | 1-7 | C07K16/18<br>C07K16/22<br>C07K16/32<br>C07K16/42 |
| A | W. HU ET AL: "Biological Roles of the Delta Family Notch Ligand Dll4 in Tumor and Endothelial Cells in Ovarian Cancer", CANCER RESEARCH, vol. 71, no. 18, 15 September 2011 (2011-09-15), pages 6030-6039, XP055095433, ISSN: 0008-5472, DOI: 10.1158/0008-5472.CAN-10-2719 * the whole document * | 1-7 | |
| A | WO 2011/117653 A1 (UCB PHARMA SA [BE]; HEYWOOD SAM PHILIP [GB]; HUMPHREYS DAVID PAUL [GB]) 29 September 2011 (2011-09-29) * page 24, line 25 - line 39; claims 1-9; figure 1; examples 1-2 * | 1-7 | TECHNICAL FIELDS SEARCHED (IPC)<br><br>A61K<br>C07K |

The present search report has been drawn up for all claims

| Place of search | Date of completion of the search | Examiner |
|---|---|---|
| The Hague | 7 February 2019 | Le Flao, Katell |

EPO FORM 1503 03.82 (P04C01)

EP 3 466 973 A1

## ANNEX TO THE EUROPEAN SEARCH REPORT
## ON EUROPEAN PATENT APPLICATION NO.

EP 18 17 1273

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2019

| Patent document cited in search report | Publication date | Patent family member(s) | Publication date |
|---|---|---|---|
| WO 2011039370 A1 | 07-04-2011 | AR 078515 A1 | 16-11-2011 |
| | | AU 2010302589 A1 | 19-04-2012 |
| | | CA 2775422 A1 | 07-04-2011 |
| | | CN 102639566 A | 15-08-2012 |
| | | CN 105037542 A | 11-11-2015 |
| | | EA 201200548 A1 | 28-12-2012 |
| | | EC SP12011835 A | 29-06-2012 |
| | | EP 2483314 A1 | 08-08-2012 |
| | | JP 5833009 B2 | 16-12-2015 |
| | | JP 2013506411 A | 28-02-2013 |
| | | JP 2016026207 A | 12-02-2016 |
| | | KR 20120101375 A | 13-09-2012 |
| | | MA 33607 B1 | 01-09-2012 |
| | | NZ 598956 A | 25-07-2014 |
| | | NZ 626302 A | 25-09-2015 |
| | | PE 10242012 A1 | 10-08-2012 |
| | | TN 2012000145 A1 | 19-09-2013 |
| | | TW 201124533 A | 16-07-2011 |
| | | US 2011172398 A1 | 14-07-2011 |
| | | US 2014120095 A1 | 01-05-2014 |
| | | UY 32920 A | 29-04-2011 |
| | | WO 2011039370 A1 | 07-04-2011 |
| US 2011212094 A1 | 01-09-2011 | AR 078795 A1 | 07-12-2011 |
| | | AU 2010319850 A1 | 14-06-2012 |
| | | BR 112012010241 A2 | 06-12-2016 |
| | | CA 2778140 A1 | 19-05-2011 |
| | | CN 102770451 A | 07-11-2012 |
| | | CO 6551686 A2 | 31-10-2012 |
| | | CR 20120266 A | 14-08-2012 |
| | | EC SP12011922 A | 31-07-2012 |
| | | EP 2493924 A2 | 05-09-2012 |
| | | GT 201200125 A | 30-09-2013 |
| | | JP 2013509189 A | 14-03-2013 |
| | | KR 20140015145 A | 06-02-2014 |
| | | NZ 600100 A | 28-03-2014 |
| | | NZ 620267 A | 26-06-2015 |
| | | NZ 707465 A | 28-08-2015 |
| | | PE 15432012 A1 | 22-12-2012 |
| | | RU 2012121813 A | 10-12-2013 |
| | | TW 201124534 A | 16-07-2011 |
| | | US 2011212094 A1 | 01-09-2011 |
| | | US 2014308286 A1 | 16-10-2014 |
| | | UY 32979 A | 28-02-2011 |
| | | WO 2011059755 A2 | 19-05-2011 |

For more details about this annex : see Official Journal of the European Patent Office, No. 12/82

page 1 of 2

251

## ANNEX TO THE EUROPEAN SEARCH REPORT
## ON EUROPEAN PATENT APPLICATION NO.

EP 18 17 1273

This annex lists the patent family members relating to the patent documents cited in the above-mentioned European search report.
The members are as contained in the European Patent Office EDP file on
The European Patent Office is in no way liable for these particulars which are merely given for the purpose of information.

07-02-2019

| Patent document cited in search report | Publication date | Patent family member(s) | | Publication date |
|---|---|---|---|---|
| WO 2011117653 A1 | 29-09-2011 | EP | 2550297 A1 | 30-01-2013 |
| | | WO | 2011117653 A1 | 29-09-2011 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61721364 B
- US 61794231 B
- US 7612181 B, Wu
- US 6258562 B
- US 6914128 B
- US 20050147610 A
- US 5736137 A
- US 5500362 A
- US 2003040426 W
- US 5677171 A
- US 4753894 A
- US 4943533 A
- WO 9640210 A
- US 6235883 B
- US 172317 A
- US 5558864 A
- WO 9520045 PCT
- US 5891996 A
- US 6506883 B
- WO 0162931 A2
- WO 0188138 PCT
- US 20100226923 A
- US 20100074900 A

### Non-patent literature cited in the description

- **MANNING et al.** *Pharm. Res.,* 1989, vol. 6, 903
- **SHIRE et al.** *J. Pharm. Sci.,* 2004, vol. 93, 1390
- **WANG et al.** *J. Pharm. Sci.,* 2007, vol. 96, 1
- Peptide and Protein Drug Delivery. Marcel Dekker, Inc, 1991, 247-301
- **JONES.** *Adv. Drug Delivery Rev.,* 1993, vol. 10, 29-90
- **HOLLIGER et al.** *Proc. Natl. Acad. Sci. USA,* 1993, vol. 90, 6444-6448
- **POLJAK et al.** *Structure,* 1994, vol. 2, 1121-1123
- **KABAT et al.** *Ann. NY Acad. Sci.,* 1971, vol. 190, 382-391
- **KABAT et al.** Sequences of Proteins of Immunological Interest. U.S. Department of Health and Human Services, 1991
- **CHOTHIA ; LESK.** *J. Mol. Biol.,* 1987, vol. 196, 901-917
- **CHOTHIA et al.** *Nature,* 1989, vol. 342, 877-883
- **PADLAN.** *FASEB J.,* 1995, vol. 9, 133-139
- **MACCALLUM.** *J. Mol. Biol.,* 1996, vol. 262 (5), 732-45
- **JONSSON et al.** *Ann. Biol. Clin.,* 1993, vol. 51, 19-26
- **JONSSON et al.** *Biotechniques,* 1991, vol. 11, 620-627
- **JONSSON et al.** *J. Mol. Recognit.,* 1995, vol. 8, 125-131
- **JOHNSSON et al.** *Anal. Biochem.,* 1991, vol. 198, 268-277
- **PRESTA.** *J. Allergy Clin. Immunol.,* 2005, vol. 116, 731-6
- **CLARK.** Antibodies for Therapeutic Applications. Cambridge University, 2000
- **MURTHY et al.** *Arch. Biochem. Biophys.,* 1987, vol. 252 (2), 549-60
- **RODECK et al.** *J. Cell. Biochem.,* 1987, vol. 35 (4), 315-20
- **KETTLEBOROUGH et al.** *Protein Eng.,* 1991, vol. 4 (7), 773-83
- **MODJTAHEDI et al.** *J. Cell. Biophys.,* 1993, vol. 22 (1-3), 129-46
- **MODJTAHEDI et al.** *Br. J. Cancer,* 1993, vol. 67 (2), 247-53
- **MODJTAHEDI et al.** *Br. J. Cancer,* 1996, vol. 73 (2), 228-35
- **MODJTAHEDI et al.** *Int. J. Cancer,* 2003, vol. 105 (2), 273-80
- **MATEO et al.** *Immunotechnol.,* 1997, vol. 3 (1), 71-81
- **JUNGBLUTH et al.** *Proc. Natl. Acad. Sci. USA,* 2003, vol. 100 (2), 639-44
- **URLAUB ; CHASIN.** *Proc. Natl. Acad. Sci. USA,* 1980, vol. 77, 4216-4220
- **KAUFMAN ; SHARP.** *J. Mol. Biol.,* 1982, vol. 159, 601-621
- **URLAUB et al.** *Som. Cell Molec. Genet.,* 1986, vol. 12, 555
- **HAYNES et al.** *Nuc. Acid. Res.,* 1983, vol. 11, 687-706
- **LAU et al.** *Mol. Cell. Biol.,* 1984, vol. 4, 1469-1475